# EUROPEAN PATENT APPLICATION

(11) **EP 4 458 818 A1**
(43) Date of publication of application: **06.11.2024**
(21) Application number: 22915053.7
(22) Date of filing: 29.12.2022
(51) Int. Cl.: C07D 401/04, A61K 31/4375, A61P 35/00

(54) **HETEROAROMATIC NITROGEN-OXIDE COMPOUND, PREPARATION METHOD THEREFOR, AND USE THEREOF**

(30) Priority: 29.12.2021 CN 202111640604
(71) Applicant: Beijing Avistone Biotechnology Co., Ltd., Beijing 100102 (CN)
(72) Inventor: LI, Gong, Beijing 100102 (CN); ZHANG, Peilong, Beijing 100102 (CN); LAN, Wenli, Beijing 100102 (CN); LI, Xiangqiu, Beijing 100102 (CN)
(74) Representative: Schiweck Weinzierl Koch Patentanwälte Partnerschaft mbB
(86) International application number: PCT/CN2022/143381
(87) International publication number: WO 2023/125803

(57) **Abstract**

Disclosed are a heteroaromatic nitrogen-oxide compound, a preparation method therefor, and a use thereof. The heteroaromatic nitrogen-oxide compound is as shown in formula I, wherein A, L, Z, X, Y, E₁, E₂, E₃ and G are as defined in the description.

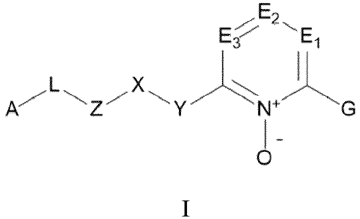

## Description

### Cross Reference to Related Applications

The present application claims priority to and the benefit of Chinese Patent Application No. 202111640604.9, filed on 29 December 2021, the content of which is hereby incorporated by reference in its entirety for all purposes.

### Field of The Invention

The present invention relates to heteroaromatic nitrogen-oxide compound, preparation method and use thereof, in particular to compounds for inhibiting, regulating and/or regulating signal transduction of c-Met kinase, preparation method thereof, pharmaceutical compositions comprising the same as well as use thereof.

### Background

Protein kinases are enzymatic components of signaling transduction pathways that catalyze the migration of terminal phosphates from ATP to hydroxyl groups of tyrosine, serine, and/or threonine residues of proteins. Therefore, compounds that inhibit protein kinase function are valuable tools for assessing the physiological consequences of protein kinase activity. Overexpression or inappropriate expression of normal or mutant protein kinases in mammals has been the subject of extensive research and has been proven to play an important role in the development of a number of diseases, including diabetes, angiogenesis, psoriasis, restenosis, ocular diseases, schizophrenia, rheumatoid arthritis, atherosclerosis, cardiovascular diseases and cancer. Protein kinase inhibitors have particular utility in the treatment of human and animal diseases.

Hepatocyte growth factor receptor (c-Met) is a transmembrane protein encoded by the MET gene, belonging to the tyrosine kinase receptor superfamily, expressed primarily in epithelial cells. Hepatocyte Growth Factor (HGF), also known as Scatter Factor (SF), is the only high affinity ligand for c-Met so far, which is widely present in various human tissues and organs, mainly expressed in mesenchymal cells. After HGF binds to c-Metat an extracellular domain, c-Met dimerization is initiated. Two catalytic active sites Tyr1234 and Tyr1235 at the activation loop of c-Met autophosphorylate, then leading to phosphoration of the carboxy-terminal Tyr1349 and Tyr1356 Various downstream cell effectors and effector molecules are recruited, and a series of downstream signal transduction pathways, such as PI3K-AKT, RAS-MAPK, STAT, Wnt/β-catenin and the like, are activated. c-Met/HGF plays an important role in promoting cell proliferation, cell growth, cell migration, invasion of blood vessels and angiogenesis. The c-Met gene abnormality mainly comprises three types: MET 14 exon skipping mutation, MET gene amplification and c-Met protein overexpression. The c-Met gene abnormality will lead to abnormal activation of c-Met pathway, thereby causing excessive activation of the downstream pathway and inducing cancer.

At present, the most widely studied and the most therapeutically potential methods to block the c-Met/HGF signaling pathway are c-Met small molecule kinase inhibitors. Small molecule kinase inhibitors can bind at an intramembrane catalytic domain to prevent protein phosphorylation, and further block signal transduction, thus achieving the goal of targeted treatment of cancer. Depending on the binding mode, c-Met small molecule kinase inhibitors can be divided into three types, namely type I, type II and type III. Type I is a c-Met kinase specific inhibitor which can competitively bind to the ATP binding domain with ATP, in which aspartic acidphenylalanine-glycine (DFG) moiety points to ATP binding domain, and representative inhibitors include Crizotinib, Capmatinib, Tepotinib, Savolinitib, PLB1001, and Glumetinib. Type I inhibitors can be further divided into type Ia and type Ib. Type Ia, such as Crizotinib, can interact with the solvent front residue G1163 in addition to targeting kinase domain. Type Ib, such as Capmatinib, Tepotinib and Savolitinib, only targets the kinase domain. Type II are ATP-competitive, multi-target kinase inhibitors, which not only competitively binds ATP at the ATP binding pocket of inactive conformation c-Met kinase, but also extends and occupies the DFG pocket, such as Cabozantinib, Glesatinib and Merestinib. Type III is a non-ATP competitive inhibitor, such as Tivantinib.

At present, the c-Met kinase inhibitors on the market comprise Crizotinib, Capmatinib, Tepotinib and Cabozantinib. Clinically, c-Met kinase inhibitors have demonstrated clinical efficacy in non-small cell lung cancer patients with MET 14 exon skipping mutation, the response rate (ORR) is between 25% and 68%, but the side effect is intolerance, and the patients inevitably generate drug resistance after 9-16 months. The drug resistance of type I kinase inhibitor is mainly caused by mutations at D1228 and Y1230 residue, the drug resistance mutation of type II kinase inhibitor is mainly caused by mutation at F 1200 and L1195 residue, and the emerging of drug resistance mutation causes weakened binding between kinase inhibitor and c-Met receptor, which makes the kinase inhibitor lose curative effect. Preclinical studies have found that the drug-resistance mutation caused by type I inhibitors is still sensitive to type II inhibitors. In contrast, the drug-resistance mutations caused by type II inhibitors are sensitive to type I inhibitors.

In order to better solve the drug resistance problem of type I and type II c-Met inhibitors, it is of great significance to develop high-activity c-Met kinase inhibitors that can overcome various drug resistance mutation mechanisms.

### Summary of the Invention

The present application provides a heteroaromatic nitrogen-oxide compound capable of overcoming c-Met gene abnormality, having a structure shown as the following formula I: 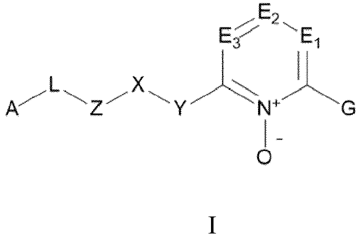 wherein, in the formula I,
A is selected from the group consisting of 5-14 membered heteroaryl substituted with R¹, 3-14 membered heterocyclyl substituted with R¹, 6-14 membered aryl substituted with R¹, and 3-12 membered cycloalkyl substituted with R¹, the 5-14 membered heteroaryl substituted with R¹, 3-14 membered heterocyclyl substituted with R¹, 6-14 membered aryl substituted with R¹, and 3-12 membered cycloalkyl substituted with R¹ are optionally substituted with one or more R², the 5-14 membered heteroaryl substituted with R¹ and 6-14 membered aryl substituted with R¹ comprise monocyclic or fused ring, the 3-14 membered heterocyclyl substituted with R¹, and 3-12 membered cycloalkyl substituted with R¹ comprise monocyclic , spirocyclic or bridged ring;
L is selected from O, S, CR^{a}R^{b},NR^{b},C(=O),SO₂ and SO;
Z is selected from the group consisiting of 6-14 membered aryl, 5-14 membered heteroaryl, 3-12 membered cycloalkyl and 3-14 membered heterocyclyl, the 6-14 membered aryl, 5-14 membered heteroaryl, 3-12 membered cycloalkyl and 3-14 membered heterocyclyl are optionally substituted with one or more R³;
X is absent or selected from the group consisiting of NR⁴, O, S, CR⁴R⁵ and C(=O);
Y is absent or selected from (CR^{a}R^{b})ₙ₁-C(=O), C(=S), C(=NR⁴), SO₂, SO, NR⁴, O, S and CR⁴R⁵;
E₁, E₂ and E₃ are each absent or each independently selected from CR^{a}R^{b}, N, C(=O), C(=S) and C(=NR⁴);
G is selected from the group consisting of (CR^{a}R^{b})ₙ₁-6-20-membered aryl, (CR^{a}R^{b})ₙ₁-5-20-membered heteroaryl, (CR^{a}R^{b})ₙ₁-3-12-membered cycloalkyl, (CR^{a}R^{b})ₙ₁-3-20-membered heterocyclyl, (CR^{a}R^{b})ₙ₁-O-(CR^{a}R^{b})ₘ₁-aryl, (CR^{a}R^{b})ₙ₁-OR⁶, (CR^{a}R^{b})ₙ₁-NR⁶R⁷, (CR^{a}R^{b})ₙ₁-NR⁶C(=O)R⁷, C₁₋₆ alkyl, C₂₋₆ alkenyl and C₂₋₆ alkynyl, wherein the (CR^{a}R^{b})ₙ₁-6-20-membered aryl, (CR^{a}R^{b})ₙ₁-5-20-membered heteroaryl, (CR^{a}R^{b})ₙ₁-3-12-membered cycloalkyl, (CR^{a}R^{b})ₙ₁-3-20-membered heterocyclyl, (CR^{a}R^{b})ₙ₁-O-(CR^{a}R^{b})ₘ₁-aryl, C₁₋₆ alkyl, C₂₋₆ alkenyl and C₂₋₆ alkynyl are optionally substituted with one or more groups independently selected from the group consisting of: halogen, nitro, oxo(=O), SO₂R^{f}, CN, OR^{e}, SR^{e}, NR^{e}R^{f}, -NR^{e}C(=O)R^{f}, (CR^{a}R^{b})ₙ₁-C(=O)R^{e}, -C(=O)NR^{e}R^{f}, -C(=O)OR^{e}, C₁₋₁₂ alkyl, halogenated C₁₋₁₂ alkyl, C₁₋₆ alkoxy-C₁₋₁₂ alkyl, halogenated C₁₋₆ alkoxy-C₁₋₁₂ alkyl, R^{a}R^{b}N-C₁₋₆ alkyl, C₂₋₈ alkenyl, halogenated C₂₋₈ alkenyl, C₂₋₈ alkynyl, 3-12 membered cycloalkyl, 3-20 membered heterocyclyl, 6-20 membered aryl, or 5-20 membered heteroaryl;
R¹ is selected from R⁸ substituted with one or more hydroxy;
R², R³ and R⁸ are each independently selected from the group consisting of hydrogen, halogen, cyano, nitro, -SO-R^{a}, -NR^{b}R^{c}, -OR^{b}, -SR^{b}, R^{a}SO-C₁₋₆ alkyl, R^{b}R^{c}N-C₁₋₆ alkyl, R^{b}R^{c}NC(=Y¹)-C₁₋₆ alkyl, R^{a}SO-C₁₋₆ alkoxy, R^{b}R^{c}N-C₁₋₆ alkoxy, R^{b}R^{c}NC(=Y¹)-C₁₋₆ alkoxy, -C(=Y¹)R^{a}, -C(=Y¹)OR^{b}, -C(=Y¹)NR^{b}R^{c}, - C(=Y¹)NR^{c}(CR^{a}R^{b})ₙ₁NR^{b}R^{c}, -OC(=Y¹)R^{a}, -OC(=Y¹)NR^{b}R^{c}, -OC(=Y¹)OR^{b}, - NR^{b}C(=Y¹)NR^{b}R^{c}, -NR^{b}C(=Y¹)OR^{c}, -NR^{b}C(=Y¹)R^{a}, -OR^{b}C(=Y¹)OR^{c}, -SO₂-NR^{b}R^{c}, - SO₂R^{a}, -NR^{b}SO₂R^{a}, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered cycloalkenyl, 3-12 membered heterocyclyl, 6-14 membered aryl, 5-10 membered heteroaryl, C₁₋₆ alkoxy-C₁₋₆ alkyl, 3-12 membered cycloalkyl-C₁₋₆ alkyl, 3-12 membered cycloalkenyl-C₁₋₆ alkyl, 3-12 membered heterocyclyl-C₁₋₆ alkyl, 6-14 membered aryl-C₁₋₆ alkyl and 5-10 membered heteroaryl-C₁₋₆ alkyl, the R^{a}SO-C₁₋₆ alkyl, R^{b}R^{c}N-C₁₋₆ alkyl, R^{b}R^{c}NC(=Y¹)-C₁₋₆ alkyl, R^{a}SO-C₁₋₆ alkoxy, R^{b}R^{c}N-C₁₋₆ alkoxy, R^{b}R^{c}NC(=Y¹)-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered cycloalkenyl, 3-12 membered heterocyclyl, 6-14 membered aryl, 5-10 membered heteroaryl, C₁₋₆ alkoxy-C₁₋₆ alkyl, 3-12 membered cycloalkyl-C₁₋₆ alkyl, 3-12 membered cycloalkenyl-C₁₋₆ alkyl, 3-12 membered heterocyclyl-C₁₋₆ alkyl, 6-14 membered aryl-C₁₋₆ alkyl and 5-10 membered heteroaryl-C₁₋₆ alkyl are optionally substituted with one or more R^{m}, the 3-12 membered cycloalkyl, 3-12 membered cycloalkenyl, 3-12 membered heterocyclyl, 3-12 membered cycloalkyl-C₁₋₆ alkyl, 3-12 membered cycloalkenyl-C₁₋₆ alkyl and 3-12 membered heterocyclyl-C₁₋₆ alkyl comprise monocyclic, spirocyclic or bridged ring, and the 6-14 membered aryl, 5-10 membered heteroaryl, 6-14 membered aryl-C₁₋₆ alkyl and 5-10 membered heteroaryl-C₁₋₆ alkyl comprise monocyclic or fused ring;
or R¹ and R² together with the atoms to which they are attached to form a 5-14 membered cyclic group, which is substituted with one or more R¹;
or R² and R⁸ together with the atoms to which they are attached to form a 5-14 membered cyclic group, which is substituted with one or more R¹;
R⁴, R⁵, R⁶ and R⁷ are each independently selected from the group consisting of hydrogen, halogen, OR^{e}, C₁₋₆ alkyl, C₂₋₆ alkenyl, C₂₋₆ alkynyl, 3-12 membered cycloalkyl, 3-20 membered heterocyclyl, 6-20 membered aryl and 5-20 membered heteroaryl, wherein the 3-12 membered cycloalkyl and 3-20 membered heterocyclyl comprise monocyclic, spirocyclic or bridged ring, the 6-20 membered aryl and 5-20 membered heteroaryl comprise monocyclic or fused ring, the C₁₋₆ alkyl, C₂₋₆ alkenyl, C₂₋₆ alkynyl, 3-12 membered cycloalkyl, 3-20 membered heterocyclyl, 6-20 membered aryl and 5-20 membered heteroaryl are optionally substituted with one or more groups independently selected from the group consisting of halogen, SO₂R^{g}, CN, OR^{e}, NR^{e}R^{f}, C(=O)NR^{e}R^{f}, CR^{e}C(=O)R^{f}, C₁₋₁₂ alkyl, C₂₋₈ alkenyl, C₂₋₈ alkynyl, 3-12 membered cycloalkyl, 3-20 membered heterocyclyl, 6-20 membered aryl or 5-20 membered heteroaryl;
or R⁴ and R⁵ together with the carbon atom to which they are both attached to form a 3-12 membered monocyclic, spirocyclic or bridged ring optionally substituted with one or more R^{m};
or R⁶ and R⁷ together with the nitrogen atom to which they are both attached to form a saturated, partially unsaturated or fully unsaturated 3-20 membered heterocycle optionally substituted with one or more R^{m}, which containing one or more heteroatoms selected from the group consisting of N, O and S;
R^{a} is absent or selected from the group consisting of hydrogen, halogen, cyano, hydroxyl, mercapto, carboxyl, nitro, -NR^{b}R^{c}, -C(=O)R^{d}, -C(=O)OR^{b}, -C(=O)NR^{b}R^{c}, - OC(=O)NR^{b}R^{c}, -NR^{b}C(=O)R^{d}, -NR^{b}C(=O)OR^{d}, -SO-NR^{b}R^{c}, -SO₂-NR^{b}R^{c}, -SOR^{d}, - SO₂R^{d}, -NR^{b}SO₂R^{d}, C₁₋₆ alkyl, C₁₋₆ alkoxy, C₂₋₈ alkenyl, C₂₋₈ alkynyl, C₁₋₆ alkyl-R', C₁₋₆ alkoxy-R', -OR', -SR', -C(=O)-R', -SO₂R', -NR^{b}C(=O)-R', 3-12 membered cycloalkyl, 3-12 membered cycloalkenyl, 3-12 membered heterocyclyl, 6-14 membered aryl and 5-10 membered heteroaryl, the C₁₋₆ alkyl, C₁₋₆ alkoxy, C₂₋₈ alkenyl, C₂₋₈ alkynyl, C₁₋₆ alkyl-R', C₁₋₆alkoxy-R', 3-12 membered cycloalkyl, 3-12 membered cycloalkenyl, 3-12 membered heterocyclyl, 6-14 membered aryl and 5-10 membered heteroaryl are optionally substituted with one or more R^{m}, the 3-12 membered cycloalkyl, 3-12 membered cycloalkenyl and 3-12 membered heterocyclyl comprise monocyclic, spirocyclic or bridged ring, the 6-14 membered aryl and 5-10 membered heteroaryl comprise monocyclic or fused ring;
R^{b}, R^{c} and R^{d} are each absent or each independently selected from the group consisting of hydrogen, hydroxyl, mercapto, C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', -OR', -C(=O)-R', -SO₂-R', 3-12 membered cycloalkyl, 3-12 membered cycloalkenyl, 3-12 membered heterocyclyl, 6-14 membered aryl and 5-10 membered heteroaryl, the C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', 3-12 membered cycloalkyl, 3-12 membered cycloalkenyl, 3-12 membered heterocyclyl, 6-14 membered aryl and 5-10 membered heteroaryl are optionally substituted with one or more R^{m}, the 3-12 membered cycloalkyl, 3-12 membered cycloalkenyl and 3-12 membered heterocyclyl comprise monocyclic, spirocyclic or bridged ring, the 6-14 membered aryl and 5-10 membered heteroaryl comprise monocyclic or fused ring;
or R^{a} and R^{b} together with the carbon atom to which they are both attached to form a 3-12 membered monocyclic, spirocyclic or bridged ring optionally substituted with one or more R^{m};
or R^{a} or R^{b} together with the carbon atom or ortho-nitrogen atom to which they are attached to form a 5-14 membered heteroaryl, 6-14 membered aryl, 3-14 membered heterocyclyl or 3-12 membered cycloalkyl optionally substituted with one or more R^{m};
or R^{b} and R^{c} together with the nitrogen atom to which they are both attached to form a 3-12 membered monocyclic, spirocyclic, bridged or fused ring optionally substituted with one or more R^{m};
R^{e} and R^{f} are each independently selected from the group consisting of hydrogen, C₁₋₆ alkyl, C₂₋₆ alkenyl, C₂₋₆ alkynyl, 3-12 membered cycloalkyl, 3-20 membered heterocyclyl, 6-20 membered aryl and 5-20 membered heteroaryl, wherein the C₁₋₆ alkyl, C₂₋₆ alkenyl, C₂₋₆ alkynyl, 3-12 membered cycloalkyl, 3-20 membered heterocyclyl, 6-20 membered aryl and 5-20 membered heteroaryl are optionally substituted with one or more C₁₋₆ alkyl or halogen;
R^{g} is selected from the group consisting of C₁₋₆ alkyl, 3-12 membered cycloalkyl, 3-20 membered heterocyclyl, amino, C₁₋₆ alkylamino, di(C₁₋₆ alkyl)amino and 6-20 membered aryl, wherein the C₁₋₆ alkyl, 3-12 membered cycloalkyl, 3-20 membered heterocyclyl, C₁₋₆ alkylamino, di(C₁₋₆ alkyl)amino and 6-20 membered aryl are optionally substituted with one or more groups independently selected from the group consisting of halogen, OR^{e} or -C(=O)NR^{e}R^{f};
R' is selected from the group consisting of hydrogen, C₁₋₆ alkyl, 3-12 membered cycloalkyl, 3-12 membered cycloalkenyl, 3-12 membered heterocyclyl, 6-14 membered aryl and 5-14 membered heteroaryl, the C₁₋₆ alkyl, 3-12 membered cycloalkyl, 3-12 membered cycloalkenyl, 3-12 membered heterocyclyl, 6-14 membered aryl and 5-14 membered heteroaryl are optionally substituted with one or more R^{m}; the 3-12 membered cycloalkyl, 3-12 membered cycloalkenyl and 3-12 membered heterocyclyl comprise monocyclic, spirocyclic or bridged ring, and the 6-14 membered aryl and 5-14 membered heteroaryl comprise monocyclic or fused ring;
R^{m} is selected from the group consisting of deuterium, halogen, cyano, nitro, - OR^{a}, -SR^{a}, -C(=Y¹)R^{a}, -C(=Y¹)OR^{a}, -C(=Y¹)NR^{b}R^{c}, -(CR^{a}R^{b})ₙ₁-NR^{b}R^{c}, - NR^{b}C(=Y¹)R^{c}, -NR^{b}C(=Y¹)OR^{c}, -NR^{d}C(=Y¹)NR^{b}R^{c}, -NR^{b}SO₂R^{a}, -OC(=Y¹)R^{a}, - OC(=Y¹)OR^{b}, -OC(=Y¹)NR^{a}R^{b}, -OSO₂(OR^{b}), -OP(=Y¹)(OR^{b})(OR^{c}), -OP(OR^{b})(OR^{c}), -SOR^{a}, -SO₂R^{a}, -SO₂NR^{b}R^{c}, -SO(OR^{b}), -SO₂(OR^{b}), -SC(=Y¹)R^{a} ,-SC(=Y¹)OR^{b}, - SC(=Y¹)NR^{b}R^{c}, C₁₋₁₂ alkyl, halogenated C₁₋₁₂ alkyl, C₁₋₁₂ alkoxy-C₁₋₁₂ alkyl, C₁₋₁₂ alkyl-C₁₋₁₂ alkoxy, C₁₋₁₂ alkoxy-C₁₋₁₂ alkoxy, C₂₋₈ alkenyl, C₂₋₈ alkynyl, 3-12 membered cycloalkyl, 5-14 membered heterocyclyl, 6-20 membered aryl, 5-20 membered heteroaryl, (CR^{a}R^{d})ₜNR^{b}R^{c}, azido and oxo, wherein the 3-12 membered cycloalkyl and the 5-14 membered heterocyclyl comprise monocyclic, spirocyclic or bridged ring, and the 6-20 membered aryl and the 5-20 membered heteroaryl comprise monocyclic or fused ring;
Y¹ is O or S;
t, m1, and n1 are each independently 0, 1, 2, 3, 4, 5, or 6;
the conditions are as follows:
when A is 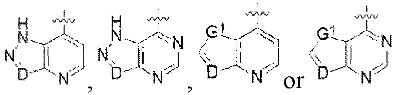 substituted with R¹, and -X-Y is -NR'-C(=O)-, R¹ is not hydroxyalkyl, except that NH is substituted with R¹, for example in the 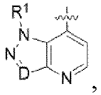 R¹ may be hydroxyC₁₋₆ alkyl, wherein D is selected from N or CH, G¹ is selected from NH, O or S;
when A is 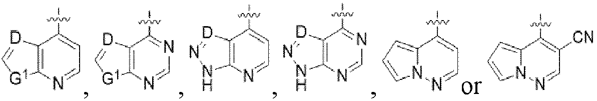 substituted with R¹, and -X-Y is-NR' -C(=O)-, R¹ is not hydroxyalkyl, wherein D is selected from N or CH, G¹ is selected from NH, O or S;
when A is 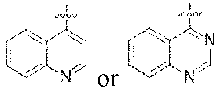 substituted with R¹, Z is a phenyl ring, and -X-Y is -NH-C(=O)-, R¹ is not hydroxyalkyl;
E₃ is not C=O, C=S or C=NR^{a}; when E₃ is CH, E₁ is not C=O and E₂ is not NR^{b}; when E₃ is absent, E₂ is not C=O, C=S or C=NR^{a};
In certain embodiments, A is selected from 5-14 membered heteroaryl substituted with R¹, the 5-14 membered heteroaryl substituted with R¹ comprises monocyclic or fused ring, optionally substituted with one or more R²;
Preferably, in formula I, A is selected from the following groups substituted with R¹, the following groups are optionally substituted with one or more R²; 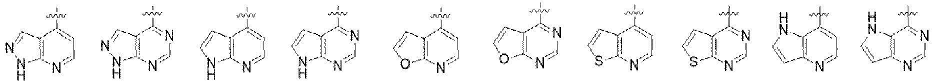 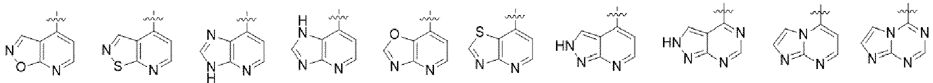 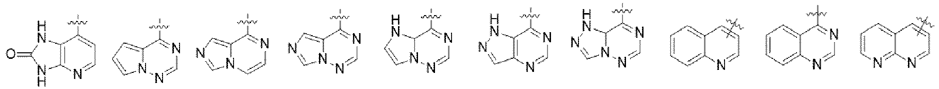 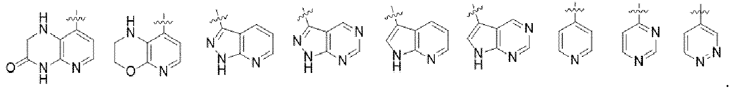
In certain embodiments, L is selected from O, S, CR^{a}R^{b}, NR^{b}, and C(=O);

Preferably, in formula I, L is selected from O, S, C(=O) and NH.

In certain embodiments, Z is selected from 6-14 membered aryl and 5-10 membered heteroaryl optionally substituted with one or more R³;
Preferably, in formula I, Z is selected from the following groups optionally substituted with one or more R³:

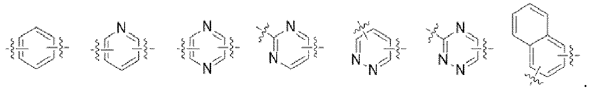

In certain embodiments, X is selected from NR⁴, O, S, or is absent;
Preferably, in formula I, X is selected from NR⁴, O and S.

In certain embodiments, Y is selected from C(=O), C(=S), C(=NR⁴), or is absent;
Preferably, in formula I, Y is selected from C(=O) and C(=S).

In certain embodiments, E₁, E₂, E₃ are each independently selected from CR^{a}R^{b}, N, C(=O), and C(=S);
Preferably, in formula I, E₁, E₂, E₃ are each independently selected from CR^{a}R^{b} and N.

In certain embodiments, G is selected from (CR^{a}R^{b})ₙ₁-6-20 membered aryl, (CR^{a}R^{b})ₙ₁-5-20 membered heteroaryl, (CR^{a}R^{b})ₙ₁-3-12 membered cycloalkyl and (CR^{a}R^{b})ₙ₁-3-20 membered heterocyclyl, the (CR^{a}R^{b})ₙ₁-6-20 membered aryl, (CR^{a}R^{b})ₙ₁-5-20 membered heteroaryl, (CR^{a}R^{b})ₙ₁-3-12 membered cycloalkyl and (CR^{a}R^{b})ₙ₁-3-20 membered heterocyclyl are optionally substituted with one or more groups independently selected from the group consisting of: halogen, nitro, oxo, CN, OR^{e}, SR^{e}, NR^{e}R^{f}, C₁₋₁₂ alkyl, halogenated C₁₋₁₂ alkyl, C₁₋₆ alkoxy-C₁₋₁₂ alkyl, halogenated C₁₋₆ alkoxy-C₁₋₁₂ alkyl, R^{a}R^{b}N-C₁₋₆ alkyl, C₂₋₈ alkenyl, halogenated C₂₋₈ alkenyl, C₂₋₈ alkynyl, 3-12 membered cycloalkyl, 3-12 membered cycloalkyl-C₁₋₆ alkyl or 3-20 membered heterocyclyl;
Preferably, in formula I, G is selected from 6-20 membered aryl and 5-20 membered heteroaryl, wherein the 6-20 membered aryl and 5-20 membered heteroaryl are optionally substituted with one or more groups independently selected from the group consisting of: halogen, nitro, oxo, CN, OR^{e}, SR^{e}, NR^{e}R^{f}, C₁₋₁₂ alkyl, halogenated C₁₋₁₂ alkyl, C₁₋₆ alkoxy-C₁₋₁₂ alkyl, halogenated C₁₋₆ alkoxy-C₁₋₁₂ alkyl, R^{a}R^{b}N-C₁₋₆ alkyl, C₂₋₈ alkenyl, halogenated C₂₋₈ alkenyl, C₂₋₈ alkynyl, 3-12 membered cycloalkyl-C₁₋₆ alkyl or 3-20 membered heterocyclyl.

In certain embodiments, R¹ is selected from R⁸ substituted with one or two hydroxy;
R⁸ is selected from the group consisting of -NR^{b}R^{c}, -OR^{b}, -SR^{b}, R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered cycloalkenyl, 3-12 membered heterocyclyl, 6-14 membered aryl, 5-10 membered heteroaryl, 3-12 membered cycloalkyl-C₁₋₆ alkyl, 3-12 membered cycloalkenyl-C₁₋₆ alkyl, 3-12 membered heterocyclyl-C₁₋₆ alkyl, 6-14 membered aryl-C₁₋₆ alkyl, and 5-10 membered heteroaryl-C₁₋₆ alkyl, the R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered cycloalkenyl, 3-12 membered heterocyclyl, 6-14 membered aryl, 5-10 membered heteroaryl, 3-12 membered cycloalkyl-C₁₋₆ alkyl, 3-12 membered cycloalkenyl-C₁₋₆ alkyl, 3-12 membered heterocyclyl-C₁₋₆ alkyl, 6-14 membered aryl-C₁₋₆ alkyl, and 5-10 membered heteroaryl-C₁₋₆ alkyl are optionally substituted with one or more R^{m}, the 3-12 membered cycloalkyl, 3-12 membered cycloalkenyl and 3-12 membered heterocyclyl comprise monocyclic, spirocyclic or bridged ring, the 6-14 membered aryl and 5-10 membered heteroaryl comprise monocyclic or fused ring;
Preferably, R⁸ is selected from the group consisting of -NR^{b}R^{c}, -OR^{b}, C₁₋₆ alkyl, R^{b}R^{c}N-C₁₋₆ alkyl, 3-12 membered heterocyclyl and 3-12 membered heterocyclyl-C₁₋₆ alkyl, the C₁₋₆ alkyl, R^{b}R^{c}N-C₁₋₆ alkyl, 3-12 membered heterocyclyl and 3-12 membered heterocyclyl-C₁₋₆ alkyl are optionally substituted with one or more R^{m}, the 3-12 membered heterocyclyl and 3-12 membered heterocyclyl-C₁₋₆ alkyl comprise monocyclic, spirocyclic or bridged ring.

In certain embodiments, R² and R³ are each independently selected from the group consisting of hydrogen, halogen, cyano, nitro, -NR^{b}R^{c}, -OR^{b}, R^{b}R^{c}N-C₁₋₆ alkyl, R^{b}R^{c}N-C₁₋₆ alkoxy, -C(=Y¹)R^{a}, -C(=Y¹)NR^{b}R^{c}, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl, 3-12 membered heterocyclyl-C₁₋₆ alkyl and 5-10 membered heteroaryl-C₁₋₆ alkyl, the R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl, 3-12 membered heterocyclyl-C₁₋₆ alkyl and 5-10 membered heteroaryl-C₁₋₆ alkyl are optionally substituted with one or more R^{m}, the 3-12 membered cycloalkyl, 3-12 membered heterocyclyl, 3-12 membered cycloalkyl-C₁₋₆ alkyl, and 3-12 membered heterocyclyl-C₁₋₆ alkyl comprise monocyclic, spirocyclic, or bridged ring, the 5-10 membered heteroaryl-C₁₋₆ alkyl comprise monocyclic or fused ring;
Preferably, R² and R³ are each independently selected from the group consisting of hydrogen, halogen, cyano, nitro, -NR^{b}R^{c}, -OR^{b}, R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl and 3-12 membered heterocyclyl-C₁₋₆ alkyl, the R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl and 3-12 membered heterocyclyl-C₁₋₆ alkyl are optionally substituted with one or more R^{m}, the 3-12 membered cycloalkyl, 3-12 membered heterocyclyl, 3-12 membered cycloalkyl-C₁₋₆ alkyl, and 3-12 membered heterocyclyl-C₁₋₆ alkyl comprise monocyclic, spirocyclic or bridged ring.

In certain embodiments, R⁴, R⁵, R⁶, and R⁷ are each independently selected from the group consisting of hydrogen, halogen, OR^{e}, C₁₋₆ alkyl, C₂₋₆ alkenyl, C₂₋₆ alkynyl, 3-20 membered cycloalkyl, 3-20 membered heterocyclyl and 6-20 membered aryl, wherein the 3-20 membered cycloalkyl and the 3-20 membered heterocyclyl comprise monocyclic, spirocyclic, or bridged ring; the C₁₋₆ alkyl, C₂₋₆ alkenyl, C₂₋₆ alkynyl, 3-20 membered cycloalkyl, 3-20 membered heterocyclyl and 6-20 membered aryl are optionally substituted with one or more groups independently selected from the group consisiting of halogen, CN, OR^{e}, NR^{e}R^{f}, C₁₋₁₂ alkyl, C₂₋₈ alkenyl, C₂₋₈ alkynyl, 3-12 membered cycloalkyl or 3-20 membered heterocyclyl;
or R⁴ and R⁵ together with the carbon atom to which they are both attached to form a 3-12 membered monocyclic, spirocyclic or bridged ring optionally substituted with one or more R^{m};
or R⁶ and R⁷ together with the nitrogen atom to which they are both attached to form a saturated, partially unsaturated or fully unsaturated 3-20 membered heterocycle optionally substituted with one or more R^{m}, which comprsing one or more heteroatoms selected from N, O or S;
Preferably, R⁴, R⁵, R⁶ and R⁷ are each independently selected from the group consisting of hydrogen, OR^{e}, C₁₋₆ alkyl, monocyclic or bicyclic of 3-12 membered cycloalkyl, 3-20 membered heterocyclyl and 6-20 membered aryl, wherein the C₁₋₆ alkyl, monocyclic or bicyclic of 3-12 membered cycloalkyl, 3-20 membered heterocyclyl and 6-20 membered aryl are optionally substituted with one or more groups independently selected from the group consisting of: halogen, CN, OR^{e}, NR^{e}R^{f}, C₁₋₁₂ alkyl, C₂₋₈ alkenyl, C₂₋₈ alkynyl, 3-12 membered cycloalkyl or 3-20 membered heterocyclyl;
or R⁴ and R⁵ together with the carbon atom to which they are both attached to form a 3-12 membered monocyclic, spirocyclic or bridged ring optionally substituted with one or more R^{m}, the 3-12 membered monocyclic, spirocyclic or bridged ring is optionally substituted with one or more groups independently selected from the group consisiting of: halogen, C₁₋₆ alkyl or C₁₋₆ alkoxy;
or R⁶ and R⁷ together with the nitrogen atom to which they are both attached to form a saturated, partially unsaturated, or fully unsaturated 3-20 membered heterocycle optionally substituted with one or more R^{m}, the 3-20 membered heterocycle is optionally substituted with one or more groups independently selected from the group consisting of halogen, C₁₋₆ alkyl or C₁₋₆ alkoxy.

In certain embodiments, R^{a} is absent or selected from the group consisting of hydrogen, halogen, cyano, hydroxyl, mercapto, carboxyl, nitro, -NR^{b}R^{c}, -C(=O)R^{d}, C₁₋₆ alkyl, C₁₋₆ alkoxy, C₂₋₈ alkenyl, C₂₋₈ alkynyl, C₁₋₆ alkyl-R', C₁₋₆ alkoxy-R', -OR', - SR', -SOR^{d}, -SO₂R^{d}, 3-12 membered cycloalkyl, 3-12 membered cycloalkenyl, 3-12 membered heterocyclyl, 6-14 membered aryl and 5-10 membered heteroaryl, the C₁₋₆ alkyl, C₁₋₆ alkoxy, C₂₋₈ alkenyl, C₂₋₈ alkynyl, C₁₋₆ alkyl-R', C₁₋₆ alkoxy-R', 3-12 membered cycloalkyl, 3-12 membered cycloalkenyl, 3-12 membered heterocyclyl, 6-14 membered aryl, and 5-10 membered heteroaryl are optionally substituted with one or more R^{m};
R^{b}, R^{c} and R^{d} are each absent or each independently selected from the group consisting of hydrogen, hydroxyl, mercapto, C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R, -OR', -C(=O)-R', 3-12 membered cycloalkyl, 3-12 membered cycloalkenyl, 3-12 membered heterocyclyl, 6-14 membered aryl and 5-10 membered heteroaryl, the C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', 3-12 membered cycloalkyl, 3-12 membered cycloalkenyl, 3-12 membered heterocyclyl, 6-14 membered aryl, and 5-10 membered heteroaryl are optionally substituted with one or more R^{m};
or R^{b} and R^{c} together with the nitrogen atom to which they are both attached to form a 3-12 membered monocyclic, spirocyclic, bridged or fused ring optionally substituted with one or more R^{m};
R^{e} and R^{f} are each independently selected from the group consisting of hydrogen, C₁₋₆ alkyl, C₂₋₆ alkenyl, 3-12 membered cycloalkyl, 3-20 membered heterocyclyl, 6-20 membered aryl, and 5-20 membered heteroaryl, wherein the C₁₋₆ alkyl, C₂₋₆ alkenyl, 3-12 membered cycloalkyl, 3-20 membered heterocyclyl, 6-20 membered aryl, and 5-20 membered heteroaryl are optionally substituted with one or more C₁₋₆ alkyl or halogen;
R^{g} is C₁₋₆ alkyl or 6-20 membered aryl, wherein the C₁₋₆ alkyl and 6-20 membered aryl are optionally substituted with one or more groups independently selected from the group consisting of halogen or OR^{e};
R' is selected from the group consisting of C₁₋₆ alkyl, 3-12 membered cycloalkyl, 3-12 membered cycloalkenyl, 3-12 membered heterocyclyl, 6-14 membered aryl and 5-10 membered heteroaryl, the C₁₋₆ alkyl, 3-12 membered cycloalkyl, 3-12 membered cycloalkenyl, 3-12 membered heterocyclyl, 6-14 membered aryl and 5-10 membered heteroaryl are optionally substituted with one or more R^{m}; the 3-12 membered cycloalkyl, 3-12 membered alkenyl and 3-12 membered heterocyclyl comprise monocyclic, spirocyclic or bridged ring, and the 6-14 membered aryl and 5-10 membered heteroaryl comprise monocyclic or fused ring.

Preferably, R^{a} is absent or selected from the group consisting of hydrogen, halogen, cyano, hydroxyl, -NR^{b}R^{c}, -C(=O)R^{d}, C₁₋₆ alkyl, C₁₋₆ alkoxy, C₂₋₈ alkenyl, C₁₋₆ alkyl-R', C₁₋₆ alkoxy-R', -OR', -SR', -SOR^{d}, -SO₂R^{d}, 3-12 membered cycloalkyl, 3-12 membered cycloalkenyl and 3-12 membered heterocyclyl, the C₁₋₆ alkyl, C₁₋₆ alkoxy, C₂₋₈ alkenyl, C₁₋₆ alkyl-R', C₁₋₆ alkoxy-R, 3-12 membered cycloalkyl, 3-12 membered cycloalkenyl and 3-12 membered heterocyclyl are optionally substituted with one or more R^{m};
R^{b}, R^{c} and R^{d} are each absent or each independently selected from the group consisting of hydrogen, hydroxyl, mercapto, C₁₋₆ alkyl, halogen, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', -OR', -C(=O)-R', 3-12 membered cycloalkyl and 3-12 membered heterocyclyl, the C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', -OR', -C(=O)-R', 3-12 membered cycloalkyl and 3-12 membered heterocyclyl are optionally substituted with one or more R^{m};
or R^{b} and R^{c} together with the nitrogen atom to which they are both attached to form a 3-12 membered monocyclic, spirocyclic, bridged or fused ring optionally substituted with one or more R^{m};
R^{e} and R^{f} are each independently selected from the group consisting of hydrogen, C₁₋₆ alkyl, C₂₋₆ alkenyl, 3-12 membered cycloalkyl and 3-20 membered heterocyclyl, wherein the C₁₋₆ alkyl, C₂₋₆ alkenyl, 3-12 membered cycloalkyl and 3-20 membered heterocyclyl are optionally substituted with one or more C₁₋₆ alkyl or halogen;
R^{g} is C₁₋₆ alkyl or 6-20 membered aryl, wherein the C₁₋₆ alkyl and 6-20 membered aryl are optionally substituted with one or more groups independently selected from: halogen or OR^{e};
R' is selected from the group consisting of C₁₋₆ alkyl, 3-12 membered cycloalkyl, 3-12 membered heterocyclyl and 5-10 membered heteroaryl, the C₁₋₆ alkyl, 3-12 membered cycloalkyl, 3-12 membered heterocyclyl and 5-10 membered heteroaryl are optionally substituted with one or more R^{m}; the 3-12 membered cycloalkyl and the 3-12 membered heterocyclyl comprise monocyclic, spirocyclic or bridged ring, and the 5-10 membered heteroaryl comprise monocyclic or fused ring.

In certain embodiments, R^{m} is independently selected from the group consisting of deuterium, halogen, cyano, nitro, -OR^{a}, -SR^{a}, -C(=Y¹)R^{a}, -C(=Y¹)OR^{a}, - C(=Y¹)NR^{b}R^{c}, -NR^{b}R^{c}, -NR^{b}C(=Y¹)R^{c}, -NR^{b}SO₂R^{a}, -OC(=Y¹)R^{a}, -SO₂R^{a}, C₁₋₁₂ alkyl, halogenerated C₁₋₁₂ alkyl, C₁₋₁₂ alkoxy-C₁₋₁₂ alkyl, C₁₋₁₂ alkyl-C₁₋₁₂ alkoxy, C₁₋₁₂ alkoxy-C₁₋₁₂ alkoxy, C₂₋₈ alkenyl, C₂₋₈ alkynyl, 3-12 membered cycloalkyl, 3-20 membered heterocyclyl, 6-20 membered aryl, 5-20 membered heteroaryl, (CR^{a}R^{d})ₜNR^{b}R^{c} and oxo, the 3-12 membered cycloalkyl and 3-20 membered heterocyclyl comprise monocyclic, spirocyclic or bridged ring, the 6-20 membered aryl and 5-20 membered heteroaryl groups comprise monocyclic or fused ring;
Preferably, R^{m} is selected from the group consisting of deuterium, halogen, cyano, nitro, hydroxyl, mercapto, amino, carboxyl, C₁₋₆ alkyl, C₁₋₆ alkoxy-C₁₋₆ alkyl, C₁₋₆ alkoxy, C₁₋₆ alkyl-C₁₋₆ alkoxy, C₁₋₆ alkoxy-C₁₋₆ alkoxy, C₁₋₆ alkylamino, (C₁₋₆ alkyl)₂amino, C₁₋₆ alkyl ester, C₁₋₆ alkylaminocarbonyl, (C₁₋₆ alkyl)₂aminocarbonyl, C₁₋₆ alkylcarbonyl, C₁₋₆ alkylcarbonyloxy, C₁₋₆ alkylcarbonylamino, C₁₋₆ alkylsulfonylamino, halogenated C₁₋₆ alkyl, halogenated C₁₋₆ alkoxy, C₁₋₆ alkylsulfonyl, C₁₋₆ alkylthio, C₂₋₈ alkenyl, C₂₋₈ alkynyl, 3-8 membered cycloalkyl, 3-8 membered heterocyclyl, 6-10 membered aryl, 3-8 membered heteroaryl, - (CR^{a}R^{d})ₜNR^{b}R^{c} and oxo.

In certain embodiments, A is selected from 5-14 membered heteroaryl substituted with R¹, the 5-14 membered heteroaryl substituted with R¹ comprises monocyclic or fused ring, optionally substituted with one or more R²;
L is selected from O, S, CR^{a}R^{b}, NR^{b} and C(=O);
Z is selected from 6-14 membered aryl and 5-10 membered heteroaryl optionally substituted with one or more R³;
X is selected from NR⁴, O, S or is absent;
Y is selected from C(=O), C(=S), C(=NR⁴) or is absent;
E₁, E₂, E₃ are each independently selected from CR^{a}R^{b}, N, C(=O) and C(=S);
G is selected from the group consisting of (CR^{a}R^{b})ₙ₁-6-20 membered aryl, (CR^{a}R^{b})ₙ₁-5-20 membered heteroaryl, (CR^{a}R^{b})ₙ₁-3-12 membered cycloalkyl and (CR^{a}R^{b})ₙ₁-3-20 membered heterocyclyl, the (CR^{a}R^{b})ₙ₁-6-20 membered aryl, (CR^{a}R^{b})ₙ₁-5-20 membered heteroaryl, (CR^{a}R^{b})ₙ₁-3-12 membered cycloalkyl and (CR^{a}R^{b})ₙ₁-3-20 membered heterocyclyl are optionally substituted with one or more groups independently selected from the group consisting of: halogen, nitro, oxo, CN, OR^{e}, SR^{e}, NR^{e}R^{f}, C₁₋₁₂ alkyl, halogenated C₁₋₁₂ alkyl, C₁₋₆ alkoxy-C₁₋₁₂ alkyl, halogenated C₁₋₆ alkoxy-C₁₋₁₂ alkyl, R^{a}R^{b}N-C₁₋₆ alkyl, C₂₋₈ alkenyl, halogenated C₂₋₈ alkenyl, C₂₋₈ alkynyl, 3-12 membered cycloalkyl or 3-20 membered heterocyclyl;
R¹ is selected from R⁸ substituted with one or two hydroxy;
R² and R³ are each independently selected from the group consisting of hydrogen, halogen, cyano, nitro, -NR^{b}R^{c}, -OR^{b}, R^{b}R^{c}N-C₁₋₆ alkyl, R^{b}R^{c}N-C₁₋₆ alkoxy, -C(=Y¹)R^{a}, -C(=Y¹)NR^{b}R^{c}, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl, 3-12 membered heterocyclyl-C₁₋₆ alkyl and 5-10 membered heteroaryl-C₁₋₆ alkyl, the R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl, 3-12 membered heterocyclyl-C₁₋₆ alkyl and 5-10 membered heteroaryl-C₁₋₆ alkyl are optionally substituted with one or more R^{m}, the 3-12 membered cycloalkyl, 3-12 membered heterocyclyl, 3-12 membered cycloalkyl-C₁₋₆ alkyl and 3-12 membered heterocyclyl-C₁₋₆ alkyl comprise monocyclic, spirocyclic or bridged ring, the 5-10 membered heteroaryl-C₁₋₆ alkyl comprises monocyclic or fused ring;
R⁴, R⁵, R⁶ and R⁷ are each independently selected from the group consisting of hydrogen, halogen, OR^{e}, C₁₋₆ alkyl, C₂₋₆ alkenyl, C₂₋₆ alkynyl, 3-20 membered cycloalkyl, 3-20 membered heterocyclyl and 6-20 membered aryl, wherein the 3-20 membered cycloalkyl and 3-20 membered heterocyclyl comprise monocyclic, spirocyclic or bridged ring; the C₁₋₆ alkyl, C₂₋₆ alkenyl, C₂₋₆ alkynyl, 3-20 membered cycloalkyl, 3-20 membered heterocyclyl and 6-20 membered aryl are optionally substituted with one or more groups independently selected from the group consisting of: halogen, CN, OR^{e}, NR^{e}R^{f}, C₁₋₁₂ alkyl, C₂₋₈ alkenyl, C₂₋₈ alkynyl, 3-12 membered cycloalkyl or 3-20 membered heterocyclyl;
or R⁴ and R⁵ together with the carbon atom to which they are both attached to form a 3-12 membered monocyclic, spirocyclic or bridged ring optionally substituted with one or more R^{m};
or R⁶ and R⁷ together with the nitrogen atom to which they are both attached to form a saturated, partially unsaturated or fully unsaturated 3-20 membered heterocycle optionally substituted with one or more R^{m}, containing one or more heteroatoms selected from N, O or S;
R⁸ is selected from the group consisting of -NR^{b}R^{c}, -OR^{b}, -SR^{b}, R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered cycloalkenyl, 3-12 membered heterocyclyl, 6-14 membered aryl, 5-10 membered heteroaryl, 3-12 membered cycloalkyl-C₁₋₆ alkyl, 3-12 membered cycloalkenyl-C₁₋₆ alkyl, 3-12 membered heterocyclyl-C₁₋₆ alkyl, 6-14 membered aryl-C₁₋₆ alkyl, and 5-10 membered heteroaryl-C₁₋₆ alkyl, the R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered cycloalkenyl, 3-12 membered heterocyclyl, 6-14 membered aryl, 5-10 membered heteroaryl, 3-12 membered cycloalkyl-C₁₋₆ alkyl, 3-12 membered cycloalkenyl-C₁₋₆ alkyl, 3-12 membered heterocyclyl-C₁₋₆ alkyl, 6-14 membered aryl-C₁₋₆ alkyl, and 5-10 membered heteroaryl-C₁₋₆ alkyl are optionally substituted with one or more R^{m}, the 3-12 membered cycloalkyl, 3-12 membered cycloalkenyl and 3-12 membered heterocyclyl comprise monocyclic, spirocyclic or bridged ring, the 6-14 membered aryl and 5-10 membered heteroaryl comprise monocyclic or fused ring;
R^{a} is absent or selected from the group consisting of hydrogen, halogen, cyano, hydroxyl, mercapto, carboxyl, nitro, -NR^{b}R^{c}, -C(=O)R^{d}, C₁₋₆ alkyl, C₁₋₆ alkoxy, C₂₋₈ alkenyl, C₂₋₈ alkynyl, C₁₋₆ alkyl-R', C₁₋₆ alkoxy-R', -OR', -SR', -SOR^{d}, -SO₂R^{d}, 3-12 membered cycloalkyl, 3-12 membered cycloalkenyl, 3-12 membered heterocyclyl, 6-14 membered aryl and 5-10 membered heteroaryl, the C₁₋₆ alkyl, C₁₋₆ alkoxy, C₂₋₈ alkenyl, C₂₋₈ alkynyl, C₁₋₆ alkyl-R', C₁₋₆ alkoxy-R', 3-12 membered cycloalkyl, 3-12 membered cycloalkenyl, 3-12 membered heterocyclyl, 6-14 membered aryl, and 5-10 membered heteroaryl are optionally substituted with one or more R^{m};
R^{b}, R^{c} and R^{d} are each absent or each independently selected from the group consisting of hydrogen, hydroxyl, mercapto, C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R, -OR', -C(=O)-R', 3-12 membered cycloalkyl, 3-12 membered cycloalkenyl, 3-12 membered heterocyclyl, 6-14 membered aryl and 5-10 membered heteroaryl, the C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', 3-12 membered cycloalkyl, 3-12 membered cycloalkenyl, 3-12 membered heterocyclyl, 6-14 membered aryl, and 5-10 membered heteroaryl are optionally substituted with one or more R^{m};
or R^{b} and R^{c} together with the nitrogen atom to which they are both attached to form a 3-12 membered monocyclic, spirocyclic, bridged or fused ring optionally substituted with one or more R^{m};
R^{e} and R^{f} are each independently selected from the group consisting of hydrogen, C₁₋₆ alkyl, C₂₋₆ alkenyl, 3-12 membered cycloalkyl, 3-20 membered heterocyclyl, 6-20 membered aryl, and 5-20 membered heteroaryl, wherein the C₁₋₆ alkyl, C₂₋₆ alkenyl, 3-12 membered cycloalkyl, 3-20 membered heterocyclyl, 6-20 membered aryl, and 5-20 membered heteroaryl are optionally substituted with one or more C₁₋₆ alkyl or halogen;
R^{g} is C₁₋₆ alkyl or 6-20 membered aryl, wherein the C₁₋₆ alkyl and 6-20 membered aryl are optionally substituted with one or more groups independently selected from the group consisting of halogen or OR^{e};
R' is selected from the group consisting of C₁₋₆ alkyl, 3-12 membered cycloalkyl, 3-12 membered cycloalkenyl, 3-12 membered heterocyclyl, 6-14 membered aryl and 5-10 membered heteroaryl, the C₁₋₆ alkyl, 3-12 membered cycloalkyl, 3-12 membered cycloalkenyl, 3-12 membered heterocyclyl, 6-14 membered aryl and 5-10 membered heteroaryl are optionally substituted with one or more R^{m}; the 3-12 membered cycloalkyl, 3-12 membered cycloalkenyl and 3-12 membered heterocyclyl comprise monocyclic, spirocyclic or bridged ring, and the 6-14 membered aryl and 5-10 membered heteroaryl comprise monocyclic or fused ring;
R^{m} is independently selected from the group consisting of deuterium, halogen, cyano, nitro, -OR^{a}, -SR^{a}, -C(=Y¹)R^{a}, -C(=Y¹)OR^{a}, -C(=Y¹)NR^{b}R^{c}, -NR^{b}R^{c}, - NR^{b}C(=Y¹)R^{c}, -NR^{b}SO₂R^{a}, -OC(=Y¹)R^{a}, -SO₂R^{a},C₁₋₁₂ alkyl, halogenated C₁₋₁₂ alkyl, C₁₋₁₂ alkoxy-C₁₋₁₂ alkyl, C₁₋₁₂ alkyl-C₁₋₁₂ alkoxy, C₁₋₁₂ alkoxy-C₁₋₁₂ alkoxy, C₂₋₈ alkenyl, C₂₋₈ alkynyl, 3-12 membered cycloalkyl, 3-20 membered heterocyclyl, 6-20 membered aryl, 5-20 membered heteroaryl, (CR^{a}R^{d})ₜNR^{b}R^{c} and oxo, and the 3-12 membered cycloalkyl and 3-20 membered heterocyclyl comprise monocyclic, spirocyclic or bridged ring, and the 6-20 membered aryl and 5-20 membered heteroaryl comprise monocyclic or fused ring.
Y¹ is O or S;
t, m1, and n1 are each independently 0, 1, 2, 3, 4, 5, or 6.

In other embodiments, A is selected from the following groups substituted with R¹, optionally substituted with one or more R²; 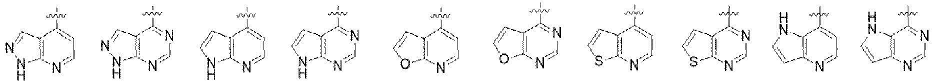 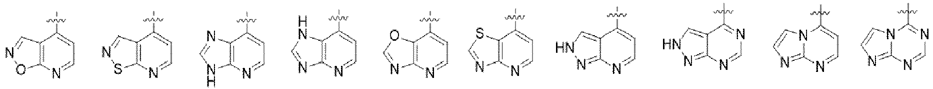 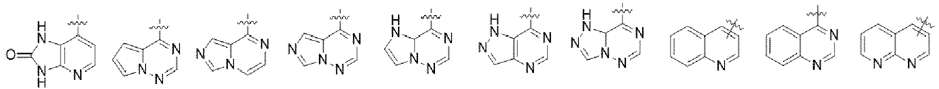 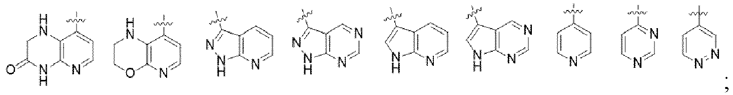
L is selected from O, S, C=O and NH;
Z is selected from the following groups optionally substituted with one or more R3: 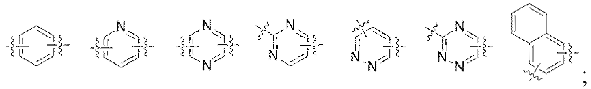
X is selected from NR⁴, O and S;
Y is selected from C(=O) and C(=S);
E₁, E₂, E₃ are each independently selected from CR^{a}R^{b} and N;
G is selected from the group consisting of 6-20 membered aryl and 5-20 membered heteroaryl, wherein the 6-20 membered aryl and 5-20 membered heteroaryl are optionally substituted with one or more groups independently selected from the group consisting of: halogen, nitro, oxo, CN, OR^{e}, SR^{e}, NR^{e}R^{f}, C₁₋₁₂ alkyl, halogenated C₁₋₁₂ alkyl, C₁₋₆ alkoxy-C₁₋₁₂ alkyl, halogenated C₁₋₆ alkoxy-C₁₋₁₂ alkyl, R^{a}R^{b}N-C₁₋₆ alkyl, C₂₋₈ alkenyl, halogenated C₂₋₈ alkenyl, C₂₋₈ alkynyl, 3-12 membered cycloalkyl, 3-12 membered cycloalkyl-C₁₋₆ alkyl or 3-20 membered heterocyclyl;
R¹ is selected from R⁸ substituted with one or two hydroxy;
R² and R³ are each independently selected from the group consisting of hydrogen, halogen, cyano, nitro, -NR^{b}R^{c}, -OR^{b}, R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl and 3-12 membered heterocyclyl-C₁₋₆ alkyl, the R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl and 3-12 membered heterocyclyl-C₁₋₆ alkyl are optionally substituted with one or more R^{m}, the 3-12 membered cycloalkyl, 3-12 membered heterocyclyl, 3-12 membered cycloalkyl-C₁₋₆ alkyl and 3-12 membered heterocyclyl-C₁₋₆ alkyl comprise monocyclic, spirocyclic or bridged ring;
R⁴, R⁵, R⁶ and R⁷ are each independently selected from the group consisting of hydrogen, OR^{e}, C₁₋₆ alkyl, monocyclic or bicyclic of 3-12 membered cycloalkyl, 3-20 membered heterocyclyl and 6-20 membered aryl, wherein the C₁₋₆ alkyl, monocyclic or bicyclic of 3-12 membered cycloalkyl, 3-20 membered heterocyclyl and 6-20 membered aryl are optionally substituted with one or more groups independently selected from the group consisting of halogen, CN, OR^{e}, NR^{e}R^{f}, C₁₋₁₂ alkyl, C₂₋₈ alkenyl, C₂₋₈ alkynyl, 3-12 membered cycloalkyl or 3-20 membered heterocyclyl;
or R⁴ and R⁵ together with the carbon atom to which they are both attached to form a 3-12 membered monocyclic, spirocyclic or bridged ring optionally substituted with one or more R^{m}, the 3-12 membered monocyclic, spirocyclic or bridged ring is optionally substituted with one or more groups independently selected from: halogen, C₁₋₆ alkyl or C₁₋₆ alkoxy;
or R⁶ and R⁷ together with the nitrogen atom to which they are both attached to form a saturated, partially unsaturated, or fully unsaturated 3-20 membered heterocycle optionally substituted with one or more R^{m}, the 3-20 membered heterocycle is optionally substituted with one or more groups independently selected from: halogen, C₁₋₆ alkyl or C₁₋₆ alkoxy;
R⁸ is selected from the group consisting of-NR^{b}R^{c}, -OR^{b}, C₁₋₆ alkyl, R^{b}R^{c}N-C₁₋₆ alkyl, 3-12 membered heterocyclyl and 3-12 membered heterocyclyl-C₁₋₆ alkyl, the C₁₋₆ alkyl, R^{b}R^{c}N-C₁₋₆ alkyl, 3-12 membered heterocyclyl and 3-12 membered heterocyclyl-C₁₋₆ alkyl are optionally substituted with one or more R^{m}, the 3-12 membered heterocyclyl and 3-12 membered heterocyclyl-C₁₋₆ alkyl comprise monocyclic, spirocyclic or bridged ring;
R^{a} is absent or selected from the group consisting of hydrogen, halogen, cyano, hydroxy, -NR^{b}R^{c}, -C(=O)R^{d},C₁₋₆ alkyl, C₁₋₆ alkoxy, C₂₋₈ alkenyl, C₁₋₆ alkyl-R', C₁₋₆ alkoxy-R', -OR', -SR', -SOR^{d}, -SO₂R^{d}, 3-12 membered cycloalkyl, 3-12 membered cycloalkenyl and 3-12 membered heterocyclyl, the C₁₋₆ alkyl, C₁₋₆ alkoxy, C₂₋₈ alkenyl, C₁₋₆ alkyl-R', C₁₋₆ alkoxy-R', 3-12 membered cycloalkyl, 3-12 membered cycloalkenyl and 3-12 membered heterocyclyl are optionally substituted with one or more R^{m};
R^{b}, R^{c} and R^{d} are each absent or each independently selected from the group consisting of hydrogen, hydroxyl, mercapto, C₁₋₆ alkyl, halogen, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', -OR', -C(=O)-R', 3-12 membered cycloalkyl and 3-12 membered heterocyclyl, the C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', -OR', -C(=O)-R', 3-12 membered cycloalkyl and 3-12 membered heterocyclyl are optionally substituted with one or more R^{m};
or R^{b} and R^{c} together with the nitrogen atom to which they are both attached to form a 3-12 membered monocyclic, spirocyclic, bridged or fused ring optionally substituted with one or more R^{m};
R^{e} and R^{f} are each independently selected from the group consisting of hydrogen, C₁₋₆ alkyl, C₂₋₆ alkenyl, 3-12 membered cycloalkyl, 3-20 membered heterocyclyl, 6-20 membered aryl and 5-20 membered heteroaryl, wherein the C₁₋₆ alkyl, C₂₋₆ alkenyl, 3-12 membered cycloalkyl, 3-20 membered heterocyclyl, 6-20 membered aryl and 5-20 membered heteroaryl are optionally substituted with one or more C₁₋₆ alkyl or halogen;
R^{g} is C₁₋₆ alkyl or 6-20 membered aryl, wherein the C₁₋₆ alkyl and 6-20 membered aryl are optionally substituted with one or more groups independently selected from: halogen or OR^{e};
R' is selected from the group consisting of C₁₋₆ alkyl, 3-12 membered cycloalkyl, 3-12 membered heterocyclyl and 5-10 membered heteroaryl, the C₁₋₆ alkyl, 3-12 membered cycloalkyl, 3-12 membered heterocyclyl and 5-10 membered heteroaryl are optionally substituted with one or more R^{m}; the 3-12 membered cycloalkyl and 3-12 membered heterocyclyl comprise monocyclic, spirocyclic or bridged ring, and the 5-10 membered heteroaryl comprises monocyclic or fused ring;
R^{m} is selected from the group consisting of deuterium, halogen, cyano, nitro, hydroxyl, mercapto, amino, carboxyl, C₁₋₆ alkyl, C₁₋₆ alkoxy-C₁₋₆ alkyl, C₁₋₆ alkoxy, C₁₋₆ alkyl-C₁₋₆ alkoxy, C₁₋₆ alkoxy-C₁₋₆ alkoxy, C₁₋₆ alkylamino, (C₁₋₆ alkyl)₂amino, C₁₋₆ alkyl ester, C₁₋₆ alkylaminocarbonyl, (C₁₋₆ alkyl)₂aminocarbonyl, C₁₋₆ alkylcarbonyl, C₁₋₆ alkylcarbonyloxy, C₁₋₆ alkylcarbonylamino, C₁₋₆ alkylsulfonyl amino, halogenated C₁₋₆ alkyl, halogenated C₁₋₆ alkoxy, C₁₋₆ alkyl sulfonyl, C₁₋₆ alkyl thio, C₂₋₈ alkenyl, C₂₋₈ alkynyl, 3-8 membered cycloalkyl, 3-8 membered heterocyclyl, 6-10 membered aryl, 3-8 membered heteroaryl, -(CR^{a}R^{d})ₜNR^{b}R^{c} and oxo.
t is 0, 1, 2, 3, 4, 5 or 6.

In still other embodiments of the present invention,
A is selected from the following groups substituted with R¹, the following groups are optionally substituted with one or more R²; 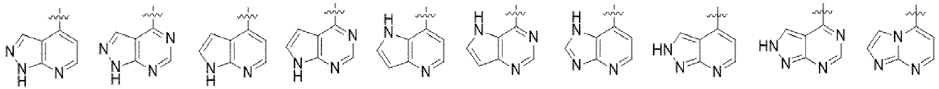 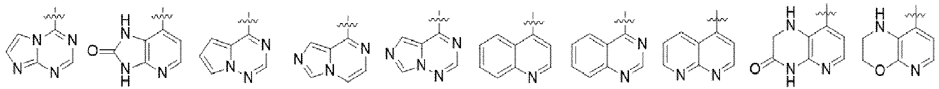 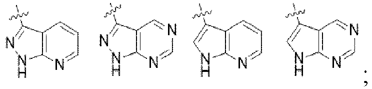
L is selected from O, C=O and NH;
Z is selected from the group consisting of benzene, pyridine and naphthalene optionally substituted with one or more R³:
   X is selected from NR⁴;
   Y is selected from C(=O);
   E₁, E₂, E₃ are each independently selected from CR^{a}R^{b} and N;
   G is selected from the group consisting of 6-20 membered aryl, pyridine and pyrazole, wherein the 6-20 membered aryl, pyridine and pyrazole are optionally substituted with one or more groups independently selected from the group consisting of: halogen, nitro, oxo, CN, methoxy, ethoxy, amino, methylamino, ethylamino, dimethylamino, methyl, ethyl, isopropyl, vinyl, CF₃, methoxymethyl, trifluoromethoxymethyl, cyclopropyl, cyclopropylmethyl;
   R¹ is selected from the following groups: 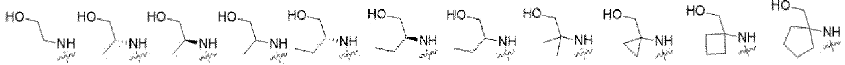 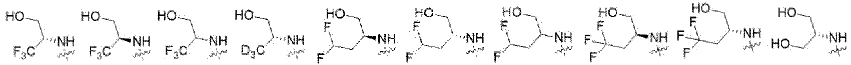 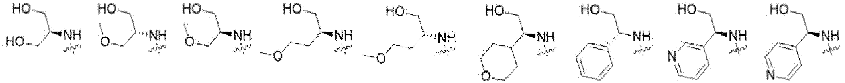 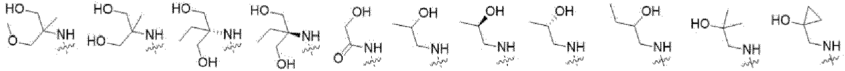 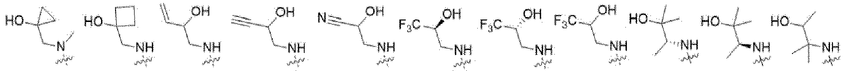 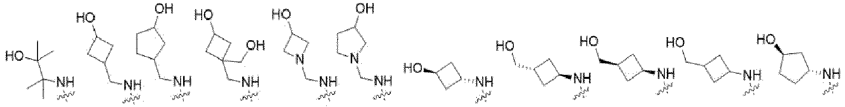 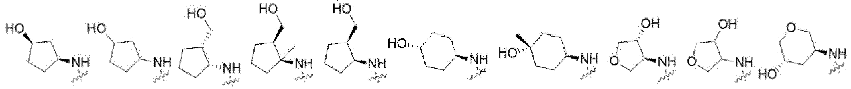 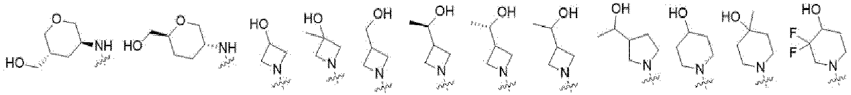 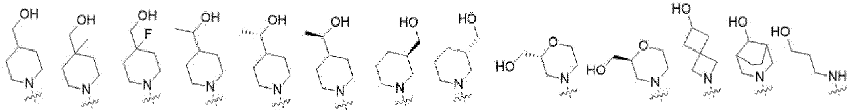 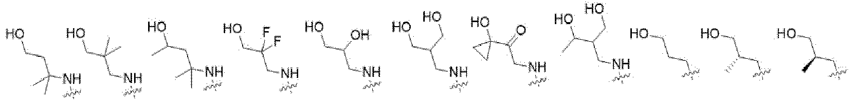 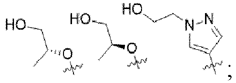
   R² and R³ are each independently selected from the group consisting of hydrogen, hydroxyl, mercapto, halogen, cyano, nitro, C₁₋₆ alkyl, C₂₋₆ alkenyl, C₂₋₆ alkynyl, hydroxyl-C₁₋₆ alkyl, amino-C₁₋₆ alkyl, halogenated-C₁₋₆ alkyl, C₁₋₆ alkoxy, halogenated-C₁₋₆ alkoxy, C₁₋₆ alkoxy-C₁₋₆ alkyl, C₁₋₆ alkyl-C₁₋₆ alkoxy, C₁₋₆ alkoxy-C₁₋₆ alkoxy, 3-12 membered cycloalkyl, 3-12 membered heterocyclyl, 3-12 membered cycloalkyl-C₁₋₆ alkyl and 3-12 membered heterocyclyl-C₁₋₆ alkyl, the C₁₋₆ alkyl, C₂₋₆ alkenyl, C₂₋₆ alkynyl, hydroxyl-C₁₋₆ alkyl, mino-C₁₋₆ alkyl, halogenated-C₁₋₆ alkyl, C₁₋₆ alkoxy, halogenated-C₁₋₆ alkoxy, C₁₋₆ alkoxy-C₁₋₆ alkyl, C₁₋₆ alkyl-C₁₋₆ alkoxy, C₁₋₆ alkoxy-C₁₋₆ alkoxy, 3-12 membered cycloalkyl, 3-12 membered heterocyclyl, 3-12 membered cycloalkyl-C₁₋₆ alkyl, and 3-12 membered heterocyclyl-C₁₋₆ alkyl are optionally substituted with one or more R^{m}; R⁴ is independently selected from the group consisting of hydrogen, methyl, ethyl, trifluoromethyl and cyclopropyl;
   R^{a} is not present or is independently selected from the group consisting of hydrogen, halogen, cyano, hydroxyl, amino, mercapto, C₁₋₆ alkyl, halogenated C₁₋₆ alkyl, C₁₋₆ alkylamino, (C₁₋₆ alkyl)₂ amino, C₁₋₆ alkoxy, halogenated C₁₋₆ alkoxy, C₁₋₆ alkylthio, C₁₋₆ alkylsulfonyl, C₁₋₆ alkylsulfidenyl, halogenated C₁₋₆ alkylsulfonyl, C₁₋₆ alkoxy-C₁₋₆ alkyl, C₂₋₈ alkenyl, 3-12 membered cycloalkyl and 3-12 membered heterocyclyl, the C₁₋₆ alkyl, halogenated C₁₋₆ alkyl, C₁₋₆ alkylamino, (C₁₋₆ alkyl)₂amino, C₁₋₆ alkoxy, halogenated C₁₋₆ alkoxy, C₁₋₆ alkylthio, C₁₋₆ alkylsulfonyl, C₁₋₆ alkylsulfidenyl, halogenated C₁₋₆ alkylsulfonyl, C₁₋₆ alkoxy-C₁₋₆ alkyl, C₂₋₈ alkenyl, 3-12 membered cycloalkyl and 3-12 membered heterocyclyl are optionally substituted with one or more R^{m};
   R^{b} is not present or is selected from the group consisting of hydrogen, C₁₋₆ alkyl, halogen, C₁₋₆ alkoxy, C₁₋₆ alkyl-3-12 membered cycloalkyl, C₁₋₆ alkyl-3-12 membered heterocyclyl, 3-12 membered cycloalkyl-C₁₋₆ alkyl, 3-12 membered heterocyclyl-C₁₋₆ alkyl, 3-12 membered cycloalkyl and 3-12 membered heterocyclyl, the C₁₋₆ alkyl, C₁₋₆ alkoxy, C₁₋₆ alkyl-3-12 membered cycloalkyl, C₁₋₆ alkyl-3-12 membered heterocyclyl, 3-12 membered cycloalkyl-C₁₋₆ alkyl, 3-12 membered heterocyclyl-C₁₋₆ alkyl, 3-12 membered cycloalkyl and 3-12 membered heterocyclyl are optionally substituted with one or more R^{m};
   or R^{a} and R^{b} together with the nitrogen atom to which they are both attached to form a 3-12 membered monocyclic, spirocyclic, bridged or fused ring optionally substituted with one or more R^{m};
   R^{m} is each independently selected from the group consisting of deuterium, halogen, cyano, nitro, hydroxyl, mercapto, amino, carboxyl, C₁₋₄ alkyl, C₁₋₄ alkoxy-C₁₋₄ alkyl, C₁₋₄ alkoxy, C₁₋₆ alkyl-C₁₋₄ alkoxy, C₁₋₄ alkoxy-C₁₋₄ alkoxy, C₁₋₄ alkylamino, (C₁₋₄ alkyl)₂amino, C₁₋₄ alkyl ester, C₁₋₄ alkylaminocarbonyl, (C₁₋₄ alkyl)₂aminocarbonyl, C₁₋₄ alkylcarbonyl, C₁₋₄ alkylcarbonyloxy, C₁₋₄ alkylcarbonylamino, C₁₋₄ alkylsulfonylamino, halogenated C₁₋₄ alkyl, halogenated C₁₋₄ alkoxy, C₁₋₄ alkylsulfonyl, C₁₋₄ alkylthio, vinyl, ethynyl, cyclopropyl, cyclobutyl, cyclopentyl, cyclohexyl, cycloheptyl, phenyl, epoxyethyl, epoxycyclobutyl, tetrahydrofuranyl, tetrahydropyranyl, oxacyclooctyl, aziridinyl, azetidinyl, tetrahydropyrrolyl, piperidinyl, piperazinyl, epoxyethylmethyl, epoxycyclobutylmethyl, tetrahydrofuranylmethyl, tetrahydropyranylmethyl, oxacyclooctylmethyl, aziridinylmethyl, azetidinylmethyl, tetrahydropyrrolylmethyl, piperidinylmethyl, piperazinylmethyl, pyrrolyl, furanyl, thienyl, oxazolyl, isoxazolyl, pyrazolyl, thiazolyl, pyridyl, pyrimidinyl, pyridazinyl and oxo.

In a particular embodiment, the compound of formula I is selected from the following compounds:

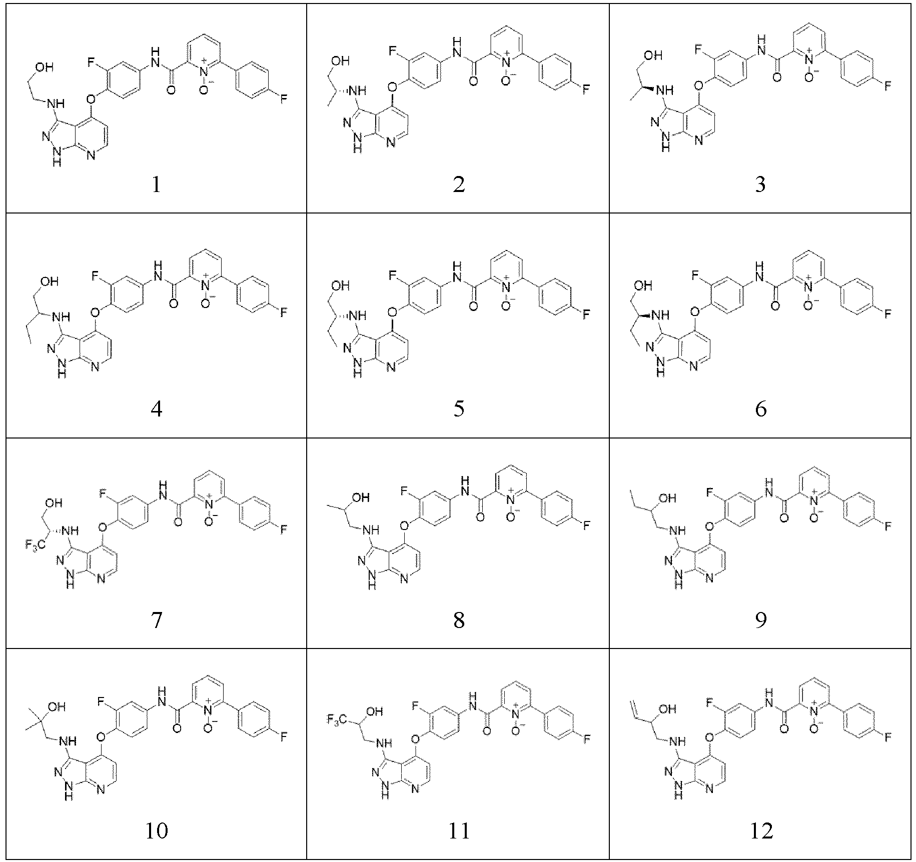

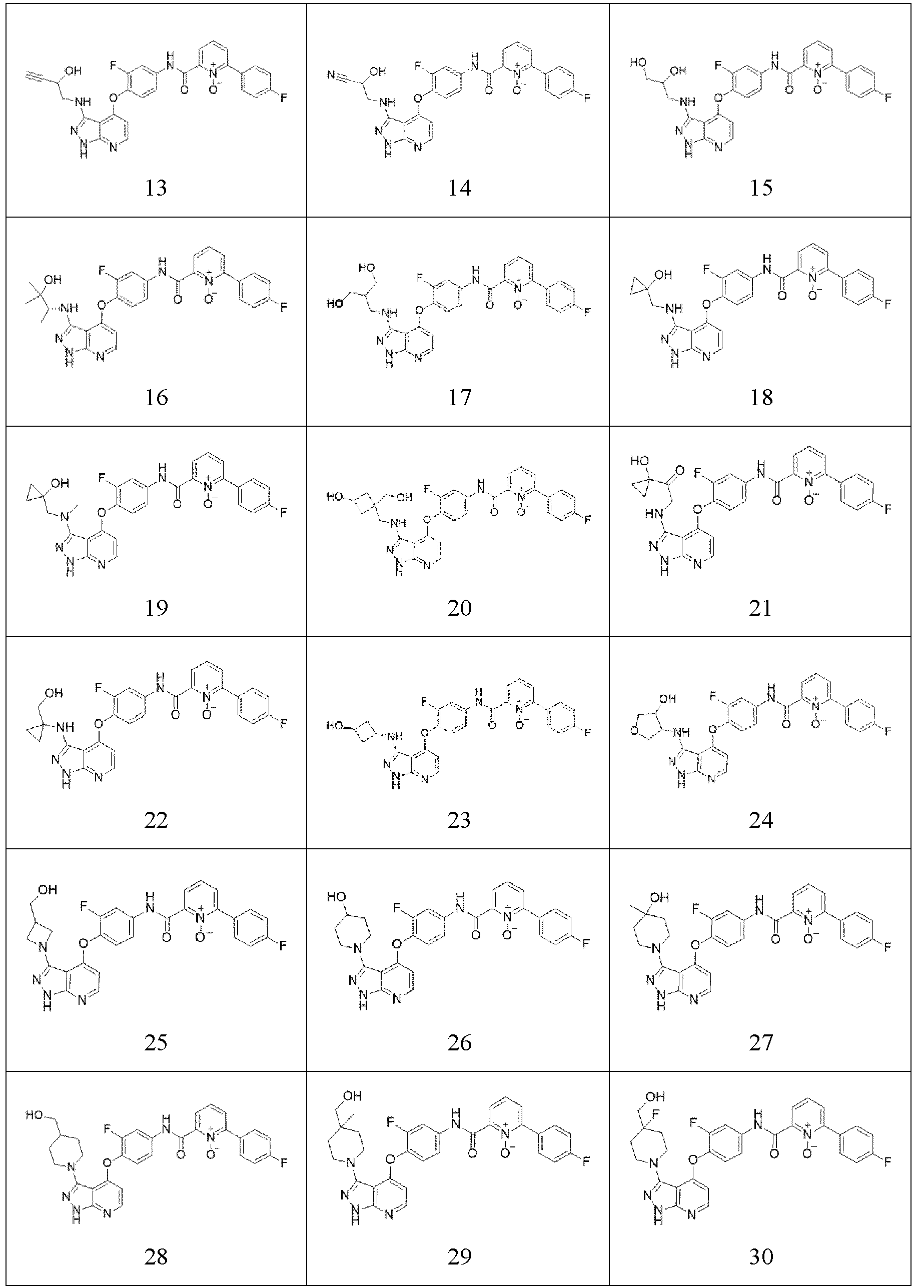

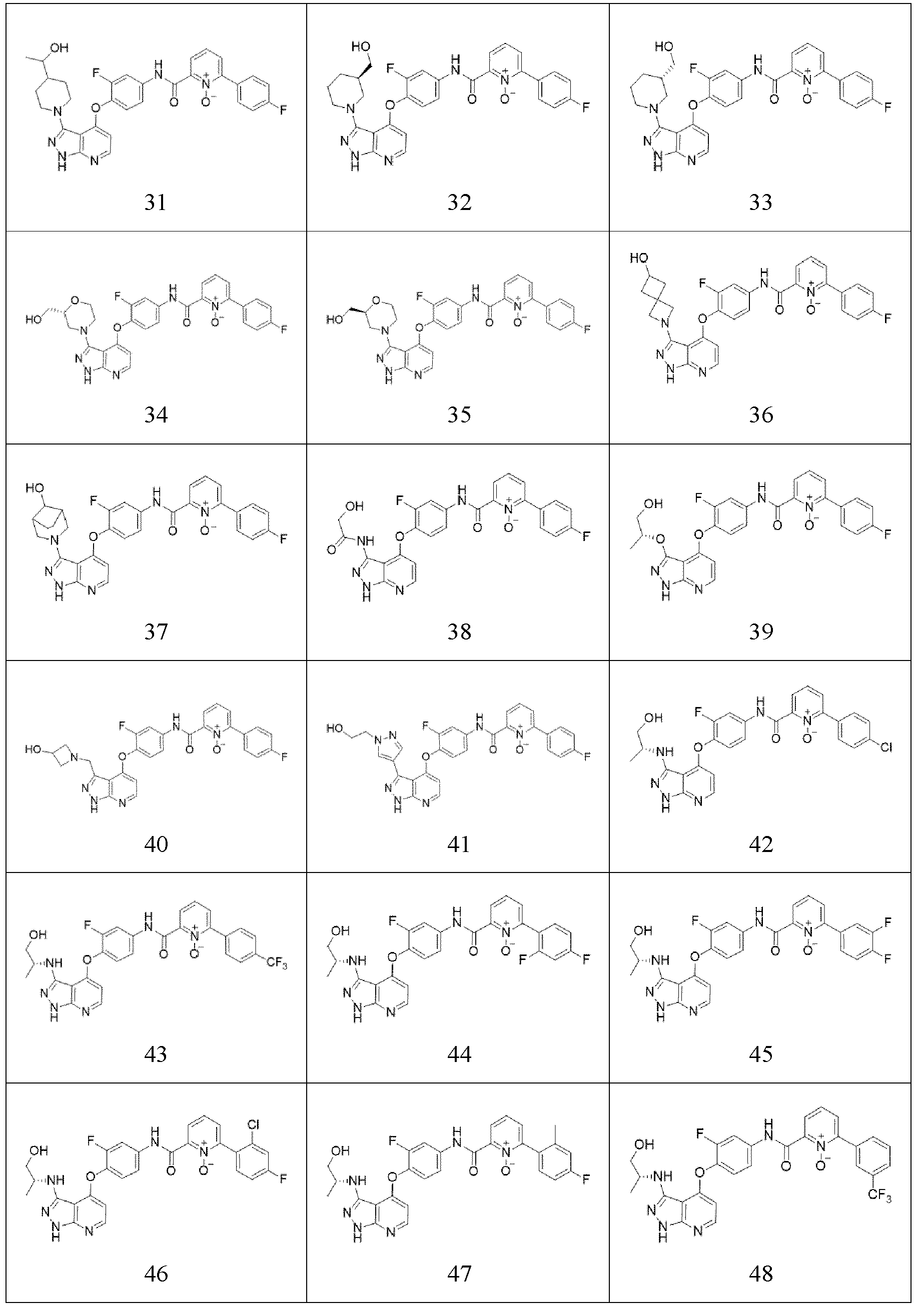

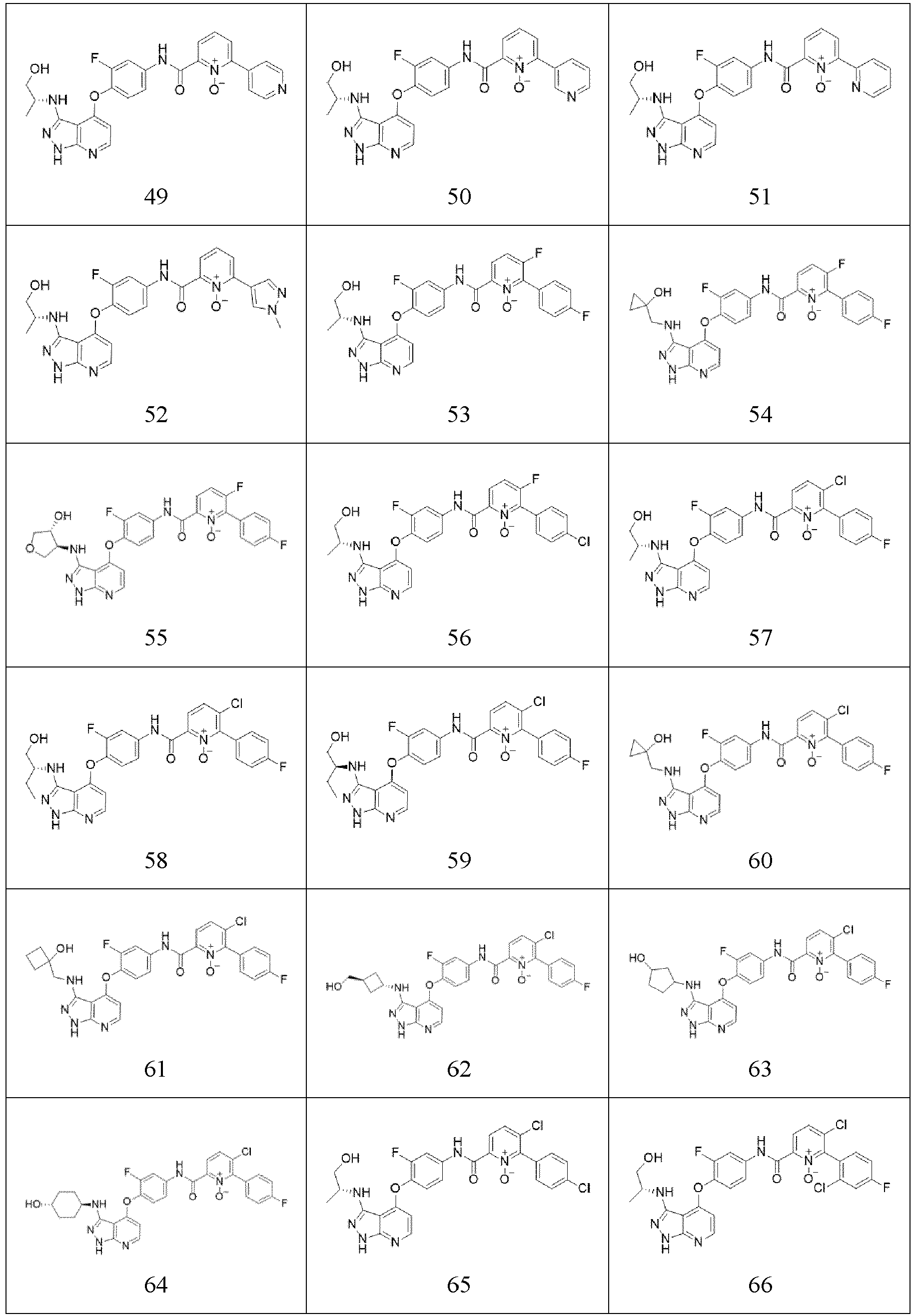

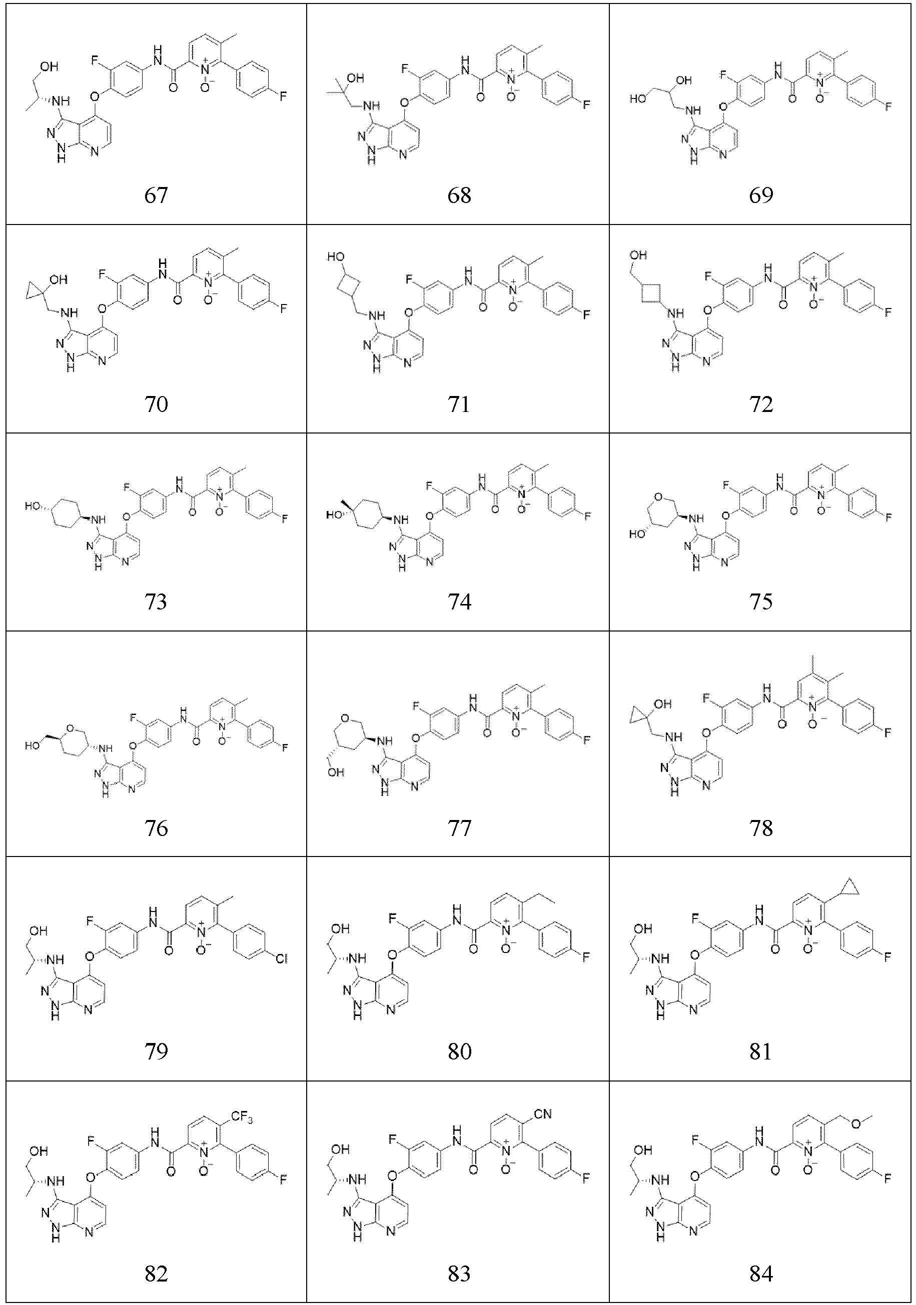

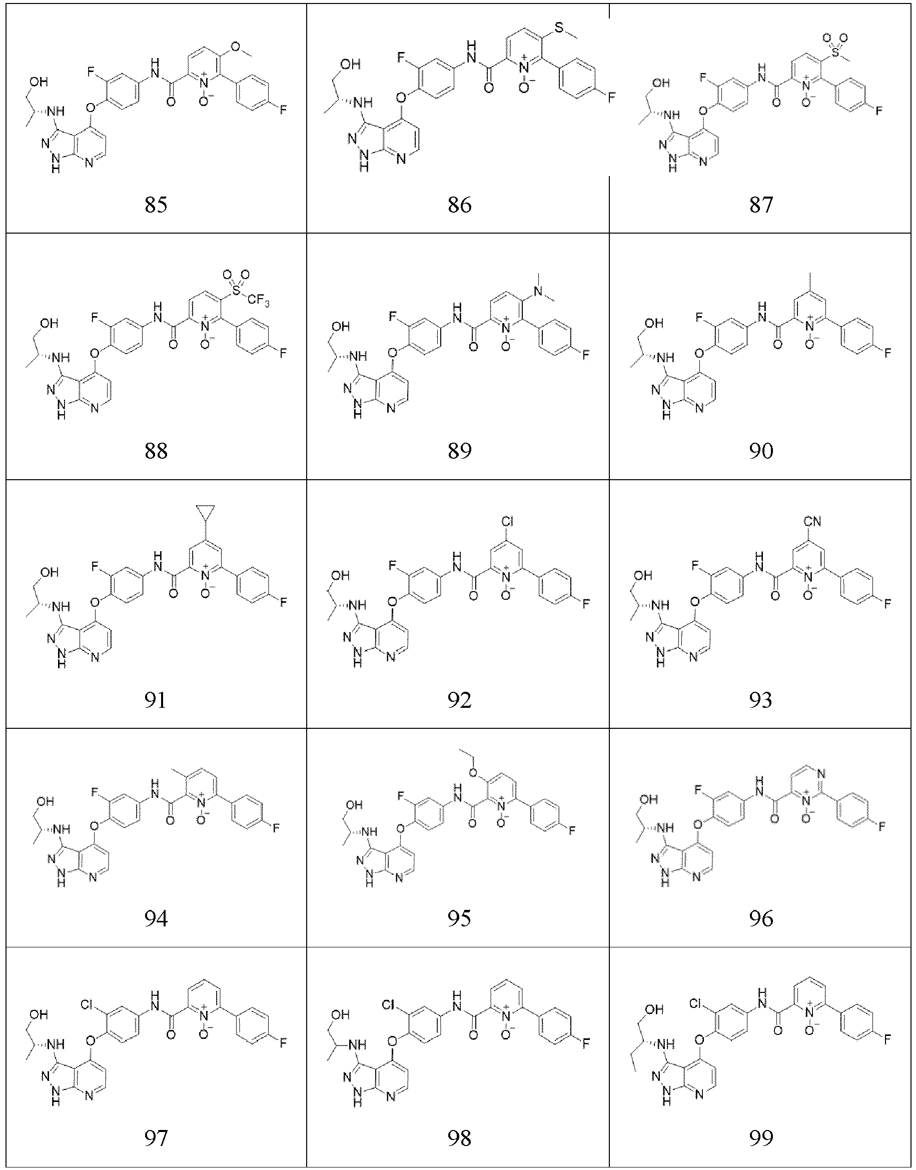

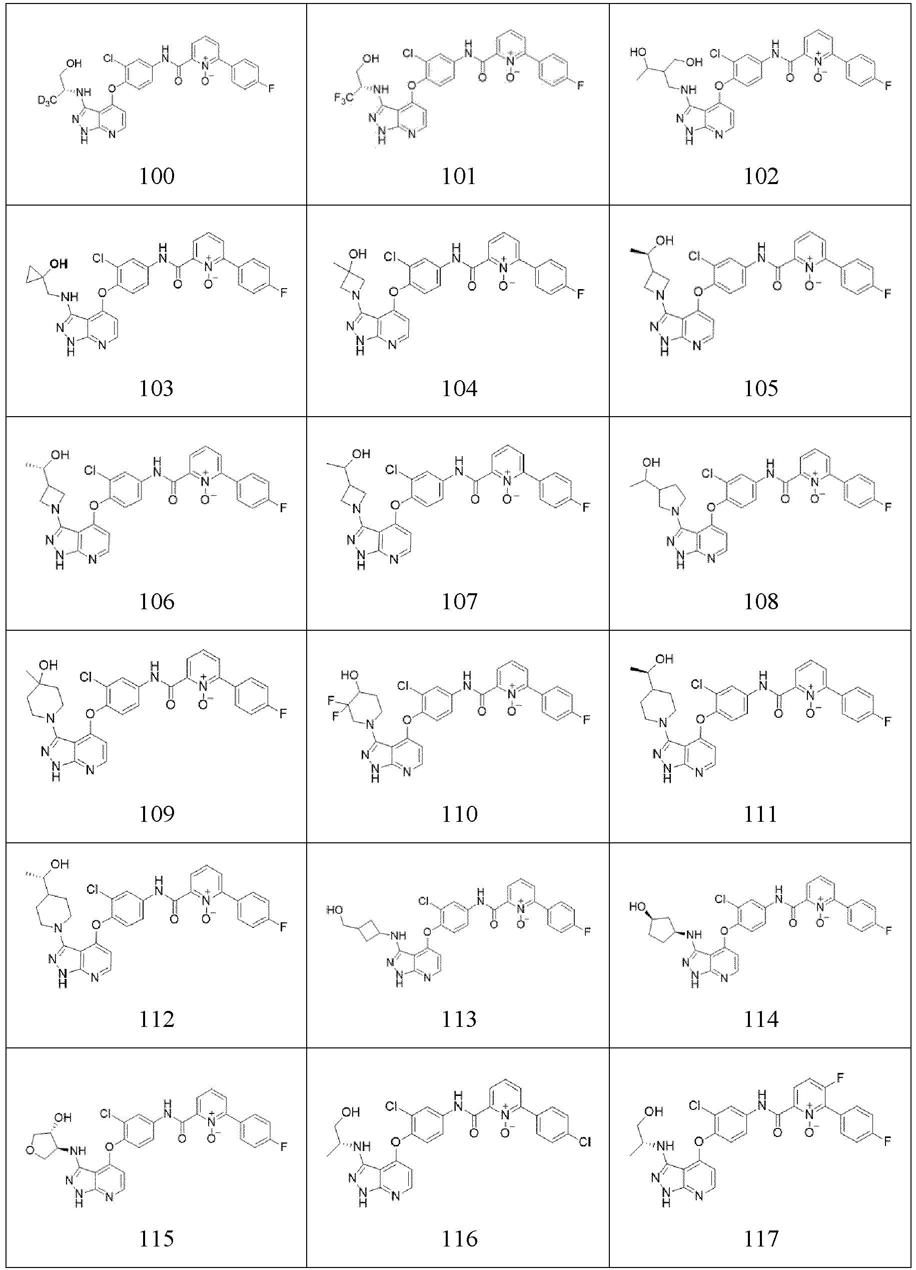

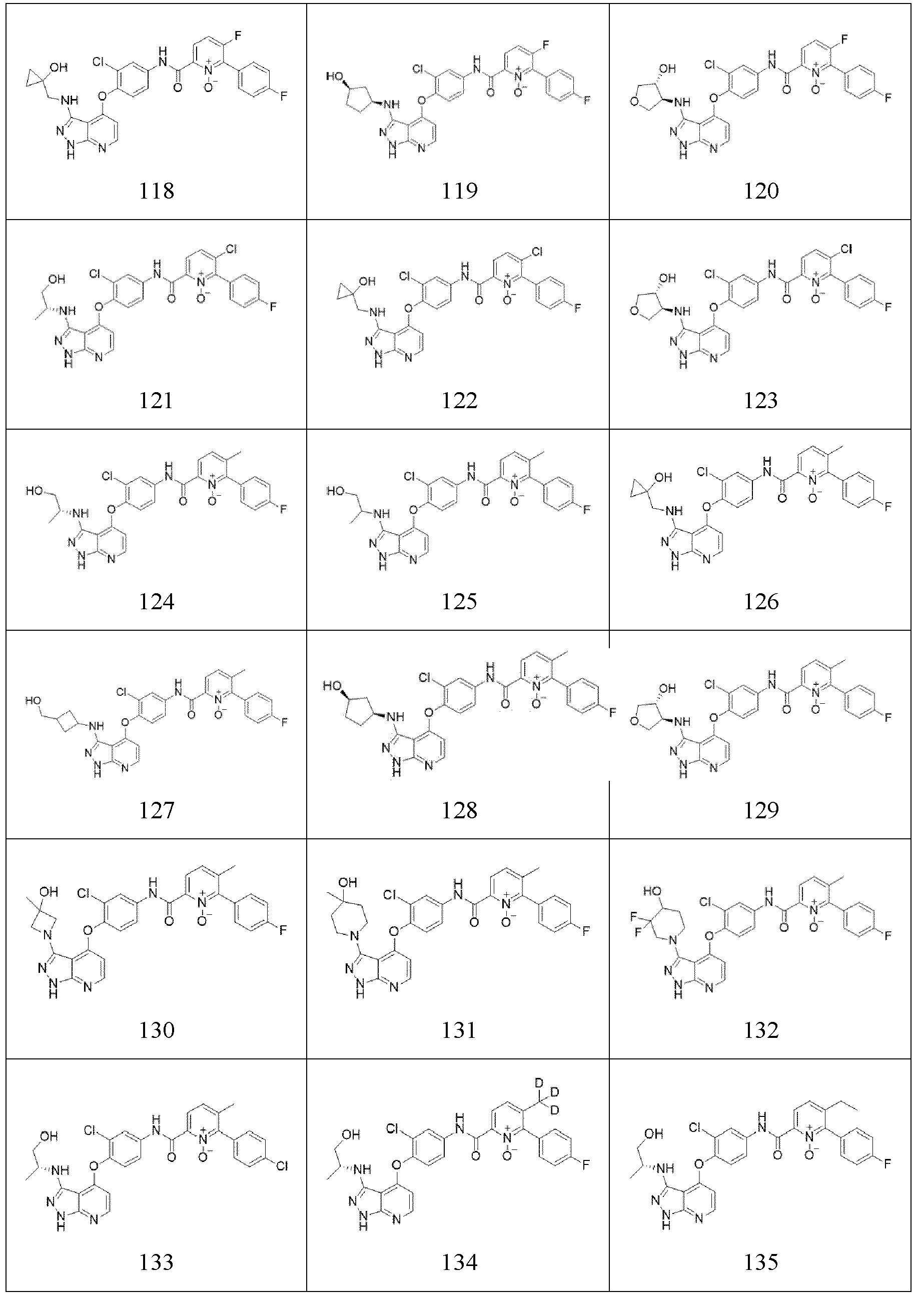

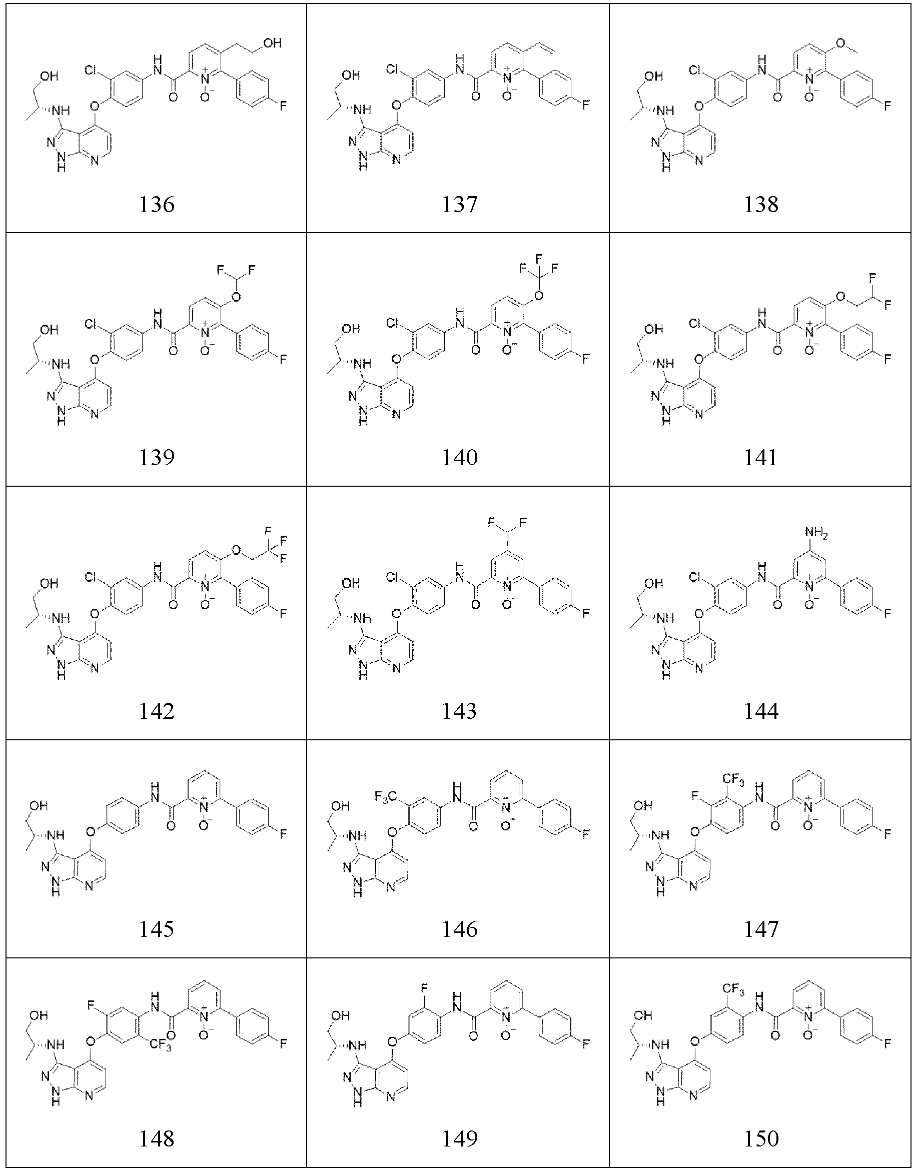

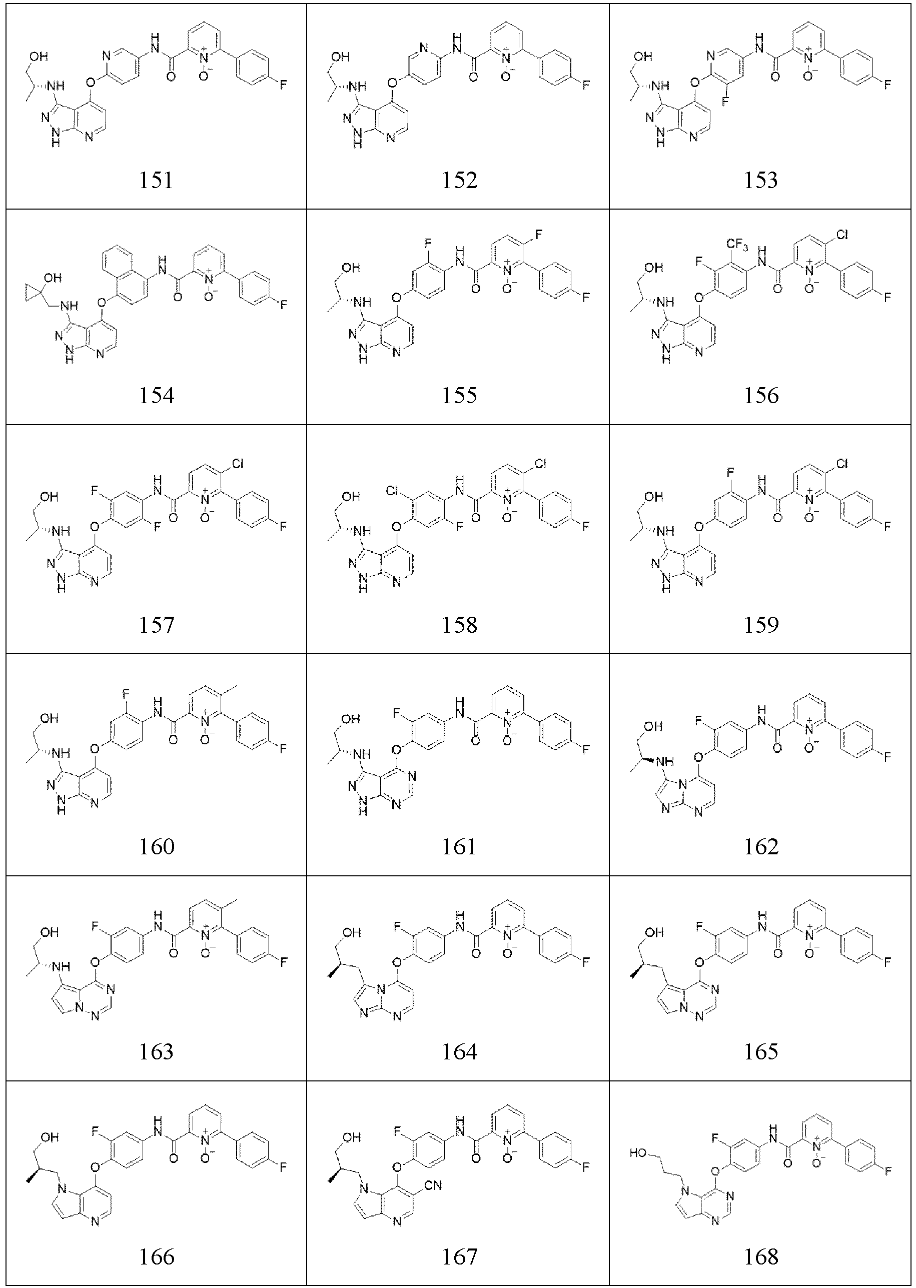

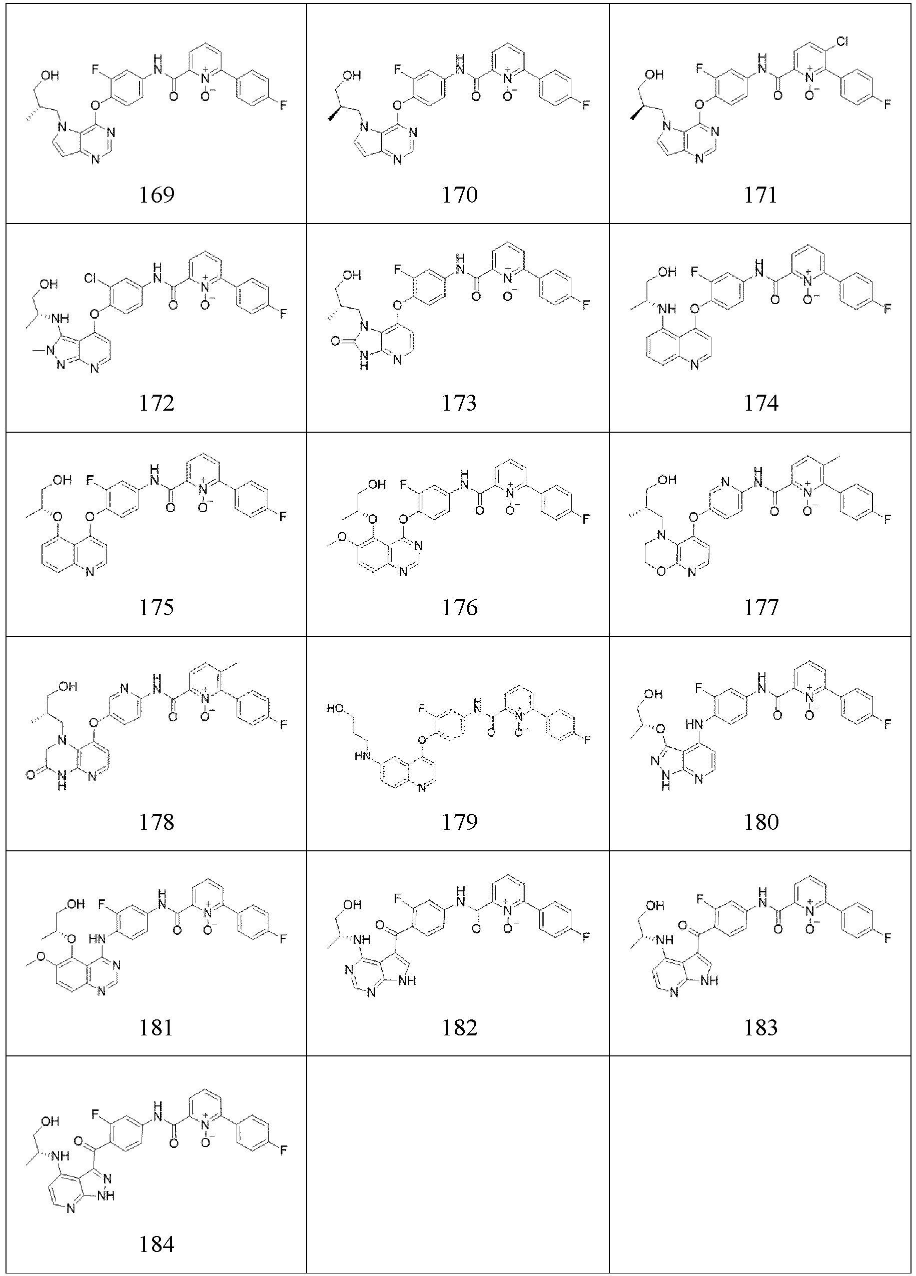

In certain embodiments, the present application provides a process for preparing a compound of formula I, comprising the following steps: reacting compound 1 with compound M under basic conditions to form compound 2, and then oxidizing compound 2 under acidic conditions to form compound 3, and reacting compound 3 with compound 4 to form a compound of formula I 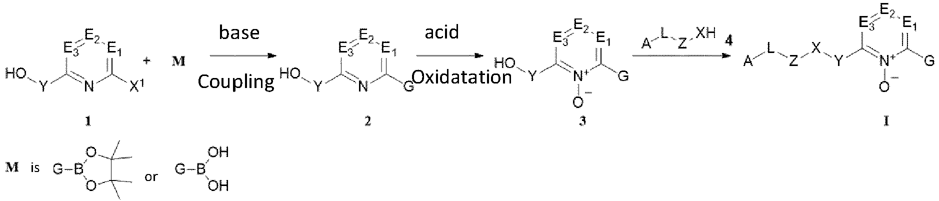
Wherein X¹ is halogen, A, L, Z, X, Y, E₁, E₂, E₃ and G are as defined herein above,
Preferably, the base is selected from cesium fluoride, cesium carbonate;
Preferably, the acid is selected from trifluoroacetic acid.

In certain embodiments, the present application provides a pharmaceutical composition comprising the compound as described above and a pharmaceutically acceptable carrier or excipient; preferably, the pharmaceutical composition is in the form of tablets, capsules, pills, granules, powders, suppositories, injections, solutions, suspensions, ointments, patches, lotions, drops, liniments and sprays

In certain embodiments, the application provides a use of the compound or the pharmaceutical composition as described above in the manufacture of a medicament for the treatment of a disease associated with a protein kinase.

In certain embodiments, the disease associated with a protein kinase is selected from a disease associated with c-Met, VEGFR-2, AXL, TAM, NTRK, or RET.

In certain embodiments, the disease associated with a protein kinase is a tumor.

In certain embodiments, the disease associated with a protein kinase includes head and neck cancer, nasopharyngeal carcinoma, melanoma, bladder cancer, esophageal cancer, anaplastic large cell lymphoma, renal cancer, breast cancer, colorectal cancer, ovarian cancer, cervical cancer, pancreatic cancer, glioma, glioblastoma, prostate cancer, leukemia, lymphoma, non-Hodgkin's lymphoma, gastric cancer, lung cancer, liver cancer, gastrointestinal stromal tumor, thyroid cancer, squamous cell carcinoma, cholangiocarcinoma, endometrial cancer, multiple myeloma, or mesothelioma; atherosclerosis and pulmonary fibrosis.

In certain embodiments, the present application provides a use of the above-described compounds and/or pharmaceutical compositions in the preparation of an anti-tumor medicament.

In certain embodiments, the tumor comprises head and neck cancer, nasopharyngeal cancer, melanoma, bladder cancer, esophageal cancer, anaplastic large cell lymphoma, renal cancer, breast cancer, colorectal cancer, ovarian cancer, cervical cancer, pancreatic cancer, glioma, glioblastoma, prostate cancer, leukemia, lymphoma, non-Hodgkin's lymphoma, gastric cancer, lung cancer, liver cancer, gastrointestinal stromal tumor, thyroid cancer, squamous cell carcinoma, cholangiocarcinoma, endometrial cancer, multiple myeloma, and mesothelioma.

In certain embodiments, the present application provides a method for treating a disease associated with a protein kinase in a subject comprising administering to the subject an effective amount of the compound described above, or a pharmaceutically acceptable salt, stereoisomer, tautomer, deuteron, prodrug molecule, hydrate, or solvate thereof, or the pharmaceutical composition described above. Preferably the subject is a mammal, preferably a human,.

In certain embodiments, the mode of administration comprises oral, mucosal, sublingual, ocular, topical, parenteral, rectal, cerebral cistern, vaginal, peritoneal, bladder, nasal administration.

In certain embodiments, the disease associated with a protein kinase is selected from a disease associated with c-Met, VEGFR-2, AXL, TAM, NTRK, or RET.

In certain embodiments, the disease associated with a protein kinase is a tumor.

In certain embodiments, the disease associated with a protein kinase includes head and neck cancer, nasopharyngeal carcinoma, melanoma, bladder cancer, esophageal cancer, anaplastic large cell lymphoma, renal cancer, breast cancer, colorectal cancer, ovarian cancer, cervical cancer, pancreatic cancer, glioma, glioblastoma, prostate cancer, leukemia, lymphoma, non-Hodgkin's lymphoma, gastric cancer, lung cancer, liver cancer, gastrointestinal stromal tumor, thyroid cancer, squamous cell carcinoma, cholangiocarcinoma, endometrial cancer, multiple myeloma, or mesothelioma; atherosclerosis and pulmonary fibrosis.

In certain embodiments, the present application provides a method for treating a tumor in a subject comprising administering to the subject an effective amount of a compound described above, or a pharmaceutically acceptable salt, stereoisomer, tautomer, deuteride, prodrug molecule, hydrate, or solvate thereof, or a pharmaceutical composition described above. The subject isa mammal, preferably a human,.

In certain embodiments, the mode of administration comprises oral, mucosal, sublingual, ocular, topical, parenteral, rectal, cerebral cistern, vaginal, peritoneal, bladder, nasal administration.

In certain embodiments, the tumor comprises: head and neck cancer, nasopharyngeal carcinoma, melanoma, bladder cancer, esophageal cancer, anaplastic large cell lymphoma, kidney cancer, breast cancer, colorectal cancer, ovarian cancer, cervical cancer, pancreatic cancer, glioma, glioblastoma, prostate cancer, leukemia, lymphoma, non-Hodgkin's lymphoma, gastric cancer, lung cancer, liver cancer, gastrointestinal stromal tumors, thyroid cancer, squamous cell carcinoma, cholangiocarcinoma, endometrial cancer, multiple myeloma, and mesothelioma.

Other features and advantages of the present application will be apparent from the detailed description that follows. The following examples and detailed description are intended to clearly illustrate the technical solutions of the present application and the technical effects and advantages thereof, and are not intended to limit the scope of the present application.

### Structure of heteroaromatic nitrogen-oxide compound as c-Met kinase inhibitors

The present application relates to a compound shown as formula I or a pharmaceutically acceptable salt, a stereoisomer, a tautomer, a deuteron, a prodrug molecule, a hydrate or a solvate thereof 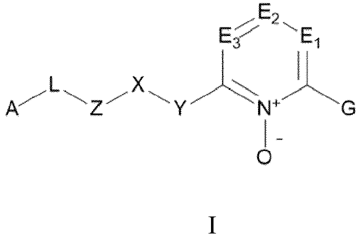 wherein, in the formula I,
A is selected from the group consisting of 5-14 membered heteroaryl substituted with R¹, 3-14 membered heterocyclyl substituted with R¹, 6-14 membered aryl substituted with R¹, and 3-12 membered cycloalkyl substituted with R¹, the 5-14 membered heteroaryl substituted with R¹, 3-14 membered heterocyclyl substituted with R¹, 6-14 membered aryl substituted with R¹, and 3-12 membered cycloalkyl substituted with R¹ are optionally substituted with one or more R², the 5-14 membered heteroaryl substituted with R¹ and 6-14 membered aryl substituted with R¹ comprise monocyclic or fused ring, the 3-14 membered heterocyclyl substituted with R¹, and 3-12 membered cycloalkyl substituted with R¹ comprise monocyclic, spirocyclic or bridged ring;
L is selected from O, S, CR^{a}R^{b}, NR^{b}, C(=O), SO₂ and SO;
Z is selected from the group consisiting of 6-14 membered aryl, 5-14 membered heteroaryl, 3-12 membered cycloalkyl and 3-14 membered heterocyclyl, the 6-14 membered aryl, 5-14 membered heteroaryl, 3-12 membered cycloalkyl and 3-14 membered heterocyclyl are optionally substituted with one or more R³;
X is absent or selected from the group consisiting of NR⁴, O, S, CR⁴R⁵ and C(=O);
Y is absent or selected from the group consisiting of (CR^{a}R^{b})ₙ₁-C(=O), C(=S), C(=NR⁴), SO₂, SO, NR⁴, O, S and CR⁴R⁵;
E₁, E₂ and E₃ are each absent or each independently selected from the group consisiting of CR^{a}R^{b}, N, C(=O), C(=S) and C(=NR⁴);
G is selected from the group consisting of (CR^{a}R^{b})ₙ₁-6-20-membered aryl, (CR^{a}R^{b})ₙ₁-5-20-membered heteroaryl, (CR^{a}R^{b})ₙ₁-3-12-membered cycloalkyl, (CR^{a}R^{b})ₙ₁-3-20-membered heterocyclyl, (CR^{a}R^{b})ₙ₁-O-(CR^{a}R^{b})ₘ₁-aryl, (CR^{a}R^{b})ₙ₁-OR⁶, (CR^{a}R^{b})ₙ₁-NR⁶R⁷, (CR^{a}R^{b})ₙ₁-NR⁶C(=O)R⁷, C₁₋₆ alkyl, C₂₋₆ alkenyl and C₂₋₆ alkynyl, wherein the (CR^{a}R^{b})ₙ₁-6-20-membered aryl, (CR^{a}R^{b})ₙ₁-5-20-membered heteroaryl, (CR^{a}R^{b})ₙ₁-3-12-membered cycloalkyl, (CR^{a}R^{b})ₙ₁-3-20-membered heterocyclyl, (CR^{a}R^{b})ₙ₁-O-(CR^{a}R^{b})ₘ₁-aryl, C₁₋₆ alkyl, C₂₋₆ alkenyl and C₂₋₆ alkynyl are optionally substituted with one or more groups independently selected from the group consisting of halogen, nitro, oxo(=O), SO₂R^{f}, CN, OR^{e}, SR^{e}, NReRf, -NR^{e}C(=O)R^{f}, (CR^{a}R^{b})ₙ₁-C(=O)R^{e}, -C(=O)NR^{e}R^{f}, -C(=O)OR^{e}, C₁₋₁₂ alkyl, halogenated C₁₋₁₂ alkyl, C₁₋₆ alkoxy-C₁₋₁₂ alkyl, halogenated C₁₋₆ alkoxy-C₁₋₁₂ alkyl, R^{a}R^{b}N-C₁₋₆ alkyl, C₂₋₈ alkenyl, halogenated C₂₋₈ alkenyl, C₂₋₈ alkynyl, 3-12 membered cycloalkyl, 3-20 membered heterocyclyl, 6-20 membered aryl, or 5-20 membered heteroaryl;
R¹ is selected from R⁸ substituted with one or more hydroxy;
R², R³ and R⁸ are each independently selected from the group consisting of hydrogen, halogen, cyano, nitro, -SO-R^{a}, -NR^{b}R^{c}, -OR^{b}, -SR^{b}, R^{a}SO-C₁₋₆ alkyl, R^{b}R^{c}N-C₁₋₆ alkyl, R^{b}R^{c}NC(=Y¹)-C₁₋₆ alkyl, R^{a}SO-C₁₋₆ alkoxy, R^{b}R^{c}N-C₁₋₆ alkoxy, R^{b}R^{c}NC(=Y¹)-C₁₋₆ alkoxy, -C(=Y¹)R^{a}, -C(=Y¹)OR^{b}, -C(=Y¹)NR^{b}R^{c}, - C(=Y¹)NR^{c}(CR^{a}R^{b})ₙ₁NR^{b}R^{c}, -OC(=Y¹)R^{a}, -OC(=Y¹)NR^{b}R^{c}, -OC(=Y¹)OR^{b}, - NR^{b}C(=Y¹)NR^{b}R^{c}, -NR^{b}C(=Y¹)OR^{c}, -NR^{b}C(=Y¹)R^{a}, -OR^{b}C(=Y¹)OR^{c}, -SO₂-N^{Rb}R^{c}, - SO₂R^{a}, -NR^{b}SO₂R^{a}, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered cycloalkenyl, 3-12 membered heterocyclyl, 6-14 membered aryl, 5-10 membered heteroaryl, C₁₋₆ alkoxy-C₁₋₆ alkyl, 3-12 membered cycloalkyl-C₁₋₆ alkyl, 3-12 membered cycloalkenyl-C₁₋₆ alkyl, 3-12 membered heterocyclyl-C₁₋₆ alkyl, 6-14 membered aryl-C₁₋₆ alkyl and 5-10 membered heteroaryl-C₁₋₆ alkyl, the R^{a}SO-C₁₋₆ alkyl, R^{b}R^{c}N-C₁₋₆ alkyl, R^{b}R^{c}NC(=Y¹)-C₁₋₆ alkyl, R^{a}SO-C₁₋₆ alkoxy, R^{b}R^{c}N-C₁₋₆ alkoxy, R^{b}R^{c}NC(=Y¹)-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered cycloalkenyl, 3-12 membered heterocyclyl, 6-14 membered aryl, 5-10 membered heteroaryl, C₁₋₆ alkoxy-C₁₋₆ alkyl, 3-12 membered cycloalkyl-C₁₋₆ alkyl, 3-12 membered cycloalkenyl-C₁₋₆ alkyl, 3-12 membered heterocyclyl-C₁₋₆ alkyl, 6-14 membered aryl-C₁₋₆ alkyl, and 5-10 membered heteroaryl-C₁₋₆ alkyl are optionally substituted with one or more R^{m}, the 3-12 membered cycloalkyl, 3-12 membered cycloalkenyl, 3-12 membered heterocyclyl, 3-12 membered cycloalkyl-C₁₋₆ alkyl, 3-12 membered cycloalkenyl-C₁₋₆ alkyl and 3-12 membered heterocyclyl-C₁₋₆ alkyl comprise monocyclic, spirocyclic or bridged ring, and the 6-14 membered aryl, 5-10 membered heteroaryl, 6-14 membered aryl-C₁₋₆ alkyl and 5-10 membered heteroaryl-C₁₋₆ alkyl comprise monocyclic or fused ring;
or R¹ and R² together with the atoms to which they are attached to form a 5-14 membered cyclic group, which is substituted with one or more R¹;
or R² and R⁸ together with the atoms to which they are attached to form a 5-14 membered cyclic group, which is substituted with one or more R¹;
R⁴, R⁵, R⁶ and R⁷ are each independently selected from the group consisting of hydrogen, halogen, OR^{e}, C₁₋₆ alkyl, C₂₋₆ alkenyl, C₂₋₆ alkynyl, 3-12 membered cycloalkyl, 3-20 membered heterocyclyl, 6-20 membered aryl and 5-20 membered heteroaryl, wherein the 3-12 membered cycloalkyl and 3-20 membered heterocyclyl comprise monocyclic, spirocyclic or bridged ring, and the 6-20 membered aryl and 5-20 membered heteroaryl comprise monocyclic or fused ring; the C₁₋₆ alkyl, C₂₋₆ alkenyl, C₂₋₆ alkynyl, 3-12 membered cycloalkyl, 3-20 membered heterocyclyl, 6-20 membered aryl and 5-20 membered heteroaryl are optionally substituted with one or more groups independently selected from the group consisting of halogen, SO₂R^{g},CN,OR^{e},NR^{e}R^{f},C(=O)NR^{e}R^{f},CR^{e}C(=O)R^{f},C₁₋₁₂ alkyl, C₂₋₈ alkenyl, C₂₋₈ alkynyl, 3-12 membered cycloalkyl, 3-20 membered heterocyclyl, 6-20 membered aryl or 5-20 membered heteroaryl;
or R⁴ and R⁵ together with the carbon atom to which they are both attached to form a 3-12 membered monocyclic, spirocyclic or bridged ring optionally substituted with one or more R^{m};
or R⁶ and R⁷ together with the nitrogen atom to which they are both attached to form a saturated, partially unsaturated or fully unsaturated 3-20-membered heterocycle optionally substituted with one or more R^{m}, containing one or more heteroatoms selected from N, O or S;
R^{a} is absent or selected from the group consisting of hydrogen, halogen, cyano, hydroxyl, mercapto, carboxyl, nitro, -NR^{b}R^{c}, -C(=O)R^{d}, -C(=O)OR^{b}, -C(=O)NR^{b}R^{c}, - OC(=O)NR^{b}R^{c}, -NR^{b}C(=O)R^{d}, -NR^{b}C(=O)OR^{d}, -SO-NR^{b}R^{c}, -SO₂₋NR^{b}R^{c}, -SOR^{d}, - SO₂R^{d}, -NR^{b}SO₂R^{d}, C₁₋₆ alkyl, C₁₋₆ alkoxy, C₂₋₈ alkenyl, C₂₋₈ alkynyl, C₁₋₆ alkyl-R', C₁₋₆ alkoxy-R', -OR', -SR', -C(=O)-R', -NR^{b}C(=O)-R', 3-12 membered cycloalkyl, 3-12 membered cycloalkenyl, 3-12 membered heterocyclyl, 6-14 membered aryl and 5-10 membered heteroaryl, the C₁₋₆ alkyl, C₁₋₆ alkoxy, C₂₋₈ alkenyl, C₂₋₈ alkynyl, C₁₋₆ alkyl-R', C₁₋₆ alkoxy-R', 3-12 membered cycloalkyl, 3-12 membered cycloalkenyl, 3-12 membered heterocyclyl, 6-14 membered aryl and 5-10 membered heteroaryl are optionally substituted with one or more R^{m}, the 3-12 membered cycloalkyl, 3-12 membered cycloalkenyl and 3-12 membered heterocyclyl comprise monocyclic, spirocyclic or bridged ring, the 6-14 membered aryl and 5-10 membered heteroaryl comprise monocyclic or fused ring;
R^{b}, R^{c} and R^{d} are each absent or each independently selected from the group consisting of hydrogen, hydroxyl, mercapto, C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', -OR', -C(=O)-R', -SO₂-R', 3-12 membered cycloalkyl, 3-12 membered cycloalkenyl, 3-12 membered heterocyclyl, 6-14 membered aryl and 5-10 membered heteroaryl, the C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', 3-12 membered cycloalkyl, 3-12 membered cycloalkenyl, 3-12 membered heterocyclyl, 6-14 membered aryl and 5-10 membered heteroaryl are optionally substituted with one or more R^{m}, the 3-12 membered cycloalkyl, 3-12 membered cycloalkenyl and 3-12 membered heterocyclyl comprise monocyclic, spirocyclic or bridged ring, the 6-14 membered aryl and 5-10 membered heteroaryl comprise monocyclic or fused ring;
or R^{a} and R^{b} together with the carbon atom to which they are both attached to form a 3-12 membered monocyclic, spirocyclic or bridged ring optionally substituted with one or more R^{m};
or R^{a} or R^{b} together with the carbon atom or ortho-nitrogen atom to which they are attached to form a 5-14 membered heteroaryl, 6-14 membered aryl, 3-14 membered heterocyclyl or 3-12 membered cycloalkyl optionally substituted with one or more R^{m};
or R^{b} and R^{c} together with the nitrogen atom to which they are both attached to form a 3-12 membered monocyclic, spirocyclic, bridged or fused ring optionally substituted with one or more R^{m};
R^{e} and R^{f} are each independently selected from the group consisting of hydrogen, C₁₋₆ alkyl, C₂₋₆ alkenyl, C₂₋₆ alkynyl, 3-12 membered cycloalkyl, 3-20 membered heterocyclyl, 6-20 membered aryl and 5-20 membered heteroaryl, wherein the C₁₋₆ alkyl, C₂₋₆ alkenyl, C₂₋₆ alkynyl, 3-12 membered cycloalkyl, 3-20 membered heterocyclyl, 6-20 membered aryl and 5-20 membered heteroaryl are optionally substituted with one or more C₁₋₆ alkyl or halogen;
R^{g} is selected from the group consisting of C₁₋₆ alkyl, 3-12 membered cycloalkyl, 3-20 membered heterocyclyl, amino, C₁₋₆ alkylamino, di(C₁₋₆ alkyl)amino and 6-20 membered aryl, wherein the C₁₋₆ alkyl, 3-12 membered cycloalkyl, 3-20 membered heterocyclyl, C₁₋₆ alkylamino, di(C₁₋₆ alkyl)amino and 6-20 membered aryl are optionally substituted with one or more groups independently selected from the group consisting of: halogen, OR^{e} or -C(=O)NR^{e}R^{f};
R' is selected from the group consisting of hydrogen, C₁₋₆ alkyl, 3-12 membered cycloalkyl, 3-12 membered cycloalkenyl, 3-12 membered heterocyclyl, 6-14 membered aryl and 5-14 membered heteroaryl, the C₁₋₆ alkyl, 3-12 membered cycloalkyl, 3-12 membered cycloalkenyl, 3-12 membered heterocyclyl, 6-14 membered aryl and 5-14 membered heteroaryl are optionally substituted with one or more R^{m}; the 3-12 membered cycloalkyl, 3-12 membered cycloalkenyl and 3-12 membered heterocyclyl comprise monocyclic, spirocyclic or bridged ring, and the 6-14 membered aryl and 5-14 membered heteroaryl comprise monocyclic or fused ring;
R^{m} is selected from the group consisting of deuterium, halogen, cyano, nitro, - OR^{a}, -SR^{a}, -C(=Y¹)R^{a}, -C(=Y¹)OR^{a}, -C(=Y¹)NR^{b}R^{c}, -(CR^{a}R^{b})ₙ₁-NR^{b}R^{c}, - NR^{b}C(=Y¹)R^{c}, -NR^{b}C(=Y¹)OR^{c}, -NR^{d}C(=Y¹)NR^{b}R^{c}, -NR^{b}SO₂R^{a}, -OC(=Y¹)R^{a}, - OC(=Y¹)OR^{b}, -OC(=Y¹)NR^{a}R^{b}, -OSO₂(OR^{b}), -OP(=Y¹)(OR^{b})(OR^{c}), -OP(OR^{b})(OR^{c}), -SOR^{a}, -SO₂R^{a}, -SO₂NR^{b}R^{c}, -SO(OR^{b}), -SO₂(OR^{b}), -SC(=Y¹)R^{a}, -SC(=Y¹)OR^{b}, - SC(=Y¹)NR^{b}R^{c}, C₁₋₁₂ alkyl, halogenated C₁₋₁₂ alkyl, C₁₋₁₂ alkoxy-C₁₋₁₂ alkyl, C₁₋₁₂ alkyl-C₁₋₁₂ alkoxy, C₁₋₁₂ alkoxy-C₁₋₁₂ alkoxy, C₂₋₈ alkenyl, C₂₋₈ alkynyl, 3-12 membered cycloalkyl, 5-14 membered heterocyclyl, 6-20 membered aryl, 5-20 membered heteroaryl, (CR^{a}R^{d})ₜNR^{b}R^{c}, azido and oxo, wherein the 3-12 membered cycloalkyl and the 5-14 membered heterocyclyl comprise monocyclic, spirocyclic or bridged ring, and the 6-20 membered aryl and the 5-20 membered heteroaryl comprise monocyclic or fused ring;
Y¹ is O or S;
t, m1, and n1 are each independently 0, 1, 2, 3, 4, 5, or 6;
the conditions are as follows:
   when A is 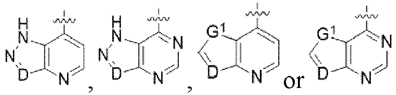 substituted with R¹, and -X-Y is -NR'-C(=O)-, R¹ is not hydroxyalkyl, except that NH is substituted with R¹, for example in the 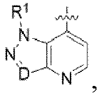 R¹ may be hydroxyl C₁₋₆ alkyl, wherein D is selected from N or CH, G¹ is selected from NH, O or S;
   when A is 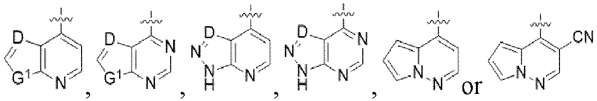 substituted with R¹, and -X-Y is-NR' -C(=O)-, R¹ is not hydroxyalkyl, wherein D is selected from N or CH, G¹ is selected from NH, O or S;
   when A is 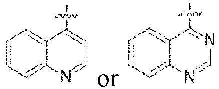 substituted with R¹, Z is a phenyl ring, and -X-Y is -NH-C(=O)-, R¹ is not hydroxyalkyl;
   E₃ is not C=O, C=S or C=NR^{a}; when E₃ is CH, E₁ is not C=O and E₂ is not NR^{b}; when E₃ is absent, E₂ is not C=O, C=S or C=NR^{a};

In certain embodiments, A is selected from 5-14 membered heteroaryl substituted with R¹, the 5-14 membered heteroaryl substituted with R¹ comprises monocyclic or fused ring, optionally substituted with one or more R²;
Preferably, in formula I, A is selected from the following groups substituted with R¹, the following groups are optionally substituted with one or more R²;

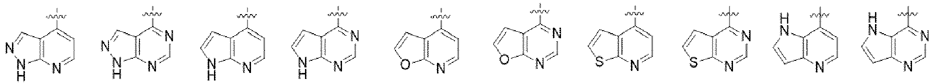

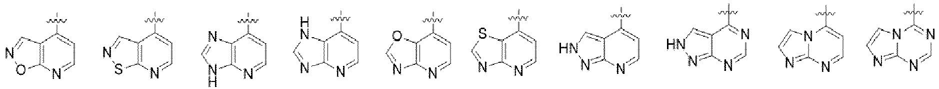

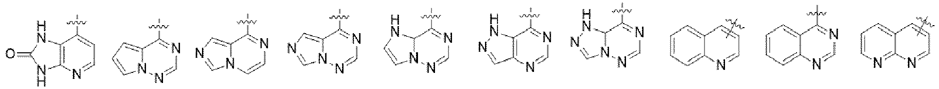

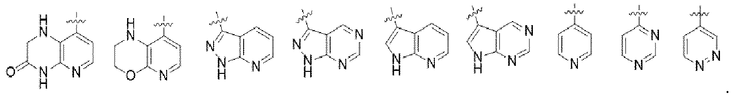

In certain embodiments, L is selected from O, S, CR^{a}R^{b}, NR^{b}, and C(=O);
Preferably, in formula I, L is selected from O, S, C(=O) and NH.

In certain embodiments, Z is selected from 6-14 membered aryl and 5-10 membered heteroaryl optionally substituted with one or more R³;
Preferably, in formula I, Z is selected from the following groups optionally substituted with one or more R³:

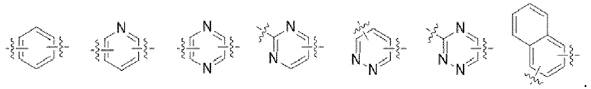

In certain embodiments, X is selected from NR⁴, O, S, or is absent;
Preferably, in formula I, X is selected from NR⁴, O and S.

In certain embodiments, Y is selected from C(=O), C(=S), C(=NR⁴), or is absent;
Preferably, in formula I, Y is selected from C(=O) and C(=S).

In certain embodiments, E₁, E₂, E₃ are each independently selected from CR^{a}R^{b}, N, C(=O), and C(=S);
Preferably, in formula I, E₁, E₂, E₃ are each independently selected from CR^{a}R^{b} and N.

In certain embodiments, G is selected from (CR^{a}R^{b})ₙ₁-6-20 membered aryl, (CR^{a}R^{b})ₙ₁-5-20 membered heteroaryl, (CR^{a}R^{b})ₙ₁-3-12 membered cycloalkyl and (CR^{a}R^{b})ₙ₁-3-20 membered heterocyclyl, the (CR^{a}R^{b})ₙ₁-6-20 membered aryl, (CR^{a}R^{b})ₙ₁-5-20 membered heteroaryl, (CR^{a}R^{b})ₙ₁-3-12 membered cycloalkyl and (CR^{a}R^{b})ₙ₁-3-20 membered heterocyclyl are optionally substituted with one or more groups independently selected from the group consisting of: halogen, nitro, oxo, CN, OR^{e}, SR^{e}, NR^{e}R^{f}, C₁₋₁₂ alkyl, halogenated C₁₋₁₂ alkyl, C₁₋₆ alkoxy-C₁₋₁₂ alkyl, halogenated C₁₋₆ alkoxy-C₁₋₁₂ alkyl, R^{a}R^{b}N-C₁₋₆ alkyl, C₂₋₈ alkenyl, halogenated C₂₋₈ alkenyl, C₂₋₈ alkynyl, 3-12 membered cycloalkyl, 3-12 membered cycloalkyl-C₁₋₆ alkyl or 3-20 membered heterocyclyl;
Preferably, in formula I, G is selected from 6-20 membered aryl and 5-20 membered heteroaryl, wherein the 6-20 membered aryl and 5-20 membered heteroaryl are optionally substituted with one or more groups independently selected from the group consisting of: halogen, nitro, oxo, CN, OR^{e}, SR^{e}, NR^{e}R^{f}, C₁₋₁₂ alkyl, halogenated C₁₋₁₂ alkyl, C₁₋₆ alkoxy-C₁₋₁₂ alkyl, halogenated C₁₋₆ alkoxy-C₁₋₁₂ alkyl, R^{a}R^{b}N-C₁₋₆ alkyl, C₂₋₈ alkenyl, halogenated C₂₋₈ alkenyl, C₂₋₈ alkynyl, 3-12 membered cycloalkyl-C₁₋₆ alkyl or 3-20 membered heterocyclyl.

In certain embodiments, R¹ is selected from R⁸ substituted with one or two hydroxy;
R⁸ is selected from the group consisting of -NR^{b}R^{c}, -OR^{b}, -SR^{b}, R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered cycloalkenyl, 3-12 membered heterocyclyl, 6-14 membered aryl, 5-10 membered heteroaryl, 3-12 membered cycloalkyl-C₁₋₆ alkyl, 3-12 membered cycloalkenyl-C₁₋₆ alkyl, 3-12 membered heterocyclyl-C₁₋₆ alkyl, 6-14 membered aryl-C₁₋₆ alkyl, and 5-10 membered heteroaryl-C₁₋₆ alkyl, the R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered cycloalkenyl, 3-12 membered heterocyclyl, 6-14 membered aryl, 5-10 membered heteroaryl, 3-12 membered cycloalkyl-C₁₋₆ alkyl, 3-12 membered cycloalkenyl-C₁₋₆ alkyl, 3-12 membered heterocyclyl-C₁₋₆ alkyl, 6-14 membered aryl-C₁₋₆ alkyl, and 5-10 membered heteroaryl-C₁₋₆ alkyl are optionally substituted with one or more R^{m}, the 3-12 membered cycloalkyl, 3-12 membered cycloalkenyl and 3-12 membered heterocyclyl comprise monocyclic, spirocyclic or bridged ring, the 6-14 membered aryl and 5-10 membered heteroaryl comprise monocyclic or fused ring;
Preferably, R⁸ is selected from the group consisting of -NR^{b}R^{c}, -OR^{b}, C₁₋₆ alkyl, R^{b}R^{c}N-C₁₋₆ alkyl, 3-12 membered heterocyclyl and 3-12 membered heterocyclyl-C₁₋₆ alkyl, the C₁₋₆ alkyl, R^{b}R^{c}N-C₁₋₆ alkyl, 3-12 membered heterocyclyl and 3-12 membered heterocyclyl-C₁₋₆ alkyl are optionally substituted with one or more R^{m}, the 3-12 membered heterocyclyl and 3-12 membered heterocyclyl-_{C1-6} alkyl comprise monocyclic, spirocyclic or bridged ring.

In certain embodiments, R² and R³ are each independently selected from the group consisting of hydrogen, halogen, cyano, nitro, -NR^{b}R^{c}, -OR^{b}, R^{b}R^{c}N-C₁₋₆ alkyl, R^{b}R^{c}N-C₁₋₆ alkoxy, -C(=Y¹)R^{a}, -C(=Y¹)NR^{b}R^{c}, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl, 3-12 membered heterocyclyl-C₁₋₆ alkyl and 5-10 membered heteroaryl-C₁₋₆ alkyl, the R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl, 3-12 membered heterocyclyl-C₁₋₆ alkyl and 5-10 membered heteroaryl-C₁₋₆ alkyl are optionally substituted with one or more R^{m}, the 3-12 membered cycloalkyl, 3-12 membered heterocyclyl, 3-12 membered cycloalkyl-C₁₋₆ alkyl, and 3-12 membered heterocyclyl-C₁₋₆ alkyl comprise monocyclic, spirocyclic, or bridged ring, the 5-10 membered heteroaryl-C₁₋₆ alkyl comprise monocyclic or fused ring;
Preferably, R² and R³ are each independently selected from the group consisting of hydrogen, halogen, cyano, nitro, -NR^{b}R^{c}, -OR^{b}, R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl and 3-12 membered heterocyclyl-C₁₋₆ alkyl, the R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl and 3-12 membered heterocyclyl-C₁₋₆ alkyl are optionally substituted with one or more R^{m}, the 3-12 membered cycloalkyl, 3-12 membered heterocyclyl, 3-12 membered cycloalkyl-C₁₋₆ alkyl, and 3-12 membered heterocyclyl-C₁₋₆ alkyl comprise monocyclic, spirocyclic or bridged ring.

In certain embodiments, R⁴, R⁵, R⁶, and R⁷ are each independently selected from the group consisting of hydrogen, halogen, OR^{e}, C₁₋₆ alkyl, C₂₋₆ alkenyl, C₂₋₆ alkynyl, 3-20 membered cycloalkyl, 3-20 membered heterocyclyl and 6-20 membered aryl, wherein the 3-20 membered cycloalkyl and the 3-20 membered heterocyclyl comprise monocyclic, spirocyclic, or bridged ring; the C₁₋₆ alkyl, C₂₋₆ alkenyl, C₂₋₆ alkynyl, 3-20 membered cycloalkyl, 3-20 membered heterocyclyl and 6-20 membered aryl are optionally substituted with one or more groups independently selected from the group consisiting of halogen, CN, OR^{e}, NR^{e}R^{f}, C₁₋₁₂ alkyl, C₂₋₈ alkenyl, C₂₋₈ alkynyl, 3-12 membered cycloalkyl or 3-20 membered heterocyclyl;
or R⁴ and R⁵ together with the carbon atom to which they are both attached to form a 3-12 membered monocyclic, spirocyclic or bridged ring optionally substituted with one or more R^{m};
or R⁶ and R⁷ together with the nitrogen atom to which they are both attached to form a saturated, partially unsaturated or fully unsaturated 3-20 membered heterocycle optionally substituted with one or more R^{m}, which comprsing one or more heteroatoms selected from N, O or S;

Preferably, R⁴, R⁵, R⁶ and R⁷ are each independently selected from the group consisting of hydrogen, OR^{e}, C₁₋₆ alkyl, monocyclic or bicyclic of 3-12 membered cycloalkyl, 3-20 membered heterocyclyl and 6-20 membered aryl, wherein the C₁₋₆ alkyl, monocyclic or bicyclic of 3-12 membered cycloalkyl, 3-20 membered heterocyclyl and 6-20 membered aryl are optionally substituted with one or more groups independently selected from the group consisting of: halogen, CN, OR^{e}, NR^{e}R^{f}, C₁₋₁₂ alkyl, C₂₋₈ alkenyl, C₂₋₈ alkynyl, 3-12 membered cycloalkyl or 3-20 membered heterocyclyl;
or R⁴ and R⁵ together with the carbon atom to which they are both attached to form a 3-12 membered monocyclic, spirocyclic or bridged ring optionally substituted with one or more R^{m}, the 3-12 membered monocyclic, spirocyclic or bridged ring is optionally substituted with one or more groups independently selected from the group consisiting of: halogen, C₁₋₆ alkyl or C₁₋₆ alkoxy;
or R⁶ and R⁷ together with the nitrogen atom to which they are both attached to form a saturated, partially unsaturated, or fully unsaturated 3-20 membered heterocycle optionally substituted with one or more R^{m}, the 3-20 membered heterocycle is optionally substituted with one or more groups independently selected from the group consisting of: halogen, C₁₋₆ alkyl or C₁₋₆ alkoxy.

In certain embodiments, R^{a} is absent or selected from the group consisting of hydrogen, halogen, cyano, hydroxyl, mercapto, carboxyl, nitro, -NR^{b}R^{c}, -C(=O)R^{d}, C₁₋₆ alkyl, C₁₋₆ alkoxy, C₂₋₈ alkenyl, C₂₋₈ alkynyl, C₁₋₆ alkyl-R', C₁₋₆ alkoxy-R', -OR', - SR', -SOR^{d}, -SO₂R^{d}, 3-12 membered cycloalkyl, 3-12 membered cycloalkenyl, 3-12 membered heterocyclyl, 6-14 membered aryl, and 5-10 membered heteroaryl, the C₁₋₆ alkyl, C₁₋₆ alkoxy, C₂₋₈ alkenyl, C₂₋₈ alkynyl, C₁₋₆ alkyl-R', C₁₋₆ alkoxy-R', 3-12 membered cycloalkyl, 3-12 membered cycloalkenyl, 3-12 membered heterocyclyl, 6-14 membered aryl, and 5-10 membered heteroaryl are optionally substituted with one or more R^{m};
R^{b}, R^{c} and R^{d} are each absent or each independently selected from the group consisting of hydrogen, hydroxyl, mercapto, C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R, -OR', -C(=O)-R', 3-12 membered cycloalkyl, 3-12 membered cycloalkenyl, 3-12 membered heterocyclyl, 6-14 membered aryl and 5-10 membered heteroaryl, the C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', 3-12 membered cycloalkyl, 3-12 membered cycloalkenyl, 3-12 membered heterocyclyl, 6-14 membered aryl, and 5-10 membered heteroaryl are optionally substituted with one or more R^{m};
or R^{b} and R^{c} together with the nitrogen atom to which they are both attached to form a 3-12 membered monocyclic, spirocyclic, bridged or fused ring optionally substituted with one or more R^{m};
R^{e} and R^{f} are each independently selected from the group consisting of hydrogen, C₁₋₆ alkyl, C₂₋₆ alkenyl, 3-12 membered cycloalkyl, 3-20 membered heterocyclyl, 6-20 membered aryl, and 5-20 membered heteroaryl, wherein the C₁₋₆ alkyl, C₂₋₆ alkenyl, 3-12 membered cycloalkyl, 3-20 membered heterocyclyl, 6-20 membered aryl, and 5-20 membered heteroaryl are optionally substituted with one or more C₁₋₆ alkyl or halogen;
R^{g} is C₁₋₆ alkyl or 6-20 membered aryl, wherein the C₁₋₆ alkyl and 6-20 membered aryl are optionally substituted with one or more groups independently selected from the group consisting of: halogen or OR^{e};
R' is selected from the group consisting of C₁₋₆ alkyl, 3-12 membered cycloalkyl, 3-12 membered cycloalkenyl, 3-12 membered heterocyclyl, 6-14 membered aryl and 5-10 membered heteroaryl, the C₁₋₆ alkyl, 3-12 membered cycloalkyl, 3-12 membered cycloalkenyl, 3-12 membered heterocyclyl, 6-14 membered aryl and 5-10 membered heteroaryl are optionally substituted with one or more R^{m}; the 3-12 membered cycloalkyl, 3-12 membered alkenyl and 3-12 membered heterocyclyl comprise monocyclic, spirocyclic or bridged ring, and the 6-14 membered aryl and 5-10 membered heteroaryl comprise monocyclic or fused ring.

Preferably, R^{a} is absent or selected from the group consisting of hydrogen, halogen, cyano, hydroxyl, -NR^{b}R^{c}, -C(=O)R^{d}, C₁₋₆ alkyl, C₁₋₆ alkoxy, C₂₋₈ alkenyl, C₁₋₆ alkyl-R', C₁₋₆ alkoxy-R', -OR', -SR', -SOR^{d}, -SO₂R^{d}, 3-12 membered cycloalkyl, 3-12 membered cycloalkenyl and 3-12 membered heterocyclyl, the C₁₋₆ alkyl, C₁₋₆ alkoxy, C₂₋₈ alkenyl, C₁₋₆ alkyl-R', C₁₋₆ alkoxy-R, 3-12 membered cycloalkyl, 3-12 membered cycloalkenyl and 3-12 membered heterocyclyl are optionally substituted with one or more R^{m};
R^{b}, R^{c} and R^{d} are each absent or each independently selected from the group consisting of hydrogen, hydroxyl, mercapto, C₁₋₆ alkyl, halogen, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', -OR', -C(=O)-R', 3-12 membered cycloalkyl and 3-12 membered heterocyclyl, the C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', -OR', -C(=O)-R', 3-12 membered cycloalkyl and 3-12 membered heterocyclyl are optionally substituted with one or more R^{m};
or R^{b} and R^{c} together with the nitrogen atom to which they are both attached to form a 3-12 membered monocyclic, spirocyclic, bridged or fused ring optionally substituted with one or more R^{m};
R^{e} and R^{f} are each independently selected from the group consisting of hydrogen, C₁₋₆ alkyl, C₂₋₆ alkenyl, 3-12 membered cycloalkyl and 3-20 membered heterocyclyl, wherein the C₁₋₆ alkyl, C₂₋₆ alkenyl, 3-12 membered cycloalkyl and 3-20 membered heterocyclyl are optionally substituted with one or more C₁₋₆ alkyl or halogen;
R^{g} is C₁₋₆ alkyl or 6-20 membered aryl, wherein the C₁₋₆ alkyl and 6-20 membered aryl are optionally substituted with one or more groups independently selected from: halogen or OR^{e};
R' is selected from the group consisting of C₁₋₆ alkyl, 3-12 membered cycloalkyl, 3-12 membered heterocyclyl and 5-10 membered heteroaryl, the C₁₋₆ alkyl, 3-12 membered cycloalkyl, 3-12 membered heterocyclyl and 5-10 membered heteroaryl are optionally substituted with one or more R^{m}; the 3-12 membered cycloalkyl and the 3-12 membered heterocyclyl comprise monocyclic, spirocyclic or bridged ring, and the 5-10 membered heteroaryl comprise monocyclic or fused ring.

In certain embodiments, R^{m} is independently selected from the group consisting of deuterium, halogen, cyano, nitro, -OR^{a}, -SR^{a}, -C(=Y¹)R^{a}, -C(=Y¹)OR^{a}, - C(=Y¹)NR^{b}R^{c}, -NR^{b}R^{c}, -NR^{b}C(=Y¹)R^{c}, -NR^{b}SO₂R^{a}, -OC(=Y¹)R^{a}, -SO₂R^{a}, C₁₋₁₂ alkyl, halogenerated C₁₋₁₂ alkyl, C₁₋₁₂ alkoxy-C₁₋₁₂ alkyl, C₁₋₁₂ alkyl-C₁₋₁₂ alkoxy, C₁₋₁₂ alkoxy-C₁₋₁₂ alkoxy, C₂₋₈ alkenyl, C₂₋₈ alkynyl, 3-12 membered cycloalkyl, 3-20 membered heterocyclyl, 6-20 membered aryl, 5-20 membered heteroaryl, (CR^{a}R^{d})ₜNR^{b}R^{c} and oxo, the 3-12 membered cycloalkyl and 3-20 membered heterocyclyl comprise monocyclic, spirocyclic or bridged ring, the 6-20 membered aryl and 5-20 membered heteroaryl groups comprise monocyclic or fused ring;

Preferably, R^{m} is selected from the group consisting of deuterium, halogen, cyano, nitro, hydroxyl, mercapto, amino, carboxyl, C₁₋₆ alkyl, C₁₋₆ alkoxy-C₁₋₆ alkyl, C₁₋₆ alkoxy, C₁₋₆ alkyl-C₁₋₆ alkoxy, C₁₋₆ alkoxy-C₁₋₆ alkoxy, C₁₋₆ alkylamino, (C₁₋₆ alkyl)₂amino, C₁₋₆ alkyl ester, C₁₋₆ alkylaminocarbonyl, (C₁₋₆ alkyl)₂aminocarbonyl, C₁₋₆ alkylcarbonyl, C₁₋₆ alkylcarbonyloxy, C₁₋₆ alkylcarbonylamino, C₁₋₆ alkylsulfonylamino, halogenated C₁₋₆ alkyl, halogenated C₁₋₆ alkoxy, C₁₋₆ alkylsulfonyl, C₁₋₆ alkylthio, C₂₋₈ alkenyl, C₂₋₈ alkynyl, 3-8 membered cycloalkyl, 3-8 membered heterocyclyl, 6-10 membered aryl, 3-8 membered heteroaryl, - (CR^{a}R^{d})ₜNR^{b}R^{c} and oxo.

Unless otherwise indicated, the following definitions are set forth to illustrate and define the meaning and scope of the various terms used to describe the present application. 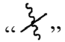 refers to a connection site.

The minimum and maximum values of the carbon atom content in a hydrocarbon group are indicated by a prefix, e.g., the prefix (C_{a-b}) alkyl indicates any alkyl containing from "a" to "b" carbon atoms. Thus, for example, (C₁₋₆) alkyl refers to alkyl containing 1 to 6 carbon atoms. The alkyl is branched or straight chain.

The atoms described in the compounds of the present application include isotopes thereof, for example, hydrogen may be deuterium or tritium.
"alkyl" refers to a straight or branched chain, monovalent, saturated hydrocarbon group including, but not limited to methyl, ethyl, n-propyl, isopropyl, n-butyl, isobutyl, sec-butyl, tert-butyl, n-pentyl, isopentyl, neopentyl, n-hexyl, and the like. preferably C₁₋₈ alkyl, more preferably C₁₋₆ alkyl, most preferably C₁₋₄ alkyl.
"cycloalkyl" refers to a saturated monocyclic, bicyclic, spirocyclic, bicyclic, or bridged cycloalkyl, possibly in combination with other groups. Cycloalkyl include, but are not limited to cyclopropyl, cyclobutyl, cyclopentyl, cyclohexyl and cycloheptyl, preferably 3-8 membered cycloalkyl, more preferably 3-6 membered cycloalkyl, most preferably 3-4 membered cycloalkyl.
"alkenyl" refers to a straight, branched or cyclic hydrocarbon groups containing one or more double bonds, including but not limited to vinyl, propenyl, (E)-2-methylvinyl, (Z)-2-methylvinyl, (E)-but-2-enyl, (Z)-but-2-enyl, (E)-but-1-enyl, (Z)-but-1-enyl, preferably C₂₋₆ alkenyl, more preferably C₂₋₄ alkenyl.
"alkynyl" refers to a straight, branched or cyclic hydrocarbon group containing one or more triple bonds, including but not limited to ethynyl, prop-1-ynyl, prop-2-ynyl, but-1-ynyl, but-2-ynyl, but-3-ynyl, preferably C₂₋₆ alkynyl, more preferably C₂₋₄ alkynyl.
"alkoxy" means a straight or branched chain, monovalent, saturated alkyl bonded to an oxygen atom, includingbut not limited to methoxy, ethoxy, propoxy, butoxy, isobutoxy, t-butoxy, and the like, preferably C₁₋₈ alkoxy, more preferably C₁₋₆ alkoxy, most preferably C₁₋₄ alkoxy.
"halogen" refers to fluorine, chlorine, bromine or iodine, preferably fluorine, chlorine and bromine.
" halogenated alkyl " refers to an alkyl as defined herein, wherein one or more hydrogens have been replaced with the same or different halogen, including but not limited to -CH₂Cl, -CHF₂, -CH₂CF₃, -CH₂CCl₃, perfluoroalkyl (e.g., -CF₃), and the like.
"aryl" refers to a substituted or unsubstituted monocyclic or polycyclic aromatic group, including but not limited to phenyl, naphthyl, preferably 6-10 membered monocyclic or bicyclic aromatic groups, more preferably phenyl or naphthyl, most preferably phenyl.
"heterocyclyl" means a substituted or unsubstituted 3-10 membered nonaromatic monocyclic saturated ring containing 1-3 heteroatoms independently selected from N, O, or S, with the remaining ring atoms being carbon atoms. Examples of heterocyclyl includes, but is not limited to azetidinyl, oxetanyl, pyrrolidinyl, piperidinyl, piperazinyl, homopiperazinyl, oxopiperidinyl, oxopiperazinyl, oxohomopiperazinyl, tetrahydrofuranyl, imidazolinyl, morphininyl, oxazolidinyl, isoxazolidinyl, thiazolidinyl, isothiazolidinyl, quinuclidinyl, thiadiazolidinyl, dihydrofuranyl, tetrahydrofuranyl, dihydropyranyl, tetrahydropyranyl, thiomorpholinyl, thiomorpholinyl sulfoxide, thiomorpholinyl sulfone, and the like, preferably 4-7 membered heterocyclyl, more preferably 4-6 membered heterocyclyl.
"heteroaryl" refers to a substituted or unsubstituted 5 or 6 membered single heteroaromatic ring, or a substituted or unsubstituted 9 or 10 membered fused or double heteroaromatic ring containing 1 to 4 heteroatoms independently selected from N, O, or S, with the remaining ring atoms being carbon atoms. Examples of heteroaryl includes, but is not limited to thienyl, furyl, imidazolyl, isoxazolyl, oxazolyl, pyrazolyl, pyrrolyl, thiadiazolyl, triazolyl, pyridyl, pyridazinyl, pyrimidinyl, indolyl, indazolyl, quinolinyl, isoquinolinyl, benzimidazolyl or benzothiazolyl.
"bridged ring" means a polycyclic group in which any two rings share two atoms not directly attached and which may contain one or more double bonds, but none of the rings have a completely conjugated Π electron system, and the ring atoms may be all carbon atoms or one or more of the ring atoms may be selected from N, O, S, SO, or SO₂, preferably 7-10 membered rings.
"spirocyclic ring" refers to a polycyclic group in which any two rings share carbon atoms and may contain one or more double bonds, but none of the rings have a completely conjugated Π electron system, and the ring atoms may be all carbon atoms or one or more of the ring atoms may be selected from N, O, S, SO, or SO₂, preferably 5-10 membered rings.

Ring may be divided into bicyclic, tricyclic, tetracyclic or polycyclic groups depending on the number of rings formed, preferably bicyclic, tricyclic or tetracyclic, more preferably bicyclic or tricyclic.

One cyclic group may be bonded to another group in a variety of ways. If the bonding mode is not specified, all possible modes are included. For example, "pyridyl" includes 2-, 3-, or 4-pyridyl, and "thienyl" includes 2-or 3-thienyl.
"pharmaceutically acceptable salt" refers to add conventional acid or base to form salts which retain the biological effectiveness and properties of the compounds of formula I. The acid or base derived from suitable non-toxic organic or inorganic acids or organic or inorganic bases. Examples of acid addition salts include those derived from inorganic acids such as hydrochloric acid, hydrobromic acid, hydroiodic acid, sulfuric acid, sulfamic acid, phosphoric acid and nitric acid, and those derived from organic acids, such as acetic acid, propionic acid, glycolic acid, oxalic acid, stearic acid, ascorbic acid, p-toluenesulfonic acid, salicylic acid, methanesulfonic acid, ethanesulfonic acid, oxalic acid, succinic acid, citric acid, maleic acid, hydroxymaleic acid, lactic acid, fumaric acid, tartaric acid, malic acid, hydroxyethyl sulfonic acid, benzenesulfonic acid, trifluoroacetic acid, mandelic acid and the like. Examples of base addition salts include those derived from inorganic acids such as ammonium salts, calcium salts, iron salts, aluminum salts, sodium salts, potassium salts, zinc salts, magnesium salts, and those derived from organic acids, including salts of primary, secondary and tertiary amines, such as trimethylamine, triethylamine, tripropylamine, diethanolamine, ethylenediamine, ethanolamine, and the like. It is a well-known technique for pharmacists to chemically modify pharmaceutical compounds (i.e. drugs) into salts to obtain improved physical and chemical stability, hygroscopicity, fluidity and solubility of the compounds.
"prodrug" refers to a prodrug that can be converted in vivo into the structure of the compound of the present application and pharmaceutically acceptable salts thereof.

### Method for preparing heteroaromatic nitrogen-oxide compound as c-Met kinase inhibitors

The present application also relates to a method for preparing the compound of the formula I. The compounds of the present application may be prepared by any conventional means. Suitable methods for synthesizing these compounds are provided in the examples. In the multi-step synthetic routes, the order of the reactions can be adjusted under certain circumstances.

In certain embodiments, the present application provides a process for preparing a compound of formula I, comprising the steps of reacting compound 1 with compound M under basic conditions to form compound 2, then oxidizing compound 2 under acidic conditions to form compound 3, and reacting compound 3 with compound 4 to form a compound of formula I; 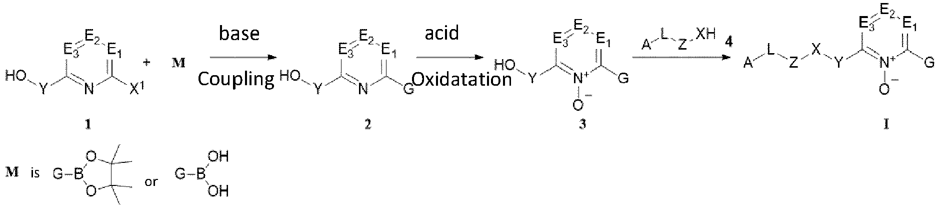 wherein X¹ is halogen, A, L, Z, X, Y, E₁, E₂, E₃ and G are as defined herein before,

Preferably, the base is selected from cesium fluoride, cesium carbonate;
Preferably, the acid is selected from trifluoroacetic acid.

### Pharmaceutical compositions of heteroaromatic nitrogen-oxide compound as c-Met kinase inhibitors

The present application also provides a pharmaceutical composition comprising heteroaromatic nitrogen-oxide compound as c-Met kinase inhibitors and pharmaceutically acceptable carriers or excipients.

The term "pharmaceutical composition" as referred to herein means the combination of one or more of the compounds of the present application or a pharmaceutically acceptable salt, solvate, hydrate or prodrug thereof with other chemical ingredients, such as a pharmaceutically acceptable carrier, excipient or diluent. The purpose of the pharmaceutical composition is to facilitate the administration process to animals.
"pharmaceutically acceptable carrier" refers to a pharmaceutically acceptable substance, ingredient or vehicle, such as a liquid or solid filler, diluent, excipient, solvent or encapsulating material, which participates in carrying or transporting a compound of the present application from a certain position, body fluid, tissue, organ (internal or external), or body part to another position, body fluid, organ (internal or external), or body part. Pharmaceutically acceptable carriers can be a vehicle, diluent, excipient or other materials that does not have undue toxicity or side effects and can be used to contact animal tissue. Typical pharmaceutically acceptable carriers include saccharides, starches, celluloses, maltose, tragacanth gum, gelatin, Ringer's solution, alginic acid, physiological saline, buffers and the like.

Each pharmaceutically acceptable carrier should be compatible with other ingredients, e.g., form a formulation with the conjugates provided herein, without undue toxicity, irritation, allergic response, immunogenicity, or other problem or complication to the living organism tissue or organ, at a reasonable benefit to risk ratio.

Some pharmaceutically acceptable carrier materials include: (1) saccharides such as lactose, glucose and sucrose; (2) starches, such as corn starch and potato starch; (3) cellulose and its derivatives, such as sodium carboxymethyl cellulose, ethyl cellulose, cellulose acetate; (4) tragacanth gum powder; (5) maltose; (6) gelatin; (7) talcum powder; (8) excipients such as cocoa butter and suppository waxes; (9) oils such as peanut oil, cottonseed oil, safflower oil, sesame oil, olive oil, corn oil and soybean oil; (10) glycols, such as propylene glycol; (11) polyols such as glycerol, sorbitol, mannitol and polyethylene glycol; (12) lipids such as ethyl oleate, ethyl laurate; (13) agar gel; (14) buffering agents such as magnesium hydroxide and aluminum hydroxide; (15) alginic acid; (16) sterile pyrogen-free water; (17) physiological saline; (18) Ringer's solution; (19) alcohols such as ethanol and propanol; (20) phosphate buffer; (21) other non-toxic compatible materials in pharmaceutical dosage forms, such as acetone.

The pharmaceutical compositions may include pharmaceutically acceptable excipients to simulate physiological conditions such as pH adjusting and buffering agents, toxicity adjusting agents and the like, such as sodium acetate, sodium chloride, potassium chloride, calcium chloride, sodium lactate and the like.

Drug ingredients can be made into any suitable dosage forms, such as solid dosage forms (e.g. tablets, capsules, powder, granules etc.) and liquid dosage forms (e.g. aqueous solution, emulsion, elixirs, syrups etc.). The methods for preparing pharmaceutical compositions has been well known, which can be prepared by conventional process, such as provided by Remington in The Science and Practice of Pharmacy (Gennaro ed. 20th edition, Williams & Wilkins PA, USA) (2000).

In certain embodiments, the compounds or pharmaceutical compositions of the present invention can be formulated into suitable dosage forms for drug release, which are administrated by injection (e.g., subcutaneous, intravenous, intramuscular, arterial, intrathecal, intracapsular, intrabox, intracardiac, intradermal, intraperitoneal, transtracheal, epidermal, intraarticular, subcapsular, subarachnoid, intraspinal, intrasternal, and/or infusion) and non-injection (e.g., oral, enteral, buccal, nasal, intranasal, mucosal, epidermal, patch, dermal, ophthalmic, pulmonary, sublingual, rectal, vaginal, or topical administration of the epidermis).

Suitable dosage forms include, but are not limited to, dosage forms for injection such as emulsions, solutions and suspensions, dosage forms for oral use such as tablets, capsules, pills, dragees, powders and granules, dosage forms for topical or transdermal absorption such as sprays, ointments, pastes, creams, lotions, gels, solutions, drug patches and inhalants, dosage forms for vaginal or rectal administration such as suppositories. These dosage forms can be prepared in accordance with compounds and suitable excipients under suitable conditions. The preparation method and process are well known, such as provided by Remingtonin in The Science and Practice of Pharmacy (Gennaro ed. 20th edition, Williams & Wilkins PA, USA) (2000).

In certain embodiments, the present application provides pharmaceutical compositions comprising the above-described compounds and a pharmaceutically acceptable carrier or excipient. In certain embodiments, the pharmaceutical composition is a tablet, capsule, pill, granule, powder, suppository, injection, solution, suspension, ointment, patch, lotion, drop, liniment, spray.

### Use of heteroaromatic nitrogen-oxide compounds as c-Met kinase inhibitors

In another aspect, the present application provides a use of a compound or pharmaceutical composition as described above for the manufacture of a medicament and for the treatment of a disease.

In certain embodiments, the present application provides a use of a compound or pharmaceutical composition as described above in the manufacture of a medicament for the treatment of a disease or condition associated with a protein kinase.

In certain embodiments, the disease associated with a protein kinase is selected from a disease associated with c-Met, VEGFR-2, AXL, TAM, NTRK, or RET.

In certain embodiments, the disease associated with a protein kinase is a tumor.

In certain embodiments, the disease associated with a protein kinase includes head and neck cancer, nasopharyngeal carcinoma, melanoma, bladder cancer, esophageal cancer, anaplastic large cell lymphoma, renal cancer, breast cancer, colorectal cancer, ovarian cancer, cervical cancer, pancreatic cancer, glioma, glioblastoma, prostate cancer, leukemia, lymphoma, non-Hodgkin's lymphoma, gastric cancer, lung cancer, liver cancer, gastrointestinal stromal tumor, thyroid cancer, squamous cell carcinoma, cholangiocarcinoma, endometrial cancer, multiple myeloma, or mesothelioma; atherosclerosis and pulmonary fibrosis.

In certain embodiments, the present application provides a use of the above-described compounds and/or pharmaceutical compositions in the preparation of an anti-tumor medicament.

In certain embodiments, the tumor comprises head and neck cancer, nasopharyngeal cancer, melanoma, bladder cancer, esophageal cancer, anaplastic large cell lymphoma, renal cancer, breast cancer, colorectal cancer, ovarian cancer, cervical cancer, pancreatic cancer, glioma, glioblastoma, prostate cancer, leukemia, lymphoma, non-Hodgkin's lymphoma, gastric cancer, lung cancer, liver cancer, gastrointestinal stromal tumor, thyroid cancer, squamous cell carcinoma, cholangiocarcinoma, endometrial cancer, multiple myeloma, and mesothelioma.

In certain embodiments, the present application provides a method for treating a tumor in a subject comprising administering to the subject an effective amount of a compound or pharmaceutical composition as described above. Preferably the subject is a mammal, preferably a human.

In certain embodiments, the mode of administration comprises oral, mucosal, sublingual, ocular, topical, parenteral, rectal, cerebral cistern, vaginal, peritoneal, bladder, nasal administration.

The compounds or pharmaceutical compositions of the present application may be administered to an organism through any suitable route, for example oral, intravenous, intranasal, topical, intramuscular, intradermal, transdermal or subcutaneous routes. In certain embodiments, the administration modes of a compound or pharmaceutical composition of the present application includes oral, mucosal, sublingual, ocular, topical, parenteral, rectal, cerebral cistern, vaginal, peritoneal, bladder, nasal administration.

In certain embodiments, the conjugates or pharmaceutical compositions of the present application may be administered simultaneously with a second active agent to achieve an additive or even synergistic effect in the body. For example, the compounds of the present application may be combined with a second active substance to form a pharmaceutical composition, or simultaneously administered as a single composition, or sequentially administered as a single composition. The second active substance for use in treating cancer that can be administered simultaneously with the compounds of the present application include, but are not limited to: Fluorouracil, Adriamycin, Daunorubicin, Tamoxifen, Leuprorelin, Goserelin, Flutamide, Nilutamide, Finasteride, Dexamethasone, Aminoglutethimide, Acridine, Anastrozole, Asparaginase, BCG, Bicalutamide, Bleomycin, Busulfan, Camptothecin, Capecitabine, Carboplatin, Carmustine, Chlorambucil, Cisplatin, Cladribine, Colchicine, cyclophosphamide, cyproterone, cytarabine, dacarbazine, actinomycin d, Daunorubicin, dienestrol, diethylstilbestrol, docetaxel, Adriamycin, Adriamycin, epirubicin, estradiol, estramustine, etoposide, exemestane, filgrastim, fludarabine, fluhydrocortisone, fluorouracil, flumethasterone, flutamide, gemcitabine, genistein, goserelin, tamoxifen, teniposide, testosterone, titanocene dichloride, topotecan, trastuzumab, tretinoin, vinblastine, hydroxyurea, idarubicin, ifosfamide, imatinib, interferon, irinotecan, letrozole, formyltetrahydrofolate, pentostatin, mithramycin, methyl benzyl hydrazine, raltitrexed, porfimer sodium, rituximab, streptozotocin, suramin, leuprorelin, levamisole, cyclohexylnitrosourea, nitrogen mustard, medroxyprogesterone, megestrol, melphalan, mercaptopurine, sodium mercaptoethanesulfonate, methotrexate, mitomycin, mitotane, mitoxantrone, nilutamide, nocodazole, octreotide, platinum, paclitaxel, pamidronic acid, thioguanine, triamcinolone, chloromethane, topotecan titanocene, trastuzumab, tretinoin, vinblastine, vindesine, vinorelbine, and vinorelbine.

In certain embodiments, the conjugates provided herein can be used concurrently with non-chemical methods for cancer therapy. In certain embodiments, the conjugates provided herein can be administered concurrently with radiation therapy. In certain embodiments, the conjugates provided herein can be used in conjunction with surgery, tumor heat treatment, ultrasound focusing therapy, cryotherapy, or any of the foregoing.

In certain embodiments, the conjugates provided herein can be used with steroids. Suitable steroids include, but are not limited toamcinolone, beclomethasone, betamethasone, budesonide, chloroprednisolone, clobetasol, corticosterone, cortisone, hydroxyprednisolide, desoximetasone, dexamethasone, diflorasone, difluorometasone, difluprednate, glycyrrhetinic acid, fluzacosone, flumethasone, flunisolide, flucloronide, fluocinonide, fluocortin butyl ester, flurandrenolone, fluperone acetate, fluprednidene acetate, fluprednisolone, flurandrenolide, fluorine propionate, formocortal, clobetasol propionate, halcinolone acetonid, halomethasone, hydrocortisone, loteprednol carbonate, methylprednisolone, medrysone, methylprednisone, 6-methylprednisolone, prednisone in the form of furoate, paramethasone, prednisolone, dexamethasone, and prednisolone 25-diethylamine acetate.

In certain embodiments, the compounds provided herein can be used concurrently with immunotherapeutic agents. Suitable immunotherapeutic agents include: tumor cell multi drug resistance reversal agents (such as verapamil), rapamycin, mycophenolate mofetil, thalidomide, cyclophosphamide, cyclosporines, and monoclonal antibodies.

### Examples

### Example 0-1. Preparation of 3-iodo-1-(4-methoxybenzyl)-1H-pyrazolo[3,4-b]pyridin-4-ol (intermediate A1)

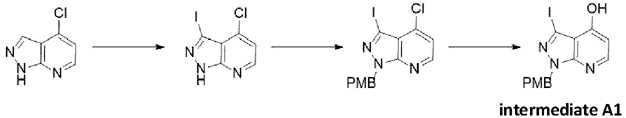

### Step 1: 4-chloro-3-iodo-1H-pyrazolo[3,4-b]pyridine

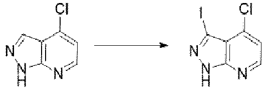

4-chloro-1H-pyrazolo[3,4-b]pyridine (20.0 g, 0.13 mmol) was dissolved in DMF (500 mL) at room temperature, and iodine (66.0 g, 0.26 mmol) and potassium hydroxide (22.0 g, 0.39 mmol) were added in batches. The reaction system was warmed to 50 °C and stirred for 2 h. After the raw materials are completely reacted through TLC monitoring, and then cooled to room temperature, the reaction solution was poured into 10% sodium bisulfite solution (500 mL), stirred for 0.5 h, filtered, the filter cake was washed with water (200 mL × 3), the filter cake was collected and dried under vacuum to obtain 4-chloro-3-iodo-1H-pyrazolo[3,4-b]pyridine (35.0 g, white solid). Yield: 96.5%. MS (ESI⁺)m/z = 279.9 [M+H]⁺.

### Step 2: 4-chloro-3-iodo-1-(4-methoxybenzyl)-1H-pyrazolo[3,4-b]pyridine

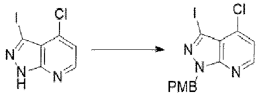

4-chloro-3-iodo-1H-pyrazolo[3,4-b]pyridine (35.0 g, 0.13 mol) was dissolved in DMF (100 mL) at room temperature, potassium carbonate (35.0 g, 0.25 mol) was added, p-methoxybenzyl chloride (29.8 g, 0.19 mol) was slowly added under ice bath, and the reaction solution was stirred overnight at room temperature. After the raw materials are completely reacted through TLC monitoring, the reaction was quenched by the addition of water (200-400 mL), the mixed system was filtered, the filter cake was washed with water (200 mL × 3), the filter cake was collected and dried in vacuum to give 4-chloro-3-iodo-1-(4-methoxybenzyl)-1H-pyrazolo[3,4-b]pyridine (50.0 g, white solid). Yield: 96.3%. MS (ESI⁺)m/z = 399.7 [M+H]⁺.

### Step 3: 3-iodo-1-(4-methoxybenzyl)-1H-pyrazolo[3,4-b]pyridin-4-ol

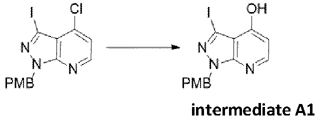

4-chloro-3-iodo-1-(4-methoxybenzyl)-1H-pyrazolo[3,4-b]pyridine (50.0 g, 0.12 mol) and a mixed solution of 20% NaOH and DMSO (1/8, 300 mL) were sequentially added to a 1-L round-bottomed flask at room temperature, and the reaction system was heated to 70 °C and stirred for 4 h. The reaction system was cooled to room temperature, 1M HCl was added to adjust the pH to 4 in an ice bath, filtered, the filter cake was washed with water (200 mL × 3), the filter cake was collected and dried in vacuum to give 3-iodo-1-(4-methoxybenzyl)-1H-pyrazolo[3,4-b]pyridin-4-ol (45.0 g, tan solid). Yield: 98.4%. MS (ESI⁺)m/z = 381.8 [M+H]⁺

### Example 0-2 Preparation of 5-bromoquinolin-4-ol (intermediate A2)

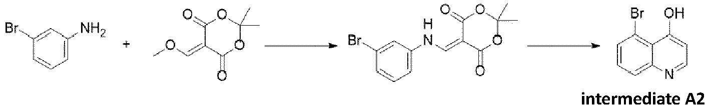

### Step 1: 5-(((3-bromophenyl)amino)methylene)-2,2-dimethyl-1,3-dioxane-4,6-dione

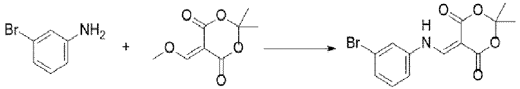

In a 100 mL dry round bottom flask, 3-bromoaniline (5.00 g, 0.029 mol) was dissolved in isopropanol (50 mL) and then 5-(methoxymethylene)-2,2-dimethyl-1,3-dioxahexane-4,6-dione (5.42 g, 0.029 mol) was added and the reation system was heated to 100 °C under nitrogen atmosphere and stirred for 2 h. After the reaction was completed, cooled to room temperature, filtered, and the filter cake was washed with methyl tert-butyl ether (50 mL) to give 5-(((3-bromophenyl)amino)methylene)-2,2-dimethyl-1,3-dioxane-4,6-dione (7.4 g, white solid), yield: 78.1 %.

### Step 2: 5-bromoquinolin-4-ol

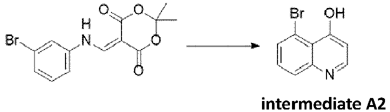

Diphenyl ether (30 mL) was added to a 100 mL dry round bottom flask, heated to 250 °C under nitrogen atmosphere, then 5-(((3-bromophenyl)amino)methylene)-2,2-dimethyl-1,3-dioxane-4,6-dione (2.0 g, 0.0061 mol) was added and stirred for 1 h. After the reaction was completed, cooled to room temperature, filtered, and the filter cake was washed with methyl tert-butyl ether (50 mL × 3) to give the crude product. The crude product was purified by high performance liquid chromatography with an eluent system of acetonitrile/water/0.1% FA [chromatographic column: XBridge-1 5 µm 19-150 mm, mobile phase: ACN-H₂O (0.1% FA) 10-37-8 min] to obtain 5-bromoquinolin-4-ol (0.88 g, yellow solid), yield: 16.0%. MS (ESI⁺)m/z = 223.9 [M+H]⁺.

### Example 0-3. Preparation of 6-carboxy-3-chloro-2-(4-fluorophenyl)pyridine 1-oxide (intermediate B1)

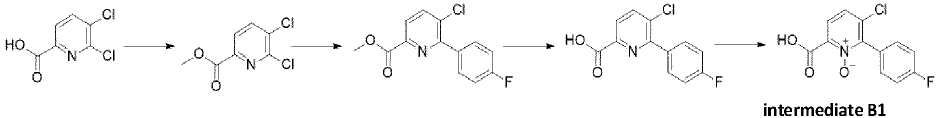

### Step 1: Methyl 5,6-dichloropicolinate

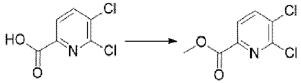

5,6-dichloropicolinic acid (1 g, 5.2 mmol) was dissolved in methanol (50 mL), thionyl chloride was added dropwise with stirring at 0 °C, and the reation system was heated to room temperature and stirred overnight. After the reaction was completed through TLC monitoring, concentrated under reduced pressure to remove methanol, the residue was dissolved in dichloromethane, washed with sodium bicarbonate solution, the organic phase was dried over anhydrous sodium sulfate, filtered and the filtrate was concentrated under reduced pressure to give 1.01 g of methyl 5,6-dichloropicolinate. Yield: 94.4%.

### Step 2: Methyl 5-chloro-6-(4-fluorophenyl)picolinate

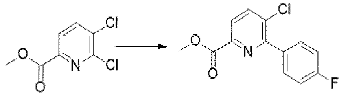

Methyl 5,6-dichloropicolinate (1.8 g, 8.74 mmol) and 4-fluorophenylboronic acid (1.5 g, 10.5 mmol) were added to a mixed system of acetonitrile/water (3/1, 32 mL) respectively, cesium fluoride (3.99 g, 26.4 mmol) and bis-triphenylphosphine palladium dichloride (0.307 g, 0.44 mmol) were then added, nitrogen was used to replace for three times, the temperature was raised to 65 °C and stirring was carried out overnight, after the reaction was completed through HPLC monitoring, cooled to room temperature and the reaction solution was slowly poured into water (20 mL), ethyl acetate was used for extraction (50 mL × 3), organic phases were combined and dried by anhydrous sodium sulfate, filtration was carried out, and the filtrate was concentrated under reduced pressure to obtain methyl 5-chloro-6-(4-fluorophenyl)picolinate (2 g). Yield: 86.2%.

### Step 3: 5-chloro-6-(4-fluorophenyl)picolinic acid

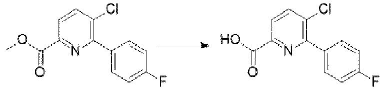

Methyl 5-chloro-6-(4-fluorophenyl)picolinate (2 g, 7.5 mmol) was added to a mixed solution of methanol/THF/H₂O (2/2/1, 62 mL) followed by lithium hydroxide monohydrate (790.5 mg, 18.8 mmol) and stirred at room temperature overnight. After the starting materials were reacted completely through TLC monitoring, concentrated under reduced pressure, and the obtained residue was added with water (50 mL), adjusted to a pH of 3 with 1M HCl, filtered, the filter cake was dissolved in ethyl acetate, allowed to stand for separation, the organic phase was dried over anhydrous sodium sulfate, filtered, and the filtrate was concentrated under reduced pressure to give 5-chloro-6-(4-fluorophenyl)picolinic acid (1.6 g). Yield: 84.6%.

### Step 4: 6-carboxy-3-chloro-2-(4-fluorophenyl)pyridine 1-oxide

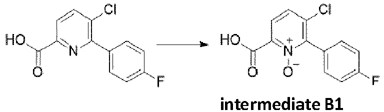

5-chloro-6-(4-fluorophenyl)picolinic acid (1.6 g, 6.36 mmol) was dissolved in acetonitrile (80 mL) and then 30% aqueous hydrogen peroxide (2.88 g, 25.4 mmol) was added in batches and TFA (5.34 g, 25.4 mmol) was added slowly dropwise in ice bath, after dropping, the temperature was raised to 65 °C and stirred overnight. After the reaction was completed through TLC monitoring, cooled to room temperature, and the reaction solution was poured into water (200 mL), filtered, the filter cake was washed once with water, then slurried with ethyl acetate, filtered and the filter cake was dried in vacuum to obtain 6-carboxy-3-chloro-2-(4-fluorophenyl)pyridine 1-oxide (1.25 g). Yield: 73.5 %. MS (ESI⁺)m/z = 268.0 [M+H]⁺.

### Examples 0-4-0-27 Preparation of intermediates B2-B25

The intermediates B2-B25 were synthesized from commercially available 6-chloropicolinic acid or formate substituted by different substituents and (hetero) aryl boric acid or borate substituted by different substituents as raw materials according to the method for synthesizing the intermediate B1 or similar methods (for example Suzuki coupling followed by oxidation, or Suzuki coupling followed by hydrolysis and oxidation, or oxidation followed by Suzuki coupling). (Table 1)

**Table1 Intermediates B2-B25**

| **Interm ediates** | **Structures** | **MS [M+H]⁺** | **Interm ediates** | **Structures** | **MS [M+H]⁺** |
|---|---|---|---|---|---|
| **B2** | 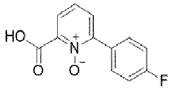 | 234.0 | **B3** | 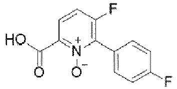 | 252.0 |
| **B4** | 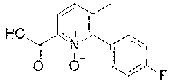 | 247.9 | **B5** | 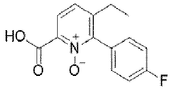 | 262.1 |
| **B6** | 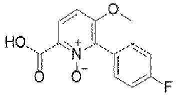 | 264.1 | **B7** | 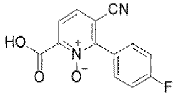 | 259.1 |
| **B8** | 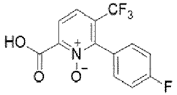 | 301.6 | **B9** | 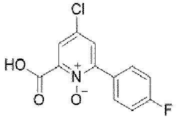 | 268.0 |
| **B10** | 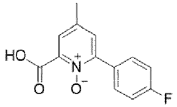 | 247.7 | **B11** | 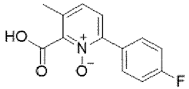 | 248.0 |
| **B12** | 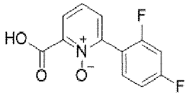 | 251.9 | **B13** | 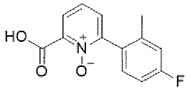 | 247.9 |
| **B14** | 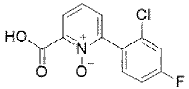 | 268.0 | **B15** | 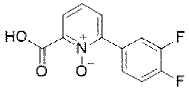 | 251.9 |
| **B16** | 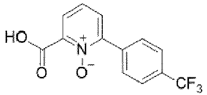 | 284.1 | **B17** | 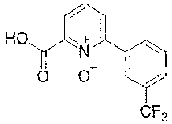 | 284.1 |
| **B18** | 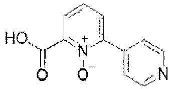 | 217.0 | **B19** | 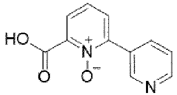 | 217.0 |
| **B20** | 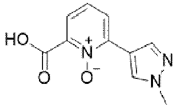 | 220.1 | **B21** | 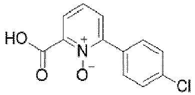 | 250.2 |
| **B22** | 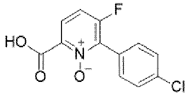 | 268.1 | **B23** | 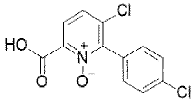 | 284.4 |
| **B24** | 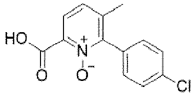 | 264.0 | **B25** | 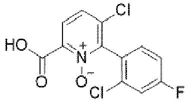 | 302.3 |

### Examples 0-28. Preparation of 6-carboxy-3-(methyl-d₃)-2-(4-fluorophenyl)pyridine 1-oxide (intermediate B26)

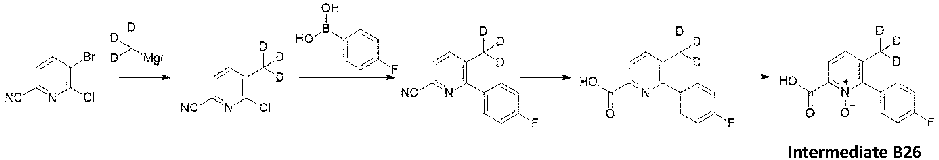

### Step 1: 2-cyano-5-(methyl-d₃)-6-chloropyridine

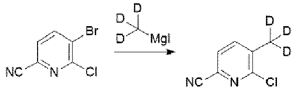

2-cyano-5-bromo-6-chloropyridine (2.0 g, 9.2 mmol) was added to anhydrous THF (30 mL), then Fe(acac)₃(227 mg, 0.6 mmol) and NMP (1.3 g, 13.8 mmol) were added and finally *d*₃-methylmagnesium iodide reagent (18.4 mL) was slowly added dropwise, the reaction solution was allowed to react overnight at room temperature, after the reaction was completed through TLC monitoring, then the reaction solution was poured into saturated aqueous ammonium chloride solution (5 mL), the mixed solution was extracted with ethyl acetate (20 mL × 3), the organic phases were combined and washed with saturated brine (10 mL), dried over anhydrous sodium sulfate, filtered, the filtrate was concentrated under reduced pressure, and the residue was purified by silica gel column chromatography with an eluent system of ethyl acetate/petroleum ether = 1/10 to give 2-cyano-5-(methyl-*d*₃)-6-chloropyridine as a yellow powder (200 mg). Yield: 14.0%.

### Step 2: 2-cyano-5-(methyl-d₃)-6-(4-fluorophenyl)pyridine

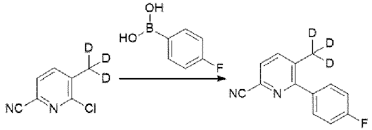

2-cyano-5-(methyl-*d*₃)-6-chloropyridine (200 mg, 1.3 mmol) and 4-fluorophenylboronic acid (234 mg, 1.7 mmol) were added to a mixed solution of toluene and ethanol (20 mL, 1/1), and then cesium carbonate (1.2 g, 3.9 mmol) was added, and finally Pd(PPh₃)₄ (74 mg, 0.065 mmol) was added, the mixture was heated to 85 °C under argon atmosphere and reacted overnight. After the reaction was completed through TLC monitoring, cooled to room temperature, the reaction solution was poured into a saturated aqueous ammonium chloride solution (5 mL), the mixed solution was extracted with ethyl acetate (10 mL × 3), the organic phases were combind and washed with saturated brine (10 mL), dried with anhydrous sodium sulfate, filtered, the filtrate was concentrated under reduced pressure, and the residue was purified with silica gel column chromatography with an eluent system of ethyl acetate/petroleum ether (1/10) to obtain 2-cyano-5-(methyl-*d*₃)-6-(4-fluorophenyl)pyridine (90 mg). Yield: 32.5%.

### Step 3: 5-(methyl-d₃)-6-(4-fluorophenyl)picolinic acid

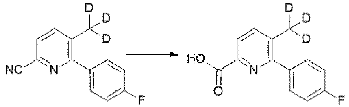

2-cyano-5-(methyl-*d*₃)-6-(4-fluorophenyl)pyridine (90 mg, 0.42 mmol) was added to dioxane hydrochloride (5 mL), water (1 mL) was added dropwise, the mixture was heated to 70 °C and reacted overnight. After completion of the reaction through TLC monitoring, cooled to room temperature and concentrated under reduced pressure, the residue was adjusted to neutral pH with saturated sodium bicarbonate, a solid was precipitated, filtered, and the residue was collected and dried to give 5-(methyl-*d*₃)-6-(4-fluorophenyl)picolinic acid (30 mg). Yield: 30.6 %.

### Step 4: 6-carboxy-3-(methyl-d₃)-2-(4-fluorophenyl)pyridine 1-oxide

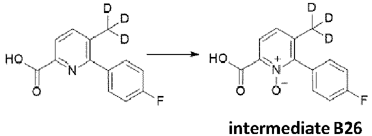

5-(methyl-*d*₃)-6-(4-fluorophenyl)picolinic acid (30 mg, 0.13 mmol) was added to acetonitrile (5 mL), carbamide peroxide (60 mg, 0.64 mmol) and TFAA (134 mg, 0.64 mmol) were added, the mixture was heated to 65 °C to react for 4 h, after the reation was completed through TLC monitoring, cooled to room temperature, and the reaction solution was poured into water (5 mL), the mixed solution was extracted with ethyl acetate (5 mL × 3), washed with saturated brine (10 mL), dried over anhydrous sodium sulfate, filtered, and the filtrate was concentrated under reduced pressure to give 6-carboxy-3-(methyl-*d*₃)-2-(4-fluorophenyl)pyridine 1-oxide (yellow solid, 30 mg). Yield: 93.8%.

### Example 0-29. Preparation of 6-carboxy-2-(4-fluorophenyl)-3-(methylthio)pyridine 1-oxide (intermediate B27)

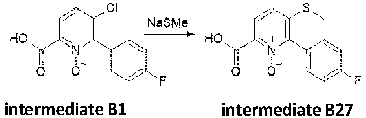

6-carboxy-3-chloro-2-(4-fluorophenyl)pyridine 1-oxide (intermediate B1,300 mg, 1.1 mmol) was dissolved in DMF (10 mL), sodium methyl mercaptide (94 mg, 1.3 mmol) was added, the reaction system was heated to 100 °C, stirred for 4 h, after the reaction was completed through TLC monitoring, cooled to room temperature, the reaction solution was poured into water (50 mL), a large amount of yellow solid was precipitated, filtered off with suction, the filter residue was washed with water, the obtained filter residue was dissolved with ethyl acetate, dried with anhydrous sodium sulfate, filtered, and the filtrate was concentrated under reduced pressure to obtain 6-carboxy-2-(4-fluorophenyl)-3-(methylthio)pyridine 1-oxide (yellow solid, 205 mg). Yield: 65.5 %.

### Example 0-30. Preparation of 6-carboxy-2-(4-fluorophenyl)-3-(methylsulfonyl)pyridine 1-oxide (intermediate B28)

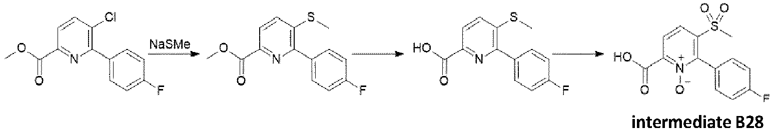

### Step 1: methyl 6-(4-fluorophenyl)-5-(methylthio)picolinate

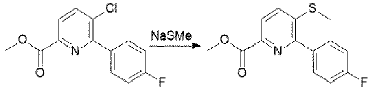

5-chloro-6-(4-fluorophenyl)methyl picolinate (500 mg, 1.9 mmol) was dissolved in DMF (10 mL), sodium methyl mercaptide (158 mg, 2.3 mmol) was added, the reaction system was heated to 100 °C, stirred for 4 h, after the reaction was completed through TLC monitoring, cooled to room temperature, the reaction solution was poured into water (50 mL), a large amount of yellow solid was precipitated, filtered off with suction, the filter residue was washed with water, the obtained filter residue was dissolved with ethyl acetate, dried with anhydrous sodium sulfate, filtered, and the filtrate was concentrated under reduced pressure to obtain methyl 6-(4-fluorophenyl)-5-(methylthio)picolinate (yellow solid, 340 mg). Yield: 65.1%.

### Step 2: 6-(4-fluorophenyl)-5-(methylthio)picolinic acid

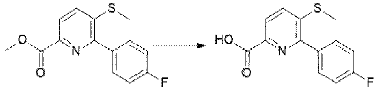

Methyl 6-(4-fluorophenyl)-5-(methylthio)picolinate (340 mg, 1.2 mmol) was dissolved in a mixed solution of methanol/THF/water (15 mL, 2/2/1), lithium hydroxide monohydrate (135 mg, 3.23 mmol) was added, stirring was carried out at room temperature for 2 h, after the reaction was completed through TLC monitoring and then concentrated under reduced pressure, the residue was adjusted to neutral pH with 1M dilute hydrochloric acid, a solid was precipitated, filtered off with suction, and the filter residue was dried to give 6-(4-fluorophenyl)-5-(methylthio)picolinic acid (280 mg). Yield: 86.7%.

### Step 3: 6-carboxy-2-(4-fluorophenyl)-3-(methylsulfonyl)pyridine 1-oxide

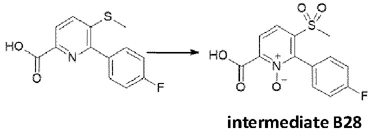

6-(4-fluorophenyl)-5-(methylthio)picolinic acid (280 mg, 1.1 mmol) was added to acetonitrile (10 mL), urea peroxide (996 mg, 10.6 mmol) and TFAA (2.2 g, 10.6 mmol) were added in batches, heated to 65 °C and stirred overnight, after the reaction was completed through TLC monitoring and the reaction solution was poured into water (20 mL), the mixture was extracted with ethyl acetate (20 mL × 3), the organic phases were combined and washed with saturated brine (20 mL), dried over anhydrous sodium sulfate, filtered, the filtrate was concentrated under reduced pressure to give a crude product, the crude product was slurried with dichloromethane (10 mL), filtered and the residue was dried to give 6-carboxy-2-(4-fluorophenyl)-3-(methylsulfonyl)pyridine 1-oxide as a pale yellow powder (140 mg). Yield: 42.3 %.

### Example 0-31. Preparation of 6-carboxy-4-cyano-2-(4-fluorophenyl)pyridine 1-oxide (intermediate B29)

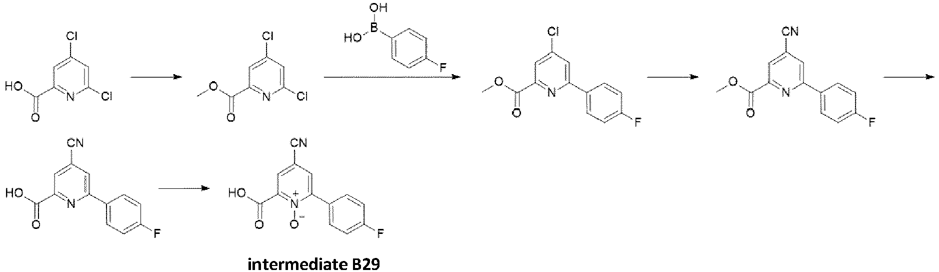

### Step 1 and Step 2: Methyl 4-chloro-6-(4-fluorophenyl)picolinate

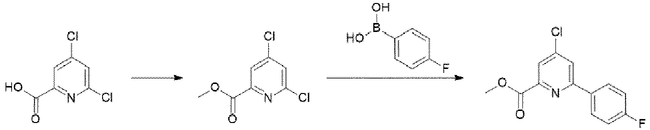

Methyl 4-chloro-6-(4-fluorophenyl)picolinate was synthesized from 4,6-dichloropicolinic acid as a starting material according to the methods of steps 1 and 2 of the synthesis of intermediate B1 in examples 0-3.

### Step 3: methyl 4-cyano-6-(4-fluorophenyl)picolinate

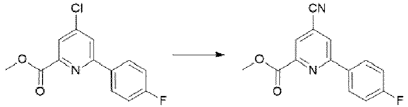

Methyl 4-chloro-6-(4-fluorophenyl)picolinate (0.425 g, 1.6 mmol), zinc cyanide (0.21 g, 1.8 mmol) were added respectively to DMAC (8 mL) and then tetratriphenyl phosphine palladium (92 mg, 0.08 mmol) was added, replaced with nitrogen three times, warmed to 65 °C overnight with stirring. After the reaction was completed through TLC monitoring, cooled to room temperature, the reaction was poured slowly into water (20 mL), extracted with ethyl acetate (40 mL × 3), the combined organic phases were dried over anhydrous sodium sulfate, filtered, the filtrate was concentrated under reduced pressure, and the residue was purified by silica gel column chromatography with an eluent system of ethyl acetate/petroleum ether = 1/10 to give methyl 4-cyano-6-(4-fluorophenyl)picolinate (0.26 g). Yield: 63.4%.

### Step 4 and Step 5: 6-carboxy-4-cyano-2-(4-fluorophenyl)pyridine 1-oxide

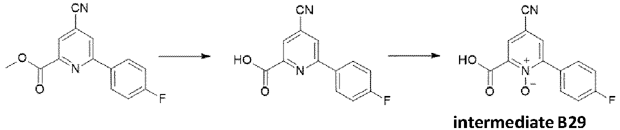

6-carboxy-4-cyano-2-(4-fluorophenyl)pyridine 1-oxide was synthesized from methyl 4-cyano-6-(4-fluorophenyl)picolinate as the starting material according to the third and fourth steps of the synthesis of intermediate B1 in example 0-3. MS (ESI⁺)m/z = 259.1 [M+H]⁺.

### Example 0-32. 6-carboxy-3-cyclopropyl-2-(4-fluorophenyl)pyridine 1-oxide (intermediate B30)

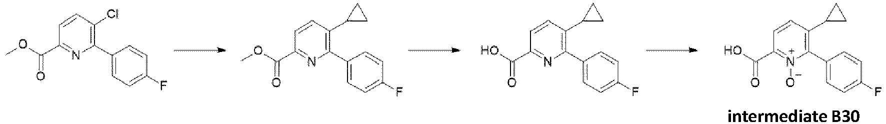

### Step 1: methyl 5-cyclopropyl-6-(4-fluorophenyl)picolinate

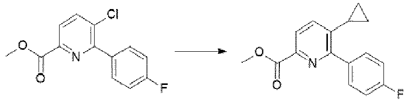

Methyl 3-chloro-6-(4-fluorophenyl)picolinate (0.2 g, 0.75 mmol), cyclopropylboronic acid (71 mg, 0.83 mmol) were added to a mixed system of toluene/water (3/1, 8 mL) respectively, potassium phosphate (477 mg, 2.25 mmol), palladium acetate (17 mg, 0.07 mmol) and SPhos (61 mg, 0.15 mmol) were added thereto, nitrogen was used for replace three times, the mixture was heated to 100 °C and stirred overnight. After the reaction was completed through HPLC monitoring, and then cooled to room temperature, the reaction solution was slowly poured into water (20 mL), extracted with ethyl acetate (30 mL × 3), the organic phases were combined and dried over anhydrous sodium sulfate, filtered, and the filtrate was concentrated under reduced pressure to give methyl 5-cyclopropyl-6-(4-fluorophenyl)picolinate (160 mg). Yield: 78.8%.

### Step 2 and Step 3: 6-carboxy-3-cyclopropyl-2-(4-fluorophenyl)pyridine 1-oxide

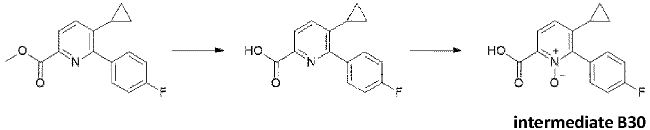

6-carboxy-3-cyclopropyl-2-(4-fluorophenyl)pyridine 1-oxide was synthesized from methyl 5-cyclopropyl-6-(4-fluorophenyl)picolinate as the starting material according to the third and fourth steps of the synthesis of intermediate B1 in example 0-3. (ESI⁺)m/z = 274.0 [M+H]⁺.

### Example 0-33. 6-carboxy-4-cyclopropyl-2-(4-fluorophenyl)pyridine 1-oxide (intermediate B31)

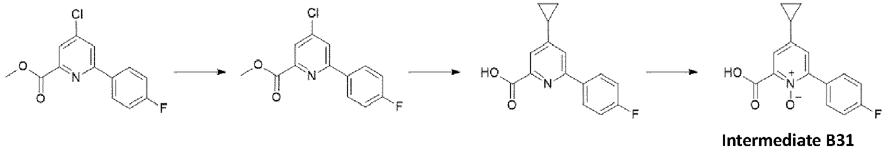

6-carboxy-4-cyclopropyl-2-(4-fluorophenyl)pyridine 1-oxide was synthesized from methyl 4-chloro-6-(4-fluorophenyl)picolinate as the starting material according to the method for synthesizing intermediate B30 in example 0-32. MS (ESI⁺)m/z = 274.0 [M+H]⁺.

### Example 0-34. Preparation of 2-carboxy-3-ethoxy-6-(4-fluorophenyl)pyridine 1-oxide (intermediate B32)

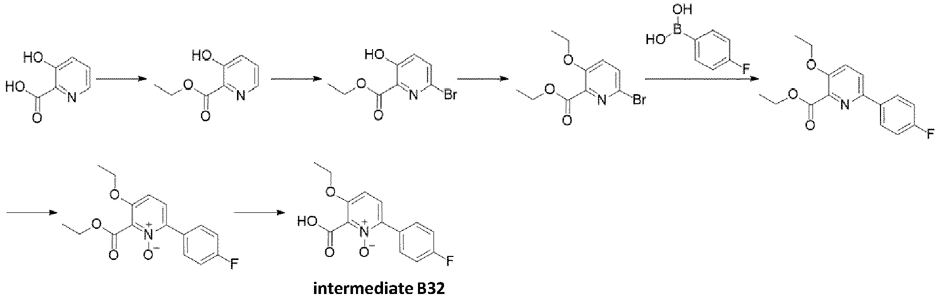

### Step 1: ethyl 3-hydroxypicolinate

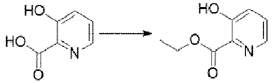

3-hydroxypicolinic acid (10 g, 72 mmol) was dissolved in ethanol (20 mL) at room temperature, concentrated sulfuric acid (10 mL) was slowly added dropwise in an ice bath, and the mixture was raised to 80 °C and stirred for 16 h. After the reaction was completed through TLC monitoring, and then cooled to room temperature, the reaction solution was slowly poured into ice water, the pH was adjusted to 8 with saturated sodium bicarbonate solution, the mixture was extracted with ethyl acetate (100 mL × 3), the organic phases were combined and washed once with saturated brine, dried over anhydrous sodium sulfate, filtered, the filtrate was concentrated under reduced pressure, and the residue was purified by silica gel column chromatography with an eluent system of methanol/dichloromethane = 1/100 to give ethyl 3-hydroxypicolinate (5 g, yellow oil), yield: 40.8 percent. MS (ESI⁺)m/z = 168.0 [M+H]⁺.

### Step 2: ethyl 6-bromo-3-hydroxypicolinate

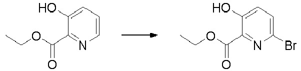

Ethyl 3-hydroxypicolinate (4.0 g, 23.9 mmol) and distilled water (40 mL) were added sequentially to a 250 mL round bottom flask at room temperature, and bromine (3.82 g, 23.9 mmol) was added slowly in ice bath, the reaction system was warmed to room temperature and stirred for 16 h. After the reaction was completed through TLC monitoring, the reaction solution was slowly poured into ice water, adjusted the pH to 8 with saturated sodium carbonate solution, then extracted with ethyl acetate (50 mL × 3), the organic phases were combined and washed once with saturated brine, dried over anhydrous sodium sulfate, filtered, the filtrate was concentrated under reduced pressure, and the residue was purified by high performance liquid chromatography with an eluent system of acetonitrile/water/0.1% TFA to give ethyl 6-bromo-3-hydroxypicolinate (2.4 g, white solid), yield: 40.1 percent. MS (ESI⁺)m/z = 245.9 [M+H]⁺.

### Step 3: ethyl 6-bromo-3-ethoxypicolinate

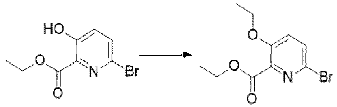

Ethyl 6-bromo-3-hydroxypicolinate (2.4 g, 9.8 mmol), potassium carbonate (2.71 g, 19.6 mmol) and DMF (25 mL) were added sequentially to a dry 50 mL round bottom flask at room temperature, iodoethane (3.06 g, 19.6 mmol) was slowly added dropwise and stirred at room temperature for 3 h. After the reaction was completed through TLC monitoring, water (50 mL) was added, the mixed solution was extracted with ethyl acetate (100 mL × 3), the organic phases were combined and washed three times with saturated brine, dried over anhydrous sodium sulfate, filtered, the filtrate was concentrated under reduced pressure, and the residue was purified by silica gel column chromatography with an eluent system of ethyl acetate/petroleum ether = 1/20 to give ethyl 6-bromo-3-ethoxypicolinate (2.5 g, white solid), yield: 89.8 percent. MS (ESI⁺)m/z = 275.9 [M+H]⁺.

### Step 4: ethyl 3-ethoxy-6-(4-fluorophenyl)picolinate

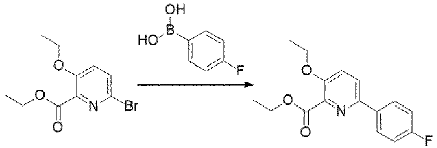

Ethyl 3-ethoxy-6-(4-fluorophenyl)picolinate (0.8 g, yellow oil) was synthesized from ethyl 6-bromo-3-ethoxypicolinate as the starting material according to the second step of synthesizing of intermediate B1 in example 0-3, yield: 72.2%. MS (ESI⁺)m/z = 290.0 [M+H]⁺.

### Step 5: 3-ethoxy-2-(ethoxycarbonyl)-6-(4-fluorophenyl)pyridine 1-oxide

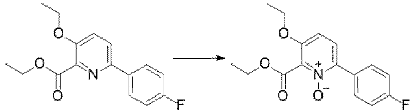

3-ethoxy-2-(ethoxycarbonyl)-6-(4-fluorophenyl)pyridine 1-oxide (320 mg, white solid) was synthesized from ethyl 3-ethoxy-6-(4-fluorophenyl)picolinate as the raw material according to the method of the fourth step of synthesizing intermediate B1 in example 0-3, yield: 72.0%. MS (ESI⁺)m/z = 306.0 [M+H]⁺.

### Step 6: 2-carboxy-3-ethoxy-6-(4-fluorophenyl)pyridine 1-oxide

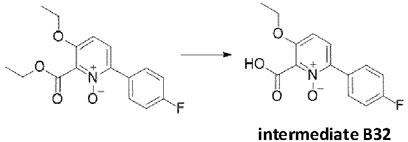

2-carboxy-3-ethoxy-6-(4-fluorophenyl)pyridine 1-oxide (210 mg, white solid) was synthesized from 3-ethoxy-2-(ethoxycarbonyl)-6-(4-fluorophenyl)pyridine 1-oxide as the raw materal in the same manner as in the third step of synthesizing of intermediate B1 in example 0-3, yield: 68.7%. MS (ESI⁺)m/z = 278.0 [M+H]⁺.

### Example 0-35. preparation of 6-carboxy-3-(dimethylamino)-2-(4-fluorophenyl)pyridine 1-oxide (intermediate B33)

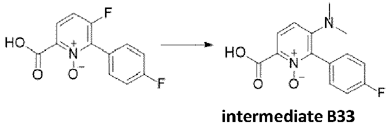

6-carboxy-3-fluoro-2-(4-fluorophenyl)pyridine 1-oxide (200 mg, 0.79 mmol) was dissolved in DMF (10 mL) in a 50 mL dry round bottom flask, 30% aqueous dimethylamine (359 mg, 2.39 mmol) was added and the reaction system was warmed to 80 °C and stirred overnight. After the reaction was completed, cooled to room temperature, the mixed solution was poured into water (30 mL), filtered, and the filter cake was washed with water and dried to obtain crude 6-carboxy-3-(dimethylamino)-2-(4-fluorophenyl)pyridine 1-oxide (160 mg, white solid), yield: 73.1%. MS(ESI⁺)m/z = 277.1 [M+H]⁺.

### Examples 0-36. Preparation of 6-carboxy-2-(4-fluorophenyl)pyrimidine 1-oxide (intermediate B34)

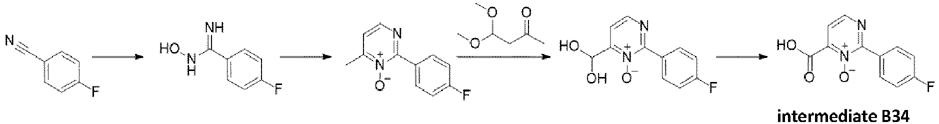

### Step 1: N-hydroxy-4-fluorobenzamidine

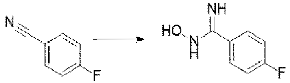

p-fluorobenzonitrile (2.0 g, 16.5 mmol), ethanol (5 mL), hydroxylamine hydrochloride (5.6 g, 80.5 mmol) and DIEA (10.8 g, 83.5 mmol) were added sequentially to a 100 mL round bottom flask at room temperature. The temperature is increased to 85 °C under nitrogen atmosphere and the mixture is stirred for 16 h. After the reaction was completed, the reaction mixture was concentrated under reduced pressure, diluted with water (50 mL), and extracted with ethyl acetate (50 mL × 3). The organic phases were combined, washed with saturated brine (30 mL), dried over anhydrous sodium sulfate, filtered, the filtrate was concentrated under reduced pressure, and the resulting residue was purified by silica gel column chromatography with an eluent of DCM/MeOH = 50/1 to give N-hydroxy-4-fluorobenzamidine (8 g, off-white solid). MS(ESI⁺)m/z = 155.0 [M+H]⁺.

### Step 2: 2-(4-fluorophenyl)-6-methylpyrimidine 1-oxide

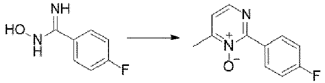

N-hydroxy-4-fluorobenzamidine (5 g, 32.4 mmol), isopropanol (100 mL), 4, 4-dimethoxy-2-butanone (4.71 g, 35.6 mmol) and trifluoroacetic acid (4.06 g, 35.6 mmol) were added sequentially to a dry 250 mL round bottom flask at room temperature and stirred at 90 °C for 16 h. After completion of the reaction, the reaction liquid was concentrated under reduced pressure, the pH of the residue was adjusted to 8 with a saturated aqueous sodium bicarbonate solution, the resulting mixture was extracted with ethyl acetate (30 mL × 3), the organic phases were combined, washed with saturated brine (20 mL), dried over anhydrous sodium sulfate, filtered, the filtrate was concentrated under reduced pressure, and the residue was purified by silica gel column chromatography with an eluent of ethyl acetate/petroleum ether = 1/5 to give 2-(4-fluorophenyl)-6-methylpyrimidine 1-oxide (1.5 g, yellow solid), yield: 22.6%. MS(ESI⁺)m/z = 205.0 [M+H]⁺.

### Step 3: 6-(dihydroxymethyl)-2-(4-fluorophenyl)pyrimidine 1-oxide

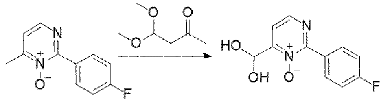

2-(4-fluorophenyl)-6-methylpyrimidine 1-oxide (300 mg, 1.47 mmol), 1,4-dioxane (5 mL) and selenium dioxide (326 mg, 2.94 mmol) were added sequentially to a 100 mL round-bottomed flask at room temperature and stirred at 100 °C for 4 h. After completion of the reaction, the mixture was adde with water (50 mL), extracted with ethyl acetate (50 mL × 3), and the organic phases were combined, washed with saturated brine (30 mL), dried over anhydrous sodium sulfate, filtered, and the filtrate was concentrated under reduced pressure to give 6-(dihydroxymethyl)-2-(4-fluorophenyl)pyrimidine 1-oxide (300 mg, yellow solid), yield: 86.4%. MS(ESI⁺)m/z = 237.0 [M+H]⁺.

### Step 4: 6-carboxy-2-(4-fluorophenyl)pyrimidine 1-oxide

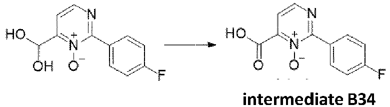

6-(dihydroxymethyl)-2-(4-fluorophenyl)pyrimidine 1-oxide (300 mg, 1.27 mmol), hydrogen peroxide (1 mL) and formic acid (3 mL) were added sequentially to a 100 mL round-bottomed flask at room temperature and stirred at room temperature for 4 h. After the reaction was completed, the mixed solution was poured into water (10 mL), filtered, and the filter cake was dried to give 6-carboxy-2-(4-fluorophenyl)pyrimidine 1-oxide (120 mg, yellow solid), yield: 40.4%. MS(ESI⁺)m/z = 235.0 [M+H]⁺.

### Examples 0-37. Preparation of 6-carboxy-3-(2,2-difluoroethoxy)-2-(4-fluorophenyl)pyridine 1-oxide intermediate B35)

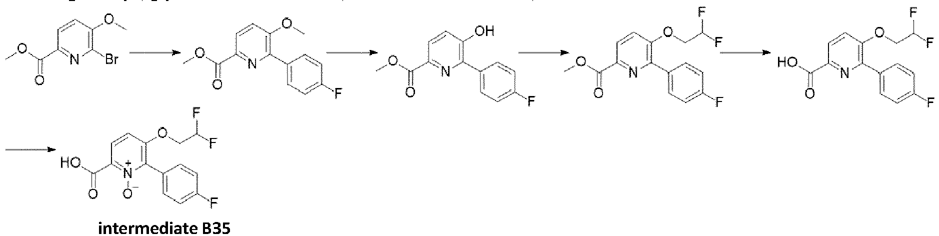

### Step 1: methyl 6-(4-fluorophenyl)-5-methoxypicolinate

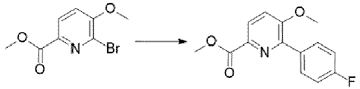

Methyl 6-(4-fluorophenyl)-5-methoxypicolinate was synthesized from methyl 6-bromo-5-methoxypicolinate according to the second step of synthesizing intermediate B1 in Example 0-3.

### Step 2: methyl 6-(4-fluorophenyl)-5-hydroxypicolinate

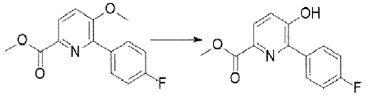

Methyl 6-(4-fluorophenyl)-5-methoxypicolinate (1.9 g, 7.27 mmol) was added to dichloromethane (40 mL) and aluminum trichloride (9.9 g, 74.4 mmol) was added in batches. The reaction system was heated to 40 °C and stirred for 48 h. After the reaction of raw materials was completely through TLC monitoring, the mixed solution was cooled to room temperature, poured into water (50 mL) under ice bath, extracted with ethyl acetate (50 mL × 3), the organic phases were combined, washed with saturated brine, dried over anhydrous sodium sulfate, concentrated under reduced pressure, and the residue was purified by silica gel column chromatography with an eluent of ethyl acetate/petroleum ether = 1/5 to give methyl 6-(4-fluorophenyl)-5-hydroxypicolinate (0.88 g, yellow solid) , yield: 48.9%.

### Step 3: methyl 5-(2,2-difluoroethoxy)-6-(4-fluorophenyl)picolinate.

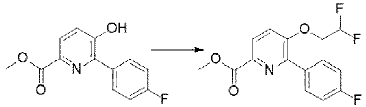

The compound methyl 6-(4-fluorophenyl)-5-hydroxypicolinate (200 mg, 0.8 mmol) was added to DMF (10 mL), followed by cesium carbonate (437 mg, 1.34 mmol) and 2,2-difluoroethyl trifluoromethanesulfonate (190 mg, 0.88 mmol), the reaction system was stirred at room temperature for 2 h, and after the reaction was completed through TLC detection, the mixed solution was poured into water (100 mL) to precipitate a white solid, filtered, and the filter cake was washed once with water and dried to obtain methyl 5-(2,2-difluoroethoxy)-6-(4-fluorophenyl)picolinate (0.25 g, white solid), yield: 99.6%.

### Step 4 and Step 5: 6-carboxy-3-(2,2-difluoroethoxy)-2-(4-fluorophenyl)pyridine 1-oxide

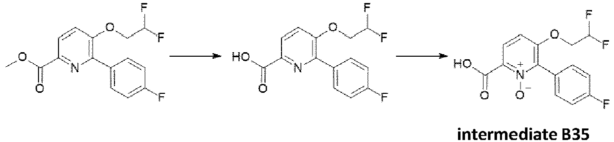

6-carboxy-3-(2,2-difluoroethoxy)-2-(4-fluorophenyl)pyridine 1-oxide was synthesized from methyl 5-(2,2-difluoroethoxy)-6-(4-fluorophenyl)picolinate as the starting material according to the third and fourth steps of the intermediate B1 synthesized in examples 0-3. MS (ESI⁺)m/z = 314.0 [M+H]⁺.

### Examples 0-38 and 0-39 preparation of intermediate B36 and intermediate B37

Intermediate B36 and intermediate B37 were prepared from sodium difluoroacetate or 2,2,2-trifluoroethyl trifluoromethanesulfonate as the raw materials by applying the method for synthesizing the intermediate B35.

| **Intermediate** | **Structure** | **MS [M+H]⁺** | **Intermediate** | **Structure** | **MS [M+H]⁺** |
|---|---|---|---|---|---|
| **B36** | 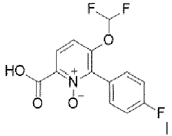 | 300.0 | **B37** | 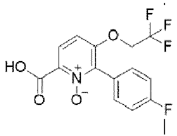 | 331.9 |

### Example 0-40. Preparation of 2-((3-chloro-4-((3-iodo-1-(4-methoxybenzyl)-1H-pyrazolo[3,4-b]pyridin-4-yl)oxy)phenyl)carbamoyl)-6-(4-fluorophenyl)pyridine1-oxide (intermediate C1)

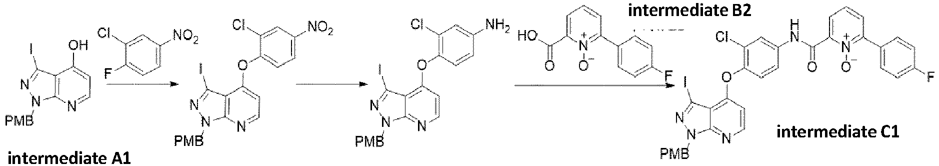

### Step 1: 4-(2-chloro-4-nitrophenoxy)-3-iodo-1-(4-methoxybenzyl)-1H-pyrazolo[3,4-b]pyridine

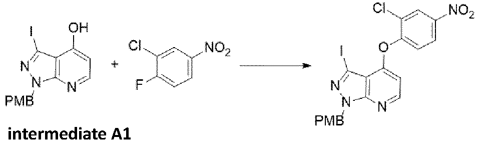

3-iodo-1-(4-methoxybenzyl)-1H-pyrazolo[3,4-b]pyridin-4-ol (3.4 g, 8.9 mmol) was dissolved in DMF (40 mL) at room temperature, followed by the addition of potassium carbonate (2.46 g, 18 mmol) and 1-fluoro-2-chloro-4-nitrobenzene (1.72 g, 9.8 mmol), the reaction system was warmed to 60 °C and stirred overnight. After the reaction was completed through TLC monitoring, the mixed solution was cooled to room temperature, poured slowly into water (150 mL), extracted with ethyl acetate (150 mL × 3), the organic phases were combined and washed with water (300 mL × 2) and saturated brine (300 mL × 1), dried over anhydrous sodium sulfate, filtered, the filtrate was concentrated under reduced pressure, and the residue was purified by silica gel column chromatography with an eluent system of dichloromethane/methanol = 200/1 to give 4-(2-chloro-4-nitrophenoxy)-3-iodo-1-(4-methoxybenzyl)-1H-pyrazolo[3,4-b]pyridine (4.1 g, yellow solid), yield: 85.6 %.

### Step 2: 3-chloro-4-((3-iodo-1-(4-methoxybenzyl)-1H-pyrazolo[3,4-b]pyridin-4-yl)oxy)aniline

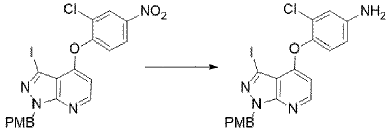

To 4-(2-chloro-4-nitrophenoxy)-3-iodo-1-(4-methoxybenzyl)-1H-pyrazolo[3,4-b]pyridine (1.0 g, 1.9 mmol) was added with saturated ammonium chloride solution (3 mL) and ethanol (15 mL) at room temperature, the temperature was raised to 80 °C, and reduced iron powder (0.53 g, 9.5 mmol) was added in batches with stirring and the mixture was stirred at 80 °C for 2 h. After the reaction was completed through TLC monitoring, the reaction system was cooled to room temperature, filtered, the filter cake was washed with dichloromethane (10 mL × 3), the filtrate was diluted with water (30 mL), extracted with dichloromethane (30 mL × 3), the organic phases were combined and washed with saturated brine (50 mL × 1), dried over anhydrous sodium sulfate, filtered, and the filtrate was concentrated under reduced pressure to give crude 3-chloro-4-((3-iodo-1-(4-methoxybenzyl)-1H-pyrazolo[3,4-b]pyridin-4-yl)oxy)aniline (0.95 g, a yellow solid). Yield: 100.0%. MS (ESI⁺)m/z = 506.9 [M+H]⁺.

### Step 3: 2-((3-chloro-4-((3-iodo-1-(4-methoxybenzyl)-1H-pyrazolo[3,4-b]pyridin-4-yl)oxy)phenyl)carbamoyl)-6-(4-fluorophenyl)pyridine 1-oxide

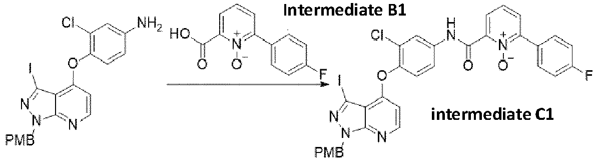

3-chloro-4-((3-iodo-1-(4-methoxybenzyl)-1H-pyrazolo[3,4-b]pyridin-4-yl)oxy)aniline (300 mg, 0.59 mmol), intermediate B2(165.7 mg, 0.71 mmol), HATU (675.3 mg, 1.78 mmol), TEA (239.6 mg, 2.37 mmol) and N, N-dimethylformamide (5 mL) were sequentially added into a 50 mL round bottom flask at room temperature and stirred at room temperature for 6 h. After the reaction was completed through TLC monitoring, diluted with water (20 mL) and the mixed solution was extracted with ethyl acetate (20 mL × 3). The organic phases were combined and washed with water (40 mL × 3) and saturated brine (40 mL × 1), dried over anhydrous sodium sulfate, filtered, the filtrate was concentrated under reduced pressure, and the residue was purified by silica gel column chromatography with an eluent system of ethyl acetate/petroleum ether = 1/1 to give 2-((3-chloro-4-((3-iodo-1-(4-methoxybenzyl)-1H-pyrazolo[3,4-b]pyridin-4-yl)oxy)phenyl)carbamoyl)-6-(4-fluorophenyl)pyridine 1-oxide (380 mg, yellow solid), yield: 88.9%. MS (ESI+)m/z = 721.9 [M+H]⁺.

### Example 0-41. Preparation of 2-((3-fluoro-4-((3-iodo-1-(4-methoxybenzyl)-1H-pyrazolo[3,4-b]pyridin-4-yl)oxy)phenyl)carbamoyl)-6-(4-fluorophenyl)pyridine 1-oxide (intermediate C2)

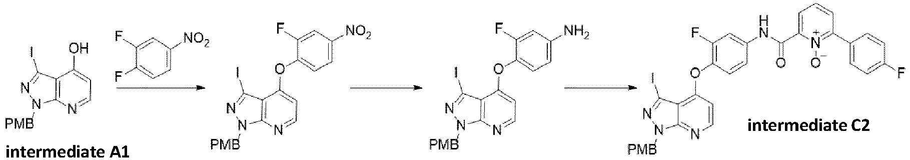

Intermediate C2 was synthesizd from intermediate A1 and 1, 2-difluoro-4-nitrobenzene as the raw materials according to the synthesis method of intermediate C1.

### Example 1 (R)-2-((3-fluoro-4-((3-((1-hydroxypropan-2-yl)amino)-1H-pyrazolo[3,4-b]pyridin-4-yl)oxy)phenyl)carbamoyl)-6-(4-fluorophenyl)pyridine 1-oxide (final product 2)

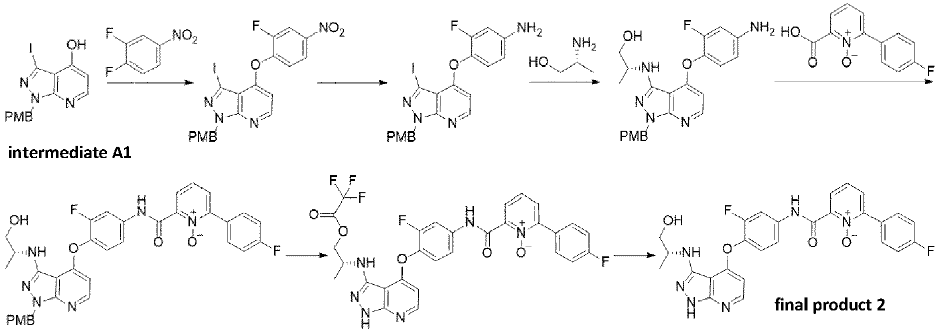

### Step 1 and Step 2: 3-fluoro-4-((3-iodo-1-(4-methoxybenzyl)-1H-pyrazolo[3,4-b]pyridin-4-yl)oxy) aniline

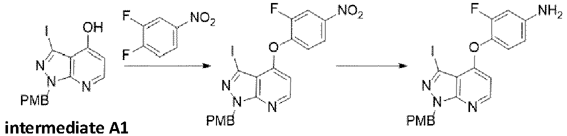

3-fluoro-4-((3-iodo-1-(4-methoxybenzyl)-1H-pyrazolo[3,4-b]pyridin-4-yl)oxy) aniline was synthesized from intermediate A1 and 1, 2-difluoro-4-nitrobenzene as the starting materials according to the method of the first and second steps of example 0-40.

### Step 3: (R)-2-((4-(4-amino-2-fluorophenoxy)-1-(4-methoxybenzyl)-1H-pyrazolo [3,4-b] pyridin-3-yl)amino)-1-propanol

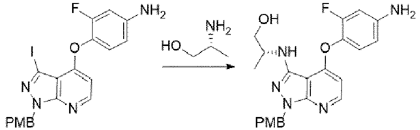

3-fluoro-4-((3-iodo-1-(4-methoxybenzyl)-1H-pyrazolo[3,4-b]pyridin-4-yl)oxy) aniline (15 g, 30.6 mmol) was dissolved in DMSO (150 mL), and (R)-aminopropanol (6.9 g, 91.8 mmol), cuprous iodide (2.3 g, 12.2 mmol), potassium carbonate (12.7 g, 91.8 mmol), and pyrrole-2-carboxylic acid (1.7 g, 15.3 mmol) were added to the mixed solution in sequence under nitrogen atmosphere, the reaction system was replaced with nitrogen three times, warmed to 100 °C and stirred overnight. After the reaction was completed through TLC monitoring, the temperature was reduced to room temperature, water (150 mL) was added, the mixed solution was extracted with ethyl acetate (200 mL × 3), the organic phases were combined and washed with saturated brine (100 mL × 3), dried over anhydrous sodium sulfate, filtered, the filtrate was concentrated under reduced pressure, and the residue was purified by silica gel column chromatography with an eluent system of ethyl acetate/petroleum ether = 1/2 to give (R)-2-((4-(4-amino-2-fluorophenoxy)-1-(4-methoxybenzyl)-1H-pyrazolo[3,4-b]pyridin-3-yl)amino)-1-propanol (9 g, yellow solid) yield: 63.7%. MS (ESI⁺)m/z = 438.2 [M+H]⁺.

### Step 4: (R)-2-((3-fluoro-4-((3-((1-hydroxypropan-2-yl)amino)-1-(4-methoxybenzyl)-1H-pyrazolo[3,4-b]pyridin-4-yl)oxy)phenyl)carbamoyl)-6-(4-fluorophenyl)pyridine 1-oxide

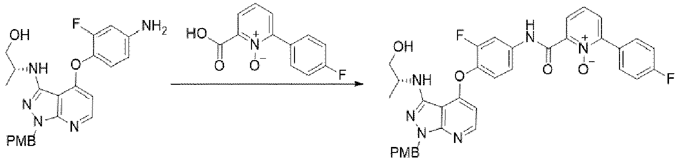

(R)-2-((4-(4-amino-2-fluorophenoxy)-1-(4-methoxybenzyl)-1H-pyrazolo[3,4-B]pyridin-3-yl)amino)-1-propanol (13.5 g, 30.9 mmol) and 2-carboxy-6-(4-fluorophenyl)pyridine 1-oxide (intermediate B2, 7.93 g, 34.0 mmol) were dissolved in DMF (135 mL) at room temperature and HOBt (6.26 g, 46.4 mmol), EDCI (8.89 g, 46.4 mmol) and TEA (6.25 g, 61.8 mmol) were added sequentially and stirred at room temperature overnight. After the reaction was completed through TLC monitoring, water (200 mL) was added, extracted with ethyl acetate (200 mL × 3), the organic phases were combined and washed with saturated brine (150 mL × 2), anhydrous sodium sulfate, filtered, the filtrate was concentrated under reduced pressure, and the residue was purified by silica gel column chromatography with an eluent system of methanol/dichloromethane = 1/200 to give (R)-2-((3-fluoro-4-((3-((1-hydroxypropan-2-yl)amino)-1-(4-methoxybenzyl)-1H-pyrazolo[3,4-b]pyridin-4-yl)oxy)phenyl)carbamoyl)-6-(4-fluorophenyl)pyridine 1-oxide (11 g, light yellow solid), yield: 51.8 %. MS (ESI⁺)m/z = 653.1 [M+H]⁺.

### Step 5: (R)-2-((3-fluoro-4-((3-((1-(2,2,2-trifluoroacetyloxy)propan-2-yl)amino)-1H-pyrazolo[3,4-b]pyridin-4-yl)oxy)phenyl)carbamoyl)-6-(4-fluorophenyl)pyridine 1-oxide

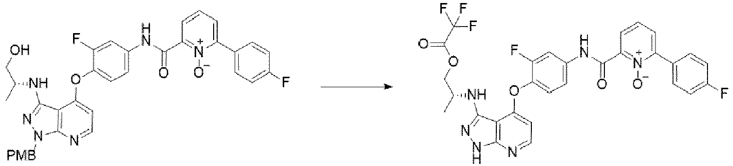

(R)-2-((3-fluoro-4-((3-((1-hydroxypropan-2-yl)amino)-1-(4-methoxybenzyl)-1H-pyrazolo[3,4-b]pyridin-4-yl)oxy)phenyl)carbamoyl)-6-(4-fluorophenyl)pyridine 1-oxide (16 g, 24.5 mmol) was dissolved in TFA (100 mL) at room temperature and stirred overnight at room temperature. After the reaction was completed through TLC monitoring, concentrated under reduced pressure to give crude (R)-2-((3-fluoro-4-((3-((1-(2,2,2-trifluoroacetoxy)propan-2-yl)amino)-1H-pyrazolo[3,4-b]pyridin-4-yl)oxy)phenyl)carbamoyl)-6-(4-fluorophenyl)pyridine 1-oxide (16 g, yellow oil). yield: 93.5%. MS (ESI⁺)m/z = 629.2 [M+H]⁺.

### Step 6: (R)-2-((3-fluoro-4-((3-((1-hydroxypropan-2-yl)amino)-1H-pyrazolo[3,4-b]pyridin-4-yl)oxy)phenyl)carbamoyl)-6-(4-fluorophenyl)pyridine 1-oxide

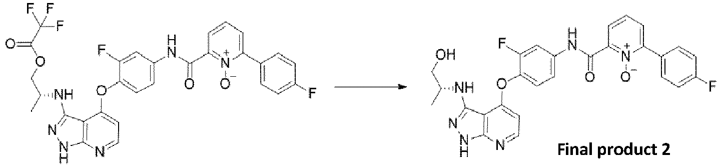

(R)-2-((3 -fluoro-4-((3 -((1-(2,2,2-trifluoroacetoxy)propan-2-yl)amino)-1H-pyrazolo[3,4-b]pyridin-4-yl)oxy)phenyl)carbamoyl)-6-(4-fluorophenyl)pyridine 1-oxide (16 g, 25.5 mmol) was dissolved in EtOH (160 mL) at room temperature followed by addition of potassium carbonate (35.24 g, 255 mmol) and stirred at room temperature for 2 h. After the reaction was completed through TLC monitoring, water (200 mL) was added, extraction was performed with ethyl acetate (200 mL × 3), the organic phases were combined and washed with saturated brine (200 mL × 2), dried over anhydrous sodium sulfate, filtered, and the filtrate was concentrated under reduced pressure to give a crude product. The crude product was purified by silica gel column chromatography with an eluent system of methanol/dichloromethane = 1/100 to give (R)-2-((3-fluoro-4-((3-((1-hydroxypropan-2-yl)amino)-1H-pyrazolo[3,4-b]pyridin-4-yl)oxy)phenyl)carbamoyl)-6-(4-fluorophenyl)pyridine 1-oxide (12.0 g).

Light yellow solid, yield 87.5%. MS (ESI⁺)m/z = 533.0 [M+H]⁺. ¹H NMR (400 MHz, CDCl₃) *δ* 14.05 (s, 1H), 8.60 (dd, *J* = 5.6, 4.8 Hz, 1H), 8.24 (d, *J* = 5.2 Hz, 1H), 8.02 (dd, *J* = 12.0, 2.8 Hz, 1H), 7.79-7.75 (m, 2H), 7.62 (dd, *J* = 6.0, 1.2 Hz, 2H), 7.50-7.47 (m, 1H), 7.32-7.25 (m, 4H), 6.13 (dd, *J* = 5.6, 0.8 Hz, 1H), 4.72 (d, *J* = 4.4 Hz, 1H), 4.05-4.04 (m, 1H), 3.84 (dd, *J* = 11.2, 3.6 Hz, 1H), 3.78-3.73 (m, 1H), 1.37 (d, *J* = 6.8 Hz, 3H).

### Examples 2-108 preparation of final products 3, 5-6, 8-12, 15-18, 20, 23-25, 27, 29-36, 42-50, 52-54, 56-67, 69-70, 79-83, 85-87, 89, 91-99, 103, 113-129, 133-135, 138-139, 141-142, 145-146, 149, 151-155, 157-160, 174

Final products 3, 5-6, 8-12, 15-18, 20, 23-25, 27, 29-36, 42-50, 52-54, 56-67, 69-70, 79-83, 85-87, 89, 91-99, 103, 113-129, 133-135, 138-139, 141-142, 145-146, 149-151-155, 157-160 and 174 were synthesized from intermediate A, commercially available fluoro-nitro (hetero) aryl compounds, commercially available hydroxylamine compounds and intermediate B as the raw materials according to the method for synthesizing final product 2. (Table 2)

**Table 2 final products 3, 5-6, 8-12, 15-18, 20, 23-25, 27, 29-36, 42-50, 52-54, 56-67, 69-70, 79-83, 85-87, 89, 91-99, 103, 113-129, 133-135, 138-139, 141-142, 145-146, 149, 151-155, 157-160, 174-160**

| **Final product Nos.** | **Inter medi ate Nos.** | **Structures of Final products** | **NMR or MS** |
|---|---|---|---|
| **Final product 3** | **B2** | 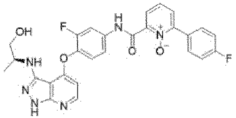 | White-like solid, yield: 52.0%. MS (ESI⁺)m/z = 532.9 [M+H]⁺. ¹H NMR (400 MHz, DMSO-*d*₆) *δ* 13.53 (s, 1H), 12.16 (s, 1H), 8.28 (dd, *J* = 8.0, 2.4 Hz, 1H), 8.10 (d, *J* = 5.6 Hz, 1H), 8.02 (dd, *J* = 12.7, 2.4 Hz, 1H), 7.87-7.83 (m, 3H), 7.70 (t, *J* = 8.0 Hz, 1H), 7.60 (d, *J* = 8.0 Hz, 1H), 7.47 (t, *J* = 8.9 Hz, 1H), 7.38 (t, *J* = 12.0 Hz, 2H), 5.99 (d, *J* = 5.4 Hz, 1H), 5.09 (d, *J* = 8.0 Hz, 1H), 4.78 (t, *J* = 4.0 Hz, 1H), 3.80-3.72 (m, 1H), 3.48-3.43 (m, 2H), 1.19-1.17 (m, 3H). |
| **Final product 5** | **B2** | 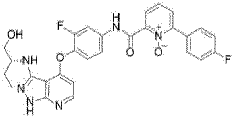 | MS (ESI⁺)m/z = 547.0 [M+H]⁺. ¹H NMR (400 MHz, DMSO-*d*₆) *δ* 13.58 (s, 1H), 12.16 (s, 1H), 8.33 (dd, *J* = 8.4, 2.4 Hz, 1H), 8.15 (d, *J* = 5.6 Hz, 1H), 8.05 (dd, *J* = 12.4, 2.4 Hz, 1H), 7.91-7.87 (m, 3H), 7.75 (t, *J* = 8.0 Hz, 1H), 7.64 (dd, *J* = 8.8, 1.6 Hz, 1H), 7.51 (t, *J* = 8.8 Hz, 1H), 7.42-7.37 (m, 2H), 6.05 (d, *J* = 5.2 Hz, 1H), 5.05 (d , *J* = 7.6 Hz, 1H), 4.72 (t, *J* = 5.6 Hz, 1H), 3.64-3.59 (m, 1H), 3.58-3.53 (m, 2H), 1.68-1.62 (m, 2H), 0.93 (t, *J* = 7.2 Hz, 3H). |
| **Final product 6** | **B2** | 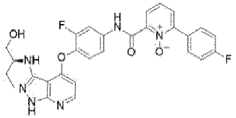 | Yellow solid, yield: 27.1%. MS (ESI⁺)m/z = 547.1 [M+H]⁺. ¹H NMR (400 MHz, DMSO-*d*₆) *δ* 13.58 (s, 1H), 12.17 (s, 1H), 8.33 (d, *J* = 8.0 Hz, 1H), 8.15 (d, *J* = 5.6 Hz, 1H), 8.06 (d, *J* = 12.4 Hz, 1H), 7.91-7.87 (m, 3H), 7.75 (t, *J* = 8.0 Hz, 1H), 7.64 (d, *J* = 8.4 Hz, 1H), 7.52 (t, *J* = 8.8 Hz, 1H), 7.40 (t, *J* = 8.8 Hz, 2H), 6.05 (d, *J* = 5.2 Hz, 1H), 5.06 (d , *J* = 7.6 Hz, 1H), 4.74 (t, *J* = 5.2 Hz, 1H), 3.64-3.52 (m, 3H), 1.68-1.61 (m, 2H), 0.93 (t, *J* = 7.2 Hz, 3H). |
| **Final product 8** | **B2** | 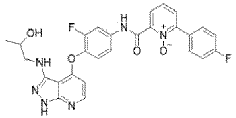 | MS (ESI⁺)m/z = 533.2 [M+H]⁺. ¹H NMR (400 MHz, CD₃OD) *δ* 8.45 (dd, *J* = 8.0, 2.4 Hz, 1H), 8.13 (d, *J* = 5.6 Hz, 1H), 8.05 (dd, *J* = 12.4, 2.4 Hz, 1H), 7.88-7.83 (m, 2H), 7.79 (dd, *J* = 8.0, 2.4 Hz, 1H), 7.73 (t, *J* = 7.9 Hz, 1H), 7.50 (dd, *J* = 2.4, 1.2 Hz, 1H), 7.40 (t, *J* = 8.8 Hz, 1H), 7.26 (t, *J* = 8.8 Hz, 2H), 6.11 (dd, *J* = 5.6, 1.0 Hz, 1H), 3.45 (dd, *J* = 13.2, 4.0 Hz, 1H), 3.22 (dd, *J* = 12.0, 8.0 Hz, 2H), 1.22 (d, *J* = 4.0 Hz, 3H). |
| **Final product 9** | **B2** | 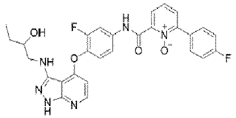 | Pale yellow solid, yield: 18.0%. MS (ESI⁺)m/z = 547.0 [M+H]⁺. ¹H NMR (400 MHz, CD₃OD) *δ* 8.45 (dd, *J* = 7.9, 2.2 Hz, 1H), 8.17-7.96 (m, 1H), 8.05 (dd, *J* = 12.4, 2.4 Hz, 1H), 7.88-7.83 (m, 2H), 7.81-7.77 (m, 1H), 7.73 (t, *J* = 7.9 Hz, 1H), 7.53-7.49 (m, 1H), 7.40 (t, *J* = 8.7 Hz, 1H), 7.26 (t, *J* = 8.8 Hz, 2H), 6.12 (d, *J* = 4.0 Hz, 1H), 3.80-3.73 (m, 1H), 3.55-3.46 (m, 1H), 3.24-3.16 (m, 1H), 1.64-1.55 (m, 1H), 1.53-1.46 (m, 1H), 0.99 (t, *J* = 8.0 Hz, 3H). |
| **Final product 10** | **B2** | 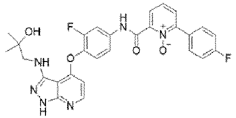 | MS (ESI⁺)m/z = 546.9 [M+H]⁺. ¹H NMR (400 MHz, DMSO-*d*₆) *δ* 13.58 (s, 1H), 12.20 (s, 1H), 8.32 (dd, *J* = 8.0, 2.1 Hz, 1H), 8.17 (s, 1H), 8.07 (d, *J* = 12.0 Hz, 1H), 7.91-7.86 (m, 3H), 7.74 (t, *J* = 7.9 Hz, 1H), 7.65 (d, *J* = 8.5 Hz, 1H), 7.54 (d, *J* = 8.8 Hz, 1H), 7.39 (t, *J* = 8.8 Hz, 2H), 6.07 (d, *J* = 5.2 Hz, 1H), 3.25 (s, 2H), 1.17 (s, 6H). |
| **Final product 11** | **B2** | 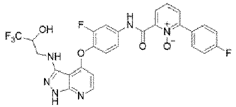 | Pale yellow solid, yield: 24.3%. MS (ESI⁺)m/z = 586.9 [M+H]⁺. ¹H NMR (400 MHz, CD₃OD) *δ* 8.45 (dd, *J* = 8.0, 2.4 Hz, 1H), 8.14 (d, *J* = 5.6 Hz, 1H), 8.04 (dd, *J* = 12.4, 2.4 Hz, 1H), 7.88-7.83 (m, 2H), 7.79 (dd, *J* = 8.0, 2.4 Hz, 1H), 7.73 (t, *J* = 8.0 Hz, 1H), 7.56-7.47 (m, 1H), 7.39 (t, *J* = 8.8 Hz, 1H), 7.30-7.23 (m, 2H), 6.12 (dd, *J* = 5.6, 0.9 Hz, 1H), 4.41-4.29 (m, 1H), 3.79 (dd, *J* = 12.0, 2.4 Hz, 1H), 3.58-3.37 (m, 1H). |
| **Final product 12** | **B2** | 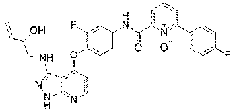 | MS (ESI⁺)m/z = 544.7 [M+H]⁺. ¹H NMR (400 MHz, CD₃OD) *δ* 8.45 (dd, *J* = 8.0, 2.4 Hz, 1H), 8.15-8.14 (m, 1H), 8.04 (dd, *J* = 12.4, 2.4 Hz, 1H), 7.88-7.83 (m, 2H), 7.79 (dd, *J* = 8.0, 2.4 Hz, 1H), 7.73 (t, *J* = 8.0 Hz, 1H), 7.56-7.47 (m, 1H), 7.39 (t, *J* = 8.8 Hz, 1H), 7.30-7.23 (m, 2H), 6.13 (dd, *J* = 5.6, 0.9 Hz, 1H), 5.95-5.91 (m, 1H), 5.33 (d, *J* = 16.0 Hz, 1H), 5.15 (d, *J* = 12.0 Hz, 1H), 4.41-4.38 (m, 1H), 3.53 (dd, *J* = 12.0, 4.0 Hz, 1H), 3.31-3.30 (m, 1H). |
| **Final product 15** | **B2** | 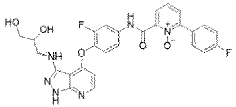 | Pale yellow solid, yield: 32.7%. MS (ESI⁺)m/z = 548.9 [M+H]⁺. ¹H NMR (400 MHz, DMSO-*d*₆) *δ* 13.56 (s, 1H), 12.21 (s, 1H), 8.32 (dd, *J* = 8.0, 2.1 Hz, 1H), 8.15 (d, *J* = 5.4 Hz, 1H), 8.06 (dd, *J* = 12.6, 2.3 Hz, 1H), 7.93-7.87 (m, 3H), 7.74 (t, *J* = 7.9 Hz, 1H), 7.64 (d, *J* = 8.8 Hz, 1H), 7.51 (t, *J* = 8.9 Hz, 1H), 7.39 (t, *J* = 8.9 Hz, 2H), 6.05 (d, *J* = 4.0 Hz, 1H), 3.78-3.72 (m, 1H), 3.40-3.38 (m, 3H), 3.22-3.16 (m, 1H). |
| **Final product 16** | **B2** | 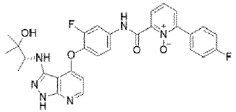 | White solid, yield: 14.9%. MS (ESI⁺)m/z = 561.1 [M+H]⁺. ¹H NMR (400 MHz, CD₃OD) *δ* 8.49 (dd, *J* = 8.0, 2.4 Hz, 1H), 8.17 (d, *J* = 5.6 Hz, 1H), 8.09 (dd, *J* = 12.4, 2.4 Hz, 1H), 7.92-7.88 (m, 2H), 7.83 (d, *J* = 8.0, 2.4 Hz, 1H), 7.77 (t, *J* = 8.0 Hz, 1H), 7.55 (dd, *J* = 8.8, 1.2 Hz, 1H), 7.45 (t, *J* = 8.8 Hz, 1H), 7.30 (t, *J* = 8.8 Hz, 2H), 6.16 (d, *J* = 5.6 Hz, 1H), 3.83-3.81 (m, 1H), 1.33-1.30 (m, 9H). |
| **Final product 17** | **B2** | 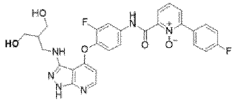 | MS (ESI⁺)m/z = 563.0 [M+H]⁺. ¹H NMR (400 MHz, CD₃OD) *δ* 8.49 (dd, *J* = 8.0, 2.0 Hz, 1H), 8.16 (d, *J* = 5.6 Hz, 1H), 8.07 (dd, *J* = 12.4, 2.4 Hz, 1H), 7.91-7.88 (m, 2H), 7.83 (dd, *J* = 7.6, 2.4 Hz, 1H), 7.77 (t, *J* = 8.0 Hz, 1H), 7.56-7.53 (m, 1H), 7.44 (t, *J* = 8.8 Hz, 1H), 7.33-7.28 (m, 2H), 6.15 (d, *J* = 5.2 Hz, 1H), 3.75-3.65 (m, 4H), 3.53-3.50 (m, 2H), 2.09-2.06 (m, 1H). |
| **Final product 18** | **B2** | 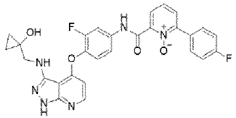 | MS (ESI⁺)m/z = 544.9 [M+H]⁺. ¹H NMR (400 MHz, DMSO-*d*₆) *δ* 13.56 (s, 1H), 12.22 (s, 1H), 8.32 (dd, *J* = 8.0, 2.1 Hz, 1H), 8.17 (d, *J* = 5.5 Hz, 1H), 8.07 (dd, *J* = 12.7, 2.4 Hz, 1H), 7.91-7.87 (m, 3H), 7.74 (t, *J* = 7.9 Hz, 1H), 7.67-7.63 (m, 1H), 7.53 (t, *J* = 8.9 Hz, 1H), 7.39 (dd, *J* = 12.4, 5.5 Hz, 2H), 6.08 (d, *J* = 5.4 Hz, 1H), 5.98 (t, *J* = 5.6 Hz, 1H), 4.09 (d, *J* = 5.7 Hz, 2H), 2.55 (dd, *J* = 8.7, 5.8 Hz, 2H), 0.96 (t, *J* = 8.7 Hz, 2H). |
| **Final product 20** | **B2** | 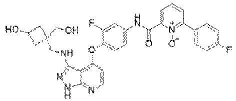 | MS (ESI⁺)m/z = 589.0 [M+H]⁺. ¹H NMR (400 MHz, DMSO-*d*₆) *δ* 13.57 (s, 1H), 12.14 (s, 1H), 8.33 (dd, *J* = 8.0, 2.0 Hz, 1H), 8.15 (d, *J* = 5.2 Hz, 1H), 8.05 (dd, *J* = 12.4, 2.0 Hz, 1H), 7.90-7.86 (m, 3H), 7.75 (t, *J* = 8.0 Hz, 1H), 7.63 (d, *J* = 8.4 Hz, 1H), 7.51 (t, *J* = 8.8 Hz, 1H), 7.40 (t, *J* = 8.8 Hz, 2H), 6.04 (d, *J* = 5.6 Hz, 1H), 5.77-5.71 (m, 1H), 5.00-4.90 (m, 2H), 4.19-4.15 (m, 1H), 4.07-4.02 (m, 1H), 2.16-2.05 (m, 2H), 1.73-1.68 (m, 1H), 1.63-1.58 (m, 1H). |
| **Final product 23** | **B2** | 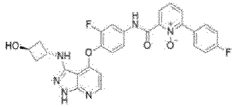 | MS (ESI⁺)m/z = 544.7 [M+H]⁺. ¹H NMR (400 MHz, CD₃OD) *δ* 8.45 (dd, *J* = 8.0, 2.4 Hz, 1H), 8.15-8.14 (m, 1H), 8.07 (dd, *J* = 12.4, 2.4 Hz, 1H), 7.88-7.83 (m, 2H), 7.79 (dd, *J* = 8.0, 2.4 Hz, 1H), 7.73 (t, *J* = 8.0 Hz, 1H), 7.56-7.47 (m, 1H), 7.39 (t, *J* = 8.8 Hz, 1H), 7.30-7.23 (m, 2H), 6.18 (d, *J* = 5.6 Hz, 1H), 4.49-4.47 (m, 1H), 4.27-4.23 (m, 1H), 2.46-2.35 (m, 4H). |
| **Final product 24** | **B2** | 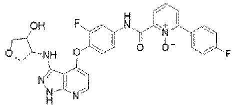 | MS (ESI⁺)m/z = 561.0 [M+H]⁺. ¹H NMR (400 MHz, DMSO-*d*₆) *δ* 13.57 (s, 1H), 12.33 (s, 1H), 8.32 (dd, *J* = 8.0, 2.0 Hz, 1H), 8.17 (d, *J* = 5.2 Hz, 1H), 8.05 (dd, *J* = 8.4, 2.0 Hz, 1H), 7.90-7.86 (m, 3H), 7.75 (t, *J* = 8.0 Hz, 1H), 7.63 (d, *J* = 8.4 Hz, 1H), 7.50 (t, *J* = 8.8 Hz, 1H), 7.39 (t, *J* = 8.8 Hz, 2H), 6.06 (d, *J* = 5.2 Hz, 1H), 5.58 (brs, 1H), 4.34 (t, *J* = 2.4 Hz, 1H), 4.09-4.05 (m, 1H), 3.99 (brs, 1H), 3.94-3.91 (m, 1H), 3.75 (dd, *J* = 8.8, 3.2 Hz, 1H), 3.56-3.52 (m, 1H). |
| **Final product 25** | **B2** | 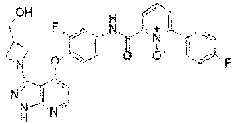 | MS (ESI⁺)m/z = 545.0 [M+H]⁺. ¹H NMR (400 MHz, CDCl₃) *δ* 14.05 (s, 1H), 8.60 (dd, *J* = 6.0, 4.8 Hz, 1H), 8.26 (d, *J* = 5.6 Hz, 1H), 8.02 (dd, *J* = 12.4, 2.4 Hz, 1H), 7.80-7.76 (m, 2H), 7.64-7.62 (m, 2H), 7.47 (d, *J* = 7.6 Hz, 1H), 7.30-7.26 (m, 3H), 6.19 (d, *J* = 5.2 Hz, 1H), 4.32 (t, *J* = 8.0 Hz, 2H), 4.04 (dd, *J* = 8.0, 5.6 Hz, 2H), 3.93 (d, *J* = 6.4 Hz, 2H), 2.99-2.96 (m, 1H). |
| **Final product 27** | **B2** | 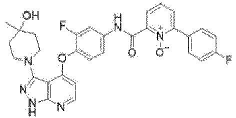 | MS (ESI⁺)m/z = 573.2 [M+H]⁺. ¹H NMR (400 MHz, DMSO-*d*₆) *δ* 13.58 (s, 1H), 12.63 (s, 1H), 8.33 (dd, *J* = 8.0, 2.2 Hz, 1H), 8.21 (d, *J* = 5.4 Hz, 1H), 8.06 (dd, *J* = 12.7, 2.4 Hz, 1H), 7.91-7.85 (m, 3H), 7.74 (t, *J* = 7.9 Hz, 1H), 7.69-7.58 (m, 1H), 7.50 (t, *J* = 8.9 Hz, 1H), 7.44-7.34 (m, 2H), 6.18 (d, *J* = 8.9 Hz, 1H), 4.25 (s, 1H), 3.47-3.44 (m, 2H), 3.29-3.18 (m, 2H), 1.68-1.55 (m, 4H), 1.15 (s, 3H). |
| **Final product 29** | **B2** | 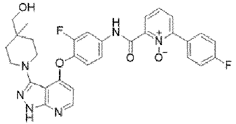 | White solid, yield: 13.1%. MS (ESI⁺)m/z = 587.1 [M+H]⁺. ¹H NMR (400 MHz, CDCl₃) *δ* 14.04 (s, 1H), 8.61 (dd, *J* = 6.0, 4.8 Hz, 1H), 8.29 (d, *J* = 5.6 Hz, 1H), 8.03 (dd, *J* = 12.0, 2.0 Hz, 1H), 7.80-7.75 (m, 2H), 7.63-7.62 (m, 2H), 7.49-7.46 (m, 1H), 7.32-7.25 (m, 3H), 6.24 (d, *J* = 5.6 Hz, 1H), 3.73-3.68 (m, 2H), 3.48 (s, 2H), 3.34-3.27 (m, 2H), 1.81-1.74 (m, 2H), 1.54-1.51 (m, 2H), 1.08 (s, 3H). |
| **Final product 30** | **B2** | 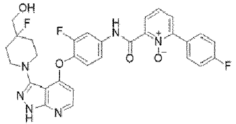 | Yellow solid, yield: 15.2%. MS (ESI⁺)m/z = 591.1 [M+H]⁺. ¹H NMR (400 MHz, DMSO-*d*₆) *δ* 13.60 (s, 1H), 12.73 (s, 1H), 8.34 (dd, *J* = 8.0, *J* = 2.0 Hz, 1H), 8.23 (d, *J* = 5.6 Hz, 1H), 8.07 (dd, *J* = 12.8, 2.4 Hz, 1H), 7.92-7.87 (m, 3H), 7.75 (t, *J* = 8.0 Hz, 1H), 7.64 (d, *J* = 10.4 Hz, 1H), 7.53 (t, *J* = 8.8 Hz, 1H), 7.40 (t, *J* = 8.8 Hz, 2H), 6.19 (d, *J* = 5.6 Hz, 1H), 4.96 (t , *J* = 6.0 Hz, 1H), 3.73 (d, *J* = 8.0 Hz, 2H), 3.47 (dd, *J* = 20.0, 6.0 Hz, 1H), 3.42 (d, *J* = 5.6 Hz, 1H), 3.09-3.06 (m, 2H), 1.89-1.78 (m, 4H). |
| **Final product 31** | **B2** | 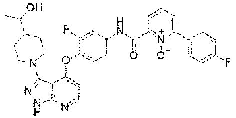 | White solid, yield: 44.3%. MS (ESI⁺)m/z = 587.2 [M+H]⁺. ¹H NMR (400 MHz, DMSO-*d*₆) *δ* 13.60 (s, 1H), 12.65 (s, 1H), 8.33 (dd, *J* = 8.0, 2.0 Hz, 1H), 8.23 (d, *J* = 5.2 Hz, 1H), 8.06 (dd, *J* = 12.4, 2.0 Hz, 1H), 7.93-7.86 (m, 3H), 7.75 (t, *J* = 8.0 Hz, 1H), 7.63 (dd, *J* = 8.8, 1.2 Hz, 1H), 7.55-7.48 (t, *J* = 9.2 Hz, 1H), 7.39 (t, *J* = 8.8 Hz, 2H), 6.17 (d, *J* = 5.2 Hz, 1H), 4.36 (d, *J* = 5.2 Hz, 1H), 3.93-3.92 (m, 2H), 3.44-3.36 (m, 1H), 2.78-2.66 (m, 2H), 1.87-1.85 (m, 1H), 1.65-1.61 (m, 1H), 1.48-1.30 (m, 3H), 1.04 (d, *J* = 6.4 Hz, 3H). |
| **Final product 32** | **B2** | 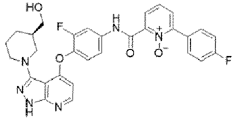 | MS (ESI⁺)m/z = 573.1 [M+H]⁺. ¹H NMR (400 MHz, DMSO-*d*₆) *δ* 13.58 (s, 1H), 12.65 (s, 1H), 8.34 (dd, *J* = 8.4, 2.4 Hz, 1H), 8.22 (d, *J* = 5.6 Hz, 1H), 8.06 (dd, *J* = 12.4, 1.6 Hz, 1H), 7.91-7.87 (m, 3H), 7.75 (t, *J* = 8.0 Hz, 1H), 7.62 (d, *J* = 9.2 Hz, 1H), 7.50 (t, *J* = 8.8 Hz, 1H), 7.40 (t, *J* = 9.2 Hz, 2H), 6.17 (d, *J* = 5.6 Hz, 1H), 4.44 (t, *J* = 5.6 Hz, 1H), 3.87 (d, *J* = 9.6 Hz, 1H), 3.75 (d, *J* = 12.4 Hz, 1H), 3.30-3.27 (m, 2H), 2.85-2.75 (m, 1H), 2.57 (t, *J* = 11.2 Hz, 1H), 1.78-1.71 (m, 3H), 1.63-1.60 (m, 1H), 1.14-1.11 (m, 1H). |
| **Final product 33** | **B2** | 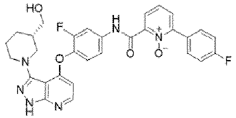 | White solid, yield: 28.7%. MS (ESI⁺)m/z = 573.0 [M+H]⁺. ¹H NMR (400 MHz, CD₃OD) *δ* 8.49 (dd, *J* = 8.0, 2.4 Hz, 1H), 8.22 (d, *J* = 5.6 Hz, 1H), 8.08 (dd, *J* = 12.4, 2.4 Hz, 1H), 7.91-7.88 (m, 2H), 7.83 (dd, *J* = 8.0, 2.4 Hz, 1H), 7.76 (t, *J* = 8.0 Hz, 1H), 7.55-7.53 (m, 1H), 7.42 (t, *J* = 8.8 Hz, 1H), 7.30 (t, *J* = 8.8 Hz, 2H), 6.26 (d, *J* = 5.2 Hz, 1H), 4.02 (d, *J* = 10.0 Hz, 1H), 3.84 (d, *J* = 11.6 Hz, 1H), 3.54-3.47 (m, 2H), 2.97-2.88 (m, 1H), 2.69 (t, *J* = 10.4 Hz, 1H), 2.00-1.72 (m, 4H), 1.27-1.18 (m, 1H). |
| **Final product 34** | **B2** | 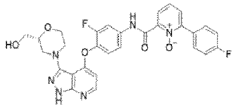 | MS (ESI⁺)m/z = 575.1 [M+H]⁺. ¹H NMR (400 MHz, CDCl₃) *δ* 14.15 (s, 1H), 8.60 (t, *J* = 5.2 Hz, 1H), 8.28 (brs, 1H), 8.09 (dd, *J* = 12.0, 2.4 Hz, 1H), 7.81-7.75 (m, 2H), 7.64 (d, *J* = 5.6 Hz, 2H), 7.51 (d, *J* = 7.6 Hz, 1H), 7.32-7.25 (m, 3H), 6.39 (d, *J* = 6.0 Hz, 1H), 4.07 (d, *J* = 11.2 Hz, 1H), 3.95-3.82 (m, 4H), 3.75-3.70 (m, 2H), 3.22-3.12 (m, 1H), 3.02 (t, *J* = 12.4 Hz, 1H). |
| **Final product 35** | **B2** | 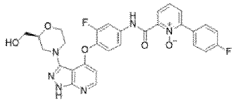 | MS (ESI⁺)m/z = 575.0 [M+H]⁺. ¹H NMR (400 MHz, CDCl₃) *δ* 14.05 (s, 1H), 8.61 (t, *J* = 5.6 Hz, 1H), 8.29 (d, *J* = 5.6 Hz, 1H), 8.03 (dd, *J* = 12.0, 2.4 Hz, 1H), 7.78 (dd, *J* = 8.8, 5.2 Hz, 2H), 7.63 (d, *J* = 4.8 Hz, 2H), 7.48 (d, *J* = 8.4 Hz, 1H), 7.32-7.29 (m, 1H), 7.28-7.25 (m, 2H), 6.27 (d, *J* = 5.6 Hz, 1H), 4.06 (d, *J* = 10.8 Hz, 1H), 3.95-3.82 (m, 4H), 3.75-3.70 (m, 2H), 3.22-3.12 (m, 1H), 2.97 (d, *J* = 12.0 Hz, 1H). |
| **Final product 36** | **B2** | 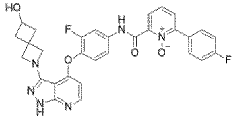 | MS (ESI⁺)m/z = 570.9 [M+H]⁺. ¹H NMR (400 MHz, CD₃OD) *δ* 8.49 (dd, *J* = 10.0, 2.0 Hz, 1H), 8.21 (d, *J* = 5.6 Hz, 1H), 8.09 (dd, *J* = 14.8, 2.4 Hz, 1H), 7.91-7.88 (m, 2H), 7.83 (dd, *J* = 10, 2.4 Hz, 1H), 7.78 (t, *J* = 15.6 Hz, 1H), 7.55 (d, *J* = 8.8 Hz, 1H), 7.42 (t, *J* = 8.8 Hz, 1H), 7.31 (t, *J* = 8.8 Hz, 2H), 6.21 (d, *J* = 5.6 Hz 1H), 4.62 (s, 1H), 4.17-4.10 (m, 4H), 2.61-2.57 (m, 2H), 2.15-2.10 (m, 2H). |
| **Final product 42** | **B21** | 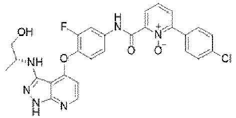 | MS (ESI⁺)m/z = 550.1 [M+H]⁺. ¹H NMR (600 MHz, DMSO-*d*₆) *δ* 13.51 (s, 1H), 12.20 (s, 1H), 8.33 (dd, *J* = 8.0, 2.2 Hz, 1H), 8.14 (d, *J* = 5.5 Hz, 1H), 8.05 (dd, *J* = 12.7, 2.4 Hz, 1H), 7.90 (dd, *J* = 7.9, 2.2 Hz, 1H), 7.88-7.83 (m, 2H), 7.74 (t, *J* = 7.9 Hz, 1H), 7.65-7.59 (m, 3H), 7.51 (t, *J* = 8.9 Hz, 1H), 6.03 (d, *J* = 5.4 Hz, 1H), 5.13 (d, *J =* 7.2 Hz, 1H), 4.81 (t, *J* = 5.4 Hz, 1H), 3.83-3.74 (m, 1H), 3.52-3.48 (m, 2H), 1.22 (d, *J* = 6.5 Hz, 3H). |
| **Final product 43** | **B16** | 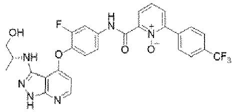 | MS (ESI⁺)m/z = 583.6 [M+H]⁺. ¹H NMR (600 MHz, DMSO-*d*₆) *δ* 13.45 (s, 1H), 12.19 (s, 1H), 8.37 (dd, *J* = 8.0, 2.0 Hz, 1H), 8.15 (d, *J* = 5.4 Hz, 1H), 8.10-8.05 (m, 3H), 7.95-7.92 (m, 3H), 7.79 (t, *J* = 8.8 Hz, 1H), 7.64 (d, *J* = 8.8 Hz, 1H), 7.52 (t, *J* = 8.9 Hz, 1H), 6.05 (d, *J* = 4.8 Hz, 1H), 5.39 (t, *J* = 12.0 Hz, 1H), 4.82 (d, *J* = 4.8 Hz, 1H), 4.00-3.83 (m, 1H), 3.28-3.25 (m, 1H), 3.18-3.05 (m, 1H), 1.12 (d, *J* = 6.2 Hz, 3H). |
| **Final product 44** | **B12** | 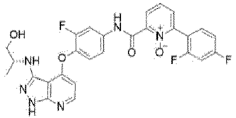 | Yellow solid, yield: 24.3%. MS (ESI⁺)m/z = 551.1 [M+H]⁺. ¹H NMR (400 MHz, CD₃OD) *δ* 8.56 (dd, *J* = 8.0, 2.8 Hz 1H), 8.16 (d, *J* = 5.6 Hz, 1H), 8.08 (dd, *J* = 12.4, 2.4 Hz, 1H), 7.85-7.75 (m, 2H), 7.71-7.63 (m, 1H), 7.56-7.51 (m, 1H), 7.43 (t, *J* = 8.8 Hz, 1H), 7.25-7.15 (m, 2H), 6.15 (dd, *J* = 5.6, 0.8 Hz, 1H), 4.00-3.90 (m, 1H), 3.76-3.64 (m, 2H), 1.33 (d, *J* = 6.8 Hz, 3H). |
| **Final product 45** | **B15** | 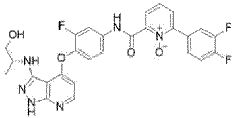 | MS (ESI⁺)m/z = 551.6 [M+H]⁺. ¹H NMR (600 MHz, DMSO-*d*₆) *δ* 13.43 (s, 1H), 12.17 (s, 1H), 8.33 (dd, *J* = 8.0, 2.1 Hz, 1H), 8.15 (d, *J* = 5.4 Hz, 1H), 8.06 (dd, *J* = 12.6, 2.4 Hz, 1H), 8.05-8.00 (m, 1H), 7.93 (dd, *J* = 7.9, 2.1 Hz, 1H), 7.75 (t, *J* = 7.9 Hz, 1H), 7.72-7.58 (m, 3H), 7.51 (t, *J* = 7.9 Hz, 1H), 6.04 (d, *J* = 5.4 Hz, 1H), 5.45-5.37 (m, 1H), 4.80 (s, 1H), 4.08-3.90 (m, 1H), 3.29-3.25 (m, 1H), 3.20-3.01 (m, 1H), 1.12 (d, *J* = 6.2 Hz, 3H) |
| **Final product 46** | **B14** | 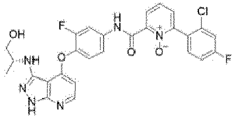 | MS (ESI⁺)m/z = 567.1 [M+H]⁺. ¹H NMR (600 MHz, DMSO-*d*₆) *δ* 13.60 (s, 1H), 12.17 (s, 1H), 8.46 (dd, *J* = 8.0, 2.2 Hz, 1H), 8.14 (d, *J* = 5.4 Hz, 1H), 8.06 (dd, *J* = 12.6, 2.4 Hz, 1H), 7.88 (dd, *J* = 7.7, 2.2 Hz, 1H), 7.79 (t, *J* = 7.9 Hz, 1H), 7.70-7.65 (m, 3H), 7.50 (t, *J* = 8.9 Hz, 1H), 7.45-7.40 (m, 1H), 6.03 (d, *J* = 5.3 Hz, 1H), 5.45-5.35 (m, 1H), 4.81 (d, *J* = 4.2 Hz, 1H), 3.95-3.85 (m, 1H), 3.29-3.25 (m, 1H), 3.17-3.06 (m, 1H), 1.11 (d, *J* = 6.2 Hz, 3H). |
| **Final product 47** | **B13** | 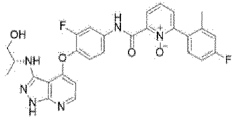 | MS (ESI⁺)m/z = 547.4 [M+H]⁺. ¹H NMR (600 MHz, DMSO-*d*₆) *δ* 13.74 (s, 1H), 12.17 (s, 1H), 8.42 (dd, *J* = 7.8, 2.4 Hz, 1H), 8.14 (d, *J* = 5.4 Hz, 1H), 8.06 (dd, *J* = 12.6, 2.4 Hz, 1H), 7.84-7.73 (m, 2H), 7.66 (dd, *J* = 8.9, 1.4 Hz, 1H), 7.50 (t, *J* = 8.9 Hz, 1H), 7.39 (dd, *J* = 8.4, 6.0 Hz, 1H), 7.26 (dd, *J* = 10.1, 2.5 Hz, 1H), 7.20-7.15 (m, 1H), 6.03 (d, *J* = 5.4 Hz, 1H), 5.41-5.38 (m, 1H), 4.80 (d, *J* = 4.8 Hz, 1H), 3.95-3.91 (m, 1H), 3.29-3.25 (m, 1H), 3.15-3.09 (m, 1H), 2.18 (s, 3H), 1.11 (d, *J* = 6.3 Hz, 3H) |
| **Final product 48** | **B17** | 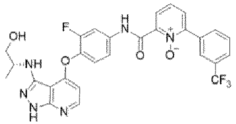 | MS (ESI⁺)m/z = 583.6 [M+H]⁺. ¹H NMR (600 MHz, DMSO-*d*₆) *δ* 13.41 (s, 1H), 12.17 (s, 1H), 8.35 (dd, *J* = 8.0, 2.1 Hz, 1H), 8.22 (s, 1H), 8.14 (d, *J* = 5.4 Hz, 1H), 8.09-8.01 (m, 2H), 7.97 (dd, *J* = 7.8, 2.1 Hz, 1H), 7.90 (d, *J* = 7.9 Hz, 1H), 7.80-7.70 (m, 2H), 7.64 (d, *J* = 8.9 Hz, 1H), 7.51 (t, *J* = 8.9 Hz, 1H), 6.04 (d, *J* = 5.4 Hz, 1H), 5.39 (t, *J* = 12.0 Hz, 1H), 4.81 (d, *J* = 4.8 Hz, 1H), 4.03-3.84 (m, 1H), 3.28-3.25 (m, 1H), 3.14-3.09 (m, 1H), 1.12 (d, *J* = 6.2 Hz, 3H). |
| **Final product 49** | **B18** | 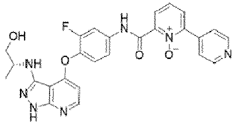 | Light yellow solid, yield: 10.1%. MS (ESI⁺)m/z = 516.0 [M+H]⁺. ¹H NMR (400 MHz, CD₃OD) *δ* 8.76 (d, *J* = 6.0 Hz, 2H), 8.56 (dd, *J* = 8.0, 2.4 Hz, 1H), 8.18 (d, *J* = 5.6 Hz, 1H), 8.09 (dd, *J* = 12.4, 2.4 Hz, 1H), 7.94-7.89 (m, 3H), 7.82 (t, *J* = 8.0 Hz, 1H), 7.58-7.53 (m, 1H), 7.44 (t, *J* = 8.4 Hz, 1H), 6.15 (d, *J* = 5.6 Hz, 1H), 4.00-3.90 (m, 1H), 3.76-3.64 (m, 2H), 1.33 (d, *J* = 6.4 Hz, 3H). |
| **Final product 50** | **B19** | 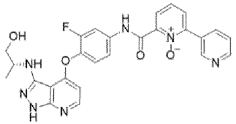 | Yellow solid, yield: 6.6%. MS (ESI⁺)m/z = 516.0 [M+H]⁺. ¹H NMR (400 MHz, CD₃OD) *δ* 9.01 (d, *J* = 1.6 Hz, 1H), 8.71 (dd, *J* = 4.8, 1.2 Hz, 1H), 8.55 (dd, *J* = 8.0, 2.4 Hz, 1H), 8.38-8.34 (m, 1H), 8.17 (d, *J* = 6.4 Hz, 1H), 8.09 (dd, *J* = 12.4, 2.4 Hz, 1H), 7.92 (dd, *J* = 8.0, 2.4 Hz, 1H), 7.81 (t, *J* = 8.0 Hz, 1H), 7.68-7.63 (m, 1H), 7.59-7.53 (m, 1H), 7.44 (t, *J* = 8.8 Hz, 1H), 6.15 (d, *J* = 5.2 Hz, 1H), 4.78 (brs, 1H), 4.00-3.90 (m, 1H), 3.76-3.64 (m, 2H), 1.33 (d, *J* = 6.4 Hz, 3H) |
| **Final product 52** | **B20** | 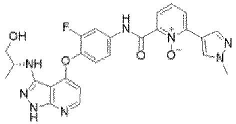 | MS (ESI⁺)m/z = 519.6 [M+H]⁺. ¹H NMR (600 MHz, DMSO-*d*₆) *δ* 13.68 (s, 1H), 12.18 (s, 1H), 8.94 (s, 1H), 8.45 (s, 1H), 8.30 (d, *J* = 5.0 Hz, 1H), 8.15 (d, *J* = 5.0 Hz, 1H), 8.12 (dd, *J* = 7.8, 2.0 Hz, 1H), 8.06 (dd, *J* = 12.6, 2.4 Hz, 1H), 7.67 (t, *J* = 8.0 Hz, 1H), 7.60 (dd, *J* = 8.8, 1.8 Hz, 1H), 7.54 (t, *J* = 8.8 Hz, 1H), 6.06 (d, *J* = 6.4 Hz, 1H), 5.51-5.32 (m, 1H), 4.81 (d, *J* = 4.8 Hz, 1H), 4.00-3.90 (m, 4H), 3.25-3.28 (m, 1H), 3.17-3.06 (m, 1H), 1.12 (d, *J* = 6.2 Hz, 3H). |
| **Final product 53** | **B3** | 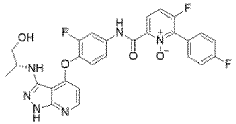 | White solid, yield: 11.5%. MS (ESI⁺)m/z = 550.9 [M+H]⁺. ¹H NMR (400 MHz, CD₃OD) *δ* 8.56-8.52 (m, 1H), 8.16 (d, *J* = 5.6 Hz, 1H), 8.05 (dd, *J* = 12.8, 2.4 Hz, 1H), 7.73-7.69 (m, 3H), 7.54-7.51 (m, 1H), 7.42 (t, *J* = 8.4 Hz, 1H), 7.36-7.32 (m, 2H), 6.14 (d, *J* = 5.6 Hz, 1H), 3.95-3.94 (m, 1H), 3.72-3.66 (m, 2H), 1.32 (d, *J* = 6.8 Hz, 3H). |
| **Final product 54** | **B3** | 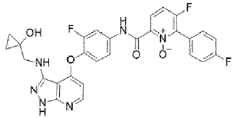 | MS (ESI⁺)m/z = 563.0 [M+H]⁺. ¹H NMR (400 MHz, DMSO-*d*₆) *δ* 13.17 (s, 1H), 12.21 (s, 1H), 8.40 (t, *J* = 8.0 Hz, 1H), 8.16 (d, *J* = 5.4 Hz, 1H), 8.05 (dd, *J* = 12.6, 2.4 Hz, 1H), 7.84 (t, *J* = 8.2 Hz, 1H), 7.72-7.67 (m, 2H), 7.65 (d, *J* = 8.5 Hz, 1H), 7.51 (t, *J* = 9.0 Hz, 1H), 7.43 (t, *J* = 8.4 Hz, 2H), 6.06 (d, *J* = 5.3 Hz, 1H), 5.96 (t, *J* = 5.6 Hz, 1H), 4.09 (d, *J* = 5.4 Hz, 2H), 2.55 (dd, *J* = 8.7, 5.8 Hz, 2H), 0.96 (t, *J* = 8.7 Hz, 2H). |
| **Final product 56** | **B22** | 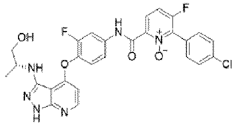 | MS (ESI⁺)m/z = 568.1 [M+H]⁺. ¹H NMR (400 MHz, DMSO-*d*₆) *δ* 13.14 (s, 1H), 12.19 (s, 1H), 8.41 (t, *J* = 8.0 Hz, 1H), 8.14 (d, *J* = 5.3 Hz, 1H), 8.05 (dd, *J* = 12.5, 2.3 Hz, 1H), 7.85 (t, *J* = 8.2 Hz, 1H), 7.78-7.56 (m, 5H), 7.50 (t, *J* = 8.7 Hz, 1H), 6.03 (d, *J* = 5.2 Hz, 1H), 5.11 (d, *J* = 7.1 Hz, 1H), 4.81-4.77 (m, 1H), 3.82-3.76 (m, 1H), 3.55-3.48 (m, 2H), 1.22 (d, *J* = 6.4 Hz, 3H). |
| **Final product 57** | **B1** | 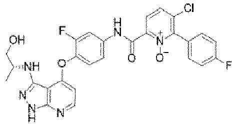 | MS (ESI⁺)m/z = 567.1 [M+H]⁺. ¹H NMR (600 MHz, DMSO-*d*₆) *δ* 13.22 (s, 1H), 12.19 (s, 1H), 8.35 (d, *J* = 8.9 Hz, 1H), 8.15 (s, 1H), 8.05 (d, *J* = 12.6 Hz, 1H), 7.98 (d, *J* = 8.9 Hz, 1H), 7.66 (d, *J* = 8.4 Hz, 1H), 7.59 (dd, *J* = 8.7, 5.5 Hz, 2H), 7.50 (t, *J* = 8.9 Hz, 1H), 7.42 (t, *J* = 8.9 Hz, 2H), 6.04 (d, *J* = 5.4 Hz, 1H), 5.35-5.31 (m, 1H), 3.99-3.90 (m, 1H), 3.29-3.28 (m, 1H), 3.11-3.07(m, 1H), 1.10 (d, *J* = 6.8 Hz, 3H). |
| **Final product 58** | **B1** | 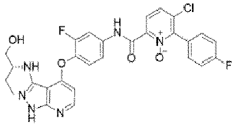 | MS (ESI⁺)m/z = 581.2 [M+H]⁺. ¹H NMR (600 MHz, DMSO-*d*₆) *δ* 13.23 (s, 1H), 12.15 (s, 1H), 8.35 (d, *J* = 8.9 Hz, 1H), 8.13 (d, *J* = 5.5 Hz, 1H), 8.04 (d, *J* = 12.7 Hz, 1H), 7.97 (d, *J* = 8.9 Hz, 1H), 7.65 (d, *J* = 9.4 Hz, 1H), 7.62-7.55 (m, 2H), 7.49 (t, *J* = 8.9 Hz, 1H), 7.41 (t, *J* = 8.8 Hz, 2H), 6.02 (d, *J* = 5.2 Hz, 1H), 5.04 (d, *J* = 7.8 Hz, 1H), 4.72 (t, *J* = 5.2 Hz, 1H), 3.66-3.48 (m, 3H), 1.70-1.60 (m, 2H), 0.91 (t, *J* = 7.4 Hz, 3H). |
| **Final product 59** | **B1** | 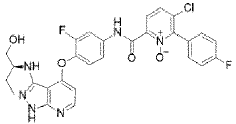 | MS (ESI⁺)m/z = 582.1 [M+H]⁺. ¹H NMR (600 MHz, DMSO) *δ* 13.23 (s, 1H), 12.16 (s, 1H), 8.35 (d, *J* = 8.9 Hz, 1H), 8.14 (d, *J* = 5.4 Hz, 1H), 8.04 (d, *J* = 10.4 Hz, 1H), 7.98 (d, *J* = 8.9 Hz, 1H), 7.66 (d, *J* = 8.1 Hz, 1H), 7.61-7.57 (m, 2H), 7.50 (t, *J* = 8.9 Hz, 1H), 7.42 (t, *J* = 8.9 Hz, 2H), 6.03 (d, *J* = 5.5 Hz, 1H), 5.06-5.02 (m, 1H), 3.56-3.52 (m, 3H), 1.69-1.59 (m, 2H), 0.92 (t, *J* = 7.4 Hz, 3H). |
| **Final product 60** | **B1** | 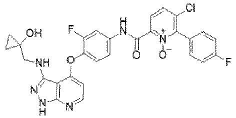 | MS (ESI⁺)m/z = 579.2 [M+H]⁺. ¹H NMR (400 MHz, DMSO-*d*₆) *δ* 13.21 (s, 1H), 12.20 (s, 1H), 8.34 (d, *J* = 9.0 Hz, 1H), 8.15 (d, *J* = 5.0 Hz, 1H), 8.04 (dd, *J* = 12.7, 2.4 Hz, 1H), 7.96 (d, *J* = 8.8 Hz, 1H), 7.66 (d, *J* = 9.0 Hz, 1 H), 7.60-7.55 (m, 2H), 7.50 (t, *J* = 8.8 Hz, 1H), 7.41 (t, *J* = 8.4 Hz, 2H), 6.05 (d, *J* = 4.9 Hz, 1H), 5.95 (t, *J* = 5.1 Hz, 1H), 4.08 (d, *J* = 5.3 Hz, 2H), 2.55-2.51 (m, 2H), 0.94 (t, *J* = 7.0 Hz, 2H). |
| **Final product 61** | **B1** | 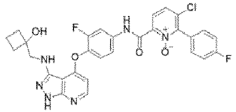 | Yellow solid, yield: 22.6%. MS (ESI⁺)m/z = 593.0 [M+H]⁺. ¹H NMR (400 MHz, CD₃OD) *δ* 8.48 (d, *J* = 8.8 Hz, 1H), 8.23 (d, *J* = 6.0 Hz, 1H), 8.09 (dd, *J* = 12.4, 2.4 Hz, 1H), 7.93 (d, *J* = 8.8 Hz, 1H), 7.58-7.52 (m, 3H), 7.47 (t, *J* = 8.8 Hz, 1H), 7.34 (t, *J* = 8.8 Hz, 2H), 6.21 (d, *J* = 6.0 Hz, 1H), 3.57 (s, 2H), 2.24-2.18 (m, 2H), 2.16-2.08 (m, 2H), 1.82-1.79 (m, 1H), 1.70-1.63 (m, 1H). |
| **Final product 62** | **B1** | 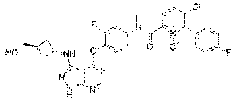 | MS (ESI⁺)m/z = 594.2 [M+H]⁺. ¹H NMR (400 MHz, DMSO-*d*₆) *δ* 13.22 (s, 1H), 12.14 (s, 1H), 8.35 (d, *J* = 8.9 Hz, 1H), 8.12 (dd, *J* = 5.5, 1.1 Hz, 1H), 8.04 (dd, *J* = 12.7, 2.5 Hz, 1H), 7.98 (d, *J* = 8.8 Hz, 1H), 7.65 (dd, *J* = 8.8, 2.4 Hz, 1H), 7.63-7.56 (m, 2H), 7.55-7.45 (m, 3H), 6.01 (d, *J* = 5.5 Hz, 1H), 5.67 (d, *J* = 7.7 Hz, 0.5H), 5.59 (d, *J* = 7.7 Hz, 0.5H), 4.56-4.53 (m, 0.5H), 4.45-4.43 (m, 0.5H), 4.45-4.42 (m, 0.5H), 4.09-4.03 (m, 0.5H), 3.49-3.46 (m, 1H), 3.39-3.37 (m, 1H), 2.13-2.09 (m, 2H), 1.81-1.69 (m, 1H), 0.90-0.81 (m, 2H). |
| **Final product 63** | **B1** | 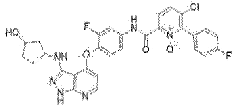 | Yellow solid, yield: 35.1%. MS (ESI⁺)m/z = 593.0 [M+H]⁺. ¹H NMR (400 MHz, DMSO-*d*₆) *δ* 13.25 (s, 1H), 8.36 (d, *J =* 8.8 Hz, 1H), 8.19 (s, 1H), 8.06 (dd, *J =* 12.4, 2.0 Hz, 1H), 7.99 (d, *J* = 8.8 Hz, 1H), 7.70-7.56 (m, 3H), 7.50 (t, *J =* 8.8 Hz, 1H), 7. 42 (t, *J =* 8.8 Hz, 2H), 6.05 (d, *J* = 5.2 Hz, 1H), 4.19-4.13 (m, 1H), 4.12-4.06 (m, 1H), 2.17-2.09 (m, 1H), 2.03-1.94 (m, 1H), 1.84-1.56 (m, 4H). |
| **Final product 64** | **B1** | 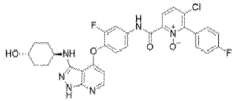 | MS (ESI⁺)m/z = 607.2 [M+H]⁺. ¹H NMR (400 MHz, DMSO-*d*₆) *δ* 13.22 (s, 1H), 12.14 (s, 1H), 8.35 (d, *J* = 8.9 Hz, 1H), 8.12 (d, *J* = 5.4 Hz, 1H), 8.03 (dd, *J* = 12.7, 2.4 Hz, 1H), 7.97 (d, *J =* 8.9 Hz, 1H), 7.67-7.55 (m, 3H), 7.52-7.36 (m, 3H), 6.01 (dd, *J =* 5.4, 1.1 Hz, 1H), 5.04 (d, *J =* 8.0 Hz, 1H), 4.52 (d, *J =* 4.4 Hz, 1H), 3.65-3.37 (m, 2H), 2.06 (d, *J* = 11.8 Hz, 2H), 1.84 (d, *J* = 11.5 Hz, 2H), 1.38-1.20 (m, 4H). |
| **Final product 65** | **B23** | 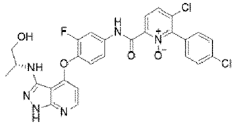 | MS (ESI⁺)m/z = 584.1 [M+H]⁺. ¹H NMR (400 MHz, DMSO-*d*₆) *δ* 13.21 (s, 1H), 12.19 (s, 1H), 8.36 (d, *J* = 8.9 Hz, 1H), 8.14 (d, *J* = 5.4 Hz, 1H), 8.04 (dd, *J* = 12.7, 2.5 Hz, 1H), 7.98 (d, *J* = 8.9 Hz, 1H), 7.68-7.63 (m, 3H), 7.59-7.53 (m, 2H), 7.51 (t, *J* = 8.9 Hz, 1H), 6.02 (dd, *J* = 5.4, 1.1 Hz, 1H), 5.11 (d, *J* = 7.2 Hz, 1H), 4.79 (t, *J* = 5.5 Hz, 1H), 3.85-3.73 (m, 1H), 3.49-3.47 (m, 2H), 1.21 (d, *J* = 6.4 Hz, 3H). |
| **Final product 66** | **B25** | 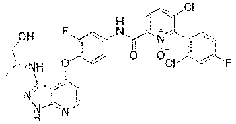 | MS (ESI⁺)m/z = 602.1 [M+H]⁺. ¹H NMR (400 MHz, DMSO-*d*₆) *δ* 13.11 (s, 1H), 12.20 (s, 1H), 8.45 (d, *J* = 8.9 Hz, 1H), 8.14 (d, *J* = 5.4 Hz, 1H), 8.09-8.03 (m, 2H), 7.75 (dd, *J* = 8.9, 2.5 Hz, 1H), 7.72-7.65 (m, 2H), 7.55-7.46 (m, 2H), 6.02 (d, *J* = 5.4 Hz, 1H), 5.13 (d, *J* = 7.2 Hz, 1H), 4.81 (t, *J* = 5.5 Hz, 1H), 3.80-3.75 (m, 1H), 3.52-3.47 (m, 2H), 1.21 (d, *J* = 6.4 Hz, 3H). |
| **Final product 67** | **B4** | 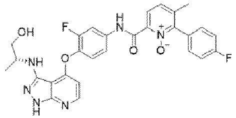 | MS (ESI⁺)m/z = 547.0 [M+H]⁺. ¹H NMR (400 MHz, CDCl₃) *δ* 13.89 (s, 1H), 8.51 (d, *J* = 8.0 Hz, 1H), 8.23 (d, *J* = 5.2 Hz, 1H), 7.99 (dd, *J* = 12.0, 2.4 Hz, 1H), 7.52 (d, *J* = 8.4 Hz, 1H), 7.46-7.23 (m, 6H), 6.11 (d, *J* = 5.2 Hz, 1H), 4.71-4.69 (m, 1H), 4.05-4.04 (m, 1H), 3.83 (dd, *J* = 11.2, 3.6 Hz, 1H), 3.75 (dd, *J* = 11.2, 6.4 Hz, ¹H ), 2.24 (s, 3H), 1.36 (d, *J* = 6.8 Hz, 3H). |
| **Final product 69** | **B4** | 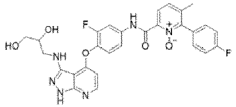 | MS (ESI⁺)m/z = 562.8 [M+H]⁺. ¹H NMR (400 MHz, DMSO-*d*₆) *δ* 13.84 (s, 1H), 12.23 (s, 1H), 8.33 (d, *J* = 8.3 Hz, 1H), 8.16 (d, *J* = 5.3 Hz, 1H), 8.06 (dd, *J* = 12.7, 2.3 Hz, 1H), 7.71 (d, *J* = 8.9 Hz, 1H), 7.65 (d, *J* = 10.0 Hz, 1H), 7.54-7.47 (m, 3H), 7.39 (t, *J* = 12.3, 5.5 Hz, 2H), 6.05 (d, *J* = 5.1 Hz, 1H), 3.73-3.42 (m, 3H), 3.21-3.16 (m, 2H), 2.14 (s, 3H). |
| **Final product 70** | **B4** | 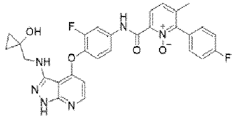 | White solid, yield: 43.8%. MS (ESI⁺)m/z = 558.7 [M+H]⁺. ¹H NMR (400 MHz, DMSO-*d*₆) *δ* 13.84 (s, 1H), 12.21 (s, 1H), 8.33 (d, *J* = 8.0 Hz, 1H), 8.16 (d, *J* = 8.0 Hz, 1H), 8.07 (dd, *J* = 12.0, 4.0 Hz, 1H), 7.72 (dd, *J* = 8.0, 0.5 Hz, 1H), 7.64-7.59 (m, 1H), 7.52-7.45 (m, 3H), 7.42-7.31 (m, 2H), 6.06 (dd, *J* = 8.0, 4.0 Hz, 1H), 5.96 (t, *J* = 4.0 Hz, 1H), 4.09 (d, *J* = 8.0 Hz, 2H), 2.14 (s, 3H), 0.96 (t, *J* = 4.0 Hz, 4H). |
| **Final product 79** | **B24** | 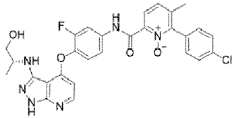 | MS (ESI⁺)m/z = 563.1 [M+H]⁺. ¹H NMR (600 MHz, DMSO-*d*₆) *δ* 13.80 (s, 1H), 12.19 (s, 1H), 8.32 (d, *J* = 2.0 Hz, 1H), 8.13 (d, *J* = 5.4 Hz, 1H), 8.04 (dd, *J* = 12.6, 2.3 Hz, 1H), 7.71 (d, *J* = 8.5 Hz, 1H), 7.65-7.60 (m, 3H), 7.52-7.46 (m, 3H), 6.02 (d, *J* = 5.3 Hz, 1H), 5.11 (d, *J* = 7.2 Hz, 1H), 4.80 (s, 1H), 3.86-3.72 (m, 1H), 3.52-3.47 (m, 2H), 2.14 (s, 3H), 1.21 (d, *J* = 6.5 Hz, 3H). |
| **Final product 80** | **B5** | 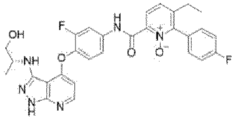 | MS (ESI⁺)m/z = 561.4 [M+H]⁺. ¹H NMR (500 MHz, DMSO-*d*₆) *δ* 13.84 (s, 1H), 12.22 (s, 1H), 8.38 (d, *J* = 8.4 Hz, 1H), 8.15 (d, *J* = 5.4 Hz, 1H), 8.07 (dd, *J* = 12.7, 2.3 Hz, 1H), 7.76 (d, *J* = 8.5 Hz, 1H), 7.65 (t, *J* = 8.1 Hz, 1H), 7.51-7.45 (m, 3H), 7.42 (t, *J* = 8.9 Hz, 2H), 6.03 (d, *J* = 5.4 Hz, 1H), 3.81-3.77 (m, 1H), 3.55-3.45 (m, 2H), 2.44-2.40 (m, 2H), 1.22 (d, *J* = 6.5 Hz, 3H), 1.06 (t, *J* = 7. 5 Hz, 3H). |
| **Final product 81** | **B30** | 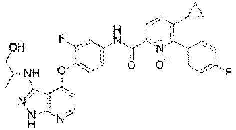 | MS (ESI⁺)m/z = 573.2 [M+H]⁺. ¹H NMR (500 MHz, DMSO-*d*₆) *δ* 13.82 (s, 1H), 12.18 (s, 1H), 8.29 (d, *J* = 8.6 Hz, 1H), 8.13 (d, *J* = 5.4 Hz, 1H), 8.05 (dd, *J* = 12.6, 2.4 Hz, 1H), 7.63 (d, *J* = 8.6 Hz, 1H), 7.55 (dd, *J* = 8.7, 5.5 Hz, 2H), 7.48 (t, *J* = 8.9 Hz, 1H), 7.41 (t, *J* = 8.9 Hz, 2H), 7.33 (d, *J* = 8.7 Hz, 1H), 6.02 (d, *J* = 5.3 Hz, 1H), 5.10 (d, *J* = 7.3 Hz, 1H), 4.79 (t, *J* = 5.5 Hz, 1H), 3.84-3.72 (m, 1H), 3.58-3.44 (m, 2H), 1.51-1.47 (m, 1H), 1.21 (d, *J* = 6.5 Hz, 3H), 0.97-0.92 (m, 2H), 0.86 (d, *J* = 4.5 Hz, 2H). |
| **Final product 82** | **B8** | 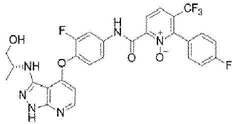 | MS (ESI⁺)m/z = 601.4 [M+H]⁺. ¹H NMR (600 MHz, DMSO-*d*₆) *δ* 13.13 (s, 1H), 12.32 (s, 1H), 8.51 (d, *J* = 8.6 Hz, 1H), 8.20 (s, 1H), 8.13 (d, *J* = 8.7 Hz, 1H), 8.06 (d, *J* = 8.9, 2.4 Hz, 1H), 7.69 (d, *J* = 8.6 Hz, 1H), 7.60-7.50 (m, 3H), 7.42 (t, *J* = 8.9 Hz, 2H), 6.07 (d, *J* = 5.6 Hz, 1H), 3.82-3.78 (m, 1H), 3.55-3.48 (m, 2H), 1.22 (d, *J* = 6.5 Hz, 3H). |
| **Final product 83** | **B7** | 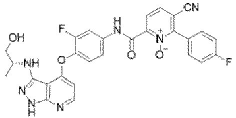 | MS (ESI⁺)m/z = 558.0 [M+H]⁺. ¹H NMR (600 MHz, DMSO-*d*₆) *δ* 12.93 (s, 1H), 12.19 (s, 1H), 8.37 (d, *J* = 8.9 Hz, 1H), 8.22 (d, *J* = 7.2 Hz, 1H), 8.14 (d, *J* = 7.2 Hz, 1H), 8.03 (dd, *J* = 12.5, 2.3 Hz, 1H), 7.80-7.75 (m, 2H), 7.65 (dd, *J* = 8.9, 2.4 Hz, 1H), 7.56-7.40 (m, 3H), 6.05 (d, *J* = 5.6 Hz, 1H), 5.07 (d, *J* = 7.2 Hz, 1H), 4.80 (t, *J* = 7.2 Hz, 1H), 3.85-3.74 (m, 1H), 3.59-3.41 (m, 2H), 1.21 (d, *J* = 6.5 Hz, 3H). |
| **Final product 85** | **B6** | 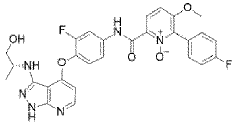 | MS (ESI⁺)m/z = 563.1 [M+H]⁺. ¹H NMR (600 MHz, DMSO-*d*₆) *δ* 13.66 (s, 1H), 12.18 (s, 1H), 8.41 (d, *J* = 9.2 Hz, 1H), 8.13 (d, *J* = 5.4 Hz, 1H), 8.05 (dd, *J* = 12.7, 2.4 Hz, 1H), 7.64-7.59 (m, 2H), 7.57-7.52 (m, 2H), 7.47 (t, *J* = 8.9 Hz, 1H), 7.35 (t, *J* = 8.9 Hz, 2H), 6.02 (d, *J* = 5.1 Hz, 1H), 5.10 (d, *J* = 7.2 Hz, 1H), 4.79 (t, *J* = 5.5 Hz, 1H), 3.89 (s, 3H), 3.82-3.74 (m, 1H), 3.57-3.45 (m, 2H), 1.21 (d, *J* = 6.5 Hz, 3H). |
| **Final product 86** | **B27** | 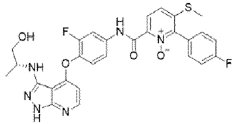 | MS (ESI⁺)m/z = 579.1 [M+H]⁺. ¹H NMR (600 MHz, DMSO-*d*₆) *δ* 13.63 (s, 1H), 12.17 (s, 1H), 8.35 (d, *J* = 8.7 Hz, 1H), 8.14 (d, *J* = 5.5 Hz, 1H), 8.05 (dd, *J* = 12.8, 2.5 Hz, 1H), 7.70-7.61 (m, 2H), 7.52-7.38 (m, 5H), 6.02 (d, *J* = 5.4 Hz, 1H), 5.10 (d, *J* = 7.2 Hz, 1H), 4.78 (t, *J* = 5.5 Hz, 1H), 3.83-3.75 (m, 1H), 3.51-3.48 (m, 2H), 2.50 (s, 3H), 1.21 (d, *J* = 6.4 Hz, 3H). |
| **Final product 87** | **B28** | 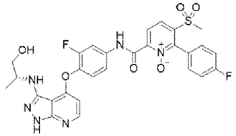 | MS (ESI⁺)m/z = 611.0 [M+H]⁺. ¹H NMR (600 MHz, DMSO-*d*₆) *δ* 13.02 (s, 1H), 12.14 (s, 1H), 8.54 (d, *J* = 8.6 Hz, 1H), 8.23 (d, *J* = 8.7 Hz, 1H), 8.12 (d, *J* = 5.4 Hz, 1H), 8.03 (dd, *J* = 12.6, 2.4 Hz, 1H), 7.67 (dd, *J* = 8.9, 1.4 Hz, 1H), 7.63-7.55 (m, 2H), 7.50 (t, *J* = 8.9 Hz, 1H), 7.41 (t, *J* = 8.9 Hz, 2H), 6.02 (d, *J* = 5.3 Hz, 1H), 5.11 (d, *J* = 7.2 Hz, 1H), 4.79 (t, *J* = 5.5 Hz, 1H), 3.85-3.72 (m, 1H), 3.53-3.46 (m, 2H), 3.00 (s, 3H), 1.21 (d, *J* = 6.5 Hz, 3H). |
| **Final product 89** | **B33** | 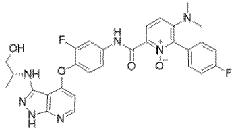 | Yellow solid, yield: 28.9%. MS (ESI⁺)m/z = 576.1 [M+H]⁺. ¹H NMR (400 MHz, DMSO-*d*₆) *δ* 13.82 (s, 1H), 12.19 (s, 1H), 8.24 (d, *J* = 9.6 Hz, 1H), 8.13 (d, *J* = 5.6 Hz, 1H), 8.05 (dd, *J* = 12.6, 2.4 Hz, 1H), 7.59-7.55 (m, 3H), 7.46 (t, *J* = 8.8 Hz, 1H), 7.37 (t, *J* = 8.8 Hz, 3H), 6.02 (d, *J* = 5.6 Hz, 1H), 5.11 (d, *J* = 7.6 Hz, 1H), 4.81 (t, *J* = 5.6 Hz, 1H), 3.79-3.76 (m, 1H), 3.51-3.44 (m, 2H), 2.58 (s, 6H), 1.21 (d, *J* = 6.4 Hz, 3H). |
| **Final product 91** | **B31** | 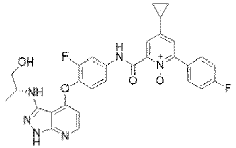 | Light yellow solid, yield: 6.0%. MS (ESI⁺)m/z = 573.0 [M+H]⁺. ¹H NMR (400 MHz, CD₃OD) *δ* 8.16-8.15 (m, 2H), 8.07 (dd, *J* = 12.4, 2.4 Hz, 1H), 7.90-7.84 (m, 2H), 7.56-7.50 (m, 2H), 7.43 (t, *J* = 8.4 Hz, 1H), 7.30 (t, *J* = 8.8 Hz, 2H), 6.15 (d, *J* = 5.6 Hz, 1H), 4.00-3.90 (m, 1H), 3.76-3.63 (m, 2H), 2.25-2.12 (m, 1H), 1.33 (d, *J* = 6.8 Hz, 3H), 1.29-1.23 (m, 2H), 1.02-0.99 (m, 2H). |
| **Final product 92** | **B9** | 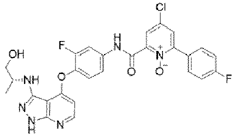 | MS (ESI⁺)m/z = 567.2 [M+H]⁺. ¹H NMR (400 MHz, DMSO-*d*₆) *δ* 13.27 (s, 1H), 12.17 (s, 1H), 8.29 (d, *J* = 3.2 Hz, 1H), 8.14 (d, *J* = 5.4 Hz, 1H), 8.09 (d, *J* = 3.2 Hz, 1H), 8.03 (dd, *J* = 12.6, 2.4 Hz, 1H), 7.95-7.88 (m, 2H), 7.63 (d, *J* = 8.6 Hz, 1H), 7.52 (d, *J* = 8.9 Hz, 1H), 7.41-7.37 (m, 2H), 6.04 (d, *J* = 5.4 Hz, 1H), 5.47-5.34 (m, 1H), 4.80-4.79 (m, 1H), 4.01-3.88 (m, 1H), 3.31-3.28 (m, 1H), 3.15-3.08 (m, 1H), 1.12 (d, *J* = 6.3 Hz, 3H). |
| **Final product 93** | **B29** | 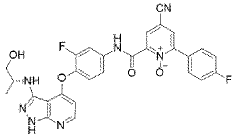 | MS (ESI⁺)m/z = 558.6 [M+H]⁺. ¹H NMR (400 MHz, DMSO-*d*₆) *δ* 12.21 (s, 1H), 12.17 (s, 1H), 8.57 (d, *J* = 2.6 Hz, 1H), 8.42 (d, *J* = 2.6 Hz, 1H), 8.14 (d, *J* = 5.4 Hz, 1H), 7.99 (dd, *J* = 12.6, 2.4 Hz, 1H), 7.96-7.87 (m, 2H), 7.61 (d, *J* = 2.4 Hz, 1H), 7.53 (t, *J* = 8.8 Hz, 1H), 7.41 (t, *J* = 8.9 Hz, 2H), 6.04 (d, *J* = 5.4 Hz, 1H), 5.48-5.36 (m, 1H), 4.80 (d, *J* = 4.8 Hz, 1H), 3.94-3.91 (m, 1H), 3.31-3.26 (m, 1H), 3.15-3.07 (m, 1H), 1.11 (d, *J* = 6.8 Hz, 3H). |
| **Final product 94** | **B11** | 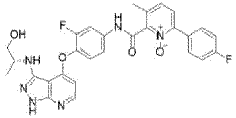 | Light yellow solid, yield: 26.8%. MS (ESI⁺)m/z = 547.0 [M+H]⁺. ¹H NMR (400 MHz, CDCl₃) *δ* 12.21 (s, 1H), 9.70 (br, 1H), 8.20 (d, *J* = 5.2 Hz, 1H), 7.97 (dd, *J* = 12.0, 2.4 Hz, 1H), 7.80-7.69 (m, 2H), 7.42 (d, *J* = 8.0 Hz, 2H), 7.35 (d, *J* = 8.0 Hz, 1H), 7.25-7.20 (m, 3H), 6.06 (d, *J* = 5.2 Hz, 1H), 4.69 (brs, 1H), 4.04-4.02 (m, 1H), 3.84 (dd, *J* = 10.8, 3.2 Hz, 1H), 3.74-3.69 (m, 1H), 2.65 (s, 3H), 1.33 (d, *J* = 6.8 Hz, 3H). |
| **Final product 95** | **B32** | 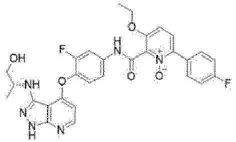 | White solid, yield: 33.8 %. MS (ESI)m/z = 558.7 [M+H]⁺. ¹H NMR (400 MHz, CD₃OD) *δ* 8.17 (d, *J* = 5.6 Hz, 1H), 7.95 (dd, *J* = 12.0, 2.0 Hz, 1H), 7.83 (dd, *J* = 8.8, 5.6 Hz, 2H), 7.68 (d, *J* = 9.2 Hz, 1H), 7.55-7.47 (m, 2H), 7.41 (t, *J* = 8.8 Hz, 1H), 7.26 (t, *J* = 8.8 Hz, 2H), 6.15 (d, *J* = 5.6 Hz, 1H), 4.35-4.26 (m, 2H), 4.00-3.91 (m, 1H), 3.76-3.64 (m, 2H), 1.45 (t, *J* = 6.8 Hz, 3H), 1.33 (d, *J* = 6.8 Hz, 3H). |
| **Final product 96** | **B34** | 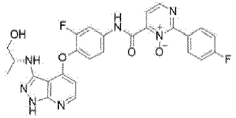 | Yellow solid, yield: 52.1%. MS (ESI⁺)m/z = 534.0 [M+H]⁺. ¹H NMR (400 MHz, DMSO-*d*₆) *δ* 12.89 (s, 1H), 12.21 (s, 1H), 8.66 (d, *J* = 4.8 Hz, 1H), 8.41 (t, *J* = 6.0 Hz, 2H), 8.16 (dd, *J* = 11.6, 4.8 Hz, 2H), 8.04 (d, *J* = 12.0 Hz, 1H), 7.65 (d, *J* = 8.8 Hz, 1H), 7.54 (t, *J* = 8.8 Hz, 1H), 7.42 (t, *J* = 8.4 Hz, 2H), 6.04 (d, *J* = 4.8 Hz, 1H), 5.14 (d, *J* = 7.2 Hz, 1H), 4.81 (t, *J* = 4.8 Hz, 1H), 3.80-3.78 (m, 1H), 3.52-3.48 (m, 2H), 1.22 (d, *J* = 6.4 Hz, 3H). |
| **Final product 97** | **B2** | 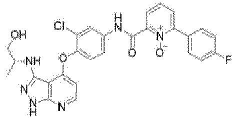 | MS (ESI⁺)m/z = 549.4 [M+H]⁺. ¹H NMR (600 MHz, DMSO-*d*₆) *δ* 13.55 (s, 1H), 12.18 (s, 1H), 8.31 (dd, *J* = 8.8, 2.5 Hz, 1H), 8.24 (d, *J* = 2.5 Hz, 1H), 8.13 (d, *J* = 5.4 Hz, 1H), 7.91-7.85 (m, 3H), 7.78-7.72 (m, 2H), 7.52 (d, *J* = 8.8 Hz, 1H), 7.38 (t, *J* = 8.9 Hz, 2H), 5.94 (d, *J* = 5.4 Hz, 1H), 5.08 (d, *J* = 7.2 Hz, 1H), 4.79 (t, *J* = 5.4 Hz, 1H), 3.87-3.68 (m, 1H), 3.50-3.40 (m, 2H), 1.22 (d, *J* = 6.5 Hz, 3H). |
| **Final product 98** | **B2** | 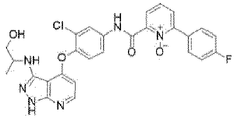 | MS (ESI⁺)m/z = 549.1 [M+H]⁺. ¹H NMR (400 MHz, DMSO-*d*₆) *δ* 13.56 (s, 1H), 12.20 (s, 1H), 8.33 (dd, *J* = 8.0, 2.2 Hz, 1H), 8.25 (d, *J* = 2.5 Hz, 1H), 8.14 (d, *J* = 5.4 Hz, 1H), 7.93-7.86 (m, 3H), 7.81-7.72 (m, 2H), 7.53 (d, *J* = 8.8 Hz, 1H), 7.43-7.36 (m, 2H), 5.95 (d, *J* = 5.4 Hz, 1H), 5.09 (d, *J* = 7.2 Hz, 1H), 4.81 (s, 1H), 3.80-3.75 (m, 1H), 3.51 (s, 2H), 1.22 (d, *J* = 6.5 Hz, 3H). |
| **Final product 99** | **B2** | 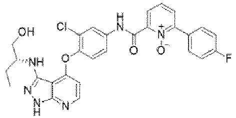 | MS (ESI⁺)m/z = 563.2 [M+H]⁺. ¹H NMR (600 MHz, DMSO-*d*₆) *δ* 13.56 (s, 1H), 12.16 (s, 1H), 8.32 (dd, *J* = 8.0, 2.1 Hz, 1H), 8.24 (d, *J* = 2.5 Hz, 1H), 8.13 (d, *J* = 5.4 Hz, 1H), 7.95-7.85 (m, 3H), 7.81-7.69 (m, 2H), 7.52 (d, *J* = 8.8 Hz, 1H), 7.39 (t, *J* = 8.9 Hz, 2H), 5.96 (d, *J* = 5.4 Hz, 1H), 5.01 (d, *J* = 7.8 Hz, 1H), 4.72 (t, *J* = 5.3 Hz, 1H), 3.60-3.45 (m, 3H), 1.67-1.62 (m, 2H), 0.93 (t, *J* = 7.4 Hz, 3H). |
| **Final product 103** | **B2** | 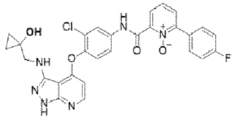 | MS (ESI⁺)m/z = 561.1 [M+H]⁺. ¹H NMR (400 MHz, DMSO-*d*₆) *δ* 13.53 (s, 1H), 12.20 (s, 1H), 8.35-8.31 (m, 1H), 8.25 (d, *J* = 2.5 Hz, 1H), 8.15 (d, *J* = 5.4 Hz, 1H), 7.93-7.86 (m, 3H), 7.81-7.71 (m, 2H), 7.53 (d, *J* = 8.8 Hz, 1H), 7.39 (t, *J* = 8.9 Hz, 2H), 5.97 (d, *J* = 5.4 Hz, 1H), 5.92 (t, *J* = 5.5 Hz, 1H), 4.11 (d, *J* = 5.6 Hz, 2H), 2.55-2.51 (m, 2H), 0.95 (d, *J* = 7.3 Hz, 2H). |
| **Final product 113** | **B2** | 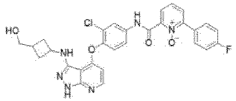 | MS (ESI⁺)m/z = 575.2 [M+H]⁺. ¹H NMR (400 MHz, DMSO-*d*₆) *δ* 13.55 (s, 1H), 12.15 (s, 1H), 8.32 (dd, *J* = 8.0, 2.1 Hz, 1H), 8.24 (t, *J* = 1.8 Hz, 1H), 8.12 (d, *J* = 5.5 Hz, 1H), 7.96-7.83 (m, 3H), 7.81-7.71 (m, 2H), 7.50 (d, *J* = 8.8 Hz, 1H), 7.39 (t, *J* = 8.7 Hz, 2H), 5.92 (d, *J* = 5.4 Hz, 1H), 5.55 (dd, *J* = 33.4, 8.0 Hz, 1H), 4.27-4.23 (m, 1H), 4.07-4.03 (m, 1H), 3.48 (d, *J =* 7.0 Hz, 1H), 3.38 (d, *J* = 6.0 Hz, 1H), 2.42-2.18 (m, 2H), 2.12 (d, *J* = 8.4 Hz, 2H), 1.76-1.72 (m, 1H). |
| **Final product 114** | **B2** | 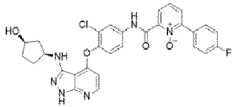 | MS (ESI⁺)m/z = 576.1 [M+H]⁺. ¹H NMR (400 MHz, DMSO-*d*₆) *δ* 13.56 (s, 1H), 12.17 (s, 1H), 8.32 (dd, *J* = 8.0, 2.2 Hz, 1H), 8.24 (d, *J* = 2.4 Hz, 1H), 8.12 (d, *J* = 5.4 Hz, 1H), 7.90-7.86 (m, 3H), 7.79-7.71 (m, 2H), 7.50 (d, *J* = 8.8 Hz, 1H), 7.39 (t, *J* = 8.7 Hz, 2H), 5.93 (d, *J* = 5.4 Hz, 1H), 5.43 (d, *J* = 8.6 Hz, 1H), 4.71 (s, 1H), 4.18-4.10 (m, 2H), 2.12-2.08 (m, 1H), 2.02-1.94 (m, 1H), 1.78-1.58 (m, 4H). |
| **Final product 115** | **B2** | 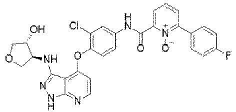 | MS (ESI⁺)m/z = 577.1 [M+H]⁺. ¹H NMR (400 MHz, DMSO-*d*₆) *δ* 13.55 (s, 1H), 12.31 (s, 1H), 8.32 (dd, *J* = 8.0, 2.2 Hz, 1H), 8.25 (d, *J* = 2.4 Hz, 1H), 8.15 (d, *J* = 5.4 Hz, 1H), 7.90-7.80 (m, 3H), 7.84-7.61 (m, 2H), 7.51 (d, *J* = 8.8 Hz, 1H), 7.39 (t, *J* = 8.9 Hz, 2H), 5.96 (d, *J* = 5.4 Hz, 1H), 5.45 (d, *J* = 5.5 Hz, 1H), 5.23 (d, *J* = 3.9 Hz, 1H), 4.33 (s, 1H), 4.10-4.01 (m, 2H), 3.93 (dd, *J* = 9.2, 4.7 Hz, 1H), 3.75 (dd, *J* = 8.8, 2.8 Hz, 1H), 3.55 (dd, *J* = 8.4, 2.2 Hz, 1H). |
| **Final product 116** | **B21** | 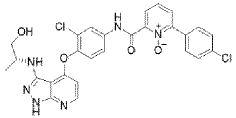 | MS (ESI⁺)m/z = 566.1 [M+H]⁺. ¹H NMR (400 MHz, DMSO-*d*₆) *δ* 13.49 (s, 1H), 12.19 (s, 1H), 8.32 (dd, *J* = 8.0, 2.1 Hz, 1H), 8.24 (d, *J* = 2.1 Hz, 1H), 8.14 (d, *J* = 5.4 Hz, 1H), 7.91 (dd, *J* = 7.8, 1.6 Hz, 1H), 7.86 (d, *J* = 8.4 Hz, 2H), 7.80-7.70 (m, 2H), 7.62 (d, *J* = 8.4 Hz, 2H), 7.54 (d, *J* = 8.8 Hz, 1H), 5.95 (d, *J* = 5.4 Hz, 1H), 5.09 (d, *J* = 7.1 Hz, 1H), 4.80 (t, *J* = 5.3 Hz, 1H), 3.80-3.75 (m, 1H), 3.53-3.49 (m, 2H), 1.22 (d, *J* = 6.5 Hz, 3H). |
| **Final product 117** | **B3** | 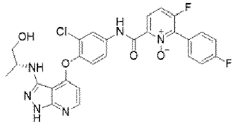 | MS (ESI⁺)m/z = 567.1 [M+H]⁺. ¹H NMR (600 MHz, DMSO-*d*₆) *δ* 13.16 (s, 1H), 12.19 (s, 1H), 8.40 (dd, *J* = 8.0, 2.0 Hz, 1H), 8.23 (d, *J* = 2.5 Hz, 1H), 8.13 (d, *J* = 5.4 Hz, 1H), 7.84 (dd, *J* = 9.2, 7.2 Hz, 1H), 7.79 (dd, *J* = 8.9, 2.5 Hz, 1H), 7.72 (dd, *J* = 8.5, 5.6 Hz, 2H), 7.51 (d, *J* = 8.8 Hz, 1H), 7.43 (t, *J* = 8.8 Hz, 2H), 5.94 (d, *J* = 5.4 Hz, 1H), 5.07 (d, *J* = 7.2 Hz, 1H), 4.80 (t, *J* = 5.5 Hz, 1H), 3.83-3.76 (m, 1H), 3.58-3.46 (m, 2H), 1.21 (d, *J* = 6.5 Hz, 3H). |
| **Final product 118** | **B3** | 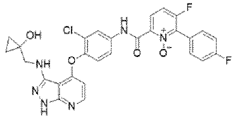 | MS (ESI⁺)m/z = 579.1 [M+H]⁺. ¹H NMR (400 MHz, DMSO-*d*₆) *δ* 13.15 (s, 1H), 12.20 (s, 1H), 8.40 (dd, *J* = 8.0, 2.0 Hz, 1H), 8.25 (d, *J* = 4.0 Hz, 1H), 8.15 (d, *J* = 4.0 Hz, 1H), 7.87-7.78 (m, 2H), 7.74-7.71 (m, 2H), 7.53 (d, *J* = 8.8 Hz, 1H), 7.47-7.39 (m, 2H), 5.99-5.88 (m, 2H), 4.11 (d, *J* = 5.6 Hz, 2H), 2.55-2.51 (m, 2H), 0.97 (t, *J* = 8.0 Hz, 2H). |
| **Final product 119** | **B3** | 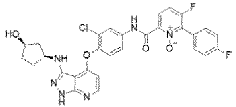 | MS (ESI⁺)m/z = 593.2 [M+H]⁺. ¹H NMR (400 MHz, DMSO-*d*₆) *δ* 13.17 (s, 1H), 12.17 (s, 1H), 8.40 (dd, *J* = 12.0, 8.0 Hz, 1H), 8.23 (d, *J* = 2.5 Hz, 1H), 8.12 (d, *J* = 5.4 Hz, 1H), 7.84-7.72 (m, 4H), 7.49 (d, *J* = 8.8 Hz, 1H), 7.46-7.40 (m, 2H), 5.92 (d, *J* = 5.4 Hz, 1H), 5.42 (d, *J* = 8.6 Hz, 1H), 4.70 (d, *J* = 3.7 Hz, 1H), 4.21-4.08 (m, 2H), 2.12-2.08 (m, 1H), 1.99-1.95 (m, 1H), 1.76-1.49 (m, 4H). |
| **Final product 120** | **B3** | 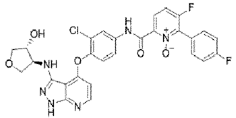 | MS (ESI⁺)m/z = 596.1 [M+H]⁺. ¹H NMR (600 MHz, DMSO-*d*₆) *δ* 13.16 (s, 1H), 12.32 (s, 1H), 8.40 (dd, *J* = 9.2, 7.3 Hz, 1H), 8.24 (d, *J* = 2.5 Hz, 1H), 8.15 (d, *J* = 5.3 Hz, 1H), 7.84 (dd, *J* = 9.2, 7.2 Hz, 1H), 7.79 (dd, *J* = 8.8, 2.5 Hz, 1H), 7.72 (dd, *J* = 8.5, 5.6 Hz, 2H), 7.50 (d, *J* = 8.8 Hz, 1H), 7.48-7.36 (m, 2H), 5.96 (d, *J* = 5.5 Hz, 1H), 5.46 (s, 1H), 4.32 (s, 1H), 4.06 (dd, *J* = 8.9, 5.5 Hz, 1H), 4.00 (d, *J* = 2.3 Hz, 1H), 3.92 (dd, *J* = 9.3, 4.7 Hz, 1H), 3.74 (dd, *J* = 8.9, 3.1 Hz, 1H), 3.53 (dd, *J* = 9.2, 2.4 Hz, 1H). |
| **Final product 121** | **B1** | 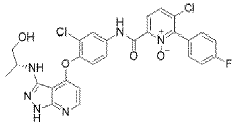 | MS (ESI⁺)m/z = 584.1 [M+H]⁺. ¹H NMR (600 MHz, DMSO-*d*₆) *δ* 13.21 (s, 1H), 12.18 (s, 1H), 8.35 (d, *J* = 8.9 Hz, 1H), 8.23 (d, *J* = 2.4 Hz, 1H), 8.13 (d, *J* = 5.4 Hz, 1H), 7.98 (d, *J* = 8.9 Hz, 1H), 7.79 (dd, *J* = 8.8, 2.4 Hz, 1H), 7.61-7.57 (m, 2H), 7.50 (d, *J* = 8.8 Hz, 1H), 7.42 (t, *J* = 8.8 Hz, 2H), 5.93 (d, *J* = 5.4 Hz, 1H), 5.07 (d, *J* = 7.2 Hz, 1H), 4.79 (t, *J* = 5.4 Hz, 1H), 3.82-3.72 (m, 1H), 3.57-3.44 (m, 2H), 1.21 (d, *J* = 6.5 Hz, 3H). |
| **Final product 122** | **B1** | 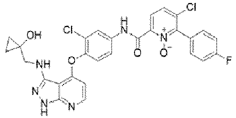 | MS (ESI⁺)m/z = 595.0 [M+H]⁺. ¹H NMR (400 MHz, DMSO-*d*₆) *δ* 13.20 (s, 1H), 12.20 (s, 1H), 8.35 (d, *J* = 8.9 Hz, 1H), 8.24 (d, *J* = 2.5 Hz, 1H), 8.15 (d, *J* = 5.4 Hz, 1H), 7.98 (d, *J* = 8.8 Hz, 1H), 7.80 (dd, *J* = 8.9, 2.5 Hz, 1H), 7.63-7.56 (m, 2H), 7.52 (d, *J* = 8.8 Hz, 1H), 7.42 (t, *J* = 8.8 Hz, 2H), 5.99-5.87 (m, 2H), 4.11 (d, *J* = 5.6 Hz, 2H), 2.58-2.52 (m, 2H), 0.97 (d, *J* = 7.3 Hz, 2H). |
| **Final product 123** | **B1** | 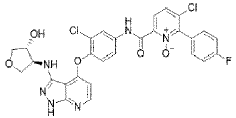 | MS (ESI⁺)m/z = 613.1 [M+H]⁺. ¹H NMR (600 MHz, DMSO-*d*₆) *δ* 13.21 (s, 1H), 12.30 (s, 1H), 8.35 (d, *J* = 8.9 Hz, 1H), 8.23 (d, *J* = 2.5 Hz, 1H), 8.14 (d, *J* = 5.4 Hz, 1H), 7.98 (d, *J* = 8.9 Hz, 1H), 7.79 (dd, *J* = 8.8, 2.5 Hz, 1H), 7.66-7.53 (m, 2H), 7.49 (d, *J* = 8.8 Hz, 1H), 7.42 (t, *J* = 8.8 Hz, 2H), 5.94 (d, *J* = 5.4 Hz, 1H), 5.44 (d, *J* = 5.6 Hz, 1H), 5.22 (d, *J* = 3.9 Hz, 1H), 4.37-4.28 (m, 1H), 4.06 (dd, *J* = 8.9, 5.5 Hz, 1H), 4.02-3.95 (m, 1H), 3.92 (dd, *J* = 9.3, 4.7 Hz, 1H), 3.74 (dd, *J* = 8.9, 3.1 Hz, 1H), 3.53 (dd, *J* = 9.2, 2.4 Hz, 1H). |
| **Final product 124** | **B4** | 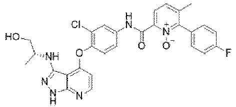 | MS (ESI⁺)m/z = 563.1 [M+H]⁺. ¹H NMR (400 MHz, DMSO-*d*₆) *δ* 13.82 (s, 1H), 12.18 (s, 1H), 8.33 (d, *J* = 8.3 Hz, 1H), 8.24 (s, 1H), 8.13 (d, *J* = 5.4 Hz, 1H), 7.77 (dd, *J* = 8.8, 2.2 Hz, 1H), 7.71 (d, *J* = 8.4 Hz, 1H), 7.53-7.47 (m, 3H), 7.40 (t, *J* = 8.4 Hz, 2H), 5.93 (d, *J* = 5.4 Hz, 1H), 5.06 (s, 1H), 4.79 (s, 1H), 3.87-3.71 (m, 1H), 3.57-3.43 (m, 2H), 2.14 (s, 3H), 1.21 (d, *J* = 6.4 Hz, 3H). |
| **Final product 125** | **B4** | 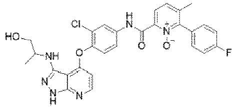 | MS (ESI⁺)m/z = 563.2 [M+H]⁺. ¹H NMR (400 MHz, DMSO-*d*₆) *δ* 13.90 (s, 1H), 12.26 (s, 1H), 8.40 (d, *J* = 8.3 Hz, 1H), 8.31 (d, *J* = 2.6 Hz, 1H), 8.20 (d, *J* = 5.4 Hz, 1H), 7.77 (dd, *J* = 8.8, 2.2 Hz, 1H), 7.71 (d, *J* = 8.4 Hz, 1H), 7.62-7.54 (m, 3H), 7.51-7.44 (m, 2H), 6.00 (d, *J* = 5.4 Hz, 1H), 5.14 (d, *J* = 7.2 Hz, 1H), 4.87 (s, 1H), 3.84-3.80 (m, 1H), 3.58-3.55 (m, 2H), 2.21 (s, 3H), 1.28 (d, *J* = 6.5 Hz, 3H). |
| **Final product 126** | **B4** | 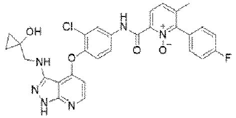 | MS (ESI⁺)m/z = 575.2 [M+H]⁺. ¹H NMR (400 MHz, DMSO-*d*₆) *δ* 13.82 (s, 1H), 12.20 (s, 1H), 8.33 (d, *J* = 8.3 Hz, 1H), 8.25 (d, *J* = 2.5 Hz, 1H), 8.14 (d, *J* = 5.4 Hz, 1H), 7.79 (dd, *J* = 8.8, 2.5 Hz, 1H), 7.71 (d, *J* = 8.4 Hz, 1H), 7.53-7.50 (m, 3H), 7.44-7.37 (m, 2H), 5.96-5.89 (m, 2H), 4.11 (d, *J* = 5.6 Hz, 2H), 2.55-2.51 (m, 2H), 2.14 (s, 3H), 0.96 (t, *J* = 7.3 Hz, 2H). |
| **Final product 127** | **B4** | 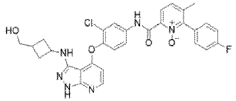 | MS (ESI⁺)m/z = 588.2 [M+H]⁺. ¹H NMR (400 MHz, DMSO-*d*₆) *δ* 13.82 (s, 1H), 12.18 (s, 1H), 8.33 (d, *J* = 8.3 Hz, 1H), 8.25 (d, *J* = 2.5 Hz, 1H), 8.13 (s, 1H), 7.78 (dd, *J* = 8.9, 2.5 Hz, 1H), 7.71 (d, *J* = 8.4 Hz, 1H), 7.53-7.48 (m, 3H), 7.42-7.38 (m, 2H), 5.92 (d, *J* = 5.6 Hz, 1H), 4.30-4.19 (m, 1H), 4.07-4.02 (m, 1H), 3.47 (d, *J* = 8.0 Hz, 1H), 3.38 (d, *J* = 8.0 Hz, 1H), 2.39-2.35 (m, 1H), 2.28-2.24 (m, 1H), 2.15-2.10 (m, 5H), 1.76-1.74 (m, 1H). |
| **Final product 128** | **B4** | 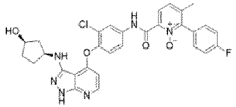 | MS (ESI⁺)m/z = 589.2 [M+H]⁺. ¹H NMR (400 MHz, DMSO-*d*₆) *δ* 13.82 (s, 1H), 12.16 (s, 1H), 8.33 (d, *J* = 8.3 Hz, 1H), 8.23 (d, *J* = 2.5 Hz, 1H), 8.12 (d, *J* = 5.4 Hz, 1H), 7.76 (dd, *J* = 8.9, 2.5 Hz, 1H), 7.71 (d, *J* = 8.5 Hz, 1H), 7.54-7.45 (m, 3H), 7.43-7.37 (m, 2H), 5.92 (d, *J* = 5.5 Hz, 1H), 5.40 (d, *J* = 8.6 Hz, 1H), 4.69 (d, *J* = 3.7 Hz, 1H), 4.17-4.08 (m, 2H), 2.14 (s, 3H), 2.12-2.06 (m, 1H), 2.01-1.94 (m, 1H), 1.77-1.55 (m, 4H). |
| **Final product 129** | **B4** | 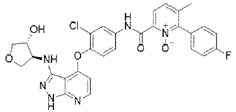 | MS (ESI⁺)m/z = 591.2 [M+H]⁺. ¹H NMR (400 MHz, DMSO-*d*₆) *δ* 13.82 (s, 1H), 12.30 (s, 2H), 8.33 (d, *J* = 8.3 Hz, 1H), 8.24 (d, *J* = 2.4 Hz, 1H), 8.15 (d, *J* = 5.4 Hz, 1H), 7.78 (dd, *J* = 8.8, 2.4 Hz, 1H), 7.72 (d, *J* = 8.4 Hz, 1H), 7.55-7.46 (m, 2H), 7.43-7.40 (m, 2H), 5.95 (d, *J* = 5.4 Hz, 1H), 5.43 (d, *J* = 5.5 Hz, 1H), 5.22 (d, *J* = 3.8 Hz, 1H), 4.32 (s, 1H), 4.20-4.10 (m, 2H), 3.92 (dd, *J* = 9.2, 4.7 Hz, 1H), 3.74 (dd, *J* = 8.8, 2.9 Hz, 1H), 3.54 (dd, *J* = 9.2, 2.2 Hz, 1H), 2.15 (s, 3H). |
| **Final product 133** | **B24** | 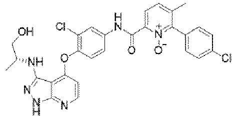 | MS (ESI⁺)m/z = 579.0 [M+H]⁺. ¹H NMR (600 MHz, DMSO-*d*₆) *δ* 13.80 (s, 1H), 12.25 (s, 1H), 8.33 (d, *J* = 8.3 Hz, 1H), 8.24 (s, 1H), 8.16 (s, 1H), 7.78 (d, *J* = 7.8 Hz, 1H), 7.72 (d, *J* = 8.4 Hz, 1H), 7.63 (d, *J* = 8.4 Hz, 2H), 7.50-7.45 (m, 3H), 5.96 (d, *J* = 5.6 Hz, 1H), 3.79-3.77 (m, 1H), 3.52-3.49 (m, 2H), 2.14 (s, 3H), 1.21 (d, *J* = 6.5 Hz, 3H). |
| **Final product 134** | **B26** | 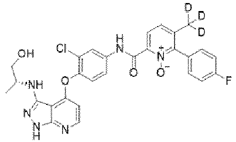 | MS (ESI⁺)m/z = 567.2 [M+H]⁺. ¹H NMR (600 MHz, CD₃OD) *δ* 14.64 (s, 1H), 13.00 (s, 1H), 9.14 (d, *J* = 8.3 Hz, 1H), 9.06 (d, *J* = 2.5 Hz, 1H), 8.94 (d, *J* = 5.4 Hz, 1H), 8.59 (dd, *J* = 8.9, 2.5 Hz, 1H), 8.53 (d, *J* = 8.4 Hz, 1H), 8.36-8.29 (m, 3H), 8.21 (t, *J* = 8.9 Hz, 2H), 6.75 (d, *J* = 5.4 Hz, 1H), 5.88 (d, *J* = 7.2 Hz, 1H), 5.61 (t, *J* = 5.4 Hz, 1H), 4.62-4.53 (m, 1H), 4.36-4.27 (m, 2H), 2.03 (d, *J* = 6.5 Hz, 3H). |
| **Final product 135** | **B5** | 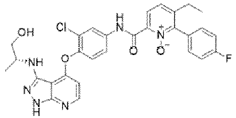 | MS (ESI⁺)m/z = 577.2 [M+H]⁺. ¹H NMR (600 MHz, DMSO-*d*₆) *δ* 13.87 (s, 1H), 12.24 (s, 1H), 8.43 (d, *J* = 8.4 Hz, 1H), 8.30 (d, *J* = 2.5 Hz, 1H), 8.19 (d, *J* = 5.4 Hz, 1H), 7.90-7.79 (m, 2H), 7.69-7.49 (m, 3H), 7.51-7.36 (m, 2H), 6.00 (d, *J* = 5.4 Hz, 1H), 5.13 (d, *J* = 7.2 Hz, 1H), 4.86 (t, *J* = 5.4 Hz, 1H), 3.93-3.76 (m, 1H), 3.60-3.53 (m, 2H), 2.51-2.44 (m, 2H), 1.27 (d, *J* = 6.5 Hz, 3H), 1.11 (t, *J* = 7.5 Hz, 3H). |
| **Final product 138** | **B6** | 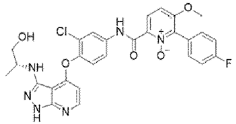 | MS (ESI⁺)m/z = 579.6 [M+H]⁺. ¹H NMR (600 MHz, DMSO-*d*₆) *δ* 13.66 (s, 1H), 12.18 (s, 1H), 8.41 (d, *J* = 9.2 Hz, 1H), 8.24 (d, *J* = 2.4 Hz, 1H), 8.11 (d, *J* = 5.5 Hz, 1H), 7.76 (dd, *J* = 8.8, 2.4 Hz, 1H), 7.61 (d, *J* = 9.4 Hz, 1H), 7.56-7.52 (m, 2H), 7.49 (d, *J* = 8.8 Hz, 1H), 7.35 (t, *J* = 8.8 Hz, 2H), 5.93 (d, *J* = 5.4 Hz, 1H), 5.07 (d, *J* = 7.1 Hz, 1H), 4.79 (t, *J* = 5.4 Hz, 1H), 3.89 (s, 3H), 3.79-3.75 (m, 1H), 3.55-3.44 (m, 2H), 1.21 (d, *J* = 6.5 Hz, 3H). |
| **Final product 139** | **B36** | 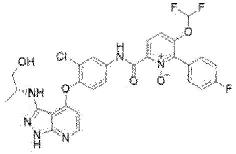 | MS (ESI⁺)m/z = 615.5 [M+H]⁺. ¹H NMR (600 MHz, DMSO-*d*₆) *δ* 13.27 (s, 1H), 12.18 (s, 1H), 8.43 (d, *J* = 9.2 Hz, 1H), 8.24 (d, *J* = 2.5 Hz, 1H), 8.13 (d, *J* = 5.4 Hz, 1H), 7.78 (dd, *J* = 13.6, 5.4 Hz, 1H), 7.74 (d, *J* = 5.4 Hz, 1H), 7.62-7.58 (m, 2H), 7.51 (d, *J* = 8.8 Hz, 1H), 7.40 (t, *J* = 5.4 Hz, 2H), 7.51-7.40 (m, 1H), 5.93 (d, *J* = 5.4 Hz, 1H), 5.07 (d, *J* = 7.1 Hz, 1H), 4.79 (t, *J* = 5.4 Hz, 1H), 3.85-3.74 (m, 1H), 3.60-3.44 (m, 2H), 1.21 (d, *J* = 6.5 Hz, 3H). |
| **Final product 141** | **B35** | 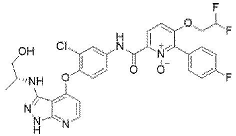 | MS (ESI⁺)m/z = 629.5 [M+H]⁺. ¹H NMR (600 MHz, DMSO-*d*₆) *δ* 13.55 (s, 1H), 12.18 (s, 1H), 8.39 (d, *J* = 9.3 Hz, 1H), 8.24 (d, *J* = 2.4 Hz, 1H), 8.13 (d, *J* = 5.4 Hz, 1H), 7.77 (dd, *J* = 8.8, 2.5 Hz, 1H), 7.69 (d, *J* = 9.4 Hz, 1H), 7.58 (dd, *J* = 8.6, 5.6 Hz, 2H), 7.49 (d, *J* = 8.8 Hz, 1H), 7.36 (t, *J* = 8.9 Hz, 2H), 6.40-6.20 (m, 1H), 5.93 (d, *J* = 5.4 Hz, 1H), 5.07 (d, *J* = 7.1 Hz, 1H), 4.80 (t, *J* = 5.4 Hz, 1H), 4.56-4.52 (m, 2H), 3.79-3.75 (m, 1H), 3.52-3.98 (m, 2H), 1.21 (d, *J* = 6.5 Hz, 3H). |
| **Final product 142** | **B37** | 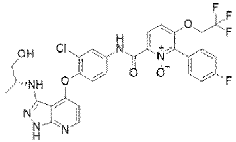 | MS (ESI⁺)m/z = 647.1 [M+H]⁺. ¹H NMR (600 MHz, DMSO-*d*₆) *δ* 13.48 (d, *J* = 6.6 Hz, 1H), 12.18 (s, 1H), 8.42 (d, *J* = 9.2Hz, 1H), 8.24 (t, *J* = 4.5 Hz, 1H), 8.13 (d, *J* = 5.4 Hz, 1H), 7.77 (dd, *J* = 8.9, 2.4 Hz, 1H), 7.71 (d, *J* = 9.4 Hz, 1H), 7.59-7.55 (m, 2H), 7.50 (d, *J* = 8.8 Hz, 1H), 7.37 (t, *J* = 8.9 Hz, 2H), 5.93 (d, *J* = 5.4 Hz, 1H), 5.07 (d, *J* = 7.1 Hz, 1H), 5.02-4.96 (m, 2H), 4.80 (t, *J* = 5.4 Hz, 1H), 3.83-3.73 (m, 1H), 3.55-3.47 (m, 2H), 1.21 (d, *J* = 6.5 Hz, 3H). |
| **Final product 145** | **B2** | 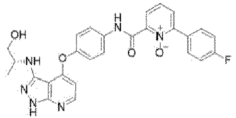 | MS (ESI⁺)m/z = 515.6 [M+H]⁺. ¹H NMR (600 MHz, DMSO-*d*₆) *δ* 13.48 (s, 1H), 12.12 (s, 1H), 8.33 (dd, *J* = 8.0, 2.1 Hz, 1H), 8.13 (d, *J* = 5.4 Hz, 1H), 7.89-7.80 (m, 5H), 7.73 (t, *J* = 7.9 Hz, 1H), 7.40-7.38 (m, 2H), 7.33 (d, *J* = 8.9 Hz, 2H), 6.03 (d, *J* = 5.4 Hz, 1H), 5.40-5.29 (m, 1H), 4.81 (d, *J* = 4.8 Hz, 1H), 3.96-3.84 (m, 1H), 3.29-3.25 (m, 1H), 3.16-3.03 (m, 1H), 1.11 (d, *J* = 6.2 Hz, 3H). |
| **Final product 146** | **B2** | 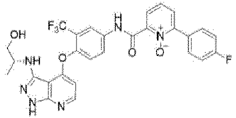 | MS (ESI⁺)m/z = 583.6 [M+H]⁺. ¹H NMR (600 MHz, DMSO-*d*₆) *δ* 13.62 (s, 1H), 12.21 (s, 1H), 8.43 (d, *J* = 2.5 Hz, 1H), 8.33 (dd, *J* = 8.0, 2.1 Hz, 1H), 8.17 (d, *J* = 5.4 Hz, 1H), 8.08 (dd, *J* = 8.9, 2.5 Hz, 1H), 7.90-8.80 (m, 3H), 7.75 (t, *J* = 7.9 Hz, 1H), 7.58 (d, *J* = 8.9 Hz, 1H), 7.39 (t, *J* = 8.9 Hz, 2H), 6.11 (d, *J* = 5.4 Hz, 1H), 5.16-5.13 (m, 1H), 4.76 (d, *J* = 8.9 Hz, 1H), 3.94-3.80 (m, 1H), 3.28-3.25 (m, 1H), 3.18-3.02 (m, 1H), 1.11 (d, *J* = 6.2 Hz, 3H). |
| **Final product 149** | **B2** | 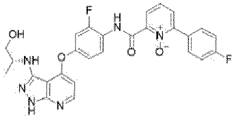 | MS (ESI⁺)m/z = 533.6 [M+H]⁺. ¹H NMR (400 MHz, DMSO-*d*₆) *δ* 14.20 (s, 1H), 12.14 (s, 1H), 8.57 (t, *J* = 8.9 Hz, 1H), 8.44 (dd, *J* = 8.0, 2.2 Hz, 1H), 8.16 (d, *J* = 5.4 Hz, 1H), 7.98-7.82 (m, 3H), 7.78 (t, *J* = 7.9 Hz, 1H), 7.47 (dd, *J* = 11.5, 2.7 Hz, 1H), 7.39 (t, *J* = 8.9 Hz, 2H), 7.24 (d, *J* = 8.9 Hz, 1H), 6.13 (d, *J* = 5.4 Hz, 1H), 5.40-5.30 (m, 1H), 4.79 (d, *J* = 4.8 Hz, 1H), 3.95-3.88 (m, 1H), 3.28-3.29 (m, 1H), 3.15-3.10 (m, 1H), 1.11 (d, *J* = 6.4 Hz, 3H). |
| **Final product 151** | **B2** | 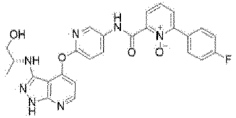 | MS (ESI⁺)m/z = 516.5 [M+H]⁺. ¹H NMR (600 MHz, DMSO-*d*₆) *δ* 13.56 (s, 1H), 12.16 (s, 1H), 8.71 (d, *J* = 2.7 Hz, 1H), 8.43 (dd, *J* = 8.8, 2.7 Hz, 1H), 8.34 (dd, *J* = 8.0, 2.1 Hz, 1H), 8.20 (d, *J* = 5.4 Hz, 1H), 7.92-7.83 (m, 3H), 7.74 (t, *J* = 7.9 Hz, 1H), 7.43-7.33 (m, 3H), 6.40 (d, *J* = 5.4 Hz, 1H), 5.27 (dd, *J* = 7.1, 4.6 Hz, 1H), 4.76 (d, *J* = 4.7 Hz, 1H), 3.88-3.87 (m, 1H), 3.28-3.17 (m, 1H), 3.12-3.01 (m, 1H), 1.08 (d, *J* = 6.2 Hz, 3H). |
| **Final product 152** | **B2** | 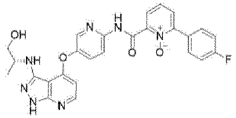 | MS (ESI⁺)m/z = 516.7 [M+H]⁺. ¹H NMR (400 MHz, DMSO-*d*₆) *δ* 14.10 (s, 1H), 12.17 (s, 1H), 8.49-8.36 (m, 3H), 8.15 (d, *J* = 5.5 Hz, 1H), 7.95-7.88 (m, 4H), 7.76 (t, *J* = 7.9 Hz, 1H), 7.47-7.32 (m, 2H), 6.10 (d, *J* = 5.4 Hz, 1H), 5.43 (dd, *J* = 7.2, 4.6 Hz, 1H), 4.79 (d, *J* = 4.7 Hz, 1H), 3.93-3.91 (m, 1H), 3.31-3.30 (m, 1H), 3.16-3.07 (m, 1H), 1.11 (d, *J* = 6.1 Hz, 3H). |
| **Final product 153** | **B2** | 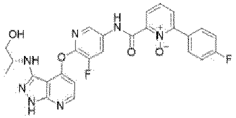 | MS (ESI⁺)m/z = 534.6 [M+H]⁺. ¹H NMR (600 MHz, DMSO-*d*₆) *δ* 13.75 (s, 1H), 12.23 (s, 1H), 8.56-8.51 (m, 2H), 8.35 (dd, *J* = 8.0, 2.1 Hz, 1H), 8.22 (d, *J* = 5.4 Hz, 1H), 7.95-7.81 (m, 3H), 7.76 (t, *J* = 7.9 Hz, 1H), 7.39 (t, *J* = 7.9 Hz, 2H), 6.42 (d, *J* = 5.4 Hz, 1H), 5.40-5.30 (m, 1H), 4.76 (d, *J* = 4.8 Hz, 1H), 3.99-3.82 (m, 1H), 3.29-3.23 (m, 1H), 3.18-2.98 (m, 1H), 1.09 (d, *J* = 6.2 Hz, 3H). |
| **Final product 154** | **B2** | 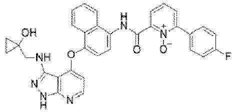 | Yellow solid, yield: 10.0 %. MS (ESI⁺)m/z = 576.7 [M+H]⁺. ¹H NMR (400 MHz, DMSO-*d*₆) *δ* 14.54 (s, 1H), 12.20 (s, 1H), 8.53 (d, *J* = 8.4 Hz, 1H), 8.49-8.45 (m, 1H), 8.15 (d, *J* = 8.5 Hz, 1H), 8.04 (d, *J* = 5.3 Hz, 1H), 7.94-7.90 (m, 4H), 7.79 (t, *J* = 7.9 Hz, 1H), 7.72 (t, *J* = 7.4 Hz, 1H), 7.63-7.60 (m, 2H), 7.40 (t, *J* = 8.8 Hz, 2H), 6.06 (brs, 1H), 5.88 (d, *J* = 5.2 Hz, 1H), 4.20-4.04 (m, 2H), 2.57-2.54 (m, 2H), 0.93 (t, *J* = 7.3 Hz, 2H). |
| **Final product 155** | **B3** | 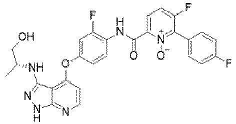 | MS (ESI⁺)m/z = 551.4 [M+H]⁺. ¹H NMR (600 MHz, DMSO-*d*₆) *δ* 13.70 (d, *J* = 2.0 Hz, 1H), 12.18 (s, 1H), 8.60-8.45 (m, 2H), 8.15 (d, *J* = 5.4 Hz, 1H), 7.87 (dd, *J* = 9.3, 7.1 Hz, 1H), 7.76-7.68 (m, 2H), 7.52-7.40 (m, 3H), 7.28-7.21 (m, 1H), 6.11 (d, *J* = 5.5 Hz, 1H), 5.04 (d, *J* = 7.1 Hz, 1H), 4.80 (s, 1H), 3.82-3.71 (m, 1H), 3.56-3.44 (m, 2H), 1.20 (d, *J* = 6.5 Hz, 3H). |
| **Final product 157** | **B1** | 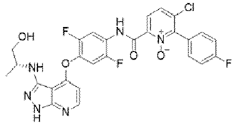 | MS (ESI⁺)m/z = 585.1 [M+H]⁺. ¹H NMR (600 MHz, DMSO-*d*₆) *δ* 13.91 (s, 1H), 12.21 (s, 1H), 8.53 (dd, *J* = 12.1, 7.1 Hz, 1H), 8.42 (d, *J* = 8.9 Hz, 1H), 8.16 (d, *J* = 5.5 Hz, 1H), 8.03 (d, *J* = 8.9 Hz, 1H), 7.76 (dd, *J* = 10.8, 7.3 Hz, 1H), 7.60-7.55 (m, 2H), 7.43 (t, *J* = 8.9 Hz, 2H), 6.13 (d, *J* = 5.2 Hz, 1H), 5.10 (d, *J* = 7.3 Hz, 1H), 4.78 (t, *J* = 5.5 Hz, 1H), 3.86-3.72 (m, 1H), 3.59-3.46 (m, 2H), 1.21 (d, *J* = 6.5 Hz, 3H). |
| **Final product 158** | **B1** | 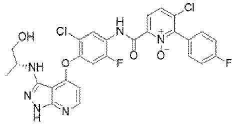 | MS (ESI⁺)m/z = 603.1 [M+H]⁺. ¹H NMR (400 MHz, DMSO-*d*₆) *δ* 13.91 (s, 1H), 12.23 (s, 1H), 8.73 (d, *J* = 7.7 Hz, 1H), 8.42 (d, *J* = 8.9 Hz, 1H), 8.15 (d, *J* = 5.5 Hz, 1H), 8.03 (d, *J* = 8.9 Hz, 1H), 7.79 (d, *J* = 10.9 Hz, 1H), 7.61-7.51 (m, 2H), 7.45-7.41 (m, 2H), 6.04 (d, *J* = 5.4 Hz, 1H), 5.07 (d, *J* = 7.2 Hz, 1H), 4.81 (s, 1H), 3.84-3.72 (m, 1H), 3.49 (s, 2H), 1.21 (d, *J* = 6.5 Hz, 3H). |
| **Final product 159** | **B1** | 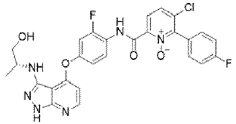 | MS (ESI⁺)m/z = 567.1 [M+H]⁺. ¹H NMR (600 MHz, DMSO-*d*₆) *δ* 13.70 (s, 1H), 12.17 (s, 1H), 8.54 (t, *J* = 8.9 Hz, 1H), 8.42 (d, *J* = 8.9 Hz, 1H), 8.15 (d, *J* = 5.4 Hz, 1H), 8.01 *(d, J* = 8.9 Hz, 1H), 7.65-7.53 (m, 2H), 7.53-7.34 (m, 3H), 7.28-7.20 (m, 1H), 6.11 (d, *J* = 5.4 Hz, 1H), 5.03 (d, *J* = 7.3 Hz, 1H), 4.79 (t, *J* = 5.5 Hz, 1H), 3.79-3.75 (m, 1H), 3.53-3.49 (m, 2H), 1.20 (d, *J* = 6.5 Hz, 3H). |
| **Final product 160** | **B4** | 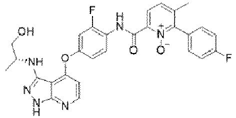 | MS (ESI⁺)m/z = 547.2 [M+H]⁺. ¹H NMR (400 MHz, DMSO-*d*₆) *δ* 14.20 (d, *J* = 2.1 Hz, 1H), 12.17 (s, 1H), 8.57 (t, *J* = 8.9 Hz, 1H), 8.37 (d, *J* = 8.4 Hz, 1H), 8.15 (d, *J* = 5.4 Hz, 1H), 7.73 (d, *J* = 8.4 Hz, 1H), 7.58-7.35 (m, 5H), 7.25-7.21 (m, 1H), 6.10 (d, *J* = 5.4 Hz, 1H), 5.04 (d, *J* = 7.3 Hz, 1H), 4.80 (s, 1H), 3.85-3.70 (m, 1H), 3.50-3.45 (m, 2H), 2.14 (s, 3H), 1.20 (d, *J* = 6.4 Hz, 3H). |
| **Final product 174** | **B2** | 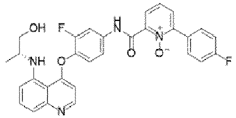 | Yellow solid, yield: 28.5%. MS (ESI⁺)m/z = 543.0 [M+H]⁺. ¹H NMR (400 MHz, DMSO-*d*₆) *δ* 13.65 (s, 1H), 10.16 (d, *J* = 7.6 Hz, 1H), 8.33 (dd, *J* = 8.0, 2.4 Hz, 1H), 8.09 (d, *J* = 12.0 Hz, 1H), 7.93-7.89 (m, 2H), 7.77-7.73 (m, 2H), 7.72-7.70 (m, 1H), 7.65-7.60 (m, 1H), 7.40 (t, *J* = 8.8 Hz, 1H), 7.24 (t, *J* = 8.4 Hz, 1H), 6.41 (d, *J* = 8.4 Hz, 1H), 6.00 (d, *J* = 7.6 Hz, 1H), 5.79 (d, *J* = 8.0 Hz, 1H), 4.87-4.84 (m, 1H), 3.58-3.50 (m, 2H), 1.19 (dd, *J* = 6.0, 3.2 Hz, 3H). |

### Example 109. Preparation of 2-((3-fluoro-4-((3-(4-hydroxypiperidin-1-yl)-1H-pyrazolo[3,4-b]pyridin-4-yl)oxy)phenyl)carbamoyl)-6-(4-fluorophenyl)pyridine 1-oxide (final product 26)

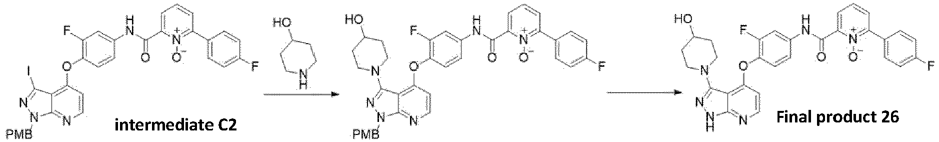

### Step 1: 2-((3-fluoro-4-((3-(4-hydroxypiperidin-1-yl)-1-(4-methoxybenzyl)-1H-pyrazolo[3,4-b]pyridin-4-yl)oxy)phenyl)carbamoyl)-6-(4-fluorophenyl)pyridine 1-oxide

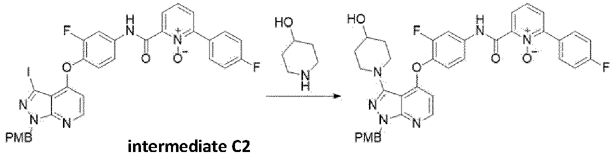

2-((3-fluoro-4-((3-iodo-1-(4-methoxybenzyl)-1H-pyrazolo[3,4-b]pyridin-4-yl)oxy)phenyl)carbamoyl)-6-(4-fluorophenyl)pyridine 1-oxide (100 mg, 0.14 mmol) was dissolved in dimethyl sulfoxide (2 mL) at room temperature, followed by piperidine-4-ol (57 mg, 0.57 mmol), pyrrole-2-carboxylic acid (6.03 mg, 0.057 mmol), anhydrous potassium carbonate (59 mg, 0.43 mmol) and cuprous iodide (11 mg, 0.057 mmol). The air was replaced with nitrogen for three times, the reaction system was heated to 75 °C, and stirred for 12 hours. After the reaction was completed through TLC monitoring, cooled to room temperature, water (20 mL) was added, extracted with ethyl acetate (20 mL × 2), the organic phases were combined and washed with saturated brine (20 mL × 1), dried over anhydrous sodium sulfate, filtered and the filtrate was concentrated under reduced pressure. The residue was isolated and purified by silica gel thin layer chromatography with an eluent of methanol/dichloromethane = 1/20 to give 2-((3-fluoro-4-((3-(4-hydroxypiperidin-1-yl)-1-(4-methoxybenzyl)-1H-pyrazolo[3,4-b]pyridin-4-yl)oxy)phenyl)carbamoyl)-6-(4-fluorophenyl)pyridine 1-oxide (30 mg, colorless oil), yield: 30.9%. MS (ESI⁺)m/z = 678.9 [M+H]⁺.

### Step 2: 2-((3-fluoro-4-((3-(4-hydroxypiperidin-1-yl)-1H-pyrazolo[3,4-b]pyridin-4-yl)oxy)phenyl)carbamoyl)-6-(4-fluorophenyl)pyridine 1-oxide

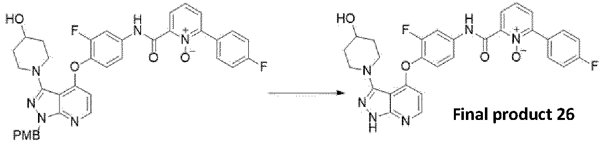

2-((3-fluoro-4-((3-(4-hydroxypiperidin-1-yl)-1-(4-methoxybenzyl)-1H-pyrazolo[3,4-b]pyridin-4-yl)oxy)phenyl)carbamoyl)-6-(4-fluorophenyl)pyridine 1-oxide (30 mg, 0.042 mmol) and trifluoroacetic acid (2 mL) were added sequentially to a 50 mL round bottom flask and stirred at room temperature for 16 h. After the reaction was completed through TLC monitoring, the reaction system was concentrated under reduced pressure, and the residue was isolated and purified by silica gel thin layer chromatography with an eluent of methanol/dichloromethane = 1/15 to give 2-((3-fluoro-4-((3-(4-hydroxypiperidin-1-yl)-1H-pyrazolo[3,4-b]pyridin-4-yl)oxy)phenyl)carbamoyl)-6-(4-fluorophenyl)pyridine 1-oxide (11.0 mg).

White solid, yield 43.2%. MS (ESI⁺)m/z = 559.0 [M+H]⁺. ¹H NMR (400 MHz, DMSO-*d*₆) *δ* 13.59 (s, 1H), 12.68 (s, 1H), 8.33 (dd, *J* = 8.0, 2.0 Hz, 1H), 8.22 (d, *J* = 5.4 Hz, 1H), 8.07 (dd, *J* = 12.7, 2.2 Hz, 1H), 7.89-7.87 (m, 3H), 7.74 (t, *J* = 7.9 Hz, 1H), 7.63 (d, *J* = 8.9 Hz, 1H), 7.51 (t, *J* = 8.9 Hz, 1H), 7.39 (t, *J* = 8.9 Hz, 2H), 6.18 (d, *J* = 4.0 Hz, 1H), 4.66 (d, *J* = 4.0 Hz, 1H), 3.66-3.61 (m, 3H), 2.97 (t, *J* = 10.2 Hz, 2H), 1.90-1.80 (m, 2H), 1.55-1.50 (m, 2H).

### Examples 110-115 Preparation of final products 1, 4, 22, 28, 102, 107

Final products 1, 4, 22, 28, 102, 107 were synthesized from intermediate C and commercially available alcohol amines according to the method for synthesing the final product 26. (Table 3).

**Table 3 final products 1, 4, 22, 28, 102, 107**

| **Final product s Nos.** | **Inter media te Nos.** | **Structures of Final products** | **NMR or MS** |
|---|---|---|---|
| **Final product 1** | **C2** | 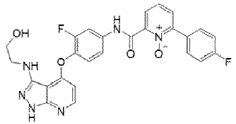 | MS (ESI⁺)m/z = 518.9 [M+H]⁺. ¹H NMR (400 MHz, DMSO-*d*₆) *δ* 13.55 (s, 1H), 12.20 (s, 1H), 8.32 (dd, *J* = 8.0, 2.1 Hz, 1H), 8.15 (brs, 1H), 8.07 (d, *J* = 12.5 Hz, 1H), 7.91-7.87 (m, 3H), 7.74 (t, *J* = 7.9 Hz, 1H), 7.64 (d, *J* = 8.4 Hz, 1H), 7.51 (t, *J* = 8.6 Hz, 1H), 7.39 (t, *J* = 9.0 Hz, 2H), 6.04 (d, *J* = 5.6 Hz, 1H), 3.64 (t, *J* = 5.9 Hz, 2H), 3.37-3.35 (m, 2H). |
| **Final product 4** | **C2** | 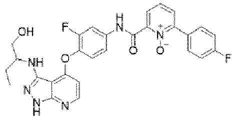 | MS (ESI⁺)m/z = 547.0 [M+H]⁺. ¹H NMR (400 MHz, DMSO-*d*₆) *δ* 13.57 (s, 1H), 12.16 (s, 1H), 8.32 (dd, *J* = 8.0, 2.2 Hz, 1H), 8.14 (d, *J* = 5.4 Hz, 1H), 8.06 (dd, *J* = 12.7, 2.4 Hz, 1H), 7.91-7.85 (m, 3H), 7.74 (t, *J* = 7.9 Hz, 1H), 7.64 (d, *J* = 7.7 Hz, 1H), 7.51 (t, *J* = 8.9 Hz, 1H), 7.39 (t, *J* = 9.0 Hz, 2H), 6.04 (d, *J* = 8.0 Hz, 1H), 5.05 (d, *J* = 8.0 Hz, 1H), 4.73 (brs, 1H), 3.62-3.56 (m, 3H), 1.67-1.62 (m, 2H), 0.92 (t, *J* = 8.0 Hz, 3H). |
| **Final product 22** | **C2** | 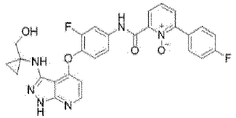 | MS (ESI⁺)m/z = 544.9 [M+H]⁺. ¹H NMR (400 MHz, DMSO-*d*₆) *δ* 13.56 (s, 1H), 12.25 (s, 1H), 8.32 (dd, *J* = 8.0, 2.1 Hz, 1H), 8.16 (brs, 1H), 8.06 (d, *J* = 12.7 Hz, 1H), 7.92-7.85 (m, 3H), 7.74 (t, *J* = 7.9 Hz, 1H), 7.64 (d, *J* = 7.5 Hz, 1H), 7.49 (t, *J* = 8.6 Hz, 1H), 7.39 (t, *J* = 8.9 Hz, 2H), 6.05 (d, *J* = 4.0 Hz, 1H), 3.59 (s, 2H), 1.23-1.19 (m, 2H), 0.80-0.77 (m, 2H). |
| **Final product 28** | **C2** | 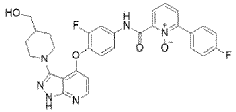 | MS (ESI⁺)m/z = 572.8 [M+H]⁺. ¹H NMR (400 MHz, DMSO-*d*₆) *δ* 13.59 (s, 1H), 12.68 (s, 1H), 8.33-8.33 (m, 1H), 8.21 (d, *J* = 5.4 Hz, 1H), 8.05 (dd, *J* = 12.7, 2.2 Hz, 1H), 7.89-7.87 (m, 3H), 7.74 (t, *J* = 7.9 Hz, 1H), 7.62 (d, *J* = 8.9 Hz, 1H), 7.51 (t, *J* = 8.9 Hz, 1H), 7.39 (t, *J* = 8.9 Hz, 2H), 6.18 (d, *J* = 4.0 Hz, 1H), 3.89-3.86 (m, 2H), 3.29-3.27 (m, 2H), 2.75-2.73 (m, 2H), 1.90-1.80 (m, 2H), 1.56-1.51 (m, 1H), 1.34-1.23 (m, 2H). |
| **Final product 102** | **C1** | 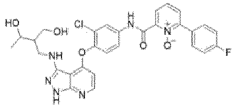 | Yellow solid, yield: 24.0%. MS (ESI⁺)m/z = 593.0 [M+H]⁺. ¹H NMR (400 MHz, CD₃OD) *δ* 8.49 (dd, *J* = 8.0, 2.4 Hz, 1H), 8.26 (d, *J* = 2.8 Hz, 1H), 8.15 (d, *J* = 5.6 Hz, 1H), 7.92-7.88 (m, 2H), 7.84 (dd, *J* = 8.0, 2.4 Hz, 1H), 7.79-7.72 (m, 2H), 7.45 (d, *J* = 8.8 Hz, 1H), 7.31 (t, *J* = 8.8 Hz, 2H), 6.05 (d, *J* = 5.6 Hz, 1H), 4.06-4.02 (m, 1H), 3.84-3.77 (m, 2H), 3.71-3.64 (m, 1H), 3.63-3.56 (m, 1H), 3.53-3.47 (m, 1H), 1.98-1.93 (m, 1H), 1.29 (d, *J* = 6.4 Hz, 3H). |
| **Final product 107** | **C1** | 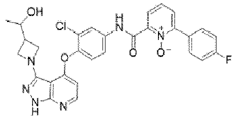 | White-like solid, yield: 10.4%. MS (EST⁺)m/z = 575.0 [M+H]⁺. ¹H NMR (400 MHz, CD₃OD) *δ* 8.49 (dd, *J* = 8.0, 2.0 Hz, 1H), 8.27 (d, *J* = 2.4 Hz, 1H), 8.19 (d, *J* = 5.6 Hz, 1H), 7.92-7.82 (m, 2H), 7.83 (dd, *J* = 7.6, 2.0 Hz, 1H), 7.79-7.72 (m, 2H), 7. 42 (d, *J* = 8.8 Hz, 1H), 7.31 (t, *J* = 8.8 Hz, 2H), 6.10 (d, *J* = 5.2 Hz, 1H), 4.27-4.20 (m, 2H), 4.11 (t, *J* = 6.4 Hz, 1H), 3.98-3.90 (m, 2H), 2.74-2.70 (m, 1H), 1. 17 (d, *J* = 6.4 Hz, 3H). |

### Example 116. Preparation of 2-((3-fluoro-4-((5-(3-hydroxypropyl)-5H-pyrrolo[3,2-d]pyrimidin-4-yl)oxy)phenyl)carbamoyl)-6-(4-fluorophenyl)pyridine 1-oxide (final product 168)

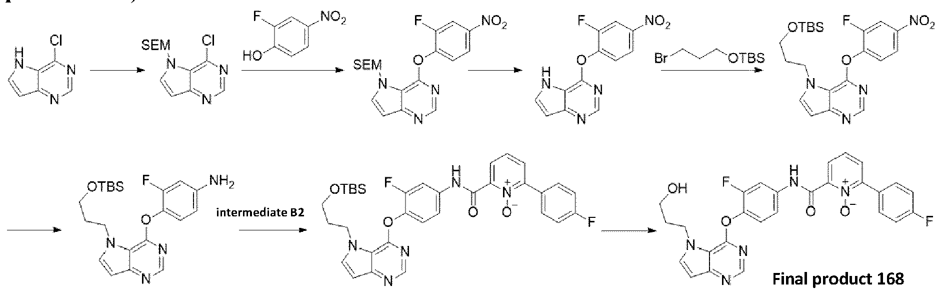

### Step 1: 4-chloro-5-((2-(trimethylsilyl)ethoxy)methyl)-5Hpyrrolo[3,2-d]pyrimidine

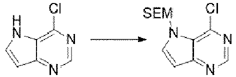

4-chloro-5H-pyrrolo[3,2-d]pyrimidine (5.0 g, 32.6 mmol) was dissolved in anhydrous DMF (50.0 mL) under nitrogen atmosphere, sodium hydride (1.56 g, 39.12 mmol) was added in batches at 0 °C, stirred for 30 min, 2-(trimethylsilyl)ethoxymethyl chloride (5.98 g, 39.86 mmol) was slowly added dropwise, and after dropping, the mixture was allowed to warm to room temperature and stirred for 3 h. After the reaction was completd through TLC monitoring, saturated ammonium chloride solution (100 mL) was added and extracted with ethyl acetate (50 mL × 2). The organic phases were combined and washed with saturated brine, dried over anhydrous sodium sulfate, filtered, and the filtrate was concentrated under reduced pressure to give 4-chloro-5-((2-(trimethylsilyl)ethoxy)methyl)-5H-pyrrolo[3,2-d]pyrimidine (2.0 g, red solid), yield: 17.6%. MS (ESI⁺)m/z = 284.1 [M+H]⁺.

### Step 2: 4-(2-fluoro-4-nitrophenoxy)-5-((2-(trimethylsilyl)ethoxy)methyl)-5H-pyrrolo [3,2-d] pyrimidine

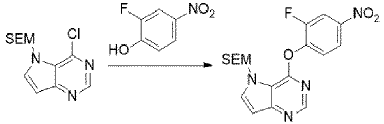

4-chloro-5-((2-(trimethylsilyl)ethoxy)methyl)-5H-pyrrolo[3,2-d]pyrimidine (2.8 g, 9.9 mmol) and 2-fluoro-4-nitrophenol (1.56 g, 9.9 mmol) were added to xylene (30.0 mL) respectively, and the mixture was warmed to 120 °C and stirred overnight. After the reaction was completed through TLC monitoring, concentrated under reduced pressure, the residue was slurried with methyl tert-butyl ether, filtered, and the filter cake was dried in vacuum to give 4-(2-fluoro-4-nitrophenoxy)-5-((2-(trimethylsilyl)ethoxy)methyl)-5H-pyrrolo[3,2-d]pyrimidine (2.2 g, Brown red solid), yield: 80.0%. MS (ESI⁺)m/z = 405.1 [M+H]⁺.

### Step 3: 4-(2-fluoro-4-nitrophenoxy)-5H-pyrrolo[3,2-d]pyrimidine

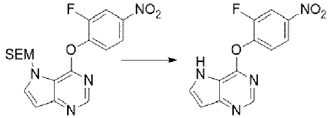

4-(2-fluoro-4-nitrophenoxy)-5-((2-(trimethylsilyl)ethoxy)methyl)-5H-pyrrolo[3,2-d]pyrimidine (2.4 g, 5.9 mmol) and trifluoroacetic acid (20.0 mL) were added to a 100 mL round bottom flask, stirred overnight at room temperature, concentrated under reduced pressure after the reaction was completed through TLC monitoring, the resulting residue was dissolved in ethanol (10.0 mL), potassium carbonate (0.82 g, 5.9 mmol) was added and stirred at room temperature for 1 h, filtrated, the filter cake was washed with ethanol (50.0 mL), the filtrate was concentrated under reduced pressure, and the residue was purified by flash silica gel chromatography with a gradient elution system of methanol/dichloromethane = 0-10% to give 4-(2-fluoro-4-nitrophenoxy)-5H-pyrrolo[3,2-d]pyrimidine (1.3 g, white solid). Yield: 93%. MS (ESI⁺)m/z = 275.1 [M+H]⁺.

### Step 4: 5-(3-((tert-butyldimethylsilyl)oxy)propyl)-4-(2-fluoro-4-nitrophenoxy)-5H-pyrrolo[3,2-d]pyrimidine

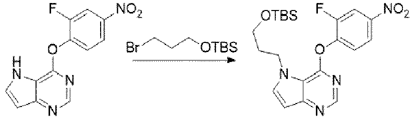

4-(2-fluoro-4-nitrophenoxy)-5H-pyrrolo[3,2-d]pyrimidine (1.3 g, 4.7 mmol) was dissolved in DMF (15.0 mL) under nitrogen atmosphere, cesium carbonate (3.06 g, 9.4 mmol) and then (3-bromopropoxy)(tert-butyl)dimethylsilane (1.31 g, 5.17 mmol) were added, and the mixture was stirred at room temperature overnight. After the reaction was completed through TLC monitoring, the reaction solution was poured into water (20 mL), extracted with ethyl acetate (50 mL × 3), the organic phases were combined and washed with brine, dried over anhydrous sodium sulfate, filtered, the filtrate was concentrated under reduced pressure and the residue was purified by flash silica gel column chromatography with a gradient elution system of ethyl acetate/petroleum ether = 0-100% to give 5-(3-((tert-butyldimethylsilyl)oxy)propyl)-4-(2-fluoro-4-nitrophenoxy)-5H-pyrrolo[3,2-d]pyrimidine (1.2 g, yellow oil). Yield: 71%. MS (ESI⁺)m/z = 447.3 [M+H]⁺.

### Step 5: 4-((5-(3-((tert-butyldimethylsilyl)oxy)propyl)-5H-pyrrolo[3,2-d]pyrimidin-4-yl)oxy)-3-fluoroaniline

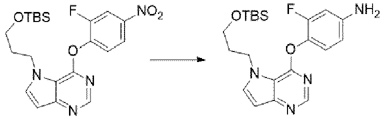

5-(3-((tert-butyldimethylsilyl)oxy)propyl)-4-(2-fluoro-4-nitrophenoxy)-5H-pyrrolo[3,2-d]pyrimidine (1.2 g, 2.7 mmol) and ammonium chloride (1.44 g, 27 mmol) were added to a mixed solution of ethanol/water (10/1, 20 mL) at room temperature, stirred to dissolve, then reduced iron powder (1.51 g, 27 mmol) was added in batches, warmed to 80 °C and stirred for 2 h. After the reaction was completed through TLC monitoring and allowed to cool to room temperature, filtered, the filter cake was washed with ethanol (300 mL) and the filtrate was concentrated under reduced pressure. The residue was purified by flash silica gel column chromatography with a gradient eluent system of methanol/dichloromethane = 0-10% to give 4-((5-(3-((tertbutyldimethylsilyl)oxy)propyl)-5H-pyrrolo[3,2-d]pyrimidin-4-yl)oxy)-3-fluoroaniline (580 mg, a light yellow solid). Yield: 90%. MS (ESI⁺)m/z = 417.3 [M+H]⁺.

### Step 6: 2-((4-((5-(3-((tert-butyldimethylsilyl)oxy)propyl)-5H-pyrrolo[3,2-d]pyrimidin-4-yl)oxy)-3-fluorophenyl)carbamoyl)-6-(4-fluorophenyl)pyridine 1-oxide

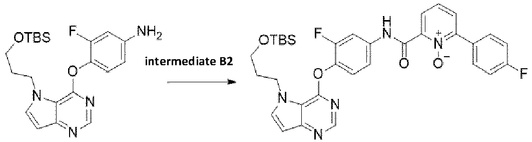

4-((5-(3-((tert-butyldimethylsilyl)oxy)propyl)-5Hpyrrolo[3,2-d]pyrimidin-4-yl)oxy)-3-fluoroaniline (300.0 mg, 0.72 mmol), intermediate B2(184.74 mg, 0.79 mmol) and HATU (410.76 mg, 1.08 mmol) were added to DMF (10.0 mL) at room temperature, stirred to dissolve, then triethylamine (145.75 mg, 0.91 mmol) was added with stirring. The reaction mixture was stirred at room temperature for 2 h. After the reaction was completed through TLC monitoring, water (30.0 mL) was added, extracted with ethyl acetate (50 mL × 2), the organic phases were combined and washed with saturated brine, dried over anhydrous sodium sulfate, filtered, the filtrate was concentrated under reduced pressure and the residue was purified by thin layer chromatography with an eluent system of methanol/dichloromethane = 1/100 to give 2-((4-((5-(3-((tert-butyldimethylsilyl)oxy)propyl)-5H-pyrrolo[3,2-d]pyrimidin-4-yl)oxy)-3-fluorophenyl)carbamoyl)-6-(4-fluorophenyl)pyridine 1-oxide (200.0 mg, yellow liquid). Yield: 39.6%. MS (ESI⁺)m/z = 631.1 [M+H]⁺.

### Step 7: 2-((3-fluoro-4-((5-(3-hydroxypropyl)-5H-pyrrolo[3,2-d]pyrimidin-4-yl)oxy)phenyl)carbamoyl)-6-(4-fluorophenyl)pyridine 1-oxide

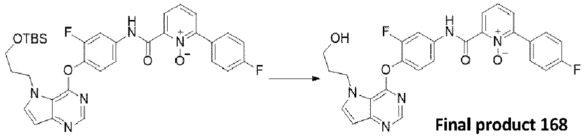

2-((4-((5-(3-((tert-butyldimethylsilyl)oxy)propyl)-5H-pyrrolo[3,2-d]pyrimidin-4-yl)oxy)-3-fluorophenyl)carbamoyl)-6-(4-fluorophenyl)pyridine 1-oxide (180.0 mg, 0.28 mmol) was dissolved in THF (5.0 mL), a solution of tetrabutylammonium fluoride in tetrahydrofuran (0.3 mL, 0.28 mmol) was added with stirring at room temperature and stirred for 1 h at room temperature. After the reaction was completed through TLC monitoring, water (10.0 mL) was added, extracted with ethyl acetate (10 mL × 2), the organic phases were combined and washed with saturated brine, dried over anhydrous sodium sulfate, filtered, the filtrate was concentrated under reduced pressure and the residue was purified by thin layer chromatography with an eluent system of methanol/dichloromethane = 1/100 to give 2-((3-fluoro-4-((5-(3-hydroxypropyl)-5H-pyrrolo[3,2-d]pyrimidin-4-yl)oxy)phenyl)carbamoyl)-6-(4-fluorophenyl)pyridine 1-oxide (24.0 mg).

White solid, yield 11.9%. MS (ESI⁺)m/z = 518.0 [M+H]⁺. ¹H NMR (400 MHz, CDCl₃) *δ* 13.99 (s, 1H), 8.60 (dd, *J* = 5.6, 4.4 Hz, 1H), 8.50 (s, 1H), 8.02 (dd, *J* = 12.0, 2.4 Hz, 1H), 7.80-7.75 (m, 2H), 7.62-7.60 (m, 2H), 7.53-7.47 (m, 2H), 7.35 (t, *J* = 8.4 Hz, 1H), 7.29-7.25 (m, 2H), 6.75 (d, *J* = 2.4 Hz, 1H), 4.46 (t, *J* = 6.8 Hz, 2H), 3.67 (t, *J* = 5.6 Hz, 2H), 2.20 (t, *J* = 6.0 Hz, 2H).

### Example 117 preparation of (S)-2-((3-fluoro-4-((5-(3-hydroxy-2-methylpropyl)-5H-pyrrolo[3,2-d]pyrimidin-4-yl)oxy)phenyl)carbamoyl)-6-(4-fluorophenyl)pyridine 1-oxide (final product 169)

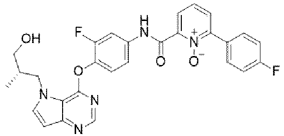

Final product 169 was prepared from (S)-3-((tert-butyldiphenylsilyl)oxy)-2-methylpropyl 4-methylbenzenesulfonate and intermediate B2 according to the above-described preparation method of final product 168.

White solid, yield 55.6%. MS (ESI⁺)m/z = 532.0 [M+H]⁺. ¹H NMR (400 MHz, CD₃OD) *δ* 8.49 (dd, *J* = 8.0, 2.0 Hz, 1H), 8.30 (s, 1H), 8.02 (dd, *J* = 12.0, 2.0 Hz, 1H), 7.91-7.88 (m, 2H), 7.82 (dd, *J* = 8.0, 2.4 Hz, 1H), 7.78-7.74 (m, 2H), 7.51 (dd, *J* = 8.8, 1.2 Hz, 1H), 7.43 (t, *J* = 8.4 Hz, 1H), 7.30 (t, *J* = 8.8 Hz, 2H), 6.67 (d, *J* = 3.2 Hz, 1H), 4.61-4.56 (m, 1H), 4.41-4.36 (m, 1H), 3.52-3.44 (m, 2H), 2.35-2.30 (m, 1H), 0.96 (d, *J* = 6.8 Hz, 3H).

### Example 118 preparation of (R)-2-((3-fluoro-4-((5-(3-hydroxy-2-methylpropyl)-5H-pyrrolo[3,2-d]pyrimidin-4-yl)oxy)phenyl)carbamoyl)-6-(4-fluorophenyl)pyridine 1-oxide (final product 170)

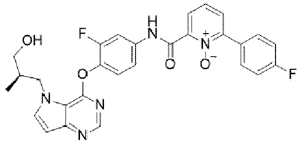

Final product 170 was prepared from (R)-3-((tert-butyl diphenylsilyl)oxy)-2-methyl propyl 4-methyl benzene sulfonate and intermediate B2 as raw materials according to the preparation method of final product 168.

Light yellow solid, yield 54.9%. MS (ESI⁺)m/z = 532.1 [M+H]⁺. ¹H NMR (400 MHz, CDCl₃) *δ* 14.02 (s, 1H), 8.61-8.58 (m, 1H), 8.54 (s, 1H), 8.04 (d, *J* = 12.0 Hz, 1H), 7.78 (dd, *J* = 8.8, 5.2 Hz, 2H), 7.63-7.58 (m, 3H), 7.49 (d, *J* = 8.8 Hz, 1H), 7.35 (t, *J* = 8.8 Hz, 1H), 7.28-7.25 (m, 2H), 6.87 (s, 1H), 4.66-4.62 (m, 1H), 4.44-4.38 (m, 1H), 3.58 (d, *J*= 4.8 Hz, 2H), 2.37-2.33 (m, 1H), 1.02 (d, *J* = 7.2 Hz, 3H).

### Example 119 preparation of (R)-3-chloro-6-((3-fluoro-4-((5-(3-hydroxy-2-methylpropyl)-5H-pyrrolo[3,2-d]pyrimidin-4-yl)oxy)phenyl)carbamoyl)-2-(4-fluorophenyl)pyridine 1-oxide (final product 171)

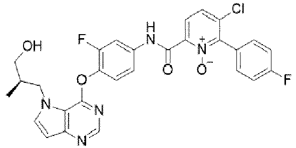

Final product 171 was prepared from (R)-3-((tert-butyldiphenylsilyl)oxy)-2-methylpropyl 4-methylbenzenesulfonate and intermediate B1 according to the above-described preparation method of final product 168.

Yellow solid, yield 24.6%. MS (ESI⁺)m/z = 565.9 [M+H]⁺. ¹H NMR (400 MHz, CD₃OD) *δ* 8.47 (d, *J* = 8.8 Hz, 1H), 8.29 (s, 1H), 8.00 (dd, *J* = 12.4, 2.4 Hz, 1H), 7.91 *(d, J* = 8.8 Hz , 1H), 7.74 (*d, J* = 3.2 Hz, 1H), 7.58-7.54 (m, 2H), 7.48-7.39 (m, 2H), 7.33 (t, *J* = 8.8 Hz, 2H), 6.66 (d, *J* = 3.6 Hz, 1H), 4.60-4.55 (m, 1H), 4.40-4.34 (m, 1H), 3.51-3.43 (m, 2H), 2.34-2.28 (m, 1H), 0.95 (d, *J* = 6.8 Hz, 3H).

### Example 120 preparation of (R)-2-((3-fluoro-4-((5-((1-hydroxypropan-2-yl)oxy)-6-methoxyquinazolin-4-yl)oxy)phenyl)carbamoyl)-6-(4-fluorophenyl)pyridine 1-oxide (final product 176)

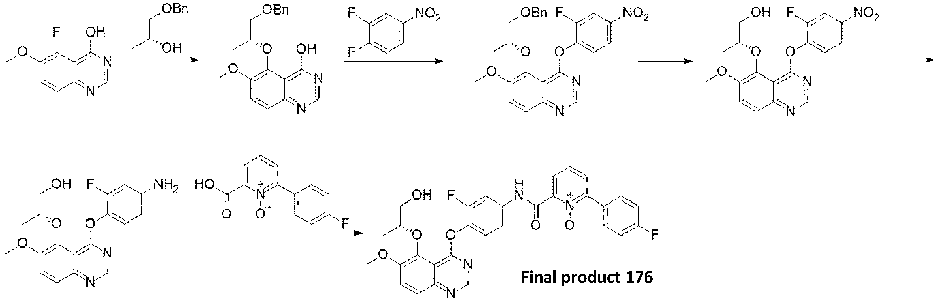

### Step 1: (R)-5-((1-(benzyloxy)propan-2-yl)oxy)-6-methoxyquinazolin-4-ol

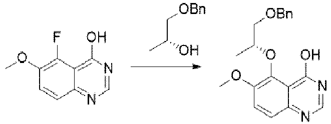

Under nitrogen atmosphere, (R)-1-(benzyloxy)propan-2-ol (390 mg, 2.3 mmol) and tetrahydrofuran (10 mL) were added to a 100 mL three-necked flask, followed by sodium hydride (470 mg, 11.7 mmol) in batches, stirred at 25 °C for 1 h, 5-fluoro-6-methoxyquinazolin-4-ol (380 mg, 2.0 mmol) was added, warmed to 65 °C and stirred for 15 h. After the reaction was completed through TLC monitoring, quenched by addition of saturated ammonium chloride (10 mL), diluted with water (50 mL), the mixture was extracted with ethyl acetate (60 mL × 3), the organic phases were combined and washed with water (60 mL) and saturated brine (60 mL), dried over anhydrous sodium sulfate, filtered, the filtrate was concentrated under reduced pressure, and the residue was purified by column chromatography an eluent system of methanol/dichloromethane = 1/10 to give (R)-5-((1-(benzyloxy)prop-2-yl)oxy)-6-methoxyquinazolin-4-ol (400 mg, yellow solid), yield: 60.0%. MS (ESI⁺)m/z = 341.0 [M+H]⁺.

### Step 2: (R)-5-((1-(benzyloxy)propan-2-yl)oxy)-4-(2-fluoro-4-nitrophenoxy)-6-methoxyquinazoline

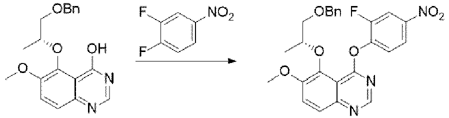

(R)-5-((1-(benzyloxy)prop-2-yl)oxy)-6-methoxyquinazolin-4-ol (200 mg, 0.59 mmol), 1, 2-difluoro-4-nitrobenzene (140 mg, 0.88 mmol), cesium carbonate (383 mg, 1.2 mmol), and DMF (5 mL) were added sequentially to a 100 mL round bottom flask at room temperature, warmed to 80 °C and stirred for 16 h. The reaction was completed through TLC monitoring, water (30 mL) was diluted for dilution, the mixture was extracted with ethyl acetate (30 mL × 3), the organic phases were combined and washed with water (30 mL × 2) and saturated brine (30 mL), dried over anhydrous sodium sulfate, filtered, and the filtrate was concentrated under reduced pressure. The residue was purified by thin layer chromatography with an eluent system of methanol/dichloromethane = 1/20 to give (R)-5-((1-(benzyloxy)propan-2-yl)oxy)-4-(2-fluoro-4-nitrophenoxy)-6-methoxyquinazoline (200 mg, yellow solid), yield: 71.0%. MS (ESI⁺)m/z = 480.0 [M+H]⁺.

### Step 3: (R)-2-((4-(2-fluoro-4-nitrophenoxy)-6-methoxyquinazolin-5-yl)oxy)propan-1-ol

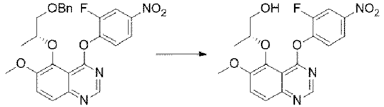

(R)-5-((1-(benzyloxy)prop-2-yl)oxy)-4-(2-fluoro-4-nitrophenoxy)-6-methoxyquinazoline (100 mg, 0.21 mmol) and 1,4-dioxane (3 mL) were added sequentially to a 50 mL round bottom flask, concentrated hydrochloric acid (3 mL) was slowly added dropwise, and the mixture was stirred for 1 h at 80 °C. after the reaction was completed through TLC monitoring, water (10 mL) was added to dilute, the mixture was adjusted to pH = 8 with saturated sodium bicarbonate, extracted with ethyl acetate (50 mL × 3), the organic phases were combined and washed with saturated brine (50 mL), dried over anhydrous sodium sulfate, filtered, and the filtrate was concentrated under reduced pressure. The residue was purified by thin layer chromatography with an eluent system of methanol/dichloromethane = 1/5 to give ((R)-2-((4-(2-fluoro-4-nitrophenoxy)-6-methoxyquinazolin-5-yl)oxy)propan-1-ol (17 mg, yellow solid), yield: 20.9%. MS (ESI⁺)m/z = 390.1 [M+H]⁺.

### Step 4: (R)-2-((4-(4-amino-2-fluorophenoxy)-6-methoxyquinazolin-5-yl)oxy)propan-1-ol

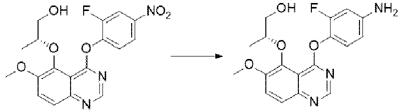

(R)-2-((4-(2-fluoro-4-nitrophenoxy)-6-methoxyquinazolin-5-yl)oxy)propan-1-ol (17 mg, 0.044 mmol), ethanol (2 mL), saturated ammonium chloride (2 mL) and iron powder (12 mg, 0.22 mmol) were added sequentially to a 50 mL round bottom flask, and the mixture was warmed to 60 °C and stirred for 2 h. After the reaction was completed through TLC monitoring, filtration was performed, and the filtrate was concentrated under reduced pressure to give crude (R)-2-((4-(4-amino-2-fluorophenoxy)-6-methoxyquinazolin-5-yl)oxy)propan-1-ol (11 mg, yellow solid), yield: 70.0%. MS (ESI⁺)m/z = 360.0 [M+H]⁺.

### Step 5: (R)-2-((3-fluoro-4-((5-((1-hydroxypropan-2-yl)oxy)-6-methoxyquinazolin-4-yl)oxy)phenyl)carbamoyl)-6-(4-fluorophenyl)pyridine 1-oxide

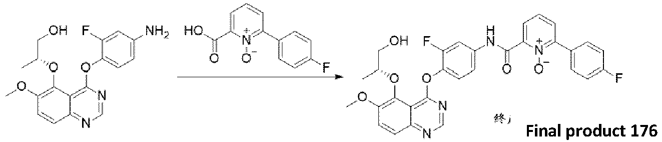

2-carboxy-6-(4-fluorophenyl)pyridine 1-oxide (intermediate B2, 7.1 mg, 0.031 mmol), DMF (3 mL), HATU (13 mg, 0.033 mmol), triethylamine (14 mg, 0.14 mmol) and (R)-2-((4-(4-amino-2-fluorophenoxy)-6-methoxyquinazolin-5-yl)oxy)propan-1-ol (10 mg, 0.028 mmol) were added sequentially to a 50 mL round bottom flask and stirred at 25 °C for 3 h. The reaction was completed through TLC monitoring, diluted with water (20 mL), the mixture was extracted with ethyl acetate (30 mL × 3), the organic phases were combined and washed with water (30 mL × 2) and saturated brine (30 mL), dried over anhydrous sodium sulfate, and filtered. The filtrate was concentrated under reduced pressure and the residue was purified by high performance liquid chromatography with an eluent system of acetonitrile/water/0.1% FA [chromatographic column: sunfire 5 µm 19-150 mm, mobile phase: ACN-H₂O (0.1% FA) 30-75-8 min]to give (R)-2-((3-fluoro-4-((5-((1-hydroxypropan-2-yl)oxy)-6-methoxyquinazolin-4-yl)oxy)phenyl)carbamoyl)-6-(4-fluorophenyl)pyridine 1-oxide (2.0 mg).

Off-white solid, yield 12.6%. MS (ESI⁺)m/z = 575.0 [M+H]⁺. ¹H NMR (400 MHz, CD₃OD) *δ* 8.49 (dd, *J* = 8.0, 2.0 Hz, 1H), 8.22 (s, 1H), 8.09 (d, *J* = 10.4 Hz, 1H), 7.94-7.87 (m, 2H), 7.85-7.81 (m, 1H), 7.79-7.74 (m, 1H), 7.62-7.50 (m, 2H), 7.31 (t, *J* = 8.8 Hz, 2H), 6.85 (d, *J* = 2.0 Hz, 1H), 6.78 (d, *J* = 2.0 Hz, 1H), 4.68-4.60 (m, 1H), 3.97 (s, 3H), 3.78-3.66 (m, 2H), 1.37 (d, *J* = 6.4 Hz, 3H).

### Example 121 (R)-2-((3-fluoro-4-(5-(1-hydroxypropan-2-yl)oxy)-6-methoxyquinazolin-4-yl)amino)phenyl)carbamoyl)-6-(4-fluorophenyl)pyridine 1-oxide (final product 181)

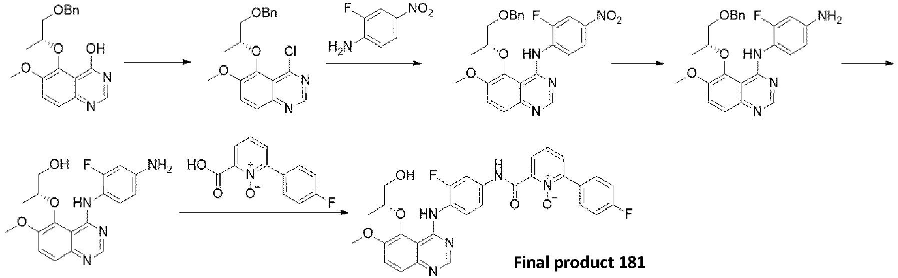

### Step 1: (R)-5-((1-(benzyloxy)propan-2-yl)oxy)-4-chloro-6-methoxyquinazoline

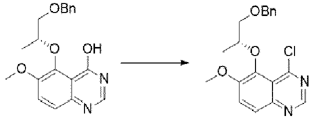

(R)-5-((1-(benzyloxy)prop-2-yl)oxy)-6-methoxyquinazolin-4-ol (400 mg, 1.17 mmol) and phosphorus oxychloride (10 mL) were added sequentially to a dry 50 mL round bottom flask, increased to 80 °C and the mixture was stirred for 16 h. After the reaction was completed through TLC monitoring, concentrated under reduced pressure to give crude (R)-5-((1-(benzyloxy)prop-2-yl)oxy)-4-chloro-6-methoxyquinazoline (500 mg, yellow oil). MS (ESI⁺)m/z = 359.1 [M+H]⁺.

### Step 2: (R)-5-((1-(benzyloxy)propan-2-yl)oxy)-N-(2-fluoro-4-nitrophenyl)-6-methoxyquinazolin-4-amine

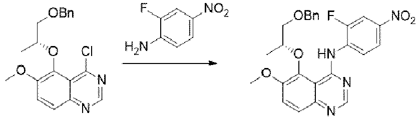

(R)-5-((1-(benzyloxy)prop-2-yl)oxy)-4-chloro-6-methoxyquinazoline (500 mg, 1.39 mmol), 2-fluoro-4-nitroaniline (261 mg, 1.67 mmol), p-toluenesulfonic acid (360 mg, 2.09 mol) and 1,4-dioxane (10 mL) were added sequentially to a dry 50 mL round bottom flask, and the mixture was warmed to 80 °C and stirred for 6 h. After the reaction was completed through TLC monitoring, quenched with water (50 mL), the mixture was extracted with ethyl acetate (50 mL × 3), the organic phases was combined and washed with saturated brine (20 mL), dried over anhydrous sodium sulfate, filtered, and the filtrate was concentrated under reduced pressure. The residue was purified by column chromatography with an eluent system of methanol/dichloromethane = 1/10 to give (R)-5-((1-(benzyloxy)propan-2-yl)oxy)-N-(2-fluoro-4-nitrophenyl)-6-methoxyquinazolin-4-amine (400 mg, yellow solid), yield: 59.9%. MS (ESI⁺)m/z = 479.2 [M+H]⁺.

### Step 3: (R)-N1-(5-((1-(benzyloxy)propan-2-yl)oxy)-6-methoxyquinazolin-4-yl)-2-fluorobenzene-1,4-diamine

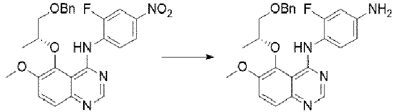

(R)-5-((1-(benzyloxy)prop-2-yl)oxy)-N-(2-fluoro-4-nitrophenyl)-6-methoxyquinazolin-4-amine (170 mg, 0.35 mmol), iron powder (59.53 mg, 1.06 mmol), saturated ammonium chloride solution (2 mL) and ethanol (6 mL) were added sequentially to a 50 mL round bottom flask at room temperature, and the mixture was warmed to 60 °C and stirred for 2 h. The reaction was complete through TLC monitoring, filtration, the filtrate was quenched with water (50 mL), the mixture was extracted with ethyl acetate (30 mL × 3), the organic phases were combined and washed with saturated brine (30 mL), dried over anhydrous sodium sulfate, filtered, and the filtrate was concentrated under reduced pressure to give crude (R)-N1-(5-((1-(benzyloxy)prop-2-yl)oxy)-6-methoxyquinazolin-4-yl)-2-fluorobenzene-1,4-diamine (150 mg, yellow oil) yield: 94.1%. MS (ESI⁺)m/z = 449.2 [M+H]⁺.

### Step 4: (R)-2-((4-((4-amino-2-fluorophenyl)amino)-6-methoxyquinazolin-5-yl)oxy)propan-1-ol

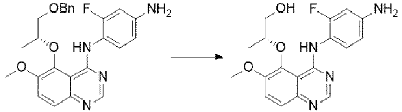

(R)-N1-(5-((1-(benzyloxy)prop-2-yl)oxy)-6-methoxyquinazolin-4-yl)-2-fluorobenzene-1,4-diamine (150 mg, 0.33 mmol), 1,4-dioxane (2 mL) and concentrated hydrochloric acid (2 mL) were added sequentially to a 50 mL round bottom flask at room temperature and stirred at 80 °C for 2 h. After the reaction was completed through TLC monitoring and allowed to cool to room temperature, the reaction was adjusted to pH = 7 with saturated sodium bicarbonate solution, and the mixture was extracted with dichloromethane (50 mL × 3). The organic phases were combined and washed with saturated brine (50 mL), dried over anhydrous sodium sulfate, filtered, the filtrate was concentrated under reduced pressure, and the residue was purified by column chromatography with an eluent system of methanol/dichloromethane = 1/20 to give (R)-2-((4-((4-amino-2-fluorophenyl)amino)-6-methoxyquinazolin-5-yl)oxy)propan-1-ol (40 mg, yellow solid), yield: 33.4%. MS (ESI⁺)m/z = 359.2 [M+H]⁺.

### Step 5: (R)-2-((3-fluoro-4-((5-((1-hydroxypropan-2-yl)oxy)-6-methoxyquinazolin-4-yl)amino)phenyl)carbamoyl)-6-(4-fluorophenyl)pyridine 1-oxide

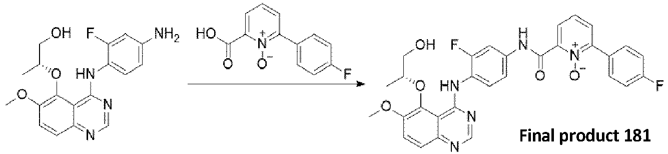

2-carboxy-6-(4-fluorophenyl)pyridine 1-oxide (intermediate B2, 27.5 mg, 0.12 mmol), N,N-dimethylformamide (5 mL), 2-(7-azabenzotriazol-1-yl)-N,N,N',N'-tetramethyluronium hexafluorophosphate (49.0 mg, 0.13 mmol), triethylamine (32.6 mg, 0.32 mmol) and (R)-2-((4-((4-amino-2-fluorophenyl)amino)-6-methoxyquinazolin-5-yl)oxy)propan-1-ol (37.0 mg, 0.11 mmol) were added sequentially to a 50 mL round bottom flask at room temperature and stirred for 2 h at 30 °C. After the reaction was completed through TLC monitoring and quenched with water (100 mL), the mixture was extracted with ethyl acetate, the organic phases were combined and washed with brine (50 mL), dried over anhydrous sodium sulfate, filtered and the filtrate was concentrated under reduced pressure to give the crude product. The crude product was slurried with methanol (10 mL), filtered, and the filter residue dried to give (R)-2-((3-fluoro-4-((5-((1-hydroxypropan-2-yl)oxy)-6-methoxyquinazolin-4-yl)amino)phenyl)carbamoyl)-6-(4-fluorophenyl)pyridine 1-oxide (11.5 mg).

Yellow solid, yield 19.4%. MS (ESI⁺)m/z = 574.0 [M+H]⁺. ¹H NMR (400 MHz, DMSO-*d*₆) *δ* 13.61 (s, 1H), 10.16 (s, 1H), 8.53-8.48 (m, 2H), 8.35 (dd, *J* = 8.1, 6.0 Hz, 1H), 8.00-7.97 (m, 2H), 7.85-7.83 (m, 2H), 7.74 (t, *J* = 9.2 Hz, 1H), 7.48-7.46 (m, 1H), 7.39 (t, *J* = 8.8 Hz, 2H), 6.83 (d, *J* = 7.2 Hz, 2H), 5.12 (t, *J* = 5.6 Hz, 1H), 4.89-4.87 (m, 1H), 3.90 (s, 3H), 3.70-3.67 (m, 2H), 1.39 (d, *J* = 6.4 Hz, 3H).

### Example 122 preparation of final product 185

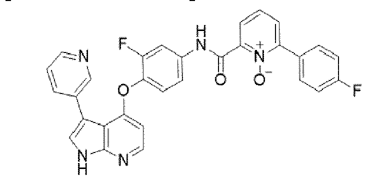

A known compound (final product 185) was synthesized according to the method of example 266 in CN 101027305A.

### Example 123

### I. kinase inhibition experiment

The activity of the compounds was determined by detecting the residual amount of ATP after reacting with wild-type or mutant MET protein kinase by using an HTRF KinEASE-TK kit. The intensity of the tested luminescence signal was positively correlated with the remaining amount of ATP in the reaction and negatively correlated with the kinase activity.

### 1. Experimental design

The compounds were determined on the selected kinase, the solvent control was set and a total of 10 concentrations were detected, diluted 3-fold, 2 replicates per concentration.

### 2. Reagents and materials

| Reagents | Supplier | Cat# |
|---|---|---|
| HTRF KinEASE-TK kit | Cisbio | 62TK0PEC/62TKPEB |
| MET | Carna | 08-151 |
| MET F1200I | Signalchem | M52-12GG/Y767-2 |
| MET D1228N | Signalchem | M52-12IG |
| DMSO | Sigma | D8418-1L or D4540-100 mL |
| ATP | Sigma or Promega | A7699 or V910B |
| DTT | Sigma | D0632 or 43816 |
| MgCl₂ | Sigma | M1028 |
| MnCl₂ | Sigma | M1787 |

| Instruments and Materials | Supplier | Cat# **or Model** |
|---|---|---|
| 384-well polystyrene white microplate with shallow mouth and round bottom plate | Greiner | 784075 |
| 384-well polystyrene transparent flat-bottom microplate with bar code plate | Labcyte | P-05525-BC |
| 96-well plate | Nunc | 249944 |
| 96-well conical transparent bottom plate | BIOFIL | VWP-033-096 |
| Shaking table | Thermo | 4625-1 CECN/THZ Q |
| Centrifuge | Eppendorf | 5810R |
| EnVision 2014 multi-label microplate detector | PerkinElmer | 2203-1060/Oct-04 |
| TECAN multi-label microplate detector | SPARK 10M | 1703004847 |
| Electro-heating standing-temperature cultivator | BOXUN | HPX-9162MBE |
| Echo pipette | Labcyte | 550 |

### 3. Experimental procedures

### 3.1 Configuration of 1X kinase buffer

1 volume of 5X kinase reaction buffer and 4 volumes of water; 5mM MgCl₂; 1mM DTT; 1mM MnCl₂; or 2500 nm SEB.

### 3.2 Compound screening

1) 10nl or 4µl of the diluted compound was transferred to each well of the reaction plate (784075 or 264706, Greiner or Thermo) with a pipette, the reaction plate was sealed with a sealing plate, and centrifuged at 1000g for 1 minute.
2) 2 X MET was prepared from 1 X enzyme reaction buffer, 5µl or 2µl kinase was added to each well of the reaction plate, the reaction plate was sealed with a sealing plate, centrifuged at 1000g for 30 seconds and incubated at room temperature for 10 min.
3) a mixed solution of 2X TK-substrate-biotin and ATP were prepared from 1X enzyme reaction buffer solution, 5 µl or 2 µl TK-substrate-biotin/ATP of the mixed solution was added into a reaction plate, the reaction plate was sealed with a sealing plate, centrifuged at 1000g for 30 seconds and reacted for 50 minutes or 10 minutes at room temperature.
4) a mixed solution of 4X or 2X Sa-XL 665 and TK-antibody-Cryptate were prepared from HTRF detection buffer, 5 or 10 µl of the mixed solution of Sa-XL 665 and TK-antibody-Cryptate was added into each well, centrifuged at 1000g for 30 seconds, and reacted at room temperature for 1 h.
5) the fluorescence signals at 615 nm (cryptate) and 665 nm (XL665) were read with Envision 2104 or TECAN.

### 4. IC₅₀ calculation

The data was analyzed using GraphPad Prism 6.0 or 7 software, and the dose-response curve was fitted with nonlinear S-curve regression, and the IC₅₀ value was calculated therefrom. Enzyme Inhibition Percent (%) = 100 - (Signalmpd - SignalAve_PC)/(SignalAve_VC - SignalAve_PC) × 100

### 5. Experimental results

**Table 4 IC₅₀ (nM) of inhibition of wild type (WT) and mutant (F1200I and D1228N) MET kinases by the compounds**

| **Final products Nos.** | **WT** | **F1200I** | **D1228N** | **Final products Nos.** | **WT** | **F1200I** | **D1228N** |
|---|---|---|---|---|---|---|---|
| **1** | 2.3 | 5.8 | 35.4 | **70** | 6.9 | / | 35.5 |
| **2** | 0.9 | 3.0 | 18.0 | **80** | 1.5 | 2.3 | 9.8 |
| **3** | 4.3 | 18.3 | 13.1 | **81** | 10.4 | 26.0 | 32.8 |
| **4** | 2.2 | 10.0 | 15.7 | **82** | 17.4 | 29.6 | 48.1 |
| **5** | 5.2 | 10.8 | 12.8 | **83** | 3.2 | 6.2 | 30.4 |
| **6** | 8.5 | 21.4 | 30.0 | **85** | 3.7 | 2.3 | 10.5 |
| **8** | 4.6 | 2.3 | 28.4 | **86** | 6.4 | 29.7 | 10.2 |
| **9** | 3.8 | 20.8 | 25.6 | **87** | 5.3 | 23.0 | 20.6 |
| **10** | 4.7 | 30.9 | 13.6 | **89** | 5.3 | 17.6 | 16.4 |
| **11** | 6.3 | 16.8 | 35.7 | **91** | 12.1 | / | 41.2 |
| **12** | 4.1 | 32.7 | 25.7 | **92** | 6.5 | 28.1 | 16.9 |
| **15** | 1.3 | 6.5 | 20.1 | **93** | 22.2 | 51.1 | 33.5 |
| **16** | 7.5 | 25.8 | 28.2 | **96** | 2.2 | 4.5 | 14.1 |
| **17** | 1.4 | 5.8 | 7.5 | **97** | 3.3 | 7.0 | 20.8 |
| **18** | 5.3 | 26.5 | 20.9 | **102** | 1.0 | 2.1 | 6.3 |
| **20** | 6.9 | 20.1 | 29.9 | **107** | 1.6 | 12.3 | 26.8 |
| **22** | 5.9 | 15.5 | 25.5 | **113** | 3.3 | 9.2 | 22.5 |
| **23** | 3.3 | 12.5 | 21.0 | **114** | 5.1 | 10.7 | 18.7 |
| **24** | 2.5 | 9.5 | 29.5 | **115** | 0.8 | 6.0 | 10.9 |
| **25** | 1.9 | 4.3 | 20.5 | **116** | 4.6 | 10.7 | 28.7 |
| **26** | 3.8 | 9.5 | 18.1 | **117** | 0.9 | 0.9 | 17.8 |
| **27** | 4.5 | 8.6 | 13.4 | **119** | 18.0 | 9.8 | 30.2 |
| **28** | 3.0 | 9.6 | 34.5 | **120** | 1.3 | 14.5 | 17.6 |
| **29** | 5.2 | 20.4 | 15.8 | **121** | 4.3 | 11.4 | 19.0 |
| **30** | 1.7 | 5.5 | 11.2 | **123** | 1.0 | 10.0 | 30.1 |
| **31** | 3.0 | 7.9 | 40.7 | **124** | 0.8 | 0.8 | 7.6 |
| **32** | 4.4 | 10.1 | 16.7 | **127** | 7.0 | 35.2 | 48.7 |
| **33** | 9.7 | 21.1 | 30.5 | **129** | 0.6 | 8.8 | 21.1 |
| **34** | 2.7 | 12.3 | 18.8 | **134** | 3.3 | 28.3 | 15.5 |
| **35** | 2.0 | 17.9 | 14.7 | **138** | 1.6 | 0.6 | 38.5 |
| **36** | 4.0 | 6.7 | 25.1 | **139** | 4.5 | 15.3 | 18.5 |
| **42** | 1.4 | 14.2 | 36.2 | **141** | 1.9 | 33.0 | 26.3 |
| **44** | 2.5 | 6.7 | 14.8 | **142** | 18.0 | 33.0 | 45.1 |
| **45** | 8.2 | 14.6 | 23.6 | **145** | 2.2 | 15.7 | 29.3 |
| **46** | 25.9 | 15.1 | 27.1 | **146** | 6.4 | 8.6 | 23.8 |
| **47** | 4.6 | 13.1 | 27.8 | **149** | 0.4 | 5.6 | 16.8 |
| **48** | 5.3 | / | 36.8 | **152** | 6.3 | 12.0 | 23.5 |
| **53** | 2.0 | 8.7 | 4.8 | **153** | 5.2 | / | 24.5 |
| **56** | 5.0 | 14.4 | 17.7 | **155** | 5.6 | 13.2 | 27.4 |
| **57** | 1.6 | 2.8 | 28.5 | **157** | 1.7 | 17.5 | 46.2 |
| **58** | 4.9 | 16.1 | 15.9 | **158** | 2.5 | 15.9 | 29.4 |
| **59** | 12.7 | 7.9 | 20.3 | **159** | 3.0 | 2.0 | 14.8 |
| **63** | 4.7 | 13.1 | 26.6 | **160** | 5.7 | 12.9 | 19.6 |
| **64** | / | 22.5 | 45.6 | **168** | 3.8 | 9.2 | 13.9 |
| **65** | 10.8 | 42.4 | 37.4 | **169** | 1.8 | 2.1 | 6.9 |
| **66** | 2.7 | 35.5 | 11.0 | **170** | 4.2 | 8.5 | 16.2 |
| **67** | 4.0 | 16.3 | 12.3 | **181** | 11.7 | / | 29.2 |
| **69** | 3.0 | 12.3 | 25.4 | **185** | 20.4 | 187.5 | 62.0 |

| | | | | | | | |
|---|---|---|---|---|---|---|---|
| **Note:** / **indicates no test activity.** | | | | | | | |

The IC₅₀ of the inhibitory activity of the present invention compounds on wild-type WT, mutant F1200I and D1228N MET kinase was shown in Table 4.

As can be seen from Table 4, the compounds of the present invention were found to show good inhibitory activity on wild-type WT, mutant F1200I and D1228N MET kinase, the IC₅₀ value reach a nanomolar level.

### II. Cell proliferation inhibition experiment

CellTiter-Glo^{™} live cell detection kit adopts luciferase as the detection substance, and the luciferase needs the participation of ATP in the luminescence process. CellTiter-Glo^{™} reagent was added into the cell culture medium and the luminescence value was measured. The light signal is in direct proportion to the amount of ATP in the system, ATP is in direct correlation with number of living cells. Therefore, the proliferation of cells can be detected by detecting the content of ATP with CellTiter-Glo kit.

In the experiment, the proliferation inhibition effect of the prepared compounds on tumor cell lines Hs746T, EBC-1 and H1993 was measured by a celltiter-glo (CTG) method, and 50% inhibition concentration IC₅₀ was calculated.

### 1. Experimental design

The compounds were determined on the selected cells, the solvent control was set and a total of 10 concentrations were detected, 2 replicates per concentration.

### 2. Reagents and materials

| **Reagents** | **Supplier** | **Cat#** |
|---|---|---|
| Hs746T | ATCC | HTB-135 |
| EBC-1 | Nanjing Kebai | CBP60091 |
| H1993 | ATCC | CRL-5909 |
| Phosphate buffer (PBS) | Gibco | 10010-031 |
| MEM Medium | HyClone | SH30024.01 |
| DMEM medium | Gibco | 11965-092 |
| Penicillin-Streptomycin | Gibco | 15140-122 |
| FBS | Gibco | 10099-141C |
| MEM NEAA | Gibco | 11140-050 |
| Sodium Pyruvate | Gibco | 11360-070 |
| TrypLE | Gibco | 12604-021 |
| CTG | Promega | G7573 |
| DMSO | Sigma | D8418-1L |
| Paciltaxel | MCE | HYB0015 |
| Staurosporine | Selleck/MCE | S1421/ HY-15141 |

| **Instruments and Materials** | **Supplier** | **Cat# or Model** |
|---|---|---|
| 384-well cell culture plate | PerkinElmer | 6007680 |
| 384-well compound dilution plate | Labcyte | PP-0200 |
| Shaking table | QILINBEIER | QB-9002 |
| CO₂ incubator | Thermo Scientific | 371 |
| Centrifuge | Eppendorf | 5810R |
| Counter | Gibco | AMQA × 1000 |
| Biosafety Cabinet | Thermo | Model 1300 Series A2 |
| Inverted microscope | OLYMPUS | CKX41 |
| Echo pipette | Labcyte | 655 |
| Envision 2104 multi-label microplate detector | PerkinElmer | EnVision 2104 |
| Vortex mixer | IKA | MS3 digital |
| Envision | PerkinElmer | 2105 |

### 3. Experimental procedures

### 3.1 Cell culture and inoculation

a) Cells in the logarithmic growth phase were harvested and counted by a platelet counter. The cell viability was detected by a trypan blue exclusion method to ensure that the cell viability was over 90%.
b) The cell concentration was adjusted; 30 µ L of cell suspension was added to a 384-well plate respectively.
c) Cells in a 384-well plate were incubated overnight at 37 °C, 5% CO₂ and 95% humidity.

### 3.2 Drug dilution and dosing

a) 10 times of drug solution was prepared with the highest concentration of 10 mM, 10 concentrations and 3 times dilution, 10 µL of drug solution was added into each well of a 384-well plate inoculated with cells, and three multiple wells were arranged for each drug concentration. DMSO was the blank control.
b) The cells in the 384-well plate were incubated for a further 72 h at 37 °C, 5% CO₂ and 95% humidity before CTG analysis.

### 3.3 Terminal reading plate

a) The CTG reagent was melted and the cell plate was equilibrated to room temperature for 30 min.
b) An equal volume of CTG solution was added to each well.
c) Cells were lysed by shaking on an orbital shaker for 5 minutes.
d) The cell plate was left at room temperature for 20 minutes to stabilize the luminescence signal.
e) The cold light value was read.

### 4. Data processing

The data was analyzed by GraphPad Prism 8 software, and the dose-response curve was fitted with nonlinear S-curve regression, and the IC₅₀ value was calculated therefrom. Cell viability (%) = (Lumdrugs to be tested - Lumblank control)/(Lumcell control - Lumblank cotrol) × 100%.

### 5. Experimental results

**Table 5 IC₅₀ (nM) of the proliferation inhibition of Hs746T, H1993 and EBC-1 cell lines by the compounds**

| **Final products Nos.** | **Hs 746T** | **H1993** | **EBC-1** | **Final products Nos.** | **Hs 746T** | **H1993** | **EBC-1** |
|---|---|---|---|---|---|---|---|
| **2** | 8.5 | 31.8 | 12.6 | **62** | 12.1 | 69.4 | 16.7 |
| **4** | 12.6 | 54.9 | 14.4 | **63** | 4.6 | / | 13.4 |
| **5** | / | 33.0 | / | **67** | 4.2 | 19.1 | 6.6 |
| **10** | 29.1 | / | 52.5 | **69** | 19.6 | 68.4 | 29.3 |
| **15** | 21.6 | 39.4 | / | **70** | 24.4 | 12.0 | / |
| **17** | / | 68.4 | / | **80** | 9.6 | 26.8 | 8.7 |
| **18** | 20.9 | / | / | **85** | 9.4 | 29.6 | 10.4 |
| **24** | 14.5 | / | 20.1 | **97** | 12.1 | 36.4 | 12.9 |
| **25** | / | 62.2 | / | **107** | / | 76.9 | / |
| **28** | 14.0 | / | 17.1 | **117** | 7.0 | 57.2 | 17.2 |
| **30** | / | 50.2 | / | **121** | / | 42.3 | / |
| **31** | / | 35.0 | / | **124** | 21.9 | 29.9 | 14.9 |
| **32** | / | 30.6 | / | **138** | 8.7 | 29.3 | 9.4 |
| **36** | 9.9 | / | 15.1 | **139** | / | 31.1 | / |
| **44** | 15.2 | 85.3 | 21.3 | **159** | / | 78.4 | / |
| **53** | 14.6 | 29.0 | 15.7 | **168** | 65.4 | / | 56.7 |
| **57** | 11.4 | 59.5 | 19.6 | **185** | 155.1 | 203.5 | 132.8 |
| **58** | 12.8 | 16.4 | 13.2 | | | | |

| | | | | | | | |
|---|---|---|---|---|---|---|---|
| **Note:** / **indicates not detected.** | | | | | | | |

The proliferation inhibitory activity IC₅₀ of the compounds of the present invention on Hs746T cell line, H1993 cell line and EBC-1 cell line, was shown in Table 5.

As can be seen from Table 5, the compounds of the present invention were shown significantly to inhibit the proliferation of Hs746T cell line, H1993 cell line and EBC-1 cell line.

## Claims

1. A compound of formula I or a pharmaceutically acceptable salt, stereoisomer, tautomer, deuteride, prodrug molecule, hydrate or solvate thereof 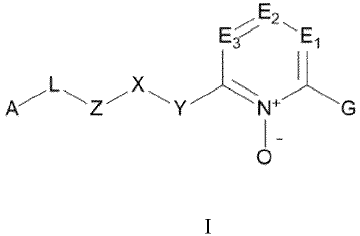 wherein, in the formula I,
A is selected from the group consisting of 5-14 membered heteroaryl substituted with R¹, 3-14 membered heterocyclyl substituted with R¹, 6-14 membered aryl substituted with R¹, and 3-12 membered cycloalkyl substituted with R¹, the 5-14 membered heteroaryl substituted with R¹, 3-14 membered heterocyclyl substituted with R¹, 6-14 membered aryl substituted with R¹, and 3-12 membered cycloalkyl substituted with R¹ are optionally substituted with one or more R², the 5-14 membered heteroaryl substituted with R¹ and 6-14 membered aryl substituted with R¹ comprise monocyclic or fused ring, the 3-14 membered heterocyclyl substituted with R¹, and 3-12 membered cycloalkyl substituted with R¹ comprise monocyclic, spirocyclic or bridged ring;
L is selected from O, S, CR^{a}R^{b}, NR^{b}, C(=O), SO₂ and SO;
Z is selected from 6-14 membered aryl, 5-14 membered heteroaryl, 3-12 membered cycloalkyl and 3-14 membered heterocyclyl, the 6-14 membered aryl, 5-14 membered heteroaryl, 3-12 membered cycloalkyl and 3-14 membered heterocyclyl are optionally substituted with one or more R³;
X is absent or selected from NR⁴, O, S, CR⁴R⁵ or C(=O);
Y is absent or selected from (CR^{a}R^{b})ₙ₁-C(=O), C(=S), C(=NR⁴), SO₂, SO, NR⁴, O, S and CR⁴R⁵;
E1, E₂ and E₃ are each absent or each independently selected from CR^{a}R^{b}, N, C(=O), C(=S) and C(=NR⁴);
G is selected from the group consisting of (CR^{a}R^{b})ₙ₁-6-20-membered aryl, (CR^{a}R^{b})ₙ₁-5-20-membered heteroaryl, (CR^{a}R^{b})ₙ₁-3-12-membered cycloalkyl, (CR^{a}R^{b})ₙ₁-3-20-membered heterocyclyl, (CR^{a}R^{b})ₙ₁-O-(CR^{a}R^{b})ₘ₁-aryl, (CR^{a}R^{b})ₙ₁-OR⁶, (CR^{a}R^{b})ₙ₁-NR⁶R⁷, (CR^{a}R^{b})ₙ₁-NR⁶C(=O)R⁷, C₁₋₆ alkyl, C₂₋₆ alkenyl and C₂₋₆ alkynyl, wherein the (CR^{a}R^{b})ₙ₁-6-20-membered aryl, (CR^{a}R^{b})ₙ₁-5-20-membered heteroaryl, (CR^{a}R^{b})ₙ₁-3-12-membered cycloalkyl, (CR^{a}R^{b})ₙ₁-3-20-membered heterocyclyl, (CR^{a}R^{b})ₙ₁-O-(CR^{a}R^{b})ₘ₁-aryl, C₁₋₆ alkyl, C₂₋₆ alkenyl and C₂₋₆ alkynyl are optionally substituted with one or more groups independently selected from the group consisting of: halogen, nitro, oxo(=O), SO₂R^{f}, CN, OR^{e}, SR^{e}, NR^{e}W^{f}, -NR^{e}C(=O)R^{f}, (CR^{a}R^{b})ₙ₁-C(=O)R^{e}, -C(=O)NR^{e}R^{f}, -C(=O)OR^{e}, C₁₋₁₂ alkyl, halogenated C₁₋₁₂ alkyl, C₁₋₆ alkoxy-C₁₋₁₂ alkyl, halogenated C₁₋₆ alkoxy-C₁₋₁₂ alkyl, R^{a}R^{b}N-C₁₋₆ alkyl, C₂₋₈ alkenyl, halogenated C₂₋₈ alkenyl, C₂₋₈ alkynyl, 3-12 membered cycloalkyl, 3-20 membered heterocyclyl, 6-20 membered aryl, or 5-20 membered heteroaryl;
R¹ is selected from R⁸ substituted with one or more hydroxy;
R², R³ and R⁸ are each independently selected from the group consisting of hydrogen, halogen, cyano, nitro, -SO-R^{a}, -NR^{b}R^{c}, -OR^{b}, -SR^{b}, R^{a}SO-C₁₋₆ alkyl, R^{b}R^{c}N-C₁₋₆ alkyl, R^{b}R^{c}NC(=Y¹)-C₁₋₆ alkyl, R^{a}SO-C₁₋₆ alkoxy, R^{b}R^{c}N-C₁₋₆ alkoxy, R^{b}R^{c}NC(=Y¹)-C₁₋₆ alkoxy, -C(=Y¹)R^{a}, -C(=Y¹)OR^{b}, -C(=Y¹)NR^{b}R^{c}, - C(=Y¹)NR^{c}(CR^{a}R^{b})ₙ₁NR^{b}R^{c}, -OC(=Y¹)R^{a}, -OC(=Y¹)NR^{b}R^{c}, -OC(=Y¹)OR^{b}, - NR^{b}C(=Y¹)NR^{b}R^{c}, -NR^{b}C(=Y¹)OR^{c}, -NR^{b}C(=Y¹)R^{a}, -OR^{b}C(=Y¹)OR^{c}, -SO₂-NR^{b}R^{c}, - SO₂R^{a}, -NR^{b}SO₂R^{a}, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered cycloalkenyl, 3-12 membered heterocyclyl, 6-14 membered aryl, 5-10 membered heteroaryl, C₁₋₆ alkoxy-C₁₋₆ alkyl, 3-12 membered cycloalkyl-C₁₋₆ alkyl, 3-12 membered cycloalkenyl-C₁₋₆ alkyl, 3-12 membered heterocyclyl-C₁₋₆ alkyl, 6-14 membered aryl-C₁₋₆ alkyl and 5-10 membered heteroaryl-C₁₋₆ alkyl, the R^{a}SO-C₁₋₆ alkyl, R^{b}R^{c}N-C₁₋₆ alkyl, R^{b}R^{c}NC(=Y¹)-C₁₋₆ alkyl, R^{a}SO-C₁₋₆ alkoxy, R^{b}R^{c}N-C₁₋₆ alkoxy, R^{b}R^{c}NC(=Y¹)-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered cycloalkenyl, 3-12 membered heterocyclyl, 6-14 membered aryl, 5-10 membered heteroaryl, C₁₋₆ alkoxy-C₁₋₆ alkyl, 3-12 membered cycloalkyl-C₁₋₆ alkyl, 3-12 membered cycloalkenyl-C₁₋₆ alkyl, 3-12 membered heterocyclyl-C₁₋₆ alkyl, 6-14 membered aryl-C₁₋₆ alkyl and 5-10 membered heteroaryl-C₁₋₆ alkyl are optionally substituted with one or more R^{m}, the 3-12 membered cycloalkyl, 3-12 membered cycloalkenyl, 3-12 membered heterocyclyl, 3-12 membered cycloalkyl-C₁₋₆ alkyl, 3-12 membered cycloalkenyl-C₁₋₆ alkyl and 3-12 membered heterocyclyl-C₁₋₆ alkyl comprise monocyclic, spirocyclic or bridged ring, and the 6-14 membered aryl, 5-10 membered heteroaryl, 6-14 membered aryl-C₁₋₆ alkyl and 5-10 membered heteroaryl-C₁₋₆ alkyl comprise monocyclic or fused ring;
or R¹ and R² together with the atoms to which they are attached to form a 5-14 membered cyclic group, which is substituted with one or more R¹;
or R² and R⁸ together with the atoms to which they are attached to form a 5-14 membered cyclic group, which is substituted with one or more R¹;
R⁴, R⁵, R⁶ and R⁷ are each independently selected from the group consisting of hydrogen, halogen, OR^{e}, C₁₋₆ alkyl, C₂₋₆ alkenyl, C₂₋₆ alkynyl, 3-12 membered cycloalkyl, 3-20 membered heterocyclyl, 6-20 membered aryl and 5-20 membered heteroaryl, wherein the 3-12 membered cycloalkyl and 3-20 membered heterocyclyl comprise monocyclic, spirocyclic or bridged ring, the 6-20 membered aryl and 5-20 membered heteroaryl comprise monocyclic or fused ring, the C₁₋₆ alkyl, C₂₋₆ alkenyl, C₂₋₆ alkynyl, 3-12 membered cycloalkyl, 3-20 membered heterocyclyl, 6-20 membered aryl and 5-20 membered heteroaryl are optionally substituted with one or more groups independently selected from the group consisting of halogen, SO₂R^{g}, CN, OR^{e}, NR^{e}R^{f}, C(=O)NR^{e}R^{f}, CR^{e}C(=O)R^{f}, C₁₋₁₂ alkyl, C₂₋₈ alkenyl, C₂₋₈ alkynyl, 3-12 membered cycloalkyl, 3-20 membered heterocyclyl, 6-20 membered aryl or 5-20 membered heteroaryl;
or R⁴ and R⁵ together with the carbon atom to which they are both attached to form a 3-12 membered monocyclic, spirocyclic or bridged ring optionally substituted with one or more R^{m};
or R⁶ and R⁷ together with the nitrogen atom to which they are both attached to form a saturated, partially unsaturated or fully unsaturated 3-20 membered heterocycle optionally substituted with one or more R^{m}, which comprising one or more heteroatoms selected from the group consisting of N, O and S; R^{a} is absent or selected from the group consisting of hydrogen, halogen, cyano, hydroxyl, mercapto, carboxyl, nitro, -NR^{b}R^{c}, -C(=O)R^{d}, -C(=O)OR^{b}, -C(=O)NR^{b}R^{c}, -OC(=O)NR^{b}R^{c}, - NR^{b}C(=O)R^{d}, -NR^{b}C(=O)OR^{d}, -SO-NR^{b}R^{c}, -SO₂-N^{b}R^{c}, -SO-R^{d}, -SO₂R^{d}, - NR^{b}SO₂R^{d}, C₁₋₆ alkyl, C₁₋₆ alkoxy, C₂₋₈ alkenyl, C₂₋₈ alkynyl, C₁₋₆ alkyl-R', C₁₋₆ alkoxy-R', -OR', -SR', -C(=O)-R', -SO₂R', -NR^{b}C(=O)-R', 3-12 membered cycloalkyl, 3-12 membered cycloalkenyl, 3-12 membered heterocyclyl, 6-14 membered aryl and 5-10 membered heteroaryl, the C₁₋₆ alkyl, C₁₋₆ alkoxy, C₂₋₈ alkenyl, C₂₋₈ alkynyl, C₁₋₆ alkyl-R', C₁₋₆alkoxy-R', 3-12 membered cycloalkyl, 3-12 membered cycloalkenyl, 3-12 membered heterocyclyl, 6-14 membered aryl and 5-10 membered heteroaryl are optionally substituted with one or more R^{m}, the 3-12 membered cycloalkyl, 3-12 membered cycloalkenyl and 3-12 membered heterocyclyl comprise monocyclic, spirocyclic or bridged ring, the 6-14 membered aryl and 5-10 membered heteroaryl comprise monocyclic or fused ring;
R^{b}, R^{c} and R^{d} are each absent or each independently selected from the group consisting of hydrogen, hydroxyl, mercapto, C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', -OR', -C(=O)-R', -SO₂-R', 3-12 membered cycloalkyl, 3-12 membered cycloalkenyl, 3-12 membered heterocyclyl, 6-14 membered aryl and 5-10 membered heteroaryl, the C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', 3-12 membered cycloalkyl, 3-12 membered cycloalkenyl, 3-12 membered heterocyclyl, 6-14 membered aryl and 5-10 membered heteroaryl are optionally substituted with one or more R^{m}, the 3-12 membered cycloalkyl, 3-12 membered cycloalkenyl and 3-12 membered heterocyclyl comprise monocyclic, spirocyclic or bridged ring, the 6-14 membered aryl and 5-10 membered heteroaryl comprise monocyclic or fused ring;
or R^{a} and R^{b} together with the carbon atom to which they are both attached to form a 3-12 membered monocyclic, spirocyclic or bridged ring optionally substituted with one or more R^{m};
or R^{a} or R^{b} together with the carbon atom or ortho-nitrogen atom to which they are attached to form a 5-14 membered heteroaryl, 6-14 membered aryl, 3-14 membered heterocyclyl or 3-12 membered cycloalkyl optionally substituted with one or more R^{m};
or R^{b} and R^{c} together with the nitrogen atom to which they are both attached to form a 3-12 membered monocyclic, spirocyclic, bridged or fused ring optionally substituted with one or more R^{m};
R^{e} and R^{f} are each independently selected from the group consisting of hydrogen, C₁₋₆ alkyl, C₂₋₆ alkenyl, C₂₋₆ alkynyl, 3-12 membered cycloalkyl, 3-20 membered heterocyclyl, 6-20 membered aryl and 5-20 membered heteroaryl, wherein the C₁₋₆ alkyl, C₂₋₆ alkenyl, C₂₋₆ alkynyl, 3-12 membered cycloalkyl, 3-20 membered heterocyclyl, 6-20 membered aryl and 5-20 membered heteroaryl are optionally substituted with one or more C₁₋₆ alkyl or halogen;
R^{g} is selected from the group consisting of C₁₋₆ alkyl, 3-12 membered cycloalkyl, 3-20 membered heterocyclyl, amino, C₁₋₆ alkylamino, di(C₁₋₆ alkyl)amino and 6-20 membered aryl, wherein the C₁₋₆ alkyl, 3-12 membered cycloalkyl, 3-20 membered heterocyclyl, C₁₋₆ alkylamino, di(C₁₋₆ alkyl)amino and 6-20 membered aryl are optionally substituted with one or more groups independently selected from the group consisting of halogen, OR^{e} or -C(=O)NR^{e}R^{f};
R' is selected from the group consisting of hydrogen, C₁₋₆ alkyl, 3-12 membered cycloalkyl, 3-12 membered cycloalkenyl, 3-12 membered heterocyclyl, 6-14 membered aryl and 5-14 membered heteroaryl, the C₁₋₆ alkyl, 3-12 membered cycloalkyl, 3-12 membered cycloalkenyl, 3-12 membered heterocyclyl, 6-14 membered aryl and 5-14 membered heteroaryl are optionally substituted with one or more R^{m}; the 3-12 membered cycloalkyl, 3-12 membered cycloalkenyl and 3-12 membered heterocyclyl comprise monocyclic, spirocyclic or bridged ring, and the 6-14 membered aryl and 5-14 membered heteroaryl comprise monocyclic or fused ring;
R^{m} is selected from deuterium, halogen, cyano, nitro, -OR^{a}, -SR^{a}, -C(=Y¹)R^{a}, - C(=Y¹)OR^{a}, -C(=Y¹)NR^{b}R^{c}, -(CR^{a}R^{b})ₙ₁-NR^{b}R^{c}, -NR^{b}C(=Y¹)R^{c}, -NR^{b}C(=Y¹)OR^{c}, - NR^{d}C(=Y¹)NR^{b}R^{c}, -NR^{b}SO₂R^{a}, -OC(=Y¹)R^{a}, -OC(=Y¹)OR^{b}, -OC(=Y¹)NR^{a}R^{b}, - OSO₂(OR^{b}), -OP(=Y¹)(OR^{b})(OR^{c}), -OP(OR^{b})(OR^{c}), -SOR^{a}, -SO₂R^{a}, -SO₂NR^{b}R^{c}, - SO(OR^{b}), -SO₂(OR^{b}), -SC(=Y¹)R^{a}, -SC(=Y¹)OR^{b}, -SC(=Y¹)N^{b}R^{c}, C₁₋₁₂ alkyl, halogenated C₁₋₁₂ alkyl, C₁₋₁₂ alkoxy-C₁₋₁₂ alkyl, C₁₋₁₂ alkyl-C₁₋₁₂ alkoxy, C₁₋₁₂ alkoxy-C₁₋₁₂ alkoxy, C₂₋₈ alkenyl, C₂₋₈ alkynyl, 3-12 membered cycloalkyl, 5-14 membered heterocyclyl, 6-20 membered aryl, 5-20 membered heteroaryl, (CR^{a}R^{d})ₜNR^{b}R^{c}, azido and oxo, wherein the 3-12 membered cycloalkyl and the 5-14 membered heterocyclyl comprise monocyclic, spirocyclic or bridged ring, and the 6-20 membered aryl and the 5-20 membered heteroaryl comprise monocyclic or fused ring;
Y¹ is O or S;
t, m1, and n1 are each independently 0, 1, 2, 3, 4, 5, or 6;
the conditions are as follows:
when A is 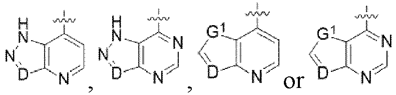 substituted with R¹, and -X-Y is -NR'-C(=O)-, R¹ is not hydroxyalkyl, except that NH is substituted with R¹, for example in the 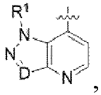 R¹ may be hydroxy alkyl, wherein D is selected from N or CH, G¹ is selected from NH, O or S;
when A is 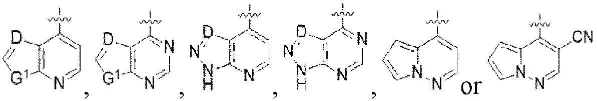 substituted with R¹, and -X-Y is-NR'-C(=O)-, R¹ is not hydroxyalkyl, wherein D is selected from N or CH, G¹ is selected from NH, O or S;
when A is 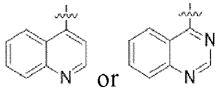 substituted with R¹, Z is a phenyl ring, and -X-Y is -NH-C(=O)-, R¹ is not hydroxyalkyl;
E₃ is not C=O, C=S or C=NR^{a}; when E₃ is CH, E₁ is not C=O and E₂ is not NR^{b}; when E₃ is absent, E₂ is not C=O, C=S or C=NR^{a}.

2. The compound, or a pharmaceutically acceptable salt, stereoisomer, tautomer, deuteride, prodrug molecule, hydrate or solvate thereof according to claim 1, wherein, in formula I, A is selected from 5-14 membered heteroaryl substituted with R¹, the 5-14 membered heteroaryl substituted with R¹ comprises monocyclic or fused ring, optionally substituted with one or more R²;
preferably, in formula I, A is selected from the following groups substituted with R¹, the following groups are optionally substituted with one or more R²; 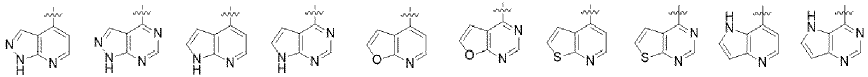 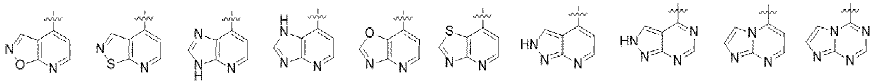 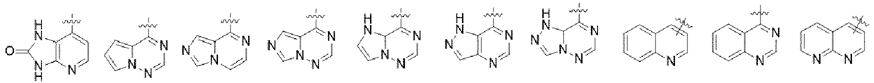 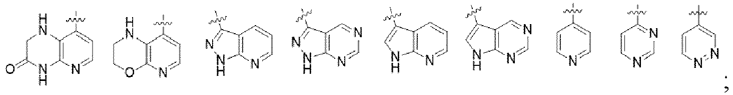
in formula I, L is selected from O, S, CR^{a}R^{b}, NR^{b}, and C(=O);
preferably, in formula I, L is selected from O, S, C(=O) and NH;
in formula I, Z is selected from 6-14 membered aryl and 5-10 membered heteroaryl optionally substituted with one or more R³;
preferably, in formula I, Z is selected from the following groups optionally substituted with one or more R³: 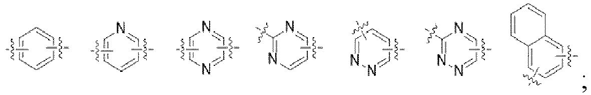
in formula I, X is selected from NR⁴, O, S, or is absent;
preferably, in formula I, X is selected from NR⁴, O and S.
in formula I, Y is selected from C(=O), C(=S), C(=NR⁴), or is absent;
preferably, in formula I, Y is selected from C(=O) and C(=S).
in formula I, E₁, E₂, E₃ are each independently selected from CR^{a}R^{b}, N, C(=O), and C(=S);
preferably, in formula I, E₁, E₂, E₃ are each independently selected from CR^{a}R^{b} and N.

3. The compound or a pharmaceutically acceptable salt, stereoisomer, tautomer, deuteride, prodrug molecule, hydrate or solvate thereof according to claim 1 or 2, wherein, in formula I, G is selected from (CR^{a}R^{b})ₙ₁-6-20 membered aryl, (CR^{a}R^{b})ₙ₁-5-20 membered heteroaryl, (CR^{a}R^{b})ₙ₁-3-12 membered cycloalkyl and (CR^{a}R^{b})ₙ₁-3-20 membered heterocyclyl, the (CR^{a}R^{b})ₙ₁-6-20 membered aryl, (CR^{a}R^{b})ₙ₁-5-20 membered heteroaryl, (CR^{a}R^{b})ₙ₁-3-12 membered cycloalkyl and (CR^{a}R^{b})ₙ₁-3-20 membered heterocyclyl are optionally substituted with one or more groups independently selected from the group consisting of halogen, nitro, oxo, CN, OR^{e}, SR^{e}, NR^{e}R^{f}, C₁₋₁₂ alkyl, halogenated C₁₋₁₂ alkyl, C₁₋₆ alkoxy-C₁₋₁₂ alkyl, halogenated C₁₋₆ alkoxy-C₁₋₁₂ alkyl, R^{a}R^{b}N-C₁₋₆ alkyl, C₂₋₈ alkenyl, halogenated C₂₋₈ alkenyl, C₂₋₈ alkynyl, 3-12 membered cycloalkyl or 3-20 membered heterocyclyl;
preferably, in formula I, G is selected from 6-20 membered aryl and 5-20 membered heteroaryl, wherein the 6-20 membered aryl and 5-20 membered heteroaryl are optionally substituted with one or more groups independently selected from the group consisting of: halogen, nitro, oxo, CN, OR^{e}, SR^{e}, NR^{e}R^{f}, C₁₋₁₂ alkyl, halogenated C₁₋₁₂ alkyl, C₁₋₆ alkoxy-C₁₋₁₂ alkyl, halogenated C₁₋₆ alkoxy-C₁₋₁₂ alkyl, R^{a}R^{b}N-C₁₋₆ alkyl, C₂₋₈ alkenyl, halogenated C₂₋₈ alkenyl, C₂₋₈ alkynyl, 3-12 membered cycloalkyl or 3-20 membered heterocyclyl;
R¹ is selected from R⁸ substituted with one or two hydroxy;
R⁸ is selected from the group consisting of -NR^{b}R^{c}, -OR^{b}, -SR^{b}, R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered cycloalkenyl, 3-12 membered heterocyclyl, 6-14 membered aryl, 5-10 membered heteroaryl, 3-12 membered cycloalkyl-C₁₋₆ alkyl, 3-12 membered cycloalkenyl-C₁₋₆ alkyl, 3-12 membered heterocyclyl-C₁₋₆ alkyl, 6-14 membered aryl-C₁₋₆ alkyl, and 5-10 membered heteroaryl-C₁₋₆ alkyl, the R^{b}R^{c}N-C₁₋₆ alkyl, R^{b}RN-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered cycloalkenyl, 3-12 membered heterocyclyl, 6-14 membered aryl, 5-10 membered heteroaryl, 3-12 membered cycloalkyl-C₁₋₆ alkyl, 3-12 membered cycloalkenyl-C₁₋₆ alkyl, 3-12 membered heterocyclyl-C₁₋₆ alkyl, 6-14 membered aryl-C₁₋₆ alkyl, and 5-10 membered heteroaryl-C₁₋₆ alkyl are optionally substituted with one or more R^{m}, the 3-12 membered cycloalkyl, 3-12 membered cycloalkenyl and 3-12 membered heterocyclyl comprise monocyclic, spirocyclic or bridged ring, the 6-14 membered aryl and 5-10 membered heteroaryl comprise monocyclic or fused ring;
preferably, R⁸ is selected from the group consisting of -NR^{b}R^{c}, -OR^{b}, C₁₋₆ alkyl, R^{b}R^{c}N-C₁₋₆ alkyl, 3-12 membered heterocyclyl and 3-12 membered heterocyclyl-C₁₋₆ alkyl, the C₁₋₆ alkyl, R^{b}R^{c}N-C₁₋₆ alkyl, 3-12 membered heterocyclyl and 3-12 membered heterocyclyl-C₁₋₆ alkyl are optionally substituted with one or more R^{m}, the 3-12 membered heterocyclyl and 3-12 membered heterocyclyl-C₁₋₆ alkyl comprise monocyclic, spirocyclic or bridged ring.
R² and R³ are each independently selected from the group consisting of hydrogen, halogen, cyano, nitro, -NR^{b}R^{c}, -OR^{b}, R^{b}R^{c}N-C₁₋₆ alkyl, R^{b}R^{c}N-C₁₋₆ alkoxy, -C(=Y¹)R^{a}, -C(=Y¹)NR^{b}R^{c}, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl, 3-12 membered heterocyclyl-C₁₋₆ alkyl and 5-10 membered heteroaryl-C₁₋₆ alkyl, the R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl, 3-12 membered heterocyclyl-C₁₋₆ alkyl and 5-10 membered heteroaryl-C₁₋₆ alkyl are optionally substituted with one or more R^{m}, the 3-12 membered cycloalkyl, 3-12 membered heterocyclyl, 3-12 membered cycloalkyl-C₁₋₆ alkyl, and 3-12 membered heterocyclyl-C₁₋₆ alkyl comprise monocyclic, spirocyclic, or bridged ring, the 5-10 membered heteroaryl-C₁₋₆ alkyl comprise monocyclic or fused ring;
preferably, R² and R³ are each independently selected from the group consisting of hydrogen, halogen, cyano, nitro, -NR^{b}R^{c}, -OR^{b}, R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl and 3-12 membered heterocyclyl-C₁₋₆ alkyl, the R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl and 3-12 membered heterocyclyl-C₁₋₆ alkyl are optionally substituted with one or more R^{m}, the 3-12 membered cycloalkyl, 3-12 membered heterocyclyl, 3-12 membered cycloalkyl-C₁₋₆ alkyl, and 3-12 membered heterocyclyl-C₁₋₆ alkyl comprise monocyclic, spirocyclic or bridged ring.

4. The compound or a pharmaceutically acceptable salt, stereoisomer, tautomer, deuteride, prodrug molecule, hydrate, or solvate thereof according to any one of claims 1 to 3, wherein,
R⁴, R⁵, R⁶, and R⁷ are each independently selected from the group consisting of hydrogen, halogen, OR^{e}, C₁₋₆ alkyl, C₂₋₆ alkenyl, C₂₋₆ alkynyl, 3-20 membered cycloalkyl, 3-20 membered heterocyclyl and 6-20 membered aryl, wherein the 3-20 membered cycloalkyl and the 3-20 membered heterocyclyl comprise monocyclic, spirocyclic, or bridged ring; the C₁₋₆ alkyl, C₂₋₆ alkenyl, C₂₋₆ alkynyl, 3-20 membered cycloalkyl, 3-20 membered heterocyclyl and 6-20 membered aryl are optionally substituted with one or more groups independently selected from the group consisiting of halogen, CN, OR^{e}, NR^{e}R^{f}, C₁₋₁₂ alkyl, C₂₋₈ alkenyl, C₂₋₈ alkynyl, 3-12 membered cycloalkyl or 3-20 membered heterocyclyl;
or R⁴ and R⁵ together with the carbon atom to which they are both attached to form a 3-12 membered monocyclic, spirocyclic or bridged ring optionally substituted with one or more R^{m};
or R⁶ and R⁷ together with the nitrogen atom to which they are both attached to form a saturated, partially unsaturated or fully unsaturated 3-20 membered heterocycle optionally substituted with one or more R^{m}, which comprsing one or more heteroatoms selected from N, O or S;
preferably, R⁴, R⁵, R⁶ and R⁷ are each independently selected from the group consisting of hydrogen, OR^{e}, C₁₋₆ alkyl, monocyclic or bicyclic of 3-12 membered cycloalkyl, 3-20 membered heterocyclyl and 6-20 membered aryl, wherein the C₁₋₆ alkyl, monocyclic or bicyclic of 3-12 membered cycloalkyl, 3-20 membered heterocyclyl and 6-20 membered aryl are optionally substituted with one or more groups independently selected from the group consisting of: halogen, CN, OR^{e}, NR^{e}R^{f}, C₁₋₁₂ alkyl, C₂₋₈ alkenyl, C₂₋₈ alkynyl, 3-12 membered cycloalkyl or 3-20 membered heterocyclyl;
or R⁴ and R⁵ together with the carbon atom to which they are both attached to form a 3-12 membered monocyclic, spirocyclic or bridged ring optionally substituted with one or more R^{m}, the 3-12 membered monocyclic, spirocyclic or bridged ring is optionally substituted with one or more groups independently selected from the group consisiting of: halogen, C₁₋₆ alkyl or C₁₋₆ alkoxy;
or R⁶ and R⁷ together with the nitrogen atom to which they are both attached to form a saturated, partially unsaturated, or fully unsaturated 3-20 membered heterocycle optionally substituted with one or more R^{m}, the 3-20 membered heterocycle is optionally substituted with one or more groups independently selected from the group consisting of halogen, C₁₋₆ alkyl or C₁₋₆ alkoxy.

5. The compound or a pharmaceutically acceptable salt, stereoisomer, tautomer, deuteride, prodrug molecule, hydrate, or solvate thereof according to any one of claims 1 to 4, wherein,
R^{a} is absent or selected from the group consisting of hydrogen, halogen, cyano, hydroxyl, mercapto, carboxyl, nitro, -NR^{b}R^{c}, -C(=O)R^{d}, C₁₋₆ alkyl, C₁₋₆ alkoxy, C₂₋₈ alkenyl, C₂₋₈ alkynyl, C₁₋₆ alkyl-R', C₁₋₆ alkoxy-R', -OR', -SR', -SOR^{d}, -SO₂R^{d}, 3-12 membered cycloalkyl, 3-12 membered cycloalkenyl, 3-12 membered heterocyclyl, 6-14 membered aryl and 5-10 membered heteroaryl, the C₁₋₆ alkyl, C₁₋₆ alkoxy, C₂₋₈ alkenyl, C₂₋₈ alkynyl, C₁₋₆ alkyl-R', C₁₋₆alkoxy-R', 3-12 membered cycloalkyl, 3-12 membered cycloalkenyl, 3-12 membered heterocyclyl, 6-14 membered aryl, and 5-10 membered heteroaryl are optionally substituted with one or more R^{m};
R^{b}, R^{c} and R^{d} are each absent or each independently selected from the group consisting of hydrogen, hydroxyl, mercapto, C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R, -OR', -C(=O)-R', 3-12 membered cycloalkyl, 3-12 membered cycloalkenyl, 3-12 membered heterocyclyl, 6-14 membered aryl and 5-10 membered heteroaryl, the C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', 3-12 membered cycloalkyl, 3-12 membered cycloalkenyl, 3-12 membered heterocyclyl, 6-14 membered aryl, and 5-10 membered heteroaryl are optionally substituted with one or more R^{m};
or R^{b} and R^{c} together with the nitrogen atom to which they are both attached to form a 3-12 membered monocyclic, spirocyclic, bridged or fused ring optionally substituted with one or more R^{m};
R^{e} and R^{f} are each independently selected from the group consisting of hydrogen, C₁₋₆ alkyl, C₂₋₆ alkenyl, 3-12 membered cycloalkyl, 3-20 membered heterocyclyl, 6-20 membered aryl, and 5-20 membered heteroaryl, wherein the C₁₋₆ alkyl, C₂₋₆ alkenyl, 3-12 membered cycloalkyl, 3-20 membered heterocyclyl, 6-20 membered aryl, and 5-20 membered heteroaryl are optionally substituted with one or more C₁₋₆ alkyl or halogen;
R^{g} is C₁₋₆ alkyl or 6-20 membered aryl, wherein the C₁₋₆ alkyl and 6-20 membered aryl are optionally substituted with one or more groups independently selected from the group consisting of: halogen or OR^{e};
R' is selected from the group consisting of C₁₋₆ alkyl, 3-12 membered cycloalkyl, 3-12 membered cycloalkenyl, 3-12 membered heterocyclyl, 6-14 membered aryl and 5-10 membered heteroaryl, the C₁₋₆ alkyl, 3-12 membered cycloalkyl, 3-12 membered cycloalkenyl, 3-12 membered heterocyclyl, 6-14 membered aryl and 5-10 membered heteroaryl are optionally substituted with one or more R^{m}; the 3-12 membered cycloalkyl, 3-12 membered alkenyl and 3-12 membered heterocyclyl comprise monocyclic, spirocyclic or bridged ring, and the 6-14 membered aryl and 5-10 membered heteroaryl comprise monocyclic or fused ring.
preferably, R^{a} is absent or selected from the group consisting of hydrogen, halogen, cyano, hydroxyl, -NR^{b}R^{c}, -C(=O)R^{d}, C₁₋₆ alkyl, C₁₋₆ alkoxy, C₂₋₈ alkenyl, C₁₋₆ alkyl-R', C₁₋₆ alkoxy-R', -OR', -SR', -SOR^{d}, -SO₂R^{d}, 3-12 membered cycloalkyl, 3-12 membered cycloalkenyl and 3-12 membered heterocyclyl, the C₁₋₆ alkyl, C₁₋₆ alkoxy, C₂₋₈ alkenyl, C₁₋₆ alkyl-R', C₁₋₆ alkoxy-R, 3-12 membered cycloalkyl, 3-12 membered cycloalkenyl and 3-12 membered heterocyclyl are optionally substituted with one or more R^{m};
R^{b}, R^{c} and R^{d} are each absent or each independently selected from the group consisting of hydrogen, hydroxyl, mercapto, C₁₋₆ alkyl, halogen, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', -OR', -C(=O)-R', 3-12 membered cycloalkyl and 3-12 membered heterocyclyl, the C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', -OR', -C(=O)-R', 3-12 membered cycloalkyl and 3-12 membered heterocyclyl are optionally substituted with one or more R^{m};
or R^{b} and R^{c} together with the nitrogen atom to which they are both attached to form a 3-12 membered monocyclic, spirocyclic, bridged or fused ring optionally substituted with one or more R^{m};
R^{e} and R^{f} are each independently selected from the group consisting of hydrogen, C₁₋₆ alkyl, C₂₋₆ alkenyl, 3-12 membered cycloalkyl and 3-20 membered heterocyclyl, wherein the C₁₋₆ alkyl, C₂₋₆ alkenyl, 3-12 membered cycloalkyl and 3-20 membered heterocyclyl are optionally substituted with one or more C₁₋₆ alkyl or halogen;
R^{g} is C₁₋₆ alkyl or 6-20 membered aryl, wherein the C₁₋₆ alkyl and 6-20 membered aryl are optionally substituted with one or more groups independently selected from: halogen or OR^{e};
R' is selected from the group consisting of C₁₋₆ alkyl, 3-12 membered cycloalkyl, 3-12 membered heterocyclyl and 5-10 membered heteroaryl, the C₁₋₆ alkyl, 3-12 membered cycloalkyl, 3-12 membered heterocyclyl and 5-10 membered heteroaryl are optionally substituted with one or more R^{m}; the 3-12 membered cycloalkyl and the 3-12 membered heterocyclyl comprise monocyclic, spirocyclic or bridged ring, and the 5-10 membered heteroaryl comprise monocyclic or fused ring.

6. The compound or a pharmaceutically acceptable salt, stereoisomer, tautomer, deuteride, prodrug molecule, hydrate, or solvate thereof according to any one of claims 1 to 5, wherein, R^{m} is independently selected from the group consisting of deuterium, halogen, cyano, nitro, -OR^{a}, -SR^{a}, -C(=Y¹)R^{a}, -C(=Y¹)OR^{a}, -C(=Y¹)NR^{b}R^{c}, -NR^{b}R^{c}, -NR^{b}C(=Y¹)R^{c}, -NR^{b}SO₂R^{a}, -OC(=Y¹)R^{a}, -SO₂R^{a}, C₁₋₁₂ alkyl, halogenerated C₁₋₁₂ alkyl, C₁₋₁₂ alkoxy-C₁₋₁₂ alkyl, C₁₋₁₂ alkyl-C₁₋₁₂ alkoxy, C₁₋₁₂ alkoxy-C₁₋₁₂ alkoxy, C₂₋₈ alkenyl, C₂₋₈ alkynyl, 3-12 membered cycloalkyl, 3-20 membered heterocyclyl, 6-20 membered aryl, 5-20 membered heteroaryl, (CR^{a}R^{d})ₜNR^{b}R^{c} and oxo, the 3-12 membered cycloalkyl and 3-20 membered heterocyclyl comprisemonocyclic, spirocyclic or bridged ring, the 6-20 membered aryl and 5-20 membered heteroaryl groups comprise monocyclic or fused ring;
preferably, R^{m} is selected from the group consisting of deuterium, halogen, cyano, nitro, hydroxyl, mercapto, amino, carboxyl, C₁₋₆ alkyl, C₁₋₆ alkoxy-C₁₋₆ alkyl, C₁₋₆ alkoxy, C₁₋₆ alkyl-C₁₋₆ alkoxy, C₁₋₆ alkoxy-C₁₋₆ alkoxy, C₁₋₆ alkylamino, (C₁₋₆ alkyl)₂amino, C₁₋₆ alkyl ester, C₁₋₆ alkylaminocarbonyl, (C₁₋₆ alkyl)₂aminocarbonyl, C₁₋₆ alkylcarbonyl, C₁₋₆ alkylcarbonyloxy, C₁₋₆ alkylcarbonylamino, C₁₋₆ alkylsulfonylamino, halogenated C₁₋₆ alkyl, halogenated C₁₋₆ alkoxy, C₁₋₆ alkylsulfonyl, C₁₋₆ alkylthio, C₂₋₈ alkenyl, C₂₋₈ alkynyl, 3-8 membered cycloalkyl, 3-8 membered heterocyclyl, 6-10 membered aryl, 3-8 membered heteroaryl, - (CR^{a}R^{d})ₜNR^{b}R^{c} and oxo.

7. The compound or a pharmaceutically acceptable salt, stereoisomer, tautomer, deuteride, prodrug molecule, hydrate, or solvate thereof according to any one of claims 1 to 6, wherein,
A is selected from 5-14 membered heteroaryl substituted with R¹, the 5-14 membered heteroaryl substituted with R¹ comprises monocyclic or fused ring, optionally substituted with one or more R²;
L is selected from O, S, CR^{a}R^{b}, NR^{b} and C(=O);
Z is selected from 6-14 membered aryl and 5-10 membered heteroaryl optionally substituted with one or more R³;
X is selected from NR⁴, O, S or is absent;
Y is selected from C(=O), C(=S), C(=NR⁴) or is absent;
E₁, E₂, E₃ are each independently selected from CR^{a}R^{b}, N, C(=O) and C(=S);
G is selected from the group consisting of (CR^{a}R^{b})ₙ₁-6-20 membered aryl, (CR^{a}R^{b})ₙ₁-5-20 membered heteroaryl, (CR^{a}R^{b})ₙ₁-3-12 membered cycloalkyl and (CR^{a}R^{b})ₙ₁-3-20 membered heterocyclyl, the (CR^{a}R^{b})ₙ₁-6-20 membered aryl, (CR^{a}R^{b})ₙ₁-5-20 membered heteroaryl, (CR^{a}R^{b})ₙ₁-3-12 membered cycloalkyl and (CR^{a}R^{b})ₙ₁-3-20 membered heterocyclyl are optionally substituted with one or more groups independently selected from the group consisting of: halogen, nitro, oxo, CN, OR^{e}, SR^{e}, NR^{e}R^{f}, C₁₋₁₂ alkyl, halogenated C₁₋₁₂ alkyl, C₁₋₆ alkoxy-C₁₋₁₂ alkyl, halogenated C₁₋₆ alkoxy-C₁₋₁₂ alkyl, R^{a}R^{b}N-C₁₋₆ alkyl, C₂₋₈ alkenyl, halogenated C₂₋₈ alkenyl, C₂₋₈ alkynyl, 3-12 membered cycloalkyl or 3-20 membered heterocyclyl;
R¹ is selected from R⁸ substituted with one or two hydroxy;
R² and R³ are each independently selected from the group consisting of hydrogen, halogen, cyano, nitro, -NR^{b}R^{c}, -OR^{b}, R^{b}R^{c}N-C₁₋₆ alkyl, R^{b}R^{c}N-C₁₋₆ alkoxy, -C(=Y¹)R^{a}, -C(=Y¹)NR^{b}R^{c}, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl, 3-12 membered heterocyclyl-C₁₋₆ alkyl and 5-10 membered heteroaryl-C₁₋₆ alkyl, the R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl, 3-12 membered heterocyclyl-C₁₋₆ alkyl and 5-10 membered heteroaryl-C₁₋₆ alkyl are optionally substituted with one or more R^{m}, the 3-12 membered cycloalkyl, 3-12 membered heterocyclyl, 3-12 membered cycloalkyl-C₁₋₆ alkyl and 3-12 membered heterocyclyl-C₁₋₆ alkyl comprise monocyclic, spirocyclic or bridged ring, the 5-10 membered heteroaryl-C₁₋₆ alkyl comprises monocyclic or fused ring;
R⁴, R⁵, R⁶ and R⁷ are each independently selected from the group consisting of hydrogen, halogen, OR^{e}, C₁₋₆ alkyl, C₂₋₆ alkenyl, C₂₋₆ alkynyl, 3-20 membered cycloalkyl, 3-20 membered heterocyclyl and 6-20 membered aryl, wherein the 3-20 membered cycloalkyl and 3-20 membered heterocyclyl comprise monocyclic, spirocyclic or bridged ring; the C₁₋₆ alkyl, C₂₋₆ alkenyl, C₂₋₆ alkynyl, 3-20 membered cycloalkyl, 3-20 membered heterocyclyl and 6-20 membered aryl are optionally substituted with one or more groups independently selected from the group consisting of: halogen, CN, OR^{e}, NR^{e}R^{f}, C₁₋₁₂ alkyl, C₂₋₈ alkenyl, C₂₋₈ alkynyl, 3-12 membered cycloalkyl or 3-20 membered heterocyclyl;
or R⁴ and R⁵ together with the carbon atom to which they are both attached to form a 3-12 membered monocyclic, spirocyclic or bridged ring optionally substituted with one or more R^{m};
or R⁶ and R⁷ together with the nitrogen atom to which they are both attached to form a saturated, partially unsaturated or fully unsaturated 3-20 membered heterocycle optionally substituted with one or more R^{m}, containing one or more heteroatoms selected from N, O or S;
R⁸ is selected from the group consisting of -NR^{b}R^{c}, -OR^{b}, -SR^{b}, R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered cycloalkenyl, 3-12 membered heterocyclyl, 6-14 membered aryl, 5-10 membered heteroaryl, 3-12 membered cycloalkyl-C₁₋₆ alkyl, 3-12 membered cycloalkenyl-C₁₋₆ alkyl, 3-12 membered heterocyclyl-C₁₋₆ alkyl, 6-14 membered aryl-C₁₋₆ alkyl, and 5-10 membered heteroaryl-C₁₋₆ alkyl, the R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆alkoxy-R^{b}, 3-12 membered cycloalkyl, 3-12 membered cycloalkenyl, 3-12 membered heterocyclyl, 6-14 membered aryl, 5-10 membered heteroaryl, 3-12 membered cycloalkyl-C₁₋₆ alkyl, 3-12 membered cycloalkenyl-C₁₋₆ alkyl, 3-12 membered heterocyclyl-C₁₋₆ alkyl, 6-14 membered aryl-C₁₋₆ alkyl, and 5-10 membered heteroaryl-C₁₋₆ alkyl are optionally substituted with one or more R^{m}, the 3-12 membered cycloalkyl, 3-12 membered cycloalkenyl and 3-12 membered heterocyclyl comprise monocyclic, spirocyclic or bridged ring, the 6-14 membered aryl and 5-10 membered heteroaryl comprise monocyclic or fused ring;
R^{a} is absent or selected from the group consisting of hydrogen, halogen, cyano, hydroxyl, mercapto, carboxyl, nitro, -NR^{b}R^{c}, -C(=O)R^{d}, C₁₋₆ alkyl, C₁₋₆ alkoxy, C₂₋₈ alkenyl, C₂₋₈ alkynyl, C₁₋₆ alkyl-R', C₁₋₆ alkoxy-R', -OR', -SR', -SOR^{d}, -SO₂R^{d}, 3-12 membered cycloalkyl, 3-12 membered cycloalkenyl, 3-12 membered heterocyclyl, 6-14 membered aryl and 5-10 membered heteroaryl, the C₁₋₆ alkyl, C₁₋₆ alkoxy, C₂₋₈ alkenyl, C₂₋₈ alkynyl, C₁₋₆ alkyl-R', C₁₋₆ alkoxy-R', 3-12 membered cycloalkyl, 3-12 membered cycloalkenyl, 3-12 membered heterocyclyl, 6-14 membered aryl, and 5-10 membered heteroaryl are optionally substituted with one or more R^{m};
R^{b}, R^{c} and R^{d} are each absent or each independently selected from the group consisting of hydrogen, hydroxyl, mercapto, C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R, -OR', -C(=O)-R', 3-12 membered cycloalkyl, 3-12 membered cycloalkenyl, 3-12 membered heterocyclyl, 6-14 membered aryl and 5-10 membered heteroaryl, the C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', 3-12 membered cycloalkyl, 3-12 membered cycloalkenyl, 3-12 membered heterocyclyl, 6-14 membered aryl, and 5-10 membered heteroaryl are optionally substituted with one or more R^{m};
or R^{b} and R^{c} together with the nitrogen atom to which they are both attached to form a 3-12 membered monocyclic, spirocyclic, bridged or fused ring optionally substituted with one or more R^{m};
R^{e} and R^{f} are each independently selected from the group consisting of hydrogen, C₁₋₆ alkyl, C₂₋₆ alkenyl, 3-12 membered cycloalkyl, 3-20 membered heterocyclyl, 6-20 membered aryl, and 5-20 membered heteroaryl, wherein the C₁₋₆ alkyl, C₂₋₆ alkenyl, 3-12 membered cycloalkyl, 3-20 membered heterocyclyl, 6-20 membered aryl, and 5-20 membered heteroaryl are optionally substituted with one or more C₁₋₆ alkyl or halogen;
R^{g} is C₁₋₆ alkyl or 6-20 membered aryl, wherein the C₁₋₆ alkyl and 6-20 membered aryl are optionally substituted with one or more groups independently selected from the group consisting of: halogen or OR^{e};
R' is selected from the group consisting of C₁₋₆ alkyl, 3-12 membered cycloalkyl, 3-12 membered cycloalkenyl, 3-12 membered heterocyclyl, 6-14 membered aryl and 5-10 membered heteroaryl, the C₁₋₆ alkyl, 3-12 membered cycloalkyl, 3-12 membered cycloalkenyl, 3-12 membered heterocyclyl, 6-14 membered aryl and 5-10 membered heteroaryl are optionally substituted with one or more R^{m}; the 3-12 membered cycloalkyl, 3-12 membered cycloalkenyl and 3-12 membered heterocyclyl comprise monocyclic, spirocyclic or bridged ring, and the 6-14 membered aryl and 5-10 membered heteroaryl comprise monocyclic or fused ring;
R^{m} is independently selected from the group consisting of deuterium, halogen, cyano, nitro, -OR^{a}, -SR^{a}, -C(=Y¹)R^{a}, -C(=Y¹)OR^{a}, -C(=Y¹)NR^{b}R^{c}, -NR^{b}R^{c}, - NR^{b}C(=Y¹)R^{c}, -NR^{b}SO₂R^{a}, -OC(=Y¹)R^{a}, -SO₂R^{a}, C₁₋₁₂ alkyl, halogenated C₁₋₁₂ alkyl, C₁₋₁₂ alkoxy-C₁₋₁₂ alkyl, C₁₋₁₂ alkyl-C₁₋₁₂ alkoxy, C₁₋₁₂ alkoxy-C₁₋₁₂ alkoxy, C₂₋₈ alkenyl, C₂₋₈ alkynyl, 3-12 membered cycloalkyl, 3-20 membered heterocyclyl, 6-20 membered aryl, 5-20 membered heteroaryl, (CR^{a}R^{d})ₜNR^{b}R^{c} and oxo, and the 3-12 membered cycloalkyl and 3-20 membered heterocyclyl comprise monocyclic, spirocyclic or bridged ring, and the 6-20 membered aryl and 5-20 membered heteroaryl comprise monocyclic or fused ring.
Y¹ is O or S;
t, m1, and n1 are each independently 0, 1, 2, 3, 4, 5, or 6.
preferably, in formula I,
A is selected from the following groups substituted with R¹, optionally substituted with one or more R²; 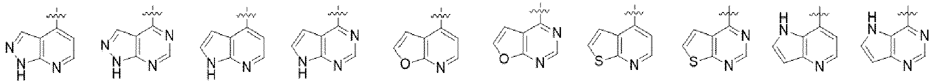 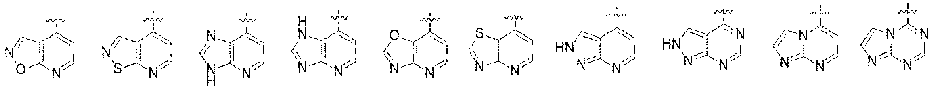 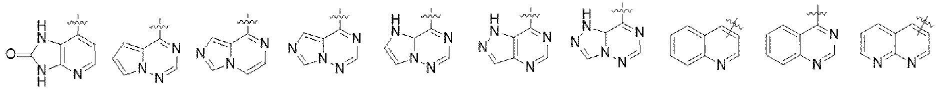 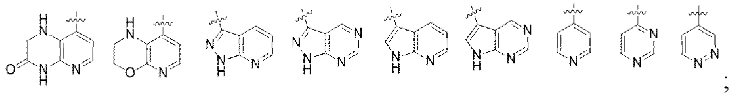
L is selected from O, S, C=O and NH;
Z is selected from the following groups optionally substituted with one or more R3: 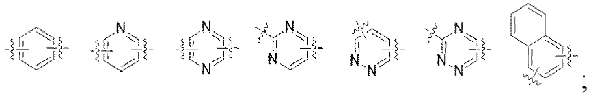
X is selected from NR⁴, O and S;
Y is selected from C(=O) and C(=S);
E₁, E₂, E₃ are each independently selected from CR^{a}R^{b} and N;
G is selected from the group consisting of 6-20 membered aryl and 5-20 membered heteroaryl, wherein the 6-20 membered aryl and 5-20 membered heteroaryl are optionally substituted with one or more groups independently selected from the group consisting of: halogen, nitro, oxo, CN, OR^{e}, SR^{e}, NR^{e}R^{f}, C₁₋₁₂ alkyl, halogenated C₁₋₁₂ alkyl, C₁₋₆ alkoxy-C₁₋₁₂ alkyl, halogenated C₁₋₆ alkoxy-C₁₋₁₂ alkyl, R^{a}R^{b}N-C₁₋₆ alkyl, C₂₋₈ alkenyl, halogenated C₂₋₈ alkenyl, C₂₋₈ alkynyl, 3-12 membered cycloalkyl, 3-12 membered cycloalkyl-C₁₋₆ alkyl or 3-20 membered heterocyclyl;
R¹ is selected from R⁸ substituted with one or two hydroxy;
R² and R³ are each independently selected from the group consisting of hydrogen, halogen, cyano, nitro, -NR^{b}R^{c}, -OR^{b}, R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl and 3-12 membered heterocyclyl-C₁₋₆ alkyl, the R^{b}R^{c}N-C₁₋₆ alkyl, R^{b}R^{c}N-C₁₋₆ alkoxy, C₁₋₆ alkyl, C₂₋₈ alkenyl, C₂₋₈ alkynyl, C₁₋₆ alkyl-R^{a}, C₁₋₆ alkoxy-R^{b}, 3-12 membered cycloalkyl, 3-12 membered heterocyclyl, 3-12 membered cycloalkyl-C₁₋₆ alkyl and 3-12 membered heterocyclyl-C₁₋₆ alkyl are optionally substituted with one or more R^{m}, the 3-12 membered cycloalkyl, 3-12 membered heterocyclyl, 3-12 membered cycloalkyl-C₁₋₆ alkyl and 3-12 membered heterocyclyl-C₁₋₆ alkyl comprise monocyclic, spirocyclic or bridged ring;
R⁴, R⁵, R⁶ and R⁷ are each independently selected from the group consisting of hydrogen, OR^{e}, C₁₋₆ alkyl, monocyclic or bicyclic of 3-12 membered cycloalkyl, 3-20 membered heterocyclyl and 6-20 membered aryl, wherein the C₁₋₆ alkyl, monocyclic or bicyclic of 3-12 membered cycloalkyl, 3-20 membered heterocyclyl and 6-20 membered aryl are optionally substituted with one or more groups independently selected from the group consisting of halogen, CN, OR^{e}, NR^{e}R^{f}, C₁₋₁₂ alkyl, C₂₋₈ alkenyl, C₂₋₈ alkynyl, 3-12 membered cycloalkyl or 3-20 membered heterocyclyl;
or R⁴ and R⁵ together with the carbon atom to which they are both attached to form a 3-12 membered monocyclic, spirocyclic or bridged ring optionally substituted with one or more R^{m}, the 3-12 membered monocyclic, spirocyclic or bridged ring is optionally substituted with one or more groups independently selected from: halogen, C₁₋₆ alkyl or C₁₋₆ alkoxy;
or R⁶ and R⁷ together with the nitrogen atom to which they are both attached to form a saturated, partially unsaturated, or fully unsaturated 3-20 membered heterocycle optionally substituted with one or more R^{m}, the 3-20 membered heterocycle is optionally substituted with one or more groups independently selected from: halogen, C₁₋₆ alkyl or C₁₋₆ alkoxy;
R⁸ is selected from the group consisting of-NR^{b}R^{c}, -OR^{b}, C₁₋₆ alkyl, R^{b}R^{c}N-C₁₋₆ alkyl, 3-12 membered heterocyclyl and 3-12 membered heterocyclyl-C₁₋₆ alkyl, the C₁₋₆ alkyl, R^{b}R^{c}N-C₁₋₆ alkyl, 3-12 membered heterocyclyl and 3-12 membered heterocyclyl-C₁₋₆ alkyl are optionally substituted with one or more R^{m}, the 3-12 membered heterocyclyl and 3-12 membered heterocyclyl-C₁₋₆ alkyl comprise monocyclic, spirocyclic or bridged ring;
R^{a} is absent or selected from the group consisting of hydrogen, halogen, cyano, hydroxy, -NR^{b}R^{c}, -C(=O)R^{d}, C₁₋₆ alkyl, C₁₋₆ alkoxy, C₂₋₈ alkenyl, C₁₋₆ alkyl-R', C₁₋₆ alkoxy-R', -OR', -SR', -SOR^{d}, -SO₂R^{d}, 3-12 membered cycloalkyl, 3-12 membered cycloalkenyl and 3-12 membered heterocyclyl, the C₁₋₆ alkyl, C₁₋₆ alkoxy, C₂₋₈ alkenyl, C₁₋₆ alkyl-R', C₁₋₆ alkoxy-R', 3-12 membered cycloalkyl, 3-12 membered cycloalkenyl and 3-12 membered heterocyclyl are optionally substituted with one or more R^{m};
R^{b}, R^{c} and R^{d} are each absent or each independently selected from the group consisting of hydrogen, hydroxyl, mercapto, C₁₋₆ alkyl, halogen, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', -OR', -C(=O)-R', 3-12 membered cycloalkyl and 3-12 membered heterocyclyl, the C₁₋₆ alkyl, C₂₋₆ alkenyl, C₂₋₆ alkynyl, C₁₋₆ alkoxy, C₁₋₆ alkyl-R', R'-C₁₋₆ alkyl, C₁₋₆ alkoxy-R', -OR', -C(=O)-R', 3-12 membered cycloalkyl and 3-12 membered heterocyclyl are optionally substituted with one or more R^{m};
or R^{b} and R^{c} together with the nitrogen atom to which they are both attached to form a 3-12 membered monocyclic, spirocyclic, bridged or fused ring optionally substituted with one or more R^{m};
R^{e} and R^{f} are each independently selected from the group consisting of hydrogen, C₁₋₆ alkyl, C₂₋₆ alkenyl, 3-12 membered cycloalkyl and 3-20 membered heterocyclyl, wherein the C₁₋₆ alkyl, C₂₋₆ alkenyl, 3-12 membered cycloalkyl and 3-20 membered heterocyclyl are optionally substituted with one or more C₁₋₆ alkyl or halogen;
R^{g} is C₁₋₆ alkyl or 6-20 membered aryl, wherein the C₁₋₆ alkyl and 6-20 membered aryl are optionally substituted with one or more groups independently selected from: halogen or OR^{e};
R' is selected from the group consisting of C₁₋₆ alkyl, 3-12 membered cycloalkyl, 3-12 membered heterocyclyl and 5-10 membered heteroaryl, the C₁₋₆ alkyl, 3-12 membered cycloalkyl, 3-12 membered heterocyclyl and 5-10 membered heteroaryl are optionally substituted with one or more R^{m}; the 3-12 membered cycloalkyl and 3-12 membered heterocyclyl comprise monocyclic, spirocyclic or bridged ring, and the 5-10 membered heteroaryl comprise monocyclic or fused ring;
R^{m} is selected from the group consisting of deuterium, halogen, cyano, nitro, hydroxyl, mercapto, amino, carboxyl, C₁₋₆ alkyl, C₁₋₆ alkoxy-C₁₋₆ alkyl, C₁₋₆ alkoxy, C₁₋₆ alkyl-C₁₋₆ alkoxy, C₁₋₆ alkoxy-C₁₋₆ alkoxy, C₁₋₆ alkylamino, (C₁₋₆ alkyl)₂amino, C₁₋₆ alkyl ester, C₁₋₆ alkylaminocarbonyl, (C₁₋₆ alkyl)₂aminocarbonyl, C₁₋₆ alkylcarbonyl, C₁₋₆ alkylcarbonyloxy, C₁₋₆ alkylcarbonylamino, C₁₋₆ alkylsulfonylamino, halogenated C₁₋₆ alkyl, halogenated C₁₋₆ alkoxy, C₁₋₆ alkylsulfonyl, C₁₋₆ alkylthio, C₂₋₈ alkenyl, C₂₋₈ alkynyl, 3-8 membered cycloalkyl, 3-8 membered heterocyclyl, 6-10 membered aryl, 3-8 membered heteroaryl, - (CR^{a}R^{d})ₜNR^{b}R° and oxo.
t is 0, 1, 2, 3, 4, 5 or 6;
more preferably, in formula I,
A is selected from the following groups substituted with R¹, the following groups are optionally substituted with one or more R²; 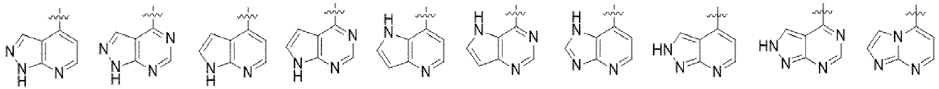 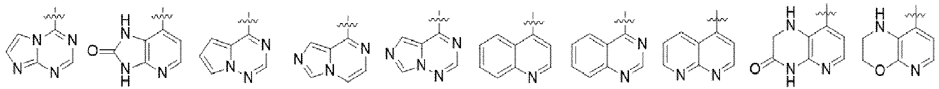 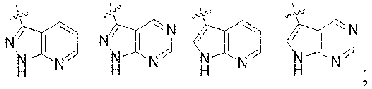
L is selected from O, C=O and NH;
Z is selected from the group consisting of benzene, pyridine and naphthalene optionally substituted with one or more R³:
X is selected from NR⁴;
Y is selected from C(=O);
E₁, E₂, E₃ are each independently selected from CR^{a}R^{b} and N;
G is selected from the group consisting of 6-20 membered aryl, pyridine and pyrazole, wherein the 6-20 membered aryl, pyridine and pyrazole are optionally substituted with one or more groups independently selected from the group consisting of: halogen, nitro, oxo, CN, methoxy, ethoxy, amino, methylamino, ethylamino, dimethylamino, methyl, ethyl, isopropyl, vinyl, CF₃, methoxymethyl, trifluoromethoxymethyl, cyclopropyl, cyclopropylmethyl;
R¹ is selected from the following groups: 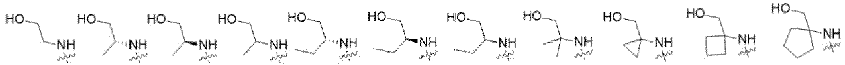 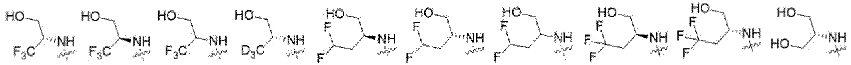 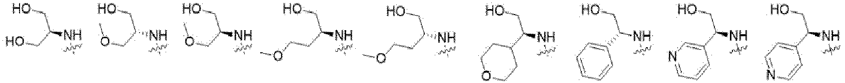 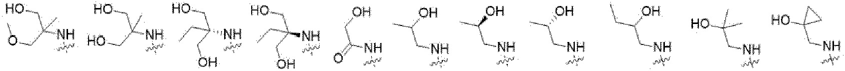 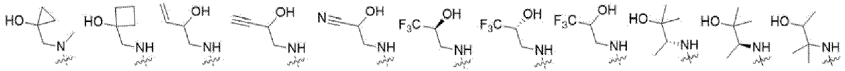 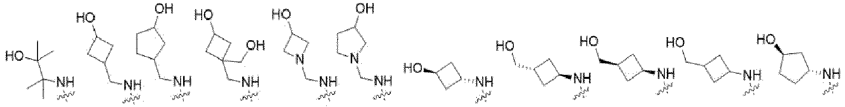 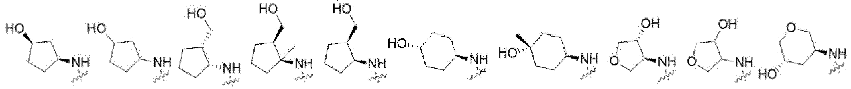 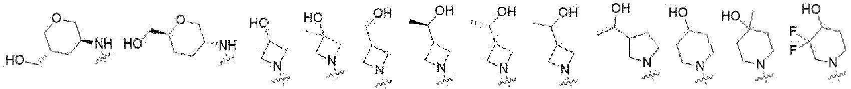 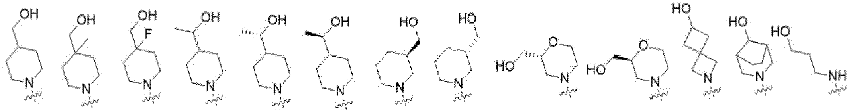 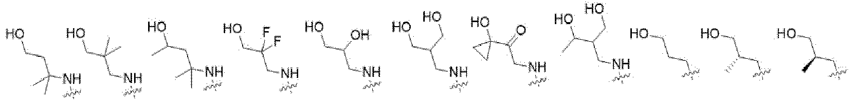 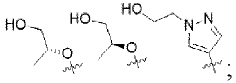
R² and R³ are each independently selected from the group consisting of hydrogen, hydroxyl, mercapto, halogen, cyano, nitro, C₁₋₆ alkyl, C₂₋₆ alkenyl, C₂₋₆ alkynyl, hydroxyl-C₁₋₆ alkyl, amino-C₁₋₆ alkyl, halogenated-C₁₋₆ alkyl, C₁₋₆ alkoxy, halogenated-C₁₋₆ alkoxy, C₁₋₆ alkoxy-C₁₋₆ alkyl, C₁₋₆ alkyl-C₁₋₆ alkoxy, C₁₋₆ alkoxy-C₁₋₆ alkoxy, 3-12 membered cycloalkyl, 3-12 membered heterocyclyl, 3-12 membered cycloalkyl-C₁₋₆ alkyl and 3-12 membered heterocyclyl-C₁₋₆ alkyl, the C₁₋₆ alkyl, C₂₋₆ alkenyl, C₂₋₆ alkynyl, hydroxyl-C₁₋₆ alkyl, amino-C₁₋₆ alkyl, halogenated-C₁₋₆ alkyl, C₁₋₆ alkoxy, halogenated-C₁₋₆ alkoxy, C₁₋₆ alkoxy-C₁₋₆ alkyl, C₁₋₆ alkyl-C₁₋₆ alkoxy, C₁₋₆ alkoxy-C₁₋₆ alkoxy, 3-12 membered cycloalkyl, 3-12 membered heterocyclyl, 3-12 membered cycloalkyl-C₁₋₆ alkyl, and 3-12 membered heterocyclyl-C₁₋₆ alkyl are optionally substituted with one or more R^{m};
R⁴ is independently selected from the group consisting of hydrogen, methyl, ethyl, trifluoromethyl and cyclopropyl;
R^{a} is not present or is independently selected from the group consisting of hydrogen, halogen, cyano, hydroxyl, amino, mercapto, C₁₋₆ alkyl, halogenated C₁₋₆ alkyl, C₁₋₆ alkylamino, (C₁₋₆ alkyl)₂amino, C₁₋₆ alkoxy, halogenated C₁₋₆ alkoxy, C₁₋₆ alkylthio, C₁₋₆ alkylsulfonyl, C₁₋₆ alkylsulfidenyl, halogenated C₁₋₆ alkylsulfonyl, C₁₋₆ alkoxy-C₁₋₆ alkyl, C₂₋₈ alkenyl, 3-12 membered cycloalkyl and 3-12 membered heterocyclyl, the C₁₋₆ alkyl, halogenated C₁₋₆ alkyl, C₁₋₆ alkylamino, (C₁₋₆ alkyl)₂amino, C₁₋₆ alkoxy, halogenated C₁₋₆ alkoxy, C₁₋₆ alkylthio, C₁₋₆ alkylsulfonyl, C₁₋₆ alkylsulfidenyl, halogenated C₁₋₆ alkylsulfonyl, C₁₋₆ alkoxy-C₁₋₆ alkyl, C₂₋₈ alkenyl, 3-12 membered cycloalkyl and 3-12 membered heterocyclyl are optionally substituted with one or more R^{m};
R^{b} is not present or is independently selected from the group consisting of hydrogen, C₁₋₆ alkyl, halogen, C₁₋₆ alkoxy, C₁₋₆ alkyl-3-12 membered cycloalkyl, C₁₋₆ alkyl-3-12 membered heterocyclyl, 3-12 membered cycloalkyl-C₁₋₆ alkyl, 3-12 membered heterocyclyl-C₁₋₆ alkyl, 3-12 membered cycloalkyl and 3-12 membered heterocyclyl, the C₁₋₆ alkyl, C₁₋₆ alkoxy, C₁₋₆ alkyl-3-12 membered cycloalkyl, C₁₋₆ alkyl-3-12 membered heterocyclyl, 3-12 membered cycloalkyl-C₁₋₆ alkyl, 3-12 membered heterocyclyl-C₁₋₆ alkyl, 3-12 membered cycloalkyl and 3-12 membered heterocyclyl are optionally substituted with one or more R^{m};
or R^{a} and R^{b} together with the nitrogen atom to which they are both attached to form a 3-12 membered monocyclic, spirocyclic, bridged or fused ring optionally substituted with one or more R^{m};
R^{m} is each independently selected from the group consisting of deuterium, halogen, cyano, nitro, hydroxyl, mercapto, amino, carboxyl, C₁₋₄ alkyl, C₁₋₄ alkoxy-C₁₋₄ alkyl, C₁₋₄ alkoxy, C₁₋₆ alkyl-C₁₋₄ alkoxy, C₁₋₄ alkoxy-C₁₋₄ alkoxy, C₁₋₄ alkylamino, (C₁₋₄ alkyl)₂amino, C₁₋₄ alkyl ester, C₁₋₄ alkylaminocarbonyl, (C₁₋₄ alkyl)₂aminocarbonyl, C₁₋₄ alkylcarbonyl, C₁₋₄ alkylcarbonyloxy, C₁₋₄ alkylcarbonylamino, C₁₋₄ alkylsulfonylamino, halogenated C₁₋₄ alkyl, halogenated C₁₋₄ alkoxy, C₁₋₄ alkylsulfonyl, C₁₋₄ alkylthio, vinyl, ethynyl, cyclopropyl, cyclobutyl, cyclopentyl, cyclohexyl, cycloheptyl, phenyl, epoxyethyl, epoxycyclobutyl, tetrahydrofuranyl, tetrahydropyranyl, oxacyclooctyl, aziridinyl, azetidinyl, tetrahydropyrrolyl, piperidinyl, piperazinyl, epoxyethylmethyl, epoxycyclobutylmethyl, tetrahydrofuranylmethyl, tetrahydropyranylmethyl, oxacyclooctylmethyl, aziridinylmethyl, azetidinylmethyl, tetrahydropyrrolylmethyl, piperidinylmethyl, piperazinylmethyl, pyrrolyl, furanyl, thienyl, oxazolyl, isoxazolyl, pyrazolyl, thiazolyl, pyridyl, pyrimidinyl, pyridazinyl and oxo.
most preferably, the compound of formula I is selected from the following compounds:

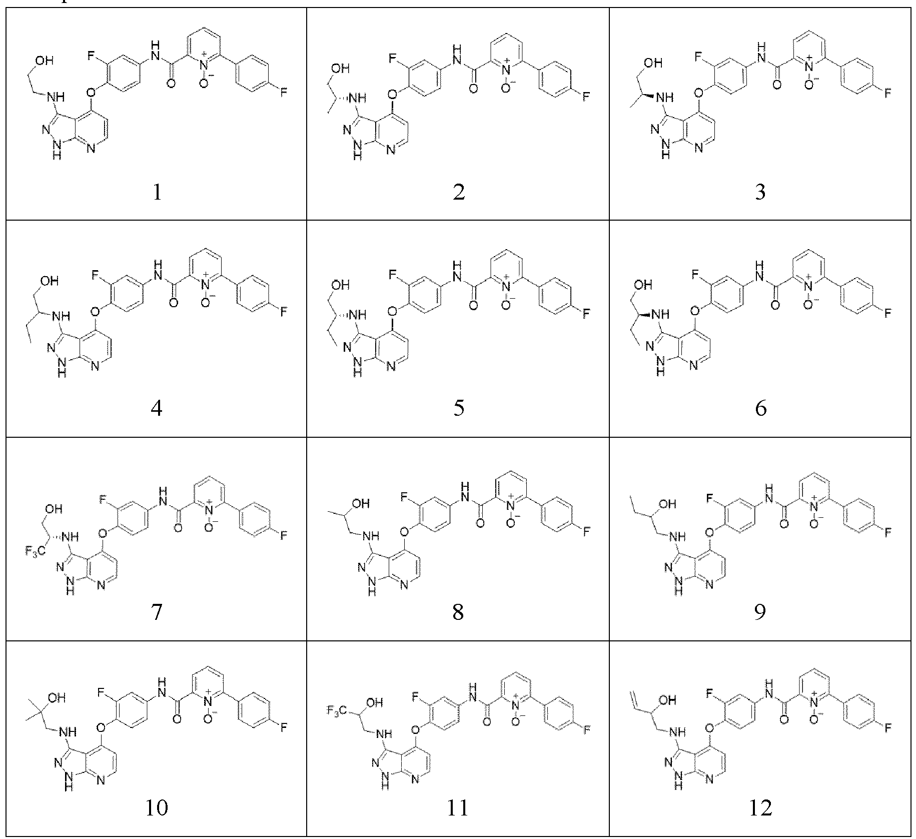

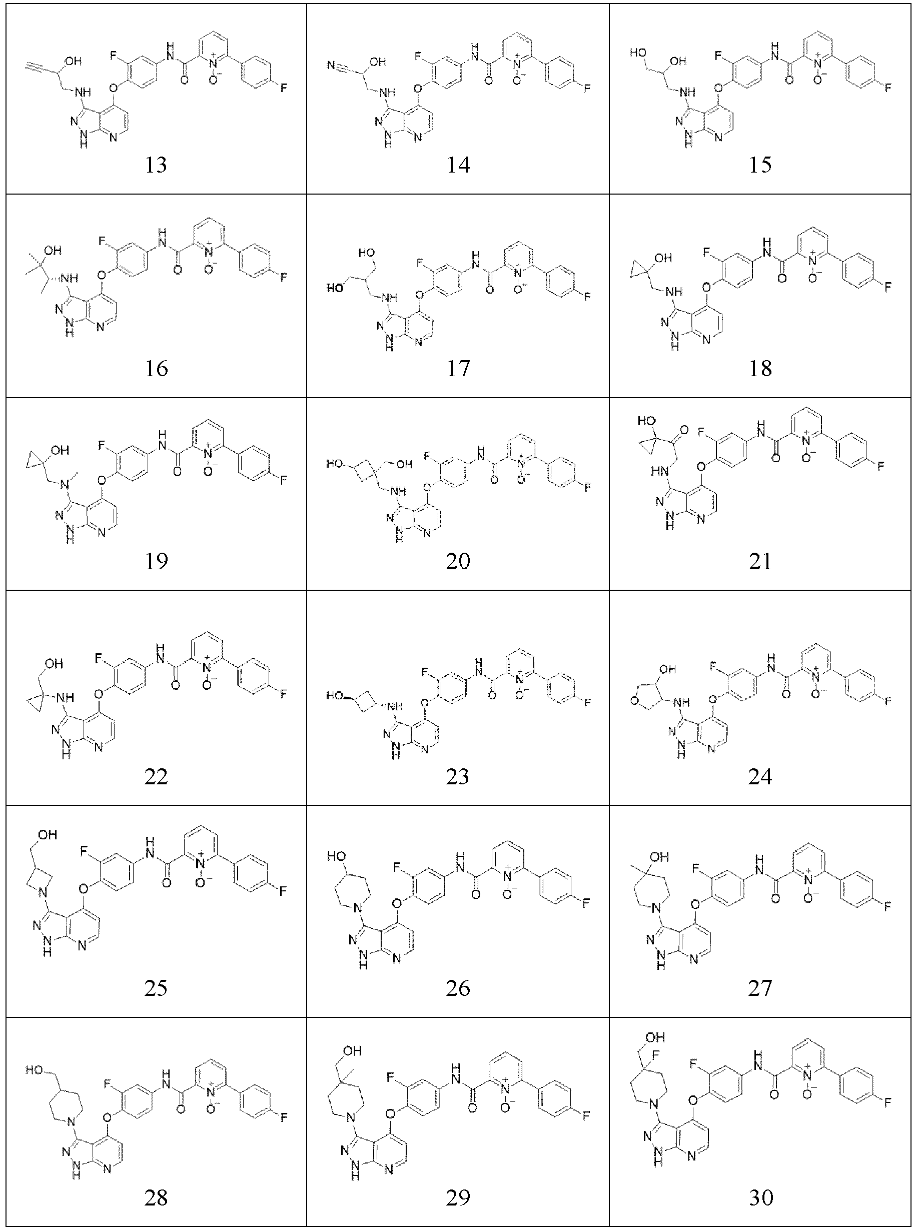

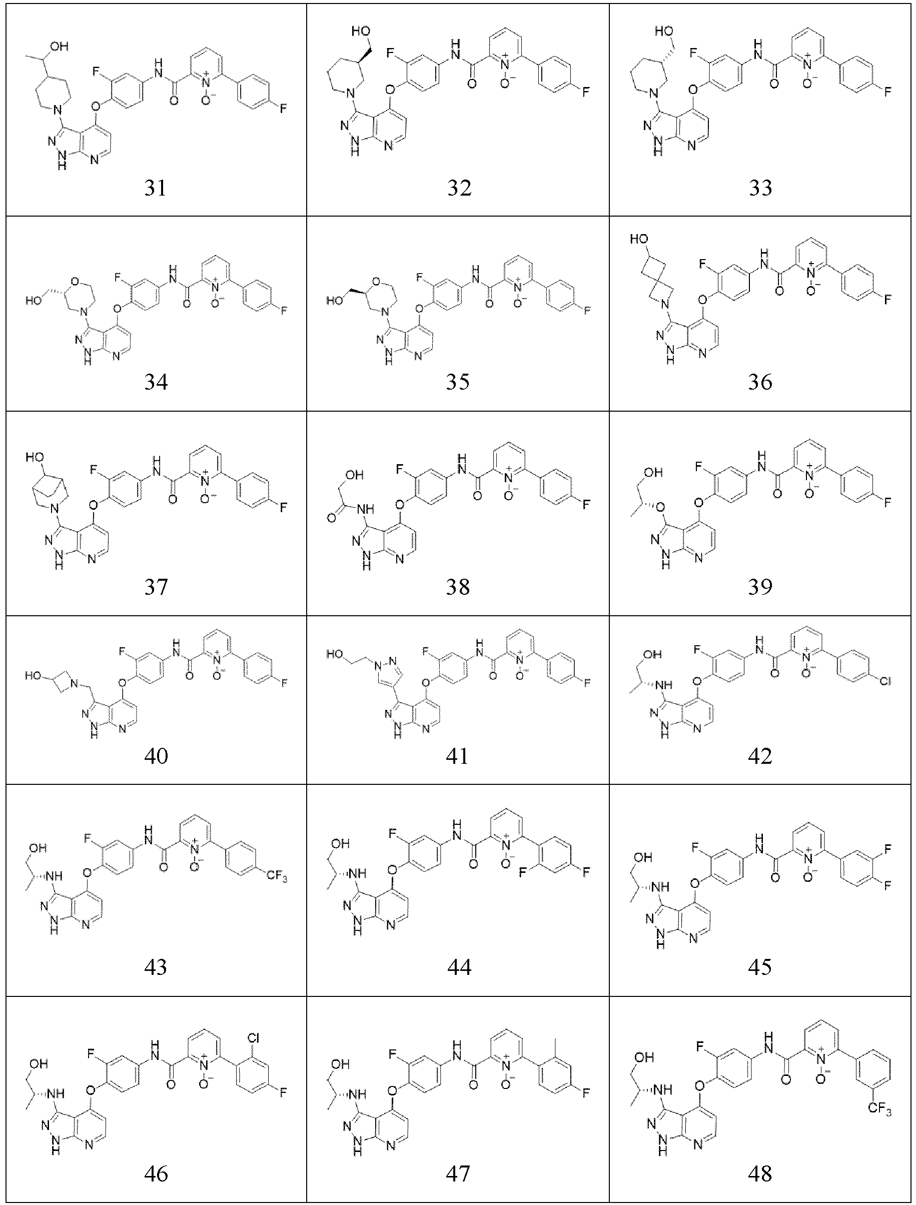

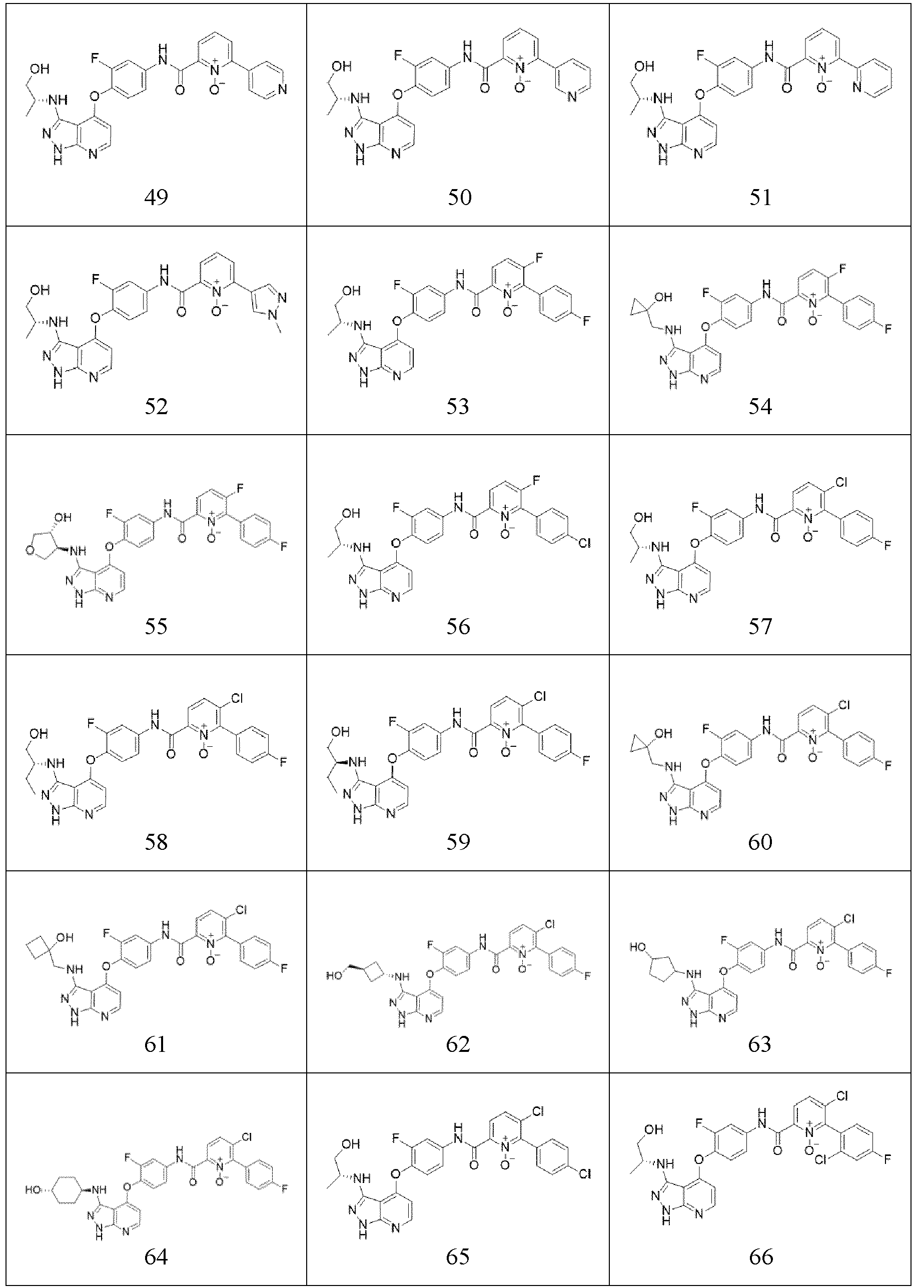

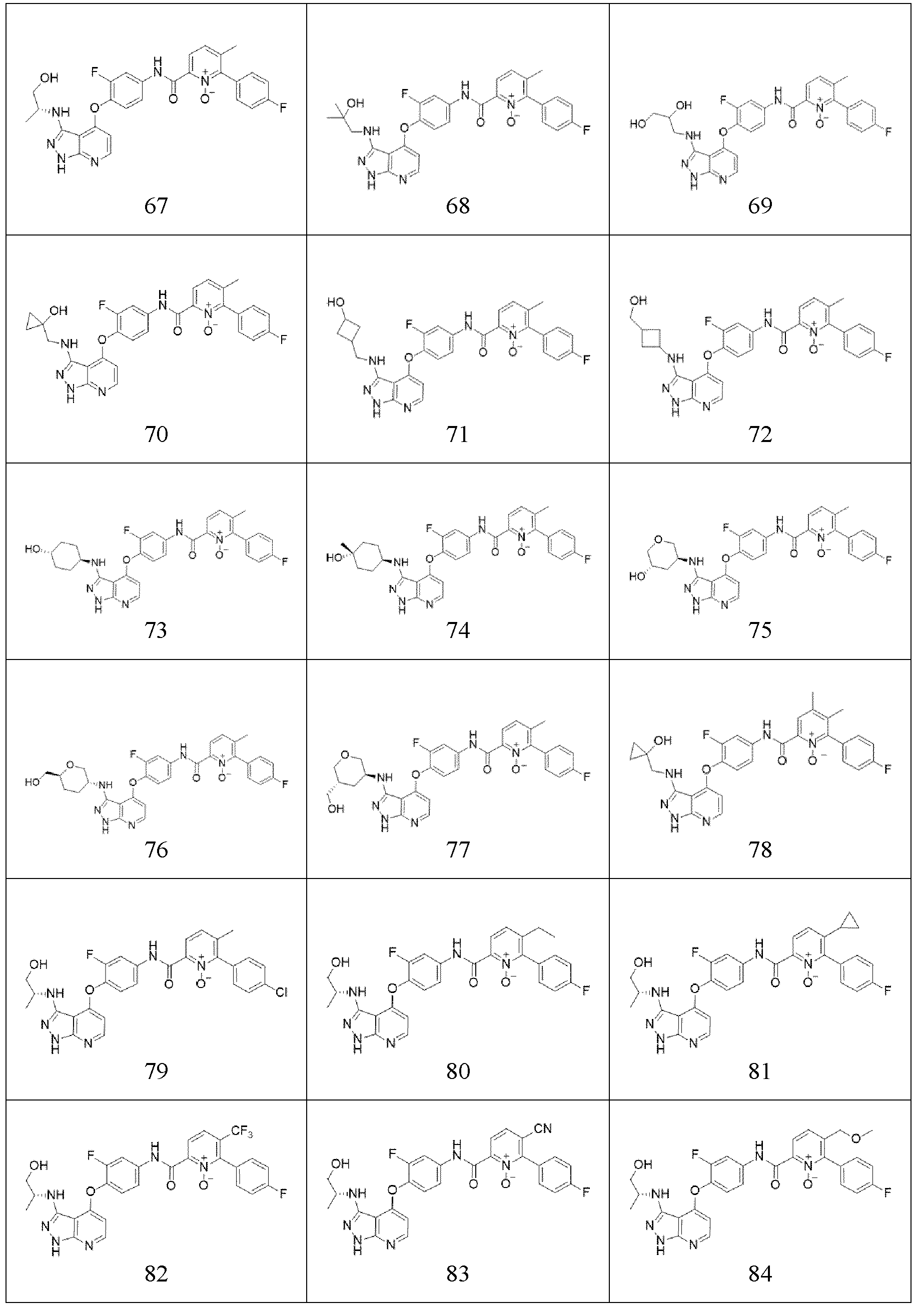

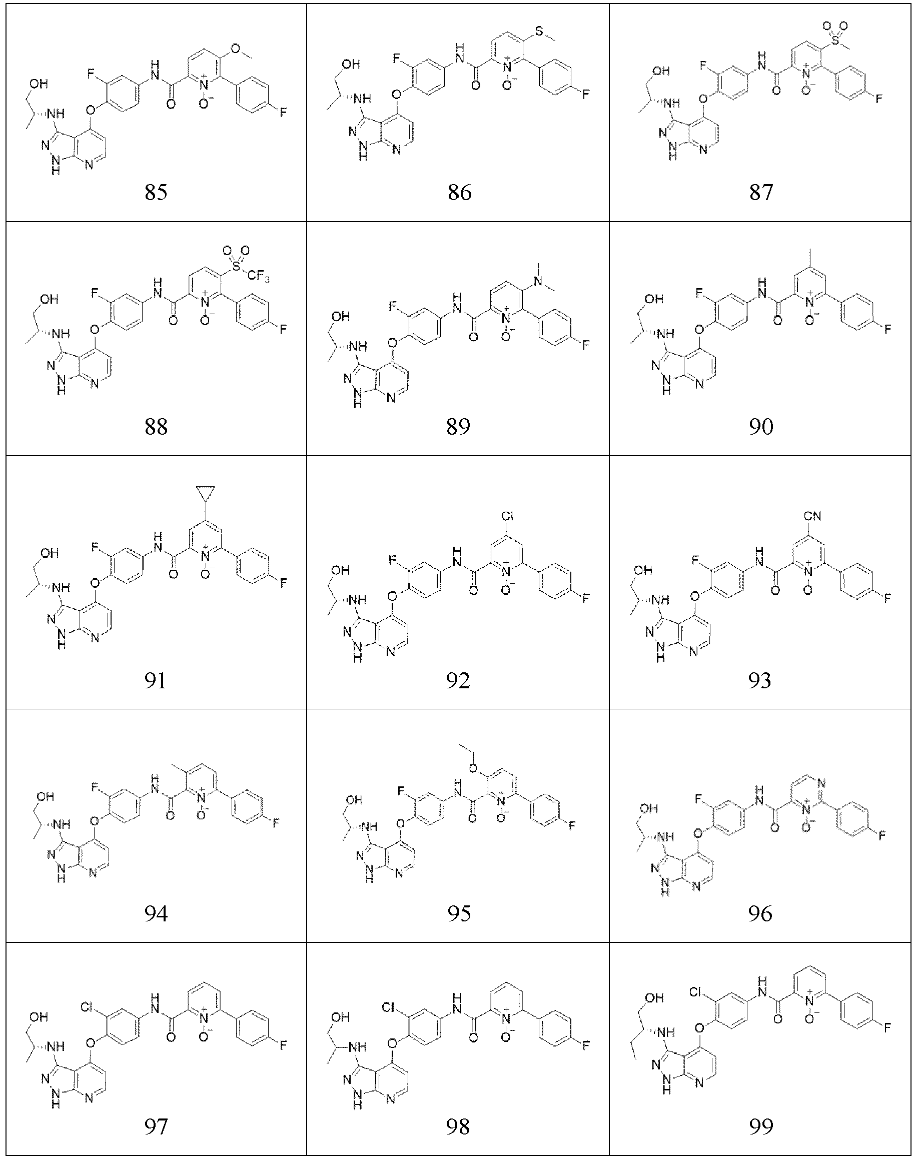

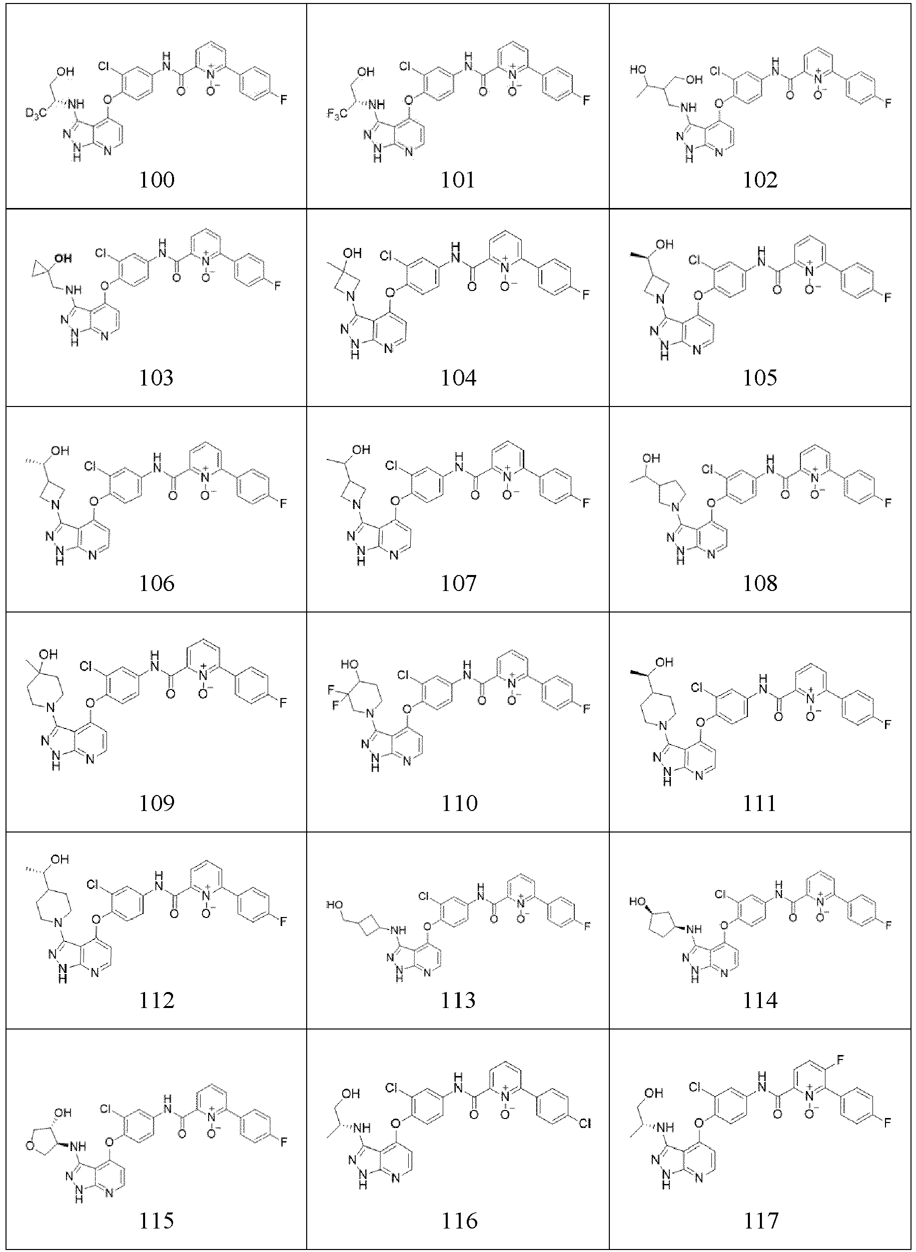

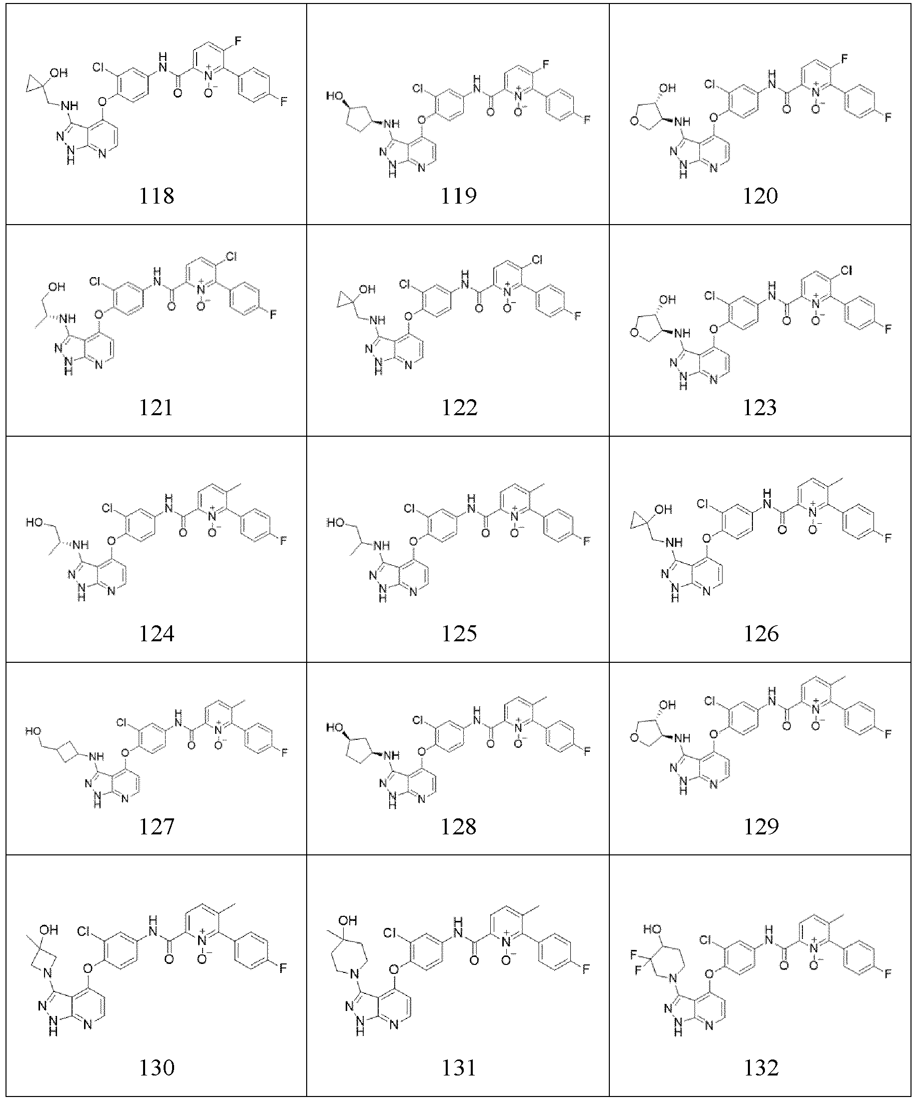

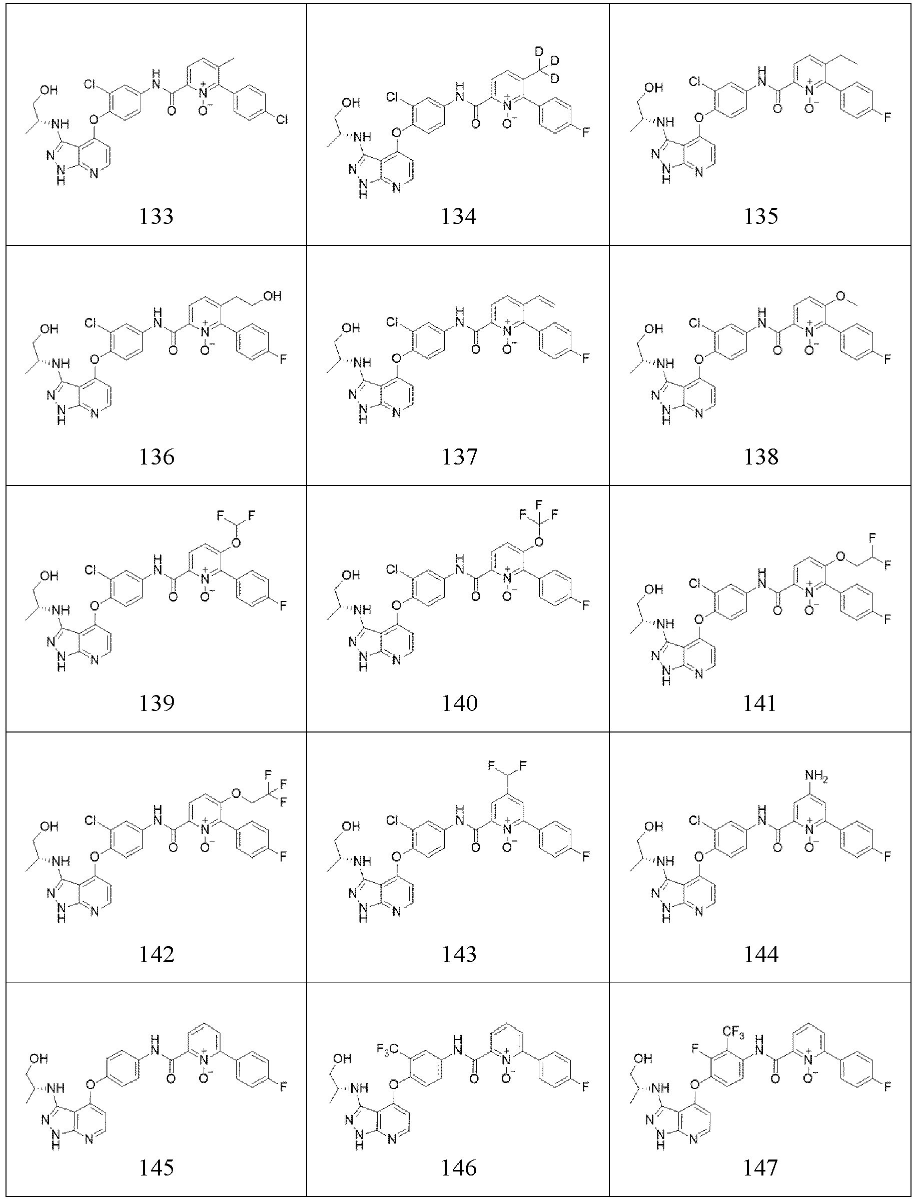

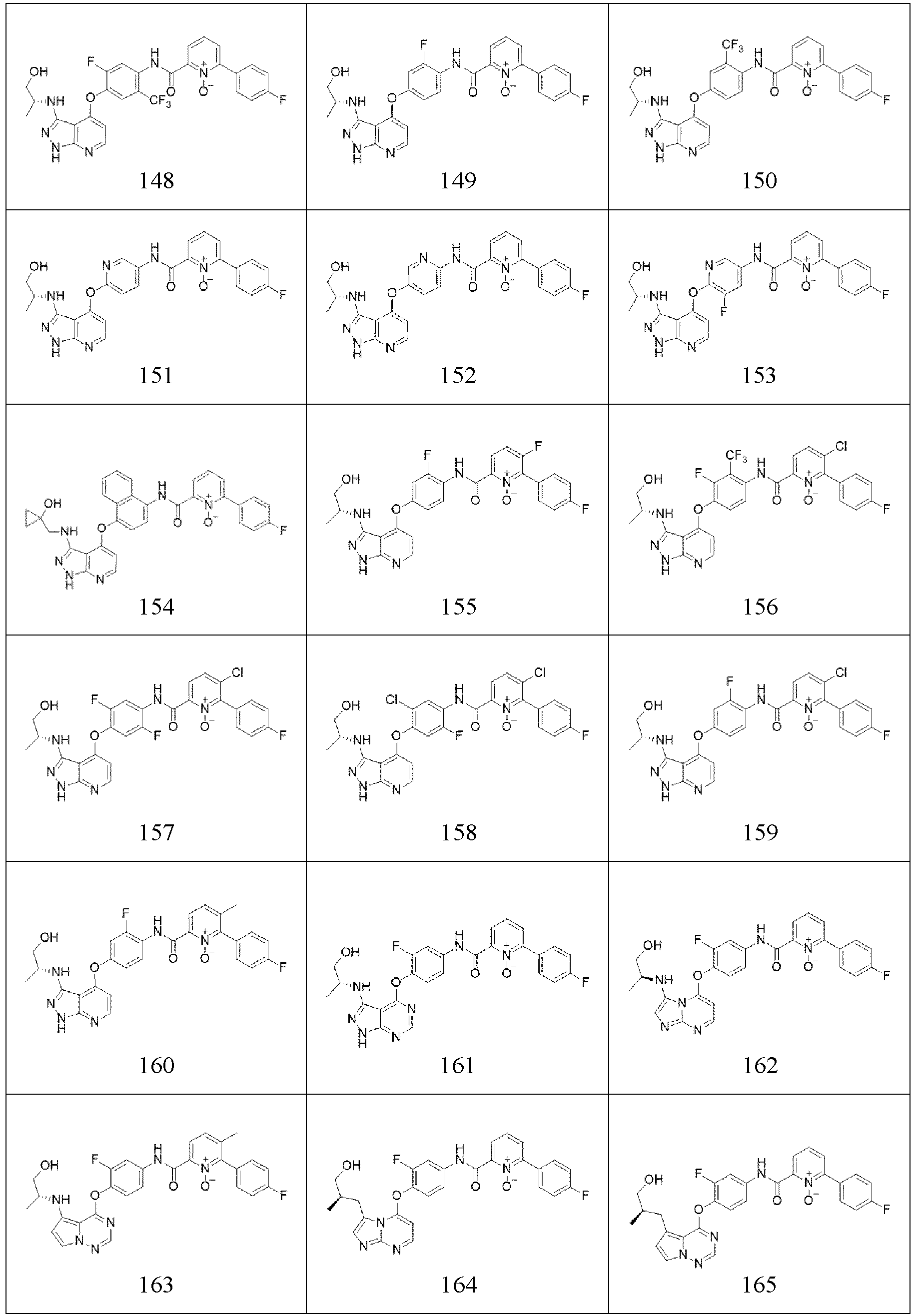

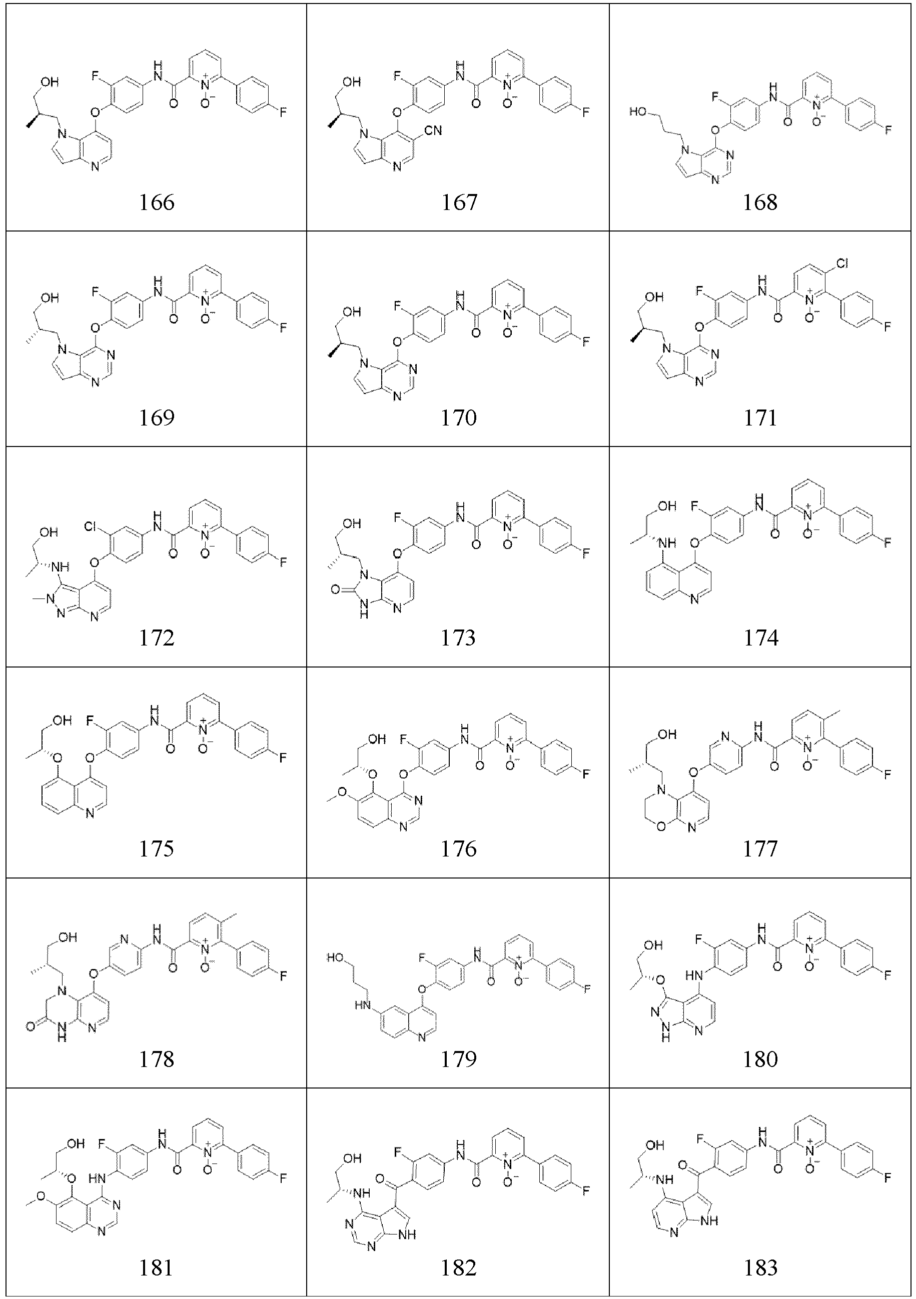

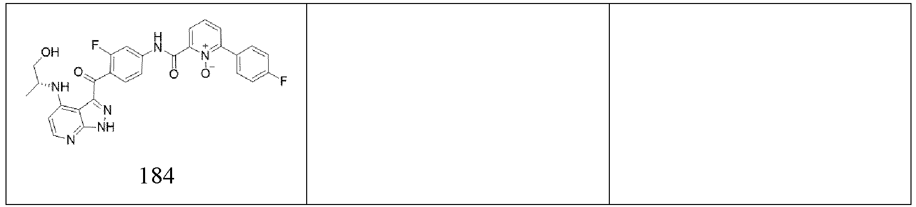

8. A method for preparing the compound according to any one of claims 1 to 7, comprising the steps of reacting compound 1 with compound M under basic conditions to form compound 2, and then oxidizing compound 2 under acidic conditions to form compound 3, and reacting compound 3 with compound 4 to form a compound of formula I; 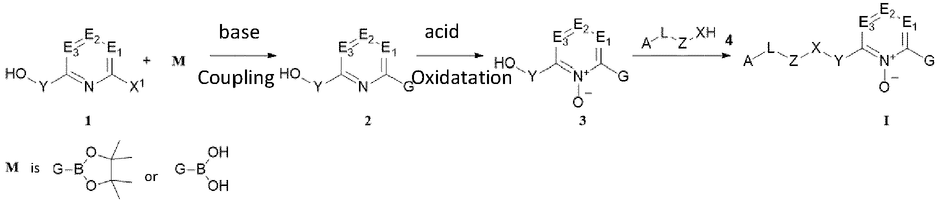 wherein, X¹ is halogen, A, L, Z, X, Y, E₁, E₂, E₃ and G are as defined in any one of claims 1 to 7,
preferably, the base is selected from cesium fluoride, cesium carbonate;
preferably, the acid is selected from trifluoroacetic acid.

9. A pharmaceutical composition comprising the compound or a pharmaceutically acceptable salt, stereoisomer, tautomer, deuteride, prodrug molecule, hydrate, or solvate thereof according to any one of claims 1-7, and a pharmaceutically acceptable carrier or excipient; preferably, the pharmaceutical composition is in the form of tablets, capsules, pills, granules, powders, suppositories, injections, solutions, suspensions, ointments, patches, lotions, drops, liniments and sprays.

10. Use of the compound or a pharmaceutically acceptable salt, stereoisomer, tautomer, deuteride, prodrug molecule, hydrate, or solvate thereof according to any one of claims 1-7, or the composition according to claim 9 in the manufacture of a medicament for the treatment of a disease associated with a protein kinase;
preferably, the disease associated with a protein kinase is selected from a disease associated with c-Met, VEGFR-2, AXL, TAM, NTRK, or RET.
preferably, the disease associated with a protein kinase is a tumor.
preferably, the disease associated with a protein kinase includes head and neck cancer, nasopharyngeal carcinoma, melanoma, bladder cancer, esophageal cancer, anaplastic large cell lymphoma, renal cancer, breast cancer, colorectal cancer, ovarian cancer, cervical cancer, pancreatic cancer, glioma, glioblastoma, prostate cancer, leukemia, lymphoma, non-Hodgkin's lymphoma, gastric cancer, lung cancer, liver cancer, gastrointestinal stromal tumor, thyroid cancer, squamous cell carcinoma, cholangiocarcinoma, endometrial cancer, multiple myeloma or mesothelioma; atherosclerosis and pulmonary fibrosis.

11. A method for treating a disease associated with a protein kinase in a subject, comprising administering to the subject an effective amount of the compound or a pharmaceutically acceptable salt, stereoisomer, tautomer, deuteron, prodrug molecule, hydrate, or solvate thereof according to any one of claims 1 to 7, or the pharmaceutical composition according to claim 9;
preferably, the subject is a mammal, preferably a human;
preferably, the disease associated with a protein kinase is selected from a disease associated with c-Met, VEGFR-2, AXL, TAM, NTRK, or RET;
preferably, the disease associated with a protein kinase is a tumor;
preferably, the disease associated with a protein kinase includes head and neck cancer, nasopharyngeal carcinoma, melanoma, bladder cancer, esophageal cancer, anaplastic large cell lymphoma, renal cancer, breast cancer, colorectal cancer, ovarian cancer, cervical cancer, pancreatic cancer, glioma, glioblastoma, prostate cancer, leukemia, lymphoma, non-Hodgkin's lymphoma, gastric cancer, lung cancer, liver cancer, gastrointestinal stromal tumor, thyroid cancer, squamous cell carcinoma, cholangiocarcinoma, endometrial cancer, multiple myeloma or mesothelioma; atherosclerosis and pulmonary fibrosis;
preferably, the mode of administration comprises oral, mucosal, sublingual, ocular, topical, parenteral, rectal, cerebral cistern, vaginal, peritoneal, bladder, nasal administration.
